(19) Europäisches Patentamt
European Patent Office
Office européen des brevets

(11) EP 4 206 322 A2

(12) EUROPEAN PATENT APPLICATION

(43) Date of publication:
**05.07.2023 Bulletin 2023/27**

(21) Application number: **22216504.5**

(22) Date of filing: **23.12.2022**

(51) International Patent Classification (IPC):
***C12N 9/06** (2006.01)* ***C12P 13/04** (2006.01)*

(52) Cooperative Patent Classification (CPC):
**C12N 9/0028; C12N 9/0016; C12P 13/04**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.12.2021 JP 2021215094**
**13.05.2022 JP 2022079712**
**13.05.2022 JP 2022079711**
**13.05.2022 JP 2022079708**

(71) Applicant: **CHUGAI SEIYAKU KABUSHIKI KAISHA**
**Tokyo 115-8543 (JP)**

(72) Inventors:
- **SHINODA, Kiyomichi**
  **Kamakura-shi, Kanagawa 247-8530 (JP)**
- **OHTA, Atsushi**
  **Kamakura-shi, Kanagawa 247-8530 (JP)**
- **SHIINA, Junichi**
  **Kanagawa, 247-8530 (JP)**
- **MATSUO, Atsushi**
  **Kanagawa, 247-8530 (JP)**
- **ISHINO, Sou**
  **138623 07-11 to 16 Synapse (SG)**
- **EJIMA, Hirotaka**
  **Kanagawa, 247-8530 (JP)**
- **ISHIYAMA, Shiori**
  **Kamakura-shi, Kanagawa 247-8530 (JP)**
- **NAKANO, Kazuhiko**
  **Kamakura-shi, Kanagawa 247-8530 (SG)**
- **TSUKADA, Keisuke**
  **Tokyo 115-8543 (JP)**

(74) Representative: **Vossius & Partner**
**Patentanwälte Rechtsanwälte mbB**
**Siebertstraße 3**
**81675 München (DE)**

Remarks:
The complete document including Reference Table(s) and the Sequence Listing(s) can be downloaded from the EPO website

# (54) POLYPEPTIDE AND METHOD FOR PRODUCING AMINO ACID USING SAME

(57) The polypeptide comprises a sequence having 90% or higher sequence identity to an amino acid sequence represented by SEQ ID NO: 1 with one amino acid residue thereof engineered, and has higher catalytic activity for the reductive amination reaction between at least one compound A represented by the formula (1) or a salt thereof and at least one compound B represented by the formula (2) or a salt thereof or the intramolecular reductive amination reaction of at least one compound B or a salt thereof than that of a polypeptide comprising the amino acid sequence represented by SEQ ID NO: 1 under at least one reaction condition.



Processed by Luminess, 75001 PARIS (FR)

## Description

### TECHNICAL FIELD

The present invention relates to a polypeptide and a method for producing an amino acid using the same.

### BACKGROUND

Amino acids such as N-alkyl amino acids are known to exist in some structures of various natural physiologically active substances. It is also known for some N-methyl amino acids, one kind of N-alkyl amino acid, that the amino acids themselves occur naturally (Non Patent Literature 1).

Various organic chemical approaches exist as methods for synthesizing N-alkyl amino acids. Methods for enzymatically synthesizing N-alkyl amino acids have been studied and reported from the viewpoint of green chemistry, etc.

Mihara et al. have screened various microbes on the basis of activity of synthesizing N-methyl phenylalanine from phenylpyruvic acid and methylamine (Patent Literature 1 and Non Patent Literatures 2 and 3). As a result, they have found N-methyl amino acid dehydrogenase (NMAADH) from a *P. putida* ATCC12633 strain having the highest level of this activity, and also successfully obtained this enzyme by cloning a gene (designated as dpkA) of the enzyme and allowing *E. coli* to express the gene, followed by purification.

The purified enzyme had activity of synthesizing, from various $\alpha$-keto acids (pyruvic acid, phenylpyruvic acid, hydroxypyruvic acid, etc.) and alkylamines (methylamine, ethylamine, propylamine, etc.), corresponding N-alkyl amino acids.

The amino acid sequence of the NMAADH derived from the *P. putida* ATCC12633 strain was very similar to that of protein PP3591 encoded by the genome of a *P. putida* KT2440 strain (Patent Literature 2 and Non Patent Literatures 2, 3 and 4).

X-ray crystallography has been conducted on *P. syringae-derived* NMAADH, which exhibits 71% amino acid sequence similarity to NMAADH derived from the *P. putida* ATCC12633 strain (Non Patent Literatures 3 and 5). The substrate specificity of purified *P. syringae-derived* NMAADH for $\alpha$-keto acid and amine has been found to be similar to that of purified NMAADH derived from the *P. putida* ATCC12633 strain, while the mechanism of reaction that is catalyzed by *P. syringae-derived* NMAADH is described on the basis of the crystal structure.

A literature disclosing that mutations were introduced to NMAADH has been reported. In the crystal structure of *P. syringae-derived* NMAADH (Non Patent Literature 5), residues contained in a substrate binding site are also conserved in NMAADH derived from the *P. putida* KT2440 strain. A mutant F 117L of NMAADH derived from the *P. putida* KT2440 strain, which exhibits improved specific activity with glyoxylic acid and methylamine or glyoxylic acid and ethylamine as substrates, has been found by introducing mutations to the amino acid residues (Non Patent Literature 6).

NMAADH derived from the *P. putida* KT2440 strain exhibits higher activity against pyruvic acid than against phenylpyruvic acid. Its mutant P262A, M141L containing two mutations has almost equivalent activity against phenylpyruvic acid and pyruvic acid (Non Patent Literature 7).

### CITATION LIST

### PATENT LITERATURE

[Patent Literature 1] International Publication No. WO 2003/072770
[Patent Literature 2] International Publication No. WO 2002/077183

### NON PATENT LITERATURE

[Non Patent Literature 1] J. F. Hyslop et al., J. Biotechnol. 2019, 293, 56-65.
[Non Patent Literature 2] H. Mihara et al., FEBS Journal 2005, 272, 1117-1123.
[Non Patent Literature 3] Hisaaki Mihara, Seikagaku (Biochemistry in English), the journal of the Japanese Biochemical Society, 2015, 87, 326-332.
[Non Patent Literature 4] K. E. Nelson et al., Environ. Microbiol. 2002, 4, 799-808.
[Non Patent Literature 5] M. Goto et al., J. Biol. Chem. 2005, 280, 40875-40884.
[Non Patent Literature 6] M. Mindet et al., Front. Bioeng. Biotechnol. 2019, 7.

[Non Patent Literature 7] A. Kerbs et al., Microorganisms 2021, 9, 824.

## SUMMARY

As far as the present inventors know, there are only three literatures, including also the literatures mentioned above, reporting that N-alkyl amino acids were synthesized using variant NMAADH. In other words, only a few reports mention variant NMAADH.

Non Patent Literatures 2 and 3 disclose that NMAADH derived from the *P. putida* ATCC12633 strain exhibits no activity against ammonia and exhibits activity against neither α-ketoisocaproic acid nor α-keto-α-methylvaleric acid having a branch at position β.

As far as the present inventors know, there exists no information on studies as to the catalytic activity, etc. of the protein PP3591 encoded by the genome of the *P. putida* KT2440 strain described in Non Patent Literature 4, though only its sequence information has been published. As far as the present inventors know, there also exists no report stating that the catalytic activity against predetermined amino acids is improved, for example, by varying the amino acid sequence of PP3591.

An object of the present invention is to provide a novel polypeptide usable as an enzyme that catalyzes reductive amination reaction. Another object of the present invention is to provide a method for producing an amino acid using the polypeptide.

The present invention relates to, for example, each of the following aspects.

[1]

[1-1] A polypeptide

comprising a sequence having 90% or higher sequence identity to an amino acid sequence represented by SEQ ID NO: 1 with one amino acid residue thereof engineered, and

having higher catalytic activity for a reductive amination reaction between at least one compound A represented by the following formula (1) or a salt thereof and at least one compound B represented by the following formula (2) or a salt thereof or an intramolecular reductive amination reaction of at least one compound B represented by the formula (2) or a salt thereof than that of a polypeptide comprising the amino acid sequence represented by SEQ ID NO: 1 under at least one reaction condition:

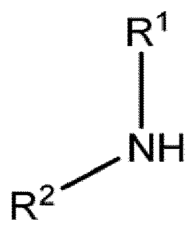

(1)

wherein

in the formula (1),

$R^1$ and $R^2$ each independently represent a hydrogen atom, an alkyl group, an alkenyl group, an alkynyl group, a cycloalkyl group, an aryl group, a heterocyclyl group, or a heteroaryl group, these groups are optionally substituted, and at least one of $R^1$ and $R^2$ is a hydrogen atom,

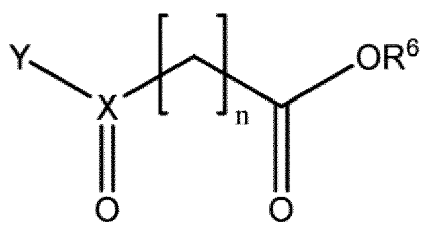

(2)

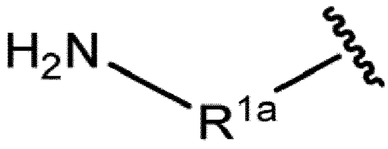

(1')

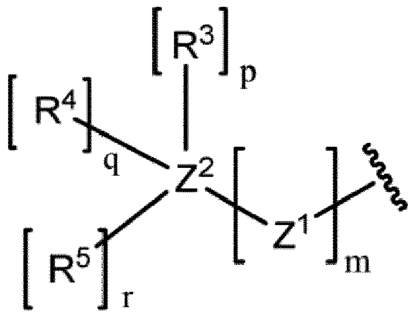

(3)

wherein
in the formula (2),

X represents a carbon atom,
Y represents a hydrogen atom, a group represented by the formula (1') or a group represented by the formula (3),
n represents an integer of between 0 and 2, and
$R^6$ represents a hydrogen atom, an optionally substituted aliphatic hydrocarbon group having between 1 and 6 carbon atoms, an optionally substituted aryl group having between 5 and 12 carbon atoms, an optionally substituted heteroaryl group having between 5 and 12 ring-constituting atoms, a group containing a nitrogen atom, or a group containing an oxygen atom;

in the formula (1'),

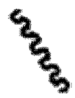

represents a binding point to X, and
$R^{1a}$ is a group formed by removing a hydrogen atom from the group represented by $R^1$ in the formula (1), and is not a hydrogen atom;

in the formula (3),

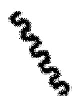

represents a binding point to X,
m represents an integer of between 0 and 6,
p is 0 or 1,
q is 0 or 1,
r is 0 or 1,
$Z^1$ represents an optionally substituted alkylene group, or an ether bond-containing group having between 1 and 6 carbon atoms, and when m is 2 or larger, a plurality of $Z^1$ are the same or different,
$Z^2$ represents a carbon atom,
$R^3$, $R^4$ and $R^5$ each independently represent a hydrogen atom, an optionally substituted aliphatic hydrocarbon group having between 1 and 6 carbon atoms, an optionally substituted aryl group

having between 5 and 12 carbon atoms, an optionally substituted heteroaryl group having between 5 and 12 ring-constituting atoms, a group containing a nitrogen atom, or a group containing an oxygen atom,

any two or more of $R^3$, $R^4$, and $R^5$ are optionally bonded to each other to form a ring structure together with $Z^2$, the ring structure is optionally a cycloalkyl group, an aryl group, a heterocyclyl group, or a heteroaryl group, and these groups are optionally substituted,

$R^3$, $R^4$, and $R^5$ each optionally form a double bond or a triple bond with $Z^2$, and when any one of $R^3$, $R^4$, and $R^5$ is bonded to $Z^2$ through a double bond or a triple bond, at least one of p, q and r is 0; and

when one of $R^1$ and $R^2$ in the formula (1) is a methyl group and the other moiety is a hydrogen atom, in the formula (2), Y is a group represented by the formula (3), m is 0, and two or more of $R^3$ to $R^5$ are not a hydrogen atom.

[1-2] The polypeptide according to [1-1], wherein the amino acid sequence represented by SEQ ID NO: 1 with one amino acid residue thereof engineered is an amino acid sequence represented by SEQ ID NO: 1 with engineering one amino acid residue positioned at a site corresponding to a histidine residue at position 44, a phenylalanine residue at position 117, a methionine residue at position 141, a threonine residue at position 156, a histidine residue at position 182, a glutamine residue at position 186, a tryptophan residue at position 253, and a lysine residue at position 260.

[1-3] The polypeptide according to [1-1] or [1-2], wherein the amino acid sequence represented by SEQ ID NO: 1 with one amino acid residue thereof engineered is the amino acid sequence represented by SEQ ID NO: 6 wherein X is a valine residue (M141V), the amino acid sequence represented by SEQ ID NO: 6 wherein X is a tyrosine residue (M141Y), the amino acid sequence represented by SEQ ID NO: 8 wherein X is a leucine residue (H182L), the amino acid sequence represented by SEQ ID NO: 11 wherein X is a histidine residue (W253H), or the amino acid sequence represented by SEQ ID NO: 12 wherein X is a glutamic acid residue (K260E).

[1-4] The polypeptide according to any of [1-1] to [1-3], wherein the polypeptide has higher catalytic activity than that of a polypeptide consisting of the amino acid sequence represented by SEQ ID NO: 17 under at least one reaction condition.

[2] A polypeptide

comprising a sequence having 90% or higher sequence identity to an amino acid sequence represented by SEQ ID NO: 1 with two amino acid residues thereof engineered, and

having higher catalytic activity for a reductive amination reaction between at least one compound A represented by the following formula (1) or a salt thereof and at least one compound B represented by the following formula (2) or a salt thereof or an intramolecular reductive amination reaction of at least one compound B represented by the formula (2) or a salt thereof than that of a polypeptide comprising the amino acid sequence represented by SEQ ID NO: 1 under at least one reaction condition:

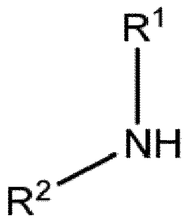
(1)

wherein

in the formula (1),

$R^1$ and $R^2$ each independently represent a hydrogen atom, an alkyl group, an alkenyl group, an alkynyl group, a cycloalkyl group, an aryl group, a heterocyclyl group, or a heteroaryl group, these groups are optionally substituted, and at least one of $R^1$ and $R^2$ is a hydrogen atom,

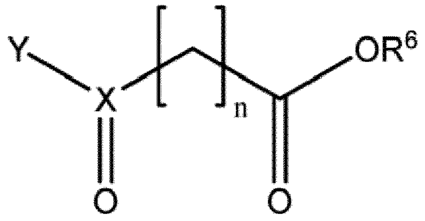

(2)

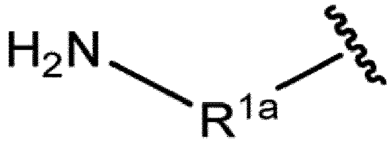

(1')

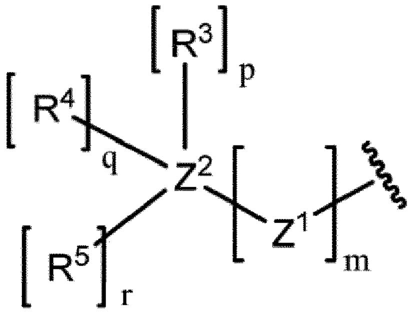

(3)

wherein
in the formula (2),

X represents a carbon atom,
Y represents a hydrogen atom, a group represented by the formula (1') or a group represented by the formula (3),
n represents an integer of between 0 and 2, and
$R^6$ represents a hydrogen atom, an optionally substituted aliphatic hydrocarbon group having between 1 and 6 carbon atoms, an optionally substituted aryl group having between 5 and 12 carbon atoms, an optionally substituted heteroaryl group having between 5 and 12 ring-constituting atoms, a group containing a nitrogen atom, or a group containing an oxygen atom;

in the formula (1'),

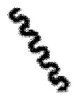

represents a binding point to X, and
$R^{1a}$ is a group formed by removing a hydrogen atom from the group represented by $R^1$ in the formula (1), and is not a hydrogen atom;

in the formula (3),

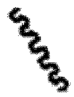

represents a binding point to X,

m represents an integer of between 0 and 6,
p is 0 or 1,
q is 0 or 1,
r is 0 or 1,
$Z^1$ represents an optionally substituted alkylene group, or an ether bond-containing group having between 1 and 6 carbon atoms, and when m is 2 or larger, a plurality of $Z^1$ are the same or different,
$Z^2$ represents a carbon atom,
$R^3$, $R^4$ and $R^5$ each independently represent a hydrogen atom, an optionally substituted aliphatic hydrocarbon group having between 1 and 6 carbon atoms, an optionally substituted aryl group having between 5 and 12 carbon atoms, an optionally substituted heteroaryl group having between 5 and 12 ring-constituting atoms, a group containing a nitrogen atom, or a group containing an oxygen atom,
any two or more of $R^3$, $R^4$, and $R^5$ are optionally bonded to each other to form a ring structure together with $Z^2$, the ring structure is optionally a cycloalkyl group, an aryl group, a heterocyclyl group, or a heteroaryl group, and these groups are optionally substituted,
$R^3$, $R^4$, and $R^5$ each optionally form a double bond or a triple bond with $Z^2$, and when any one of $R^3$, $R^4$, and $R^5$ is bonded to $Z^2$ through a double bond or a triple bond, at least one of p, q and r is 0; and
when one of $R^1$ and $R^2$ in the formula (1) is a methyl group and the other moiety is a hydrogen atom, in the formula (2), Y is a group represented by the formula (3), m is 0, and two or more of $R^3$ to $R^5$ are not a hydrogen atom.

[3] A polypeptide according to [1],

comprising a sequence having 90% or higher sequence identity to an amino acid sequence represented by SEQ ID NO: 1 with two amino acid residues thereof engineered, and
having higher catalytic activity for a reductive amination reaction between at least one compound A represented by the following formula (1) or a salt thereof and at least one compound B represented by the following formula (2) or a salt thereof or an intramolecular reductive amination reaction of at least one compound B represented by the formula (2) or a salt thereof than that of a polypeptide comprising the amino acid sequence represented by SEQ ID NO: 1 under at least one reaction condition:

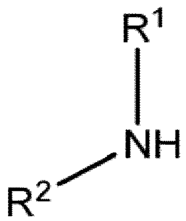

(1)

wherein
in the formula (1),
$R^1$ and $R^2$ each independently represent a hydrogen atom, an alkyl group, an alkenyl group, an alkynyl group, a cycloalkyl group, an aryl group, a heterocyclyl group, or a heteroaryl group, these groups are optionally substituted, and at least one of $R^1$ and $R^2$ is a hydrogen atom,

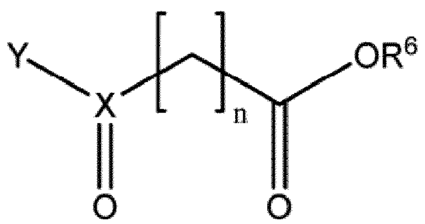

(2)

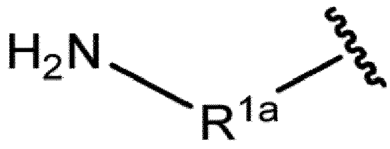

(1')

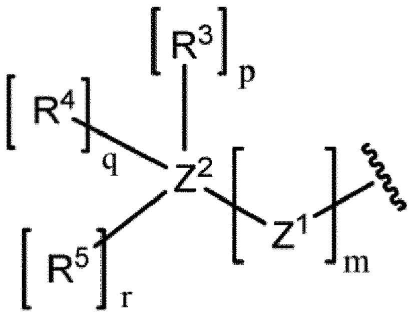

(3)

wherein
in the formula (2),

X represents a carbon atom,
Y represents a hydrogen atom, a group represented by the formula (1') or a group represented by the formula (3),
n represents an integer of between 0 and 2, and
$R^6$ represents a hydrogen atom, an optionally substituted aliphatic hydrocarbon group having between 1 and 6 carbon atoms, an optionally substituted aryl group having between 5 and 12 carbon atoms, an optionally substituted heteroaryl group having between 5 and 12 ring-constituting atoms, a group containing a nitrogen atom, or a group containing an oxygen atom;

in the formula (1'),

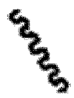

represents a binding point to X, and
$R^{1a}$ is a group formed by removing a hydrogen atom from the group represented by $R^1$ in the formula (1), and is not a hydrogen atom;

in the formula (3),

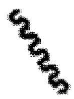

represents a binding point to X,
m represents an integer of between 0 and 6,
p is 0 or 1,
q is 0 or 1,
r is 0 or 1,
$Z^1$ represents an optionally substituted alkylene group, or an ether bond-containing group having between 1 and 6 carbon atoms, and when m is 2 or larger, a plurality of $Z^1$ are the same or different,
$Z^2$ represents a carbon atom,
$R^3$, $R^4$ and $R^5$ each independently represent a hydrogen atom, an optionally substituted aliphatic hydrocarbon group having between 1 and 6 carbon atoms, an optionally substituted aryl group having between

5 and 12 carbon atoms, an optionally substituted heteroaryl group having between 5 and 12 ring-constituting atoms, a group containing a nitrogen atom, or a group containing an oxygen atom,
any two or more of $R^3$, $R^4$, and $R^5$ are optionally bonded to each other to form a ring structure together with $Z^2$, the ring structure is optionally a cycloalkyl group, an aryl group, a heterocyclyl group, or a heteroaryl group, and these groups are optionally substituted,
$R^3$, $R^4$, and $R^5$ each optionally form a double bond or a triple bond with $Z^2$, and when any one of $R^3$, $R^4$, and $R^5$ is bonded to $Z^2$ through a double bond or a triple bond, at least one of p, q and r is 0; and
when one of $R^1$ and $R^2$ in the formula (1) is a methyl group and the other moiety is a hydrogen atom, in the formula (2), Y is a group represented by the formula (3), m is 0, and two or more of $R^3$ to $R^5$ are not a hydrogen atom.

[4] A polypeptide

comprising a sequence having 90% or higher sequence identity to an amino acid sequence represented by SEQ ID NO: 1 with three amino acid residues thereof engineered, and
having higher catalytic activity for a reductive amination reaction between at least one compound A represented by the following formula (1) or a salt thereof and at least one compound B represented by the following formula (2) or a salt thereof or an intramolecular reductive amination reaction of at least one compound B represented by the formula (2) or a salt thereof than that of a polypeptide comprising the amino acid sequence represented by SEQ ID NO: 1 under at least one reaction condition:

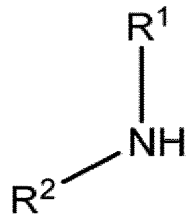

(1)

wherein
in the formula (1),
$R^1$ and $R^2$ each independently represent a hydrogen atom, an alkyl group, an alkenyl group, an alkynyl group, a cycloalkyl group, an aryl group, a heterocyclyl group, or a heteroaryl group, these groups are optionally substituted, and at least one of $R^1$ and $R^2$ is a hydrogen atom,

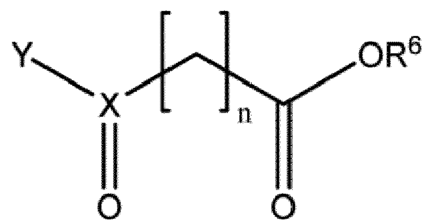

(2)

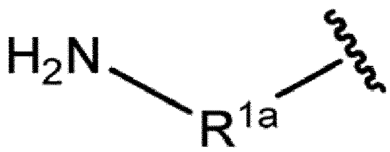

(1')

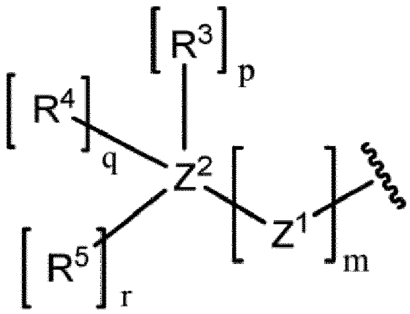

(3)

wherein
in the formula (2),

X represents a carbon atom,
Y represents a hydrogen atom, a group represented by the formula (1') or a group represented by the formula (3),
n represents an integer of between 0 and 2, and
$R^6$ represents a hydrogen atom, an optionally substituted aliphatic hydrocarbon group having between 1 and 6 carbon atoms, an optionally substituted aryl group having between 5 and 12 carbon atoms, an optionally substituted heteroaryl group having between 5 and 12 ring-constituting atoms, a group containing a nitrogen atom, or a group containing an oxygen atom;

in the formula (1'),

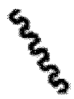

represents a binding point to X, and
$R^{1a}$ is a group formed by removing a hydrogen atom from the group represented by $R^1$ in the formula (1), and is not a hydrogen atom;

in the formula (3),

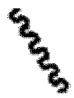

represents a binding point to X,
m represents an integer of between 0 and 6,
p is 0 or 1,
q is 0 or 1,
r is 0 or 1,
$Z^1$ represents an optionally substituted alkylene group, or an ether bond-containing group having between 1 and 6 carbon atoms, and when m is 2 or larger, a plurality of $Z^1$ are the same or different,
$Z^2$ represents a carbon atom,
$R^3$, $R^4$ and $R^5$ each independently represent a hydrogen atom, an optionally substituted aliphatic hydrocarbon group having between 1 and 6 carbon atoms, an optionally substituted aryl group having between 5 and 12 carbon atoms, an optionally substituted heteroaryl group having between 5 and 12 ring-constituting atoms, a group containing a nitrogen atom, or a group containing an oxygen atom,
any two or more of $R^3$, $R^4$, and $R^5$ are optionally bonded to each other to form a ring structure together with $Z^2$, the ring structure is optionally a cycloalkyl group, an aryl group, a heterocyclyl group, or a heteroaryl group, and these groups are optionally substituted,
$R^3$, $R^4$, and $R^5$ each optionally form a double bond or a triple bond with $Z^2$, and when any one of $R^3$, $R^4$,

and $R^5$ is bonded to $Z^2$ through a double bond or a triple bond, at least one of p, q and r is 0; and when one of $R^1$ and $R^2$ in the formula (1) is a methyl group and the other moiety is a hydrogen atom, in the formula (2), Y is a group represented by the formula (3), m is 0, and two or more of $R^3$ to $R^5$ are not a hydrogen atom.

[5] A polypeptide according to [1],

comprising a sequence having 90% or higher sequence identity to an amino acid sequence represented by SEQ ID NO: 1 with three amino acid residues thereof engineered, and having higher catalytic activity for a reductive amination reaction between at least one compound A represented by the following formula (1) or a salt thereof and at least one compound B represented by the following formula (2) or a salt thereof or an intramolecular reductive amination reaction of at least one compound B represented by the formula (2) or a salt thereof than that of a polypeptide comprising the amino acid sequence represented by SEQ ID NO: 1 under at least one reaction condition:

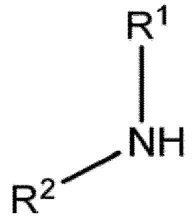

(1)

wherein
in the formula (1),
$R^1$ and $R^2$ each independently represent a hydrogen atom, an alkyl group, an alkenyl group, an alkynyl group, a cycloalkyl group, an aryl group, a heterocyclyl group, or a heteroaryl group, these groups are optionally substituted, and at least one of $R^1$ and $R^2$ is a hydrogen atom,

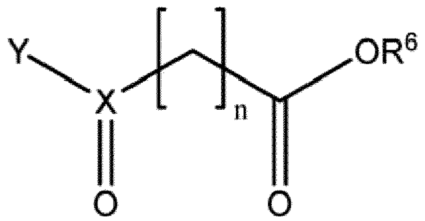

(2)

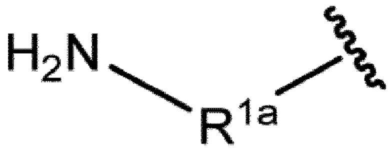

(1')

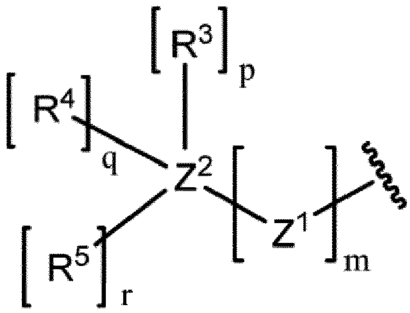

(3)

wherein
in the formula (2),

X represents a carbon atom,
Y represents a hydrogen atom, a group represented by the formula (1') or a group represented by the formula (3),
n represents an integer of between 0 and 2, and
$R^6$ represents a hydrogen atom, an optionally substituted aliphatic hydrocarbon group having between 1 and 6 carbon atoms, an optionally substituted aryl group having between 5 and 12 carbon atoms, an optionally substituted heteroaryl group having between 5 and 12 ring-constituting atoms, a group containing a nitrogen atom, or a group containing an oxygen atom;

in the formula (1'),

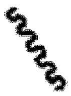

represents a binding point to X, and
$R^{1a}$ is a group formed by removing a hydrogen atom from the group represented by $R^1$ in the formula (1), and is not a hydrogen atom;

in the formula (3),

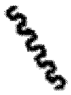

represents a binding point to X,
m represents an integer of between 0 and 6,
p is 0 or 1,
q is 0 or 1,
r is 0 or 1,
$Z^1$ represents an optionally substituted alkylene group, or an ether bond-containing group having between 1 and 6 carbon atoms, and when m is 2 or larger, a plurality of $Z^1$ are the same or different,
$Z^2$ represents a carbon atom,
$R^3$, $R^4$ and $R^5$ each independently represent a hydrogen atom, an optionally substituted aliphatic hydrocarbon group having between 1 and 6 carbon atoms, an optionally substituted aryl group having between 5 and 12 carbon atoms, an optionally substituted heteroaryl group having between 5 and 12 ring-constituting atoms, a group containing a nitrogen atom, or a group containing an oxygen atom,
any two or more of $R^3$, $R^4$, and $R^5$ are optionally bonded to each other to form a ring structure together with $Z^2$, the ring structure is optionally a cycloalkyl group, an aryl group, a heterocyclyl group, or a heteroaryl group, and these groups are optionally substituted,
$R^3$, $R^4$, and $R^5$ each optionally form a double bond or a triple bond with $Z^2$, and when any one of $R^3$, $R^4$, and $R^5$ is bonded to $Z^2$ through a double bond or a triple bond, at least one of p, q and r is 0; and
when one of $R^1$ and $R^2$ in the formula (1) is a methyl group and the other moiety is a hydrogen atom, in the formula (2), Y is a group represented by the formula (3), m is 0, and two or more of $R^3$ to $R^5$ are not a hydrogen atom.

[6]

[6-1] A polypeptide
comprising a sequence having 90% or higher sequence identity to an amino acid sequence represented by SEQ ID NO: 1 with one amino acid residue thereof engineered, and having higher catalytic activity for a reductive amination reaction between an alkylamine or a salt thereof and at least one compound represented by the following formula (2') or a salt thereof than that of a polypeptide comprising the amino acid sequence represented

by SEQ ID NO: 1 under at least one reaction condition:

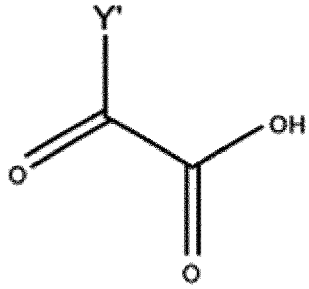

(2')

wherein Y' represents a $C_3$ to $C_8$ cycloalkyl group or a $C_6$ to $C_9$ aralkyl group, and the aralkyl group is optionally substituted by a $C_1$ to $C_3$ alkyl group or a halogen atom.

[6-2] The polypeptide according to [6-1], wherein the polypeptide has higher catalytic activity than that of a polypeptide consisting of the amino acid sequence represented by SEQ ID NO: 17 under at least one reaction condition.

[7] A polypeptide comprising a sequence having 90% or higher sequence identity to an amino acid sequence represented by SEQ ID NO: 1 with two amino acid residues thereof engineered, and having higher catalytic activity for a reductive amination reaction between an alkylamine or a salt thereof and at least one compound represented by the following formula (2') or a salt thereof than that of a polypeptide comprising the amino acid sequence represented by SEQ ID NO: 1 under at least one reaction condition:

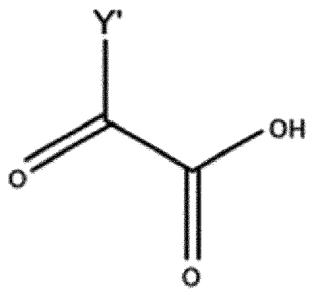

(2')

wherein Y' represents a $C_3$ to $C_8$ cycloalkyl group or a $C_6$ to $C_9$ aralkyl group, and the aralkyl group is optionally substituted by a $C_1$ to $C_3$ alkyl group or a halogen atom.

[8] A polypeptide according to [6],

comprising a sequence having 90% or higher sequence identity to an amino acid sequence represented by SEQ ID NO: 1 with two amino acid residues thereof engineered, and having higher catalytic activity for a reductive amination reaction between an alkylamine or a salt thereof and at least one compound represented by the following formula (2') or a salt thereof than that of a polypeptide comprising the amino acid sequence represented by SEQ ID NO: 1 under at least one reaction condition:

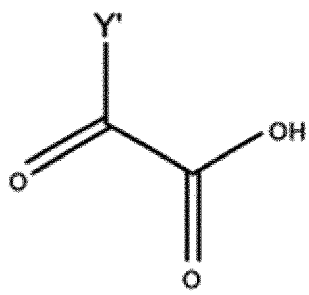

(2')

wherein Y' represents a $C_3$ to $C_8$ cycloalkyl group or a $C_6$ to $C_9$ aralkyl group, and the aralkyl group is optionally substituted by a $C_1$ to $C_3$ alkyl group or a halogen atom.

[9] A polypeptide comprising a sequence having 90% or higher sequence identity to an amino acid sequence represented by SEQ ID NO: 1 with three amino acid residues thereof engineered, and having higher catalytic activity for a reductive amination reaction between an alkylamine or a salt thereof and at least one compound represented by the following formula (2') or a salt thereof than that of a polypeptide comprising the amino acid sequence represented by SEQ ID NO: 1 under at least one reaction condition:

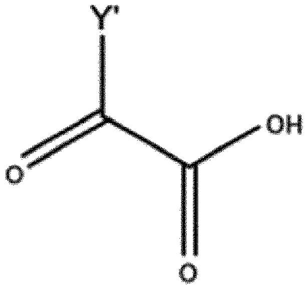

(2')

wherein Y' represents a $C_3$ to $C_8$ cycloalkyl group or a $C_6$ to $C_9$ aralkyl group, and the aralkyl group is optionally substituted by a $C_1$ to $C_3$ alkyl group or a halogen atom.

[10] A polypeptide according to [6],
comprising a sequence having 90% or higher sequence identity to an amino acid sequence represented by SEQ ID NO: 1 with three amino acid residues thereof engineered, and having higher catalytic activity for a reductive amination reaction between an alkylamine or a salt thereof and at least one compound represented by the following formula (2') or a salt thereof than that of a polypeptide comprising the amino acid sequence represented by SEQ ID NO: 1 under at least one reaction condition:

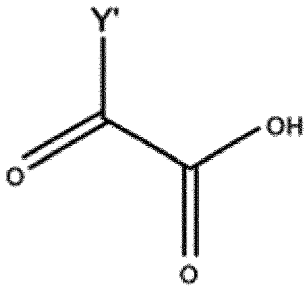

(2')

wherein Y' represents a $C_3$ to $C_8$ cycloalkyl group or a $C_6$ to $C_9$ aralkyl group, and the aralkyl group is optionally substituted by a $C_1$ to $C_3$ alkyl group or a halogen atom.

[11] A polypeptide according to any one of [1] to [10],
comprising a sequence having 90% or higher sequence identity to an amino acid sequence represented by SEQ ID NO: 17 with one amino acid residue thereof engineered.

[12] A polypeptide according to any one of [1] to [11],
comprising a sequence having 90% or higher sequence identity to an amino acid sequence represented by SEQ ID NO: 17 with two amino acid residues thereof engineered.

[13] A polypeptide according to any one of [1] to [12],
comprising a sequence having 90% or higher sequence identity to an amino acid sequence represented by SEQ ID NO: 17 with three amino acid residues thereof engineered.

[14] A polypeptide
comprising a sequence having the engineering of an amino acid residue positioned at a site corresponding to at least one amino acid residue selected from the group consisting of a histidine residue at position 44, a phenylalanine residue at position 117, a methionine residue at position 141, a threonine residue at position 156, a histidine residue at position 182, a glutamine residue at position 186, a tryptophan residue at position 253, and a lysine residue at position 260 in the amino acid sequence represented by SEQ ID NO: 1.

[15] A polypeptide according to any one of [1] to [13],
comprising a sequence having the engineering of an amino acid residue positioned at a site corresponding to at least one amino acid residue selected from the group consisting of a histidine residue at position 44, a phenylalanine residue at position 117, a methionine residue at position 141, a threonine residue at position 156, a histidine residue at position 182, a glutamine residue at position 186, a tryptophan residue at position 253, and a lysine residue at position 260 in the amino acid sequence represented by SEQ ID NO: 1.
[16] The polypeptide according to any of [1] to [15], wherein the polypeptide comprises a sequence having the engineering of an amino acid residue positioned at a site corresponding to at least one amino acid residue selected from the group consisting of a methionine residue at position 141, a histidine residue at position 182, a tryptophan residue at position 253, and a lysine residue at position 260 in the amino acid sequence represented by SEQ ID NO: 1.
[17] A polypeptide
comprising a sequence

having the engineering of an amino acid residue positioned at a site corresponding to at least one amino acid residue selected from the group consisting of a methionine residue at position 141, a histidine residue at position 182, and a tryptophan residue at position 253 in the amino acid sequence represented by SEQ ID NO: 1 as a first engineering site, and
having the engineering of an amino acid residue positioned at a site corresponding to at least one amino acid residue selected from the group consisting of a histidine residue at position 182, a tryptophan residue at position 253, and a lysine residue at position 260 in the amino acid sequence represented by SEQ ID NO: 1 as a second engineering site, wherein the amino acid reside engineered at the second engineering site is positioned at a site corresponding to one amino acid residue different from the amino acid residue engineered at the first engineering site.

[18] A polypeptide according to any one of [1] to [16],

comprising a sequence comprising the engineering of an amino acid residue positioned at a site corresponding to at least one amino acid residue selected from the group consisting of a methionine residue at position 141, a histidine residue at position 182, and a tryptophan residue at position 253 in the amino acid sequence represented by SEQ ID NO: 1 as a first engineering site, and
the engineering of an amino acid residue positioned at a site corresponding to at least one amino acid residue selected from the group consisting of a histidine residue at position 182, a tryptophan residue at position 253, and a lysine residue at position 260 in the amino acid sequence represented by SEQ ID NO: 1 as a second engineering site, wherein the amino acid reside engineered at the second engineering site is positioned at a site corresponding to one amino acid residue different from the amino acid residue engineered at the first engineering site.

[19] A polypeptide

comprising a sequence comprising the engineering of an amino acid residue positioned at a site corresponding to at least one amino acid residue selected from the group consisting of a methionine residue at position 141, and a histidine residue at position 182 in the amino acid sequence represented by SEQ ID NO: 1 as a first engineering site,
the engineering of an amino acid residue positioned at a site corresponding to at least one amino acid residue selected from the group consisting of a histidine residue at position 182, and a tryptophan residue at position 253 in the amino acid sequence represented by SEQ ID NO: 1 as a second engineering site, wherein the amino acid reside engineered at the second engineering site is positioned at a site corresponding to one amino acid residue different from the amino acid residue engineered at the first engineering site, and
the engineering of an amino acid residue positioned at a site corresponding to at least one amino acid residue selected from the group consisting of a tryptophan residue at position 253, and a lysine residue at position 260 in the amino acid sequence represented by SEQ ID NO: 1 as a third engineering site, wherein the amino acid reside engineered at the third engineering site is positioned at a site corresponding to one amino acid residue different from the amino acid residue engineered at the first engineering site and the second engineering site.

[20] A polypeptide according to any one of [1] to [16],
comprising a sequence

having the engineering of an amino acid residue positioned at a site corresponding to at least one amino acid

residue selected from the group consisting of a methionine residue at position 141, and a histidine residue at position 182 in the amino acid sequence represented by SEQ ID NO: 1 as a first engineering site,
having the engineering of an amino acid residue positioned at a site corresponding to at least one amino acid residue selected from the group consisting of a histidine residue at position 182, and a tryptophan residue at position 253 in the amino acid sequence represented by SEQ ID NO: 1 as a second engineering site, wherein the amino acid reside engineered at the second engineering site is positioned at a site corresponding to one amino acid residue different from the amino acid residue engineered at the first engineering site, and
having the engineering of an amino acid residue positioned at a site corresponding to at least one amino acid residue selected from the group consisting of a tryptophan residue at position 253, and a lysine residue at position 260 in the amino acid sequence represented by SEQ ID NO: 1 as a third engineering site, wherein the amino acid reside engineered at the third engineering site is positioned at a site corresponding to one amino acid residue different from the amino acid residue engineered at the first engineering site and the second engineering site.

[21] The polypeptide according to any of [1], [6], [11] and [14] to [16], wherein the polypeptide comprises a sequence having the engineering of an amino acid residue positioned at a site corresponding to at least one amino acid residue selected from the group consisting of a methionine residue at position 141, a tryptophan residue at position 253, and a lysine residue at position 260 in the amino acid sequence represented by SEQ ID NO: 1.
[22] The polypeptide according to any one of [1], [6], [11], [14] to [16], and [21], wherein the amino acid sequence represented by SEQ ID NO: 1 has one engineering site.
[23] The polypeptide according to any of [1], [6], [11], [14] to [16], and [21] to [22], wherein the polypeptide comprises a sequence having the substitution of an amino acid residue positioned at a site corresponding to a histidine residue at position 44 in the amino acid sequence represented by SEQ ID NO: 1 by a methionine residue.
[24] The polypeptide according to any of [1], [6], [11], [14] to [16], and [21] to [23], wherein the polypeptide comprises a sequence having the substitution of an amino acid residue positioned at a site corresponding to a phenylalanine residue at position 117 in the amino acid sequence represented by SEQ ID NO: 1 by a leucine residue.
[25] The polypeptide according to any of [1], [6], [11], [14] to [16], and [21] to [24], wherein the polypeptide comprises a sequence having the substitution of an amino acid residue positioned at a site corresponding to a methionine residue at position 141 in the amino acid sequence represented by SEQ ID NO: 1 by at least one amino acid residue selected from the group consisting of a tyrosine residue, a tryptophan residue, a valine residue, a threonine residue, a serine residue, an arginine residue, a leucine residue, a lysine residue, an isoleucine residue, a histidine residue, a phenylalanine residue and an alanine residue.
[26] The polypeptide according to any of [1], [6], [11], [14] to [16], and [21] to [25], wherein the polypeptide comprises a sequence having the substitution of an amino acid residue positioned at a site corresponding to a methionine residue at position 141 in the amino acid sequence represented by SEQ ID NO: 1 by at least one amino acid residue selected from the group consisting of a tyrosine residue, a tryptophan residue, a valine residue, a lysine residue, an isoleucine residue, a phenylalanine residue and an alanine residue.
[27] The polypeptide according to any of [1], [6], [11], [14] to [16], and [21] to [26], wherein the polypeptide comprises a sequence having the substitution of an amino acid residue positioned at a site corresponding to a threonine residue at position 156 in the amino acid sequence represented by SEQ ID NO: 1 by a serine residue.
[28] The polypeptide according to any of [1], [6], [11], [14] to [16], and [21] to [27], wherein the polypeptide comprises a sequence having the substitution of an amino acid residue positioned at a site corresponding to a histidine residue at position 182 in the amino acid sequence represented by SEQ ID NO: 1 by at least one amino acid residue selected from the group consisting of a tyrosine residue, a glutamine residue, a methionine residue, a leucine residue, a glycine residue, a phenylalanine residue and an alanine residue.
[29] The polypeptide according to any of [1], [6], [11], [14] to [16], and [21] to [28], wherein the polypeptide comprises a sequence having the substitution of an amino acid residue positioned at a site corresponding to a histidine residue at position 182 in the amino acid sequence represented by SEQ ID NO: 1 by at least one amino acid residue selected from the group consisting of a methionine residue, a leucine residue and a phenylalanine residue.
[30] The polypeptide according to any of [1], [6], [11], [14] to [16], and [21] to [29], wherein the polypeptide comprises a sequence having the substitution of an amino acid residue positioned at a site corresponding to a glutamine residue at position 186 in the amino acid sequence represented by SEQ ID NO: 1 by at least one amino acid residue selected from the group consisting of a methionine residue and a glutamic acid residue.
[31] The polypeptide according to any of [1], [6], [11], [14] to [16], and [21] to [30], wherein the polypeptide comprises a sequence having the substitution of an amino acid residue positioned at a site corresponding to a tryptophan residue at position 253 in the amino acid sequence represented by SEQ ID NO: 1 by at least one amino acid residue selected from the group consisting of a tyrosine residue, a valine residue, a threonine residue, a serine residue, an arginine residue, a glutamine residue, a proline residue, an asparagine residue, a methionine residue, a leucine

residue, a lysine residue, an isoleucine residue, a histidine residue, a phenylalanine residue and an alanine residue.

[32] The polypeptide according to any of [1], [6], [11], [14] to [16], and [21] to [31], wherein the polypeptide comprises a sequence having the substitution of an amino acid residue positioned at a site corresponding to a tryptophan residue at position 253 in the amino acid sequence represented by SEQ ID NO: 1 by at least one amino acid residue selected from the group consisting of a leucine residue, an isoleucine residue and a histidine residue.

[33] The polypeptide according to any of [1], [6], [11], [14] to [16], and [21] to [32], wherein the polypeptide comprises a sequence having the substitution of an amino acid residue positioned at a site corresponding to a tryptophan residue at position 253 in the amino acid sequence represented by SEQ ID NO: 1 by a histidine residue.

[34] The polypeptide according to any of [1], [6], [11], [14] to [16], and [21] to [33], wherein the polypeptide comprises a sequence having the substitution of an amino acid residue positioned at a site corresponding to a lysine residue at position 260 in the amino acid sequence represented by SEQ ID NO: 1 by at least one amino acid residue selected from the group consisting of a tyrosine residue, a tryptophan residue, a threonine residue, a serine residue, an arginine residue, a glutamine residue, an asparagine residue, a methionine residue, a leucine residue, a histidine residue, a glycine residue, a phenylalanine residue, a glutamic acid residue and an alanine residue.

[35] The polypeptide according to any of [1], [6], [11], [14] to [16], and [21] to [34], wherein the polypeptide comprises a sequence having the substitution of an amino acid residue positioned at a site corresponding to a lysine residue at position 260 in the amino acid sequence represented by SEQ ID NO: 1 by at least one amino acid residue selected from the group consisting of a glutamine residue, a methionine residue, a glutamic acid residue and asparagine residue.

[36] The polypeptide according to any of [1], [6], [11], [14] to [16], and [21] to [35], wherein the amino acid sequence represented by SEQ ID NO: 1 with one amino acid residue thereof engineered is the amino acid sequence represented by SEQ ID NO: 6 wherein X is a valine residue (M141V), the amino acid sequence represented by SEQ ID NO: 6 wherein X is a tyrosine residue (M141Y), the amino acid sequence represented by SEQ ID NO: 8 wherein X is a leucine residue (H182L), the amino acid sequence represented by SEQ ID NO: 11 wherein X is a histidine residue (W253H), or the amino acid sequence represented by SEQ ID NO: 12 wherein X is a glutamic acid residue (K260E).

[37]

[37-1] The polypeptide according to any of [1], [6], [11], [14] to [16], and [21] to [36], wherein the polypeptide comprises a sequence having 90% or higher sequence identity to a sequence represented by SEQ ID NO: 6 wherein X is a valine residue (M141V), the amino acid sequence represented by SEQ ID NO: 6 wherein X is a tyrosine residue (M141Y), the amino acid sequence represented by SEQ ID NO: 8 wherein X is a leucine residue (H182L), the amino acid sequence represented by SEQ ID NO: 11 wherein X is a histidine residue (W253H), or the amino acid sequence represented by SEQ ID NO: 12 wherein X is a glutamic acid residue (K260E).

[37-2] The polypeptide according to any of [1], [6], [11], [14] to [16], [21] to [36], and [37-1], wherein the polypeptide comprises a sequence having 98% or higher sequence identity to a sequence represented by SEQ ID NO: 6 wherein X is a valine residue (M141V), the amino acid sequence represented by SEQ ID NO: 6 wherein X is a tyrosine residue (M141Y), the amino acid sequence represented by SEQ ID NO: 8 wherein X is a leucine residue (H182L), the amino acid sequence represented by SEQ ID NO: 11 wherein X is a histidine residue (W253H), or the amino acid sequence represented by SEQ ID NO: 12 wherein X is a glutamic acid residue (K260E).

[37-3] The polypeptide according to any of [1], [6], [11], [14] to [16], [21] to [36], and [37-1] to [37-2], wherein the polypeptide comprises a sequence having 99% or higher sequence identity to a sequence represented by SEQ ID NO: 6 wherein X is a valine residue (M141V), the amino acid sequence represented by SEQ ID NO: 6 wherein X is a tyrosine residue (M141Y), the amino acid sequence represented by SEQ ID NO: 8 wherein X is a leucine residue (H182L), the amino acid sequence represented by SEQ ID NO: 11 wherein X is a histidine residue (W253H), or the amino acid sequence represented by SEQ ID NO: 12 wherein X is a glutamic acid residue (K260E).

[38] The polypeptide according to any of [1], [6], [11], [14] to [16], and [21] to [37], wherein the polypeptide comprises the amino acid sequence represented by SEQ ID NO: 6 wherein X is a valine residue (M141V), the amino acid sequence represented by SEQ ID NO: 6 wherein X is a tyrosine residue (M141Y), the amino acid sequence represented by SEQ ID NO: 8 wherein X is a leucine residue (H182L), the amino acid sequence represented by SEQ ID NO: 11 wherein X is a histidine residue (W253H), or the amino acid sequence represented by SEQ ID NO: 12 wherein X is a glutamic acid residue (K260E).

[39] The polypeptide according to any of [1], [6], [11], [14] to [16], and [21] to [36], wherein the polypeptide has the amino acid sequence represented by SEQ ID NO: 18, SEQ ID NO: 19, SEQ ID NO: 20, SEQ ID NO: 21, or SEQ ID NO: 22.

[40] The polypeptide according to any one of [2], [3], [7], [8], [12], [17] and [18], wherein the amino acid sequence represented by SEQ ID NO: 1 has two or more engineering sites.

[41] The polypeptide according to any one of [2], [3], [7], [8], [12], [17], [18] and [40], wherein the amino acid sequence represented by SEQ ID NO: 1 has two or more engineering sites.

[42] A polypeptide according to any one of [2], [3], [7], [8], [12], [17], [18], [40] and [41],
comprising a sequence having the engineering of an amino acid residue positioned at a site corresponding to a methionine residue at position 141 and a histidine residue at position 182, the engineering of an amino acid residue positioned at a site corresponding to a methionine residue at position 141 and a tryptophan residue at position 253, the engineering of an amino acid residue positioned at a site corresponding to a methionine residue at position 141 and a lysine residue at position 260, the engineering of an amino acid residue positioned at a site corresponding to a histidine residue at position 182 and a tryptophan residue at position 253, the engineering of an amino acid residue positioned at a site corresponding to a histidine residue at position 182 and a lysine residue at position 260, or the engineering of an amino acid residue positioned at a site corresponding to a tryptophan residue at position 253 and a lysine residue at position 260 in the amino acid sequence represented by SEQ ID NO: 1.

[43] A polypeptide according to any one of [2], [3], [7], [8], [12], [17], [18] and [40] to [42],
comprising a sequence having the engineering of an amino acid residue positioned at a site corresponding to a methionine residue at position 141 and a lysine residue at position 260, the engineering of an amino acid residue positioned at a site corresponding to a histidine residue at position 182 and a lysine residue at position 260, or the engineering of an amino acid residue positioned at a site corresponding to a tryptophan residue at position 253 and a lysine residue at position 260 in the amino acid sequence represented by SEQ ID NO: 1.

[44] The polypeptide according to any of [2], [3], [7], [8], [12], [17], [18] and [40] to [43], wherein the polypeptide comprises a sequence having the substitution of an amino acid residue positioned at a site corresponding to a methionine residue at position 141 in the amino acid sequence represented by SEQ ID NO: 1 by one amino acid residue selected from the group consisting of a tyrosine residue, a tryptophan residue, a valine residue, a threonine residue, an arginine residue, a lysine residue, an isoleucine residue and an alanine residue.

[45] The polypeptide according to any of [2], [3], [7], [8], [12], [17], [18] and [40] to [44], wherein the polypeptide comprises a sequence having the substitution of an amino acid residue positioned at a site corresponding to a methionine residue at position 141 in the amino acid sequence represented by SEQ ID NO: 1 by one amino acid residue selected from the group consisting of a tyrosine residue, a valine residue and an alanine residue.

[46] The polypeptide according to any of [2], [3], [7], [8], [12], [17], [18] and [40] to [45], wherein the polypeptide comprises a sequence having the substitution of an amino acid residue positioned at a site corresponding to a histidine residue at position 182 in the amino acid sequence represented by SEQ ID NO: 1 by one amino acid residue selected from the group consisting of a tyrosine residue, a methionine residue, a leucine residue and a phenylalanine residue.

[47] The polypeptide according to any of [2], [3], [7], [8], [12], [17], [18] and [40] to [46], wherein the polypeptide comprises a sequence having the substitution of an amino acid residue positioned at a site corresponding to a histidine residue at position 182 in the amino acid sequence represented by SEQ ID NO: 1 by one amino acid residue selected from the group consisting of a methionine residue and a leucine residue.

[48] The polypeptide according to any of [2], [3], [7], [8], [12], [17], [18] and [40] to [47], wherein the polypeptide comprises a sequence having the substitution of an amino acid residue positioned at a site corresponding to a tryptophan residue at position 253 in the amino acid sequence represented by SEQ ID NO: 1 by one amino acid residue selected from the group consisting of a tyrosine residue, a threonine residue, a serine residue, an asparagine residue, a methionine residue, a histidine residue and an alanine residue.

[49] The polypeptide according to any of [2], [3], [7], [8], [12], [17], [18] and [40] to [48], wherein the polypeptide comprises a sequence having the substitution of an amino acid residue positioned at a site corresponding to a tryptophan residue at position 253 in the amino acid sequence represented by SEQ ID NO: 1 by a histidine residue.

[50] The polypeptide according to any of [2], [3], [7], [8], [12], [17], [18] and [40] to [49], wherein the polypeptide comprises a sequence having the substitution of an amino acid residue positioned at a site corresponding to a lysine residue at position 260 in the amino acid sequence represented by SEQ ID NO: 1 by one amino acid residue selected from the group consisting of a serine residue, a glutamine residue, an asparagine residue, a leucine residue, an isoleucine residue, a histidine residue, a glycine residue, a phenylalanine residue, a glutamic acid residue and an alanine residue.

[51] The polypeptide according to any of [2], [3], [7], [8], [12], [17], [18] and [40] to [50], wherein the polypeptide comprises a sequence having the substitution of an amino acid residue positioned at a site corresponding to a lysine residue at position 260 in the amino acid sequence represented by SEQ ID NO: 1 by one amino acid residue selected from the group consisting of a glutamine residue and a glutamic acid residue.

[52] The polypeptide according to any of [2], [3], [7], [8], [12], [17], [18] and [40] to [51], wherein

the amino acid sequence represented by SEQ ID NO: 1 with two amino acid residues thereof engineered comprises:

an amino acid sequence represented by SEQ ID NO: 25, wherein X at the site corresponding to the amino acid residue at position 141 is an alanine residue and X at the site corresponding to the amino acid residue at position 260 is a glutamic acid residue(M141A_K260E);
an amino acid sequence represented by SEQ ID NO: 25, wherein X at the site corresponding to the amino acid residue at position 141 is an alanine residue and X at the site corresponding to the amino acid residue at position 260 is a glutamine residue (M141A_K260Q);
an amino acid sequence represented by SEQ ID NO: 25, wherein X at the site corresponding to the amino acid residue at position 141 is a tyrosine residue and X at the site corresponding to the amino acid residue at position 260 is a glutamic acid residue(M141Y_K260E);
an amino acid sequence represented by SEQ ID NO: 25, wherein X at the site corresponding to the amino acid residue at position 141 is a valine residue, and X at the site corresponding to the amino acid residue at position 260 is a glutamic acid residue (M141V_K260E);
an amino acid sequence represented by SEQ ID NO: 27, wherein X at the site corresponding to the amino acid residue at position 182 is a leucine residue and X at the site corresponding to the amino acid residue at position 260 is a glutamic acid residue (H182L K260E);
an amino acid sequence represented by SEQ ID NO: 27, wherein X at the site corresponding to the amino acid residue at position 182 is a leucine residue and X at the site corresponding to the amino acid residue at position 260 is a glutamine residue (H182L_K260Q);
an amino acid sequence represented by SEQ ID NO: 27, wherein X at the position corresponding to the amino acid residue at position 182 is a methionine residue, and X at the position corresponding to the amino acid residue at position 260 is a glutamic acid residue (H182M_K260E);
an amino acid sequence represented by SEQ ID NO: 27, wherein X at the position corresponding to the amino acid residue at position 182 is a methionine residue and X at the site corresponding to the amino acid residue at position 260 is a glutamine residue (H182M_K260Q); or
an amino acid sequence represented by SEQ ID NO: 28, wherein X at the position corresponding to the amino acid residue at position 253 is a histidine residue and X at the site corresponding to the amino acid residue at position 260 is a glutamic acid residue (W253H _K260E).

[53] A polypeptide according to any of [2], [3], [7], [8], [12], [17], [18] and [40] to [52],

comprising a sequence having 90% or higher sequence identity to an amino acid sequence represented by SEQ ID NO: 25, wherein X at the site corresponding to the amino acid residue at position 141 is an alanine residue and X at the site corresponding to the amino acid residue at position 260 is a glutamic acid residue(M141A_K260E),
an amino acid sequence represented by SEQ ID NO: 25, wherein X at the site corresponding to the amino acid residue at position 141 is an alanine residue and X at the site corresponding to the amino acid residue at position 260 is a glutamine residue (M141A_K260Q),
an amino acid sequence represented by SEQ ID NO: 25, wherein X at the site corresponding to the amino acid residue at position 141 is a tyrosine residue and X at the site corresponding to the amino acid residue at position 260 is a glutamic acid residue (M141Y_K260E),
an amino acid sequence represented by SEQ ID NO: 25, wherein X at the site corresponding to the amino acid residue at position 141 is a valine residue, and X at the site corresponding to the amino acid residue at position 260 is a glutamic acid residue (M141V_K260E),
an amino acid sequence represented by SEQ ID NO: 27, wherein X at the site corresponding to the amino acid residue at position 182 is a leucine residue and X at the site corresponding to the amino acid residue at position 260 is a glutamic acid residue (H182L_K260E),
an amino acid sequence represented by SEQ ID NO: 27, wherein X at the site corresponding to the amino acid residue at position 182 is a leucine residue and X at the site corresponding to the amino acid residue at position 260 is a glutamine residue (H182L_K260Q),
an amino acid sequence represented by SEQ ID NO: 27, wherein X at the position corresponding to the amino acid residue at position 182 is a methionine residue, and X at the position corresponding to the amino acid residue at position 260 is a glutamic acid residue (H182M_K260E),
an amino acid sequence represented by SEQ ID NO: 27, wherein X at the position corresponding to the amino acid residue at position 182 is a methionine residue and X at the site corresponding to the amino acid residue at position 260 is a glutamine residue (H182M_K260Q), or
an amino acid sequence represented by SEQ ID NO: 28, wherein X at the position corresponding to the amino acid residue at position 253 is a histidine residue and X at the site corresponding to the amino acid residue at position 260 is a glutamic acid residue (W253H _K260E).

[54] A polypeptide according to any of [2], [3], [7], [8], [12], [17], [18] and [40] to [53], comprising

an amino acid sequence represented by SEQ ID NO: 25, wherein X at the site corresponding to the amino acid residue at position 141 is an alanine residue and X at the site corresponding to the amino acid residue at position 260 is a glutamic acid residue(M141A_K260E),
an amino acid sequence represented by SEQ ID NO: 25, wherein X at the site corresponding to the amino acid residue at position 141 is an alanine residue and X at the site corresponding to the amino acid residue at position 260 is a glutamine residue (M141A_K260Q),
an amino acid sequence represented by SEQ ID NO: 25, wherein X at the site corresponding to the amino acid residue at position 141 is a tyrosine residue and X at the site corresponding to the amino acid residue at position 260 is a glutamic acid residue (M141Y_K260E),
an amino acid sequence represented by SEQ ID NO: 25, wherein X at the site corresponding to the amino acid residue at position 141 is a valine residue, and X at the site corresponding to the amino acid residue at position 260 is a glutamic acid residue (M141V_K260E),
an amino acid sequence represented by SEQ ID NO: 27, wherein X at the site corresponding to the amino acid residue at position 182 is a leucine residue and X at the site corresponding to the amino acid residue at position 260 is a glutamic acid residue (H182L_K260E),
an amino acid sequence represented by SEQ ID NO: 27, wherein X at the site corresponding to the amino acid residue at position 182 is a leucine residue and X at the site corresponding to the amino acid residue at position 260 is a glutamine residue (H182L_K260Q),
an amino acid sequence represented by SEQ ID NO: 27, wherein X at the position corresponding to the amino acid residue at position 182 is a methionine residue, and X at the position corresponding to the amino acid residue at position 260 is a glutamic acid residue (H182M_K260E),
an amino acid sequence represented by SEQ ID NO: 27, wherein X at the position corresponding to the amino acid residue at position 182 is a methionine residue and X at the site corresponding to the amino acid residue at position 260 is a glutamine residue (H182M_K260Q), or
an amino acid sequence represented by SEQ ID NO: 28, wherein X at the position corresponding to the amino acid residue at position 253 is a histidine residue and X at the site corresponding to the amino acid residue at position 260 is a glutamic acid residue (W253H _K260E).

[55] The polypeptide according to any of [2], [3], [7], [8], [12], [17], [18] and [40] to [54], wherein the polypeptide has the amino acid sequence represented by any of SEQ ID NO: 36 to 44.
[56] The polypeptide according to any of [4], [5], [9], [10], [13], [19] and [20], wherein the amino acid sequence represented by SEQ ID NO: 1 has three or more engineering sites.
[57] The polypeptide according to any one of [4], [5], [9], [10], [13], [19], [20] and [56], wherein the amino acid sequence represented by SEQ ID NO: 1 has three engineering sites.
[58] A polypeptide according to any one of [4], [5], [9], [10], [13], [19], [20], [56] and [57],
comprising a sequence having the engineering of an amino acid residue positioned at a site corresponding to a methionine residue at position 141, a histidine residue at position 182, and a tryptophan residue at position 253, the engineering of an amino acid residue positioned at a site corresponding to a methionine residue at position 141, a histidine residue at position 182 and a lysine residue at position 260, the engineering of an amino acid residue positioned at a site corresponding to a methionine residue at position 141, a tryptophan residue at position 253 and a lysine residue at position 260, or the engineering of an amino acid residue positioned at a site corresponding to a histidine residue at position 182, a tryptophan residue at position 253 and a lysine residue at position 260 in the amino acid sequence represented by SEQ ID NO: 1.
[59] A polypeptide according to any one of [4], [5], [9], [10], [13], [19], [20] and [56] to [58],
comprising a sequence having the engineering of an amino acid residue positioned at a site corresponding to a methionine residue at position 141, a histidine residue at position 182 and a lysine residue at position 260, the engineering of an amino acid residue positioned at a site corresponding to a methionine residue at position 141, a tryptophan residue at position 253 and a lysine residue at position 260, or the engineering of an amino acid residue positioned at a site corresponding to a histidine residue at position 182, a tryptophan residue at position 253 and a lysine residue at position 260 in the amino acid sequence represented by SEQ ID NO: 1.
[60] The polypeptide according to any of [4], [5], [9], [10], [13], [19], [20] and [56] to [59],
wherein the amino acid sequence represented by SEQ ID NO: 1 has three engineering sites and the polypeptide comprises a sequence having the substitution of an amino acid residue positioned at a site corresponding to a methionine residue at position 141 in the amino acid sequence represented by SEQ ID NO: 1 by one amino acid residue selected from the group consisting of a tyrosine residue, a valine residue, a threonine residue, a serine residue, a leucine residue, a lysine residue, an isoleucine residue and an alanine residue.

[61] The polypeptide according to any of [4], [5], [9], [10], [13], [19], [20] and [56] to [60], wherein the amino acid sequence represented by SEQ ID NO: 1 has three engineering sites and the polypeptide comprises a sequence having the substitution of an amino acid residue positioned at a site corresponding to a methionine residue at position 141 in the amino acid sequence represented by SEQ ID NO: 1 by one amino acid residue selected from the group consisting of a tyrosine residue, a valine residue and an alanine residue.
[62] The polypeptide according to any of [4], [5], [9], [10], [13], [19], [20] and [56] to [61], wherein the amino acid sequence represented by SEQ ID NO: 1 has three engineering sites and the polypeptide comprises a sequence having the substitution of an amino acid residue positioned at a site corresponding to a histidine residue at position 182 in the amino acid sequence represented by SEQ ID NO: 1 by one amino acid residue selected from the group consisting of a methionine residue, a leucine residue and a phenylalanine residue.
[63] The polypeptide according to any of [4], [5], [9], [10], [13], [19], [20] and [56] to [62], wherein the amino acid sequence represented by SEQ ID NO: 1 has three engineering sites and the polypeptide comprises a sequence having the substitution of an amino acid residue positioned at a site corresponding to a histidine residue at position 182 in the amino acid sequence represented by SEQ ID NO: 1 by one amino acid residue selected from the group consisting of a methionine residue and a leucine residue.
[64] The polypeptide according to any of [4], [5], [9], [10], [13], [19], [20] and [56] to [63], wherein the amino acid sequence represented by SEQ ID NO: 1 has three engineering sites and the polypeptide comprises a sequence having the substitution of an amino acid residue positioned at a site corresponding to a tryptophan residue at position 253 in the amino acid sequence represented by SEQ ID NO: 1 by one amino acid residue selected from the group consisting of a valine residue, a threonine residue, a serine residue, a glutamine residue, a methionine residue, a leucine residue, a histidine residue, a phenylalanine residue and a glutamic acid residue.
[65] The polypeptide according to any of [4], [5], [9], [10], [13], [19], [20] and [56] to [64], wherein the amino acid sequence represented by SEQ ID NO: 1 has three engineering sites and the polypeptide comprises a sequence having the substitution of an amino acid residue positioned at a site corresponding to a tryptophan residue at position 253 in the amino acid sequence represented by SEQ ID NO: 1 by one amino acid residue selected from the group consisting of a glutamine residue, a methionine residue, a leucine residue and a histidine residue.
[66] The polypeptide according to any of [4], [5], [9], [10], [13], [19], [20] and [56] to [65], wherein the amino acid sequence represented by SEQ ID NO: 1 has three engineering sites and the polypeptide comprises a sequence having the substitution of an amino acid residue positioned at a site corresponding to a lysine residue at position 260 in the amino acid sequence represented by SEQ ID NO: 1 by one amino acid residue selected from the group consisting of a glutamine residue and a glutamic acid residue.
[67] The polypeptide according to any of [4], [5], [9], [10], [13], [19], [20] and [56] to [66], wherein

the amino acid sequence represented by SEQ ID NO: 1 with three amino acid residues thereof engineered comprises:
an amino acid sequence represented by SEQ ID NO: 33, wherein X at the site corresponding to the amino acid residue at position 141 is a tyrosine residue, X at the site corresponding to the amino acid residue at position 182 is a methionine residue, and X at the site corresponding to the amino acid residue at position 260 is a glutamic acid residue (M141Y_H182M_K260E);
an amino acid sequence represented by SEQ ID NO: 31, wherein X at the site corresponding to the amino acid residue at position 141 is a valine residue, X at the site corresponding to the amino acid residue at position 253 is a methionine residue, and X at the site corresponding to the amino acid residue at position 260 is a glutamic acid residue(M141V_W253M_K260E);
an amino acid sequence represented by SEQ ID NO: 32, wherein X at the site corresponding to the amino acid residue at position 182 is a leucine residue, X at the site corresponding to the amino acid residue at position 253 is a histidine residue, and X at the site corresponding to the amino acid residue at position 260 is a glutamic acid residue (H182L W253H K260E);
an amino acid sequence represented by SEQ ID NO: 33, wherein X at the site corresponding to the amino acid residue at position 141 is a tyrosine residue, X at the site corresponding to the amino acid residue at position 182 is a methionine residue, and X at the site corresponding to the amino acid residue at position 260 is a glutamine residue (M141Y_H182M_K260Q);
an amino acid sequence represented by SEQ ID NO: 33, wherein X at the site corresponding to the amino acid residue at position 141 is a valine residue, X at the site corresponding to the amino acid residue at position 182 is a methionine residue, and X at site corresponding to the amino acid residue at position 260 is a glutamic acid residue (M141V_H182M_K260E);
an amino acid sequence represented by SEQ ID NO: 33, wherein X at the site corresponding to the amino acid

residue at position 141 is a valine residue, X at the site corresponding to the amino acid residue at position 182 is a leucine residue, and X at the site corresponding to the amino acid residue at position 260 is a glutamine residue (M141V_H182L_K260Q);

An amino acid sequence represented by SEQ ID NO: 33, wherein X at the site corresponding to the amino acid residue at position 141 is a tyrosine residue, X at the site corresponding to the amino acid residue at position 182 is a leucine residue, and X at the site corresponding to the amino acid residue at position 260 is a glutamine residue (M141Y_H182L_ K260Q);

an amino acid sequence represented by SEQ ID NO: 31, wherein X at the site corresponding to the amino acid residue at position 141 is a leucine residue, X at the site corresponding to the amino acid residue at position 253 is a glutamine residue, and X at the site corresponding to the amino acid residue at position 260 is a glutamic acid residue (M141L_W253Q_K260E);

an amino acid sequence represented by SEQ ID NO: 32, wherein X at the site corresponding to the amino acid residue at position 182 is a leucine residue, X at the site corresponding to the amino acid residue at position 253 is a glutamine residue, and X at the site corresponding to the amino acid residue at position 260 is a glutamic acid residue (H182L W253Q K260E);

an amino acid sequence represented by SEQ ID NO: 31, wherein X at the site corresponding to the amino acid residue at position 141 is a isoleucine residue, X at the site corresponding to the amino acid residue at position 253 is a histidine residue, and X at the site corresponding to the amino acid residue at position 260 is a glutamic acid residue (M141I_W253H_K260E);

an amino acid sequence represented by SEQ ID NO: 33, wherein X at the site corresponding to the amino acid residue at position 141 is a valine residue, X at the site corresponding to the amino acid residue at position 182 is a leucine residue, and X at the site corresponding to the amino acid residue at position 260 is a glutamic acid residue (M141V_H182L_K260E);

an amino acid sequence represented by SEQ ID NO: 33, wherein X at the site corresponding to the amino acid residue at position 141 is a tyrosine residue, X at the site corresponding to the amino acid residue at position 182 is a leucine residue, and X at the site corresponding to the amino acid residue at position 260 is a glutamic acid residue (M141Y_H182L_K260E);

an amino acid sequence represented by SEQ ID NO: 32, wherein X at the site corresponding to the amino acid residue at position 182 is a leucine residue, X at the site corresponding to the amino acid residue at position 253 is a methionine residue, and X at the site corresponding to the amino acid residue at position 260 is a glutamic acid residue (H182L_W253M_K260E);

an amino acid sequence represented by SEQ ID NO: 31, wherein X at the site corresponding to the amino acid residue at position 141 is a valine residue, X at the site corresponding to the amino acid residue at position 253 is a glutamine residue, and X at the site corresponding to the amino acid residue at position 260 is a glutamine residue (M141V_W253Q_K260Q);

an amino acid sequence represented by SEQ ID NO: 31, wherein X at the site corresponding to the amino acid residue at position 141 is a valine residue, X at the site corresponding to the amino acid residue at position 253 is a histidine residue, and X at the site corresponding to the amino acid residue at position 260 is a glutamine residue (M141V_W253H_K260Q);

an amino acid sequence represented by SEQ ID NO: 31, wherein X at the site corresponding to the amino acid residue at position 141 is a valine residue, X at the site corresponding to the amino acid residue at position 253 is a leucine residue, and X at the site corresponding to the amino acid residue at position 260 is a glutamine residue (M141V_W253L_K260Q);

an amino acid sequence represented by SEQ ID NO: 31, wherein X at the site corresponding to the amino acid residue at site 141 is isoleucine, X at the site corresponding to the amino acid residue at position 253 is a histidine residue, and X at the site corresponding to the amino acid residue at position 260 is a glutamine residue (M141I_W253H_K260Q);

an amino acid sequence represented by SEQ ID NO: 31, wherein X at the site corresponding to the amino acid residue at site 141 is an isoleucine residue, X at the site corresponding to the amino acid residue at position 253 is a histidine residue, and X at the site corresponding to the amino acid residue at position 260 is a glutamine residue (M141I_W253H_K260Q); or

an amino acid sequence represented by SEQ ID NO: 31, wherein X at the site corresponding to the amino acid residue at site 141 is a valine residue, X at the site corresponding to the amino acid residue at position 253 is a methionine residue, and X at the site corresponding to the amino acid residue at position 260 is a glutamine residue (M141V_W253M_K260Q).

[68] A polypeptide according to any of [4], [5], [9], [10], [13], [19], [20] and [56] to [67],

comprising a sequence having 90% or higher sequence identity to an amino acid sequence represented by SEQ ID NO: 33, wherein X at the site corresponding to the amino acid residue at position 141 is a tyrosine residue, X at the site corresponding to the amino acid residue at position 182 is a methionine residue, and X at the site corresponding to the amino acid residue at position 260 is a glutamic acid residue (M141Y_H182M_K260E);

an amino acid sequence represented by SEQ ID NO: 31, wherein X at the site corresponding to the amino acid residue at position 141 is a valine residue, X at the site corresponding to the amino acid residue at position 253 is a methionine residue, and X at the site corresponding to the amino acid residue at position 260 is a glutamic acid residue(M141V_W253M_K260E);

an amino acid sequence represented by SEQ ID NO: 32, wherein X at the site corresponding to the amino acid residue at position 182 is a leucine residue, X at the site corresponding to the amino acid residue at position 253 is a histidine residue, and X at the site corresponding to the amino acid residue at position 260 is a glutamic acid residue (H182L W253H K260E);

an amino acid sequence represented by SEQ ID NO: 33, wherein X at the site corresponding to the amino acid residue at position 141 is a tyrosine residue, X at the site corresponding to the amino acid residue at position 182 is a methionine residue, and X at the site corresponding to the amino acid residue at position 260 is a glutamine residue (M141Y_H182M_K260Q);

an amino acid sequence represented by SEQ ID NO: 33, wherein X at the site corresponding to the amino acid residue at position 141 is a valine residue, X at the site corresponding to the amino acid residue at position 182 is a methionine residue, and X at site corresponding to the amino acid residue at position 260 is a glutamic acid residue (M141V_H182M_K260E);

an amino acid sequence represented by SEQ ID NO: 33, wherein X at the site corresponding to the amino acid residue at position 141 is a valine residue, X at the site corresponding to the amino acid residue at position 182 is a leucine residue, and X at the site corresponding to the amino acid residue at position 260 is a glutamine residue (M141V_H182L_K260Q) ;

An amino acid sequence represented by SEQ ID NO: 33, wherein X at the site corresponding to the amino acid residue at position 141 is a tyrosine residue, X at the site corresponding to the amino acid residue at position 182 is a leucine residue, and X at the site corresponding to the amino acid residue at position 260 is a glutamine residue (M141Y_H182L_ K260Q);

an amino acid sequence represented by SEQ ID NO: 31, wherein X at the site corresponding to the amino acid residue at position 141 is a leucine residue, X at the site corresponding to the amino acid residue at position 253 is a glutamine residue, and X at the site corresponding to the amino acid residue at position 260 is a glutamic acid residue (M141L_W253Q_K260E);

an amino acid sequence represented by SEQ ID NO: 32, wherein X at the site corresponding to the amino acid residue at position 182 is a leucine residue, X at the site corresponding to the amino acid residue at position 253 is a glutamine residue, and X at the site corresponding to the amino acid residue at position 260 is a glutamic acid residue (H182L W253Q K260E);

an amino acid sequence represented by SEQ ID NO: 31, wherein X at the site corresponding to the amino acid residue at position 141 is a isoleucine residue, X at the site corresponding to the amino acid residue at position 253 is a histidine residue, and X at the site corresponding to the amino acid residue at position 260 is a glutamic acid residue (M141I_W253H_K260E);

an amino acid sequence represented by SEQ ID NO: 33, wherein X at the site corresponding to the amino acid residue at position 141 is a valine residue, X at the site corresponding to the amino acid residue at position 182 is a leucine residue, and X at the site corresponding to the amino acid residue at position 260 is a glutamic acid residue (M141V_H182L_K260E);

an amino acid sequence represented by SEQ ID NO: 33, wherein X at the site corresponding to the amino acid residue at position 141 is a tyrosine residue, X at the site corresponding to the amino acid residue at position 182 is a leucine residue, and X at the site corresponding to the amino acid residue at position 260 is a glutamic acid residue (M141Y_H182L_K260E);

an amino acid sequence represented by SEQ ID NO: 32, wherein X at the site corresponding to the amino acid residue at position 182 is a leucine residue, X at the site corresponding to the amino acid residue at position 253 is a methionine residue, and X at the site corresponding to the amino acid residue at position 260 is a glutamic acid residue (H182L_W253M_K260E);

an amino acid sequence represented by SEQ ID NO: 31, wherein X at the site corresponding to the amino acid residue at position 141 is a valine residue, X at the site corresponding to the amino acid residue at position 253 is a glutamine residue, and X at the site corresponding to the amino acid residue at position 260 is a glutamine residue (M141V_W253Q_K260Q);

an amino acid sequence represented by SEQ ID NO: 31, wherein X at the site corresponding to the amino acid

residue at position 141 is a valine residue, X at the site corresponding to the amino acid residue at position 253 is a histidine residue, and X at the site corresponding to the amino acid residue at position 260 is a glutamine residue (M141V_W253H_K260Q);
an amino acid sequence represented by SEQ ID NO: 31, wherein X at the site corresponding to the amino acid residue at position 141 is a valine residue, X at the site corresponding to the amino acid residue at position 253 is a leucine residue, and X at the site corresponding to the amino acid residue at position 260 is a glutamine residue (M141V_W253L_K260Q);
an amino acid sequence represented by SEQ ID NO: 31, wherein X at the site corresponding to the amino acid residue at site 141 is isoleucine, X at the site corresponding to the amino acid residue at position 253 is a histidine residue, and X at the site corresponding to the amino acid residue at position 260 is a glutamine residue (M141I_W253H_K260Q);
an amino acid sequence represented by SEQ ID NO: 31, wherein X at the site corresponding to the amino acid residue at site 141 is an isoleucine residue, X at the site corresponding to the amino acid residue at position 253 is a histidine residue, and X at the site corresponding to the amino acid residue at position 260 is a glutamine residue (M141I_W253H_K260Q); or
an amino acid sequence represented by SEQ ID NO: 31, wherein X at the site corresponding to the amino acid residue at site 141 is a valine residue, X at the site corresponding to the amino acid residue at position 253 is a methionine residue, and X at the site corresponding to the amino acid residue at position 260 is a glutamine residue (M141V_W253M_K260Q).

[69] A polypeptide according to any of [4], [5], [9], [10], [13], [19], [20] and [56] to [68], comprising

an amino acid sequence represented by SEQ ID NO: 33, wherein X at the site corresponding to the amino acid residue at position 141 is a tyrosine residue, X at the site corresponding to the amino acid residue at position 182 is a methionine residue, and X at the site corresponding to the amino acid residue at position 260 is a glutamic acid residue (M141Y_H182M_K260E);
an amino acid sequence represented by SEQ ID NO: 31, wherein X at the site corresponding to the amino acid residue at position 141 is a valine residue, X at the site corresponding to the amino acid residue at position 253 is a methionine residue, and X at the site corresponding to the amino acid residue at position 260 is a glutamic acid residue(M141V_W253M_K260E);
an amino acid sequence represented by SEQ ID NO: 32, wherein X at the site corresponding to the amino acid residue at position 182 is a leucine residue, X at the site corresponding to the amino acid residue at position 253 is a histidine residue, and X at the site corresponding to the amino acid residue at position 260 is a glutamic acid residue (H182L W253H K260E);
an amino acid sequence represented by SEQ ID NO: 33, wherein X at the site corresponding to the amino acid residue at position 141 is a tyrosine residue, X at the site corresponding to the amino acid residue at position 182 is a methionine residue, and X at the site corresponding to the amino acid residue at position 260 is a glutamine residue (M141Y_H182M_K260Q);
an amino acid sequence represented by SEQ ID NO: 33, wherein X at the site corresponding to the amino acid residue at position 141 is a valine residue, X at the site corresponding to the amino acid residue at position 182 is a methionine residue, and X at site corresponding to the amino acid residue at position 260 is a glutamic acid residue (M141V_H182M_K260E);
an amino acid sequence represented by SEQ ID NO: 33, wherein X at the site corresponding to the amino acid residue at position 141 is a valine residue, X at the site corresponding to the amino acid residue at position 182 is a leucine residue, and X at the site corresponding to the amino acid residue at position 260 is a glutamine residue (M141V_H182L_K260Q);
An amino acid sequence represented by SEQ ID NO: 33, wherein X at the site corresponding to the amino acid residue at position 141 is a tyrosine residue, X at the site corresponding to the amino acid residue at position 182 is a leucine residue, and X at the site corresponding to the amino acid residue at position 260 is a glutamine residue (M141Y_H182L_ K260Q);
an amino acid sequence represented by SEQ ID NO: 31, wherein X at the site corresponding to the amino acid residue at position 141 is a leucine residue, X at the site corresponding to the amino acid residue at position 253 is a glutamine residue, and X at the site corresponding to the amino acid residue at position 260 is a glutamic acid residue (M141L_W253Q_K260E);
an amino acid sequence represented by SEQ ID NO: 32, wherein X at the site corresponding to the amino acid residue at position 182 is a leucine residue, X at the site corresponding to the amino acid residue at position 253 is a glutamine residue, and X at the site corresponding to the amino acid residue at position 260 is a glutamic acid residue (H182L W253Q K260E);

an amino acid sequence represented by SEQ ID NO: 31, wherein X at the site corresponding to the amino acid residue at position 141 is a isoleucine residue, X at the site corresponding to the amino acid residue at position 253 is a histidine residue, and X at the site corresponding to the amino acid residue at position 260 is a glutamic acid residue (M141I_W253H_K260E);

an amino acid sequence represented by SEQ ID NO: 33, wherein X at the site corresponding to the amino acid residue at position 141 is a valine residue, X at the site corresponding to the amino acid residue at position 182 is a leucine residue, and X at the site corresponding to the amino acid residue at position 260 is a glutamic acid residue (M141V_H182L_K260E);

an amino acid sequence represented by SEQ ID NO: 33, wherein X at the site corresponding to the amino acid residue at position 141 is a tyrosine residue, X at the site corresponding to the amino acid residue at position 182 is a leucine residue, and X at the site corresponding to the amino acid residue at position 260 is a glutamic acid residue (M141Y_H182L_K260E);

an amino acid sequence represented by SEQ ID NO: 32, wherein X at the site corresponding to the amino acid residue at position 182 is a leucine residue, X at the site corresponding to the amino acid residue at position 253 is a methionine residue, and X at the site corresponding to the amino acid residue at position 260 is a glutamic acid residue (H182L_W253M_K260E);

an amino acid sequence represented by SEQ ID NO: 31, wherein X at the site corresponding to the amino acid residue at position 141 is a valine residue, X at the site corresponding to the amino acid residue at position 253 is a glutamine residue, and X at the site corresponding to the amino acid residue at position 260 is a glutamine residue (M141V_W253Q_K260Q);

an amino acid sequence represented by SEQ ID NO: 31, wherein X at the site corresponding to the amino acid residue at position 141 is a valine residue, X at the site corresponding to the amino acid residue at position 253 is a histidine residue, and X at the site corresponding to the amino acid residue at position 260 is a glutamine residue (M141V_W253H_K260Q);

an amino acid sequence represented by SEQ ID NO: 31, wherein X at the site corresponding to the amino acid residue at position 141 is a valine residue, X at the site corresponding to the amino acid residue at position 253 is a leucine residue, and X at the site corresponding to the amino acid residue at position 260 is a glutamine residue (M141V_W253L_K260Q);

an amino acid sequence represented by SEQ ID NO: 31, wherein X at the site corresponding to the amino acid residue at site 141 is isoleucine, X at the site corresponding to the amino acid residue at position 253 is a histidine residue, and X at the site corresponding to the amino acid residue at position 260 is a glutamine residue (M141I_W253H_K260Q);

an amino acid sequence represented by SEQ ID NO: 31, wherein X at the site corresponding to the amino acid residue at site 141 is an isoleucine residue, X at the site corresponding to the amino acid residue at position 253 is a histidine residue, and X at the site corresponding to the amino acid residue at position 260 is a glutamine residue (M141I_W253H_K260Q); or

an amino acid sequence represented by SEQ ID NO: 31, wherein X at the site corresponding to the amino acid residue at site 141 is a valine residue, X at the site corresponding to the amino acid residue at position 253 is a methionine residue, and X at the site corresponding to the amino acid residue at position 260 is a glutamine residue (M141V_W253M_K260Q).

[70] The polypeptide according to any of [4], [5], [9], [10], [13], [19], [20] and [56] to [69], wherein the polypeptide has the amino acid sequence represented by any of SEQ ID NO: 45 to 62.

[71] The polypeptide according to any of [6] to [70], wherein the polypeptide has higher catalytic activity for the reductive amination reaction between at least any one compound A represented by the formula (1) or a salt thereof and at least any one compound B represented by the formula (2) or a salt thereof or the intramolecular reductive amination reaction of at least any one compound B represented by the formula (2) or a salt thereof than that of a polypeptide having the amino acid sequence represented by SEQ ID NO: 1 under at least one reaction condition.

[72] The polypeptide according to any of [1] to [70], wherein the polypeptide has higher catalytic activity for the reductive amination reaction between at least any one compound A represented by the formula (1) or a salt thereof and at least any one compound B represented by the formula (2) or a salt thereof or the intramolecular reductive amination reaction of at least any one compound B represented by the formula (2) or a salt thereof than that of a polypeptide having the amino acid sequence represented by SEQ ID NO: 17 under at least one reaction condition.

[73] The polypeptide according to any one of [6] to [10], wherein the alkylamine or a salt thereof is one or more selected from the group consisting of methylamine, ethylamine and salts thereof.

[74] The polypeptide according to any one of [6] to [10] and [73], wherein the compound represented by the formula (2') or a salt thereof is one or more selected from the group consisting of phenylpyruvic acid, 2-oxo-3-(p-tolyl)propanoic acid, 2-cyclopentyl-2-oxo-acetic acid and salts thereof.

[75] The polypeptide according to any of [1] to [74], wherein the polypeptide has higher catalytic activity for the reductive amination reaction between ethylamine or a salt thereof and phenylpyruvic acid or a salt thereof than that of a polypeptide consisting of the amino acid sequence represented by SEQ ID NO: 17 under at least one reaction condition.

[76] The polypeptide according to any of [1] to [75], wherein the polypeptide has higher catalytic activity for the reductive amination reaction between ethylamine or a salt thereof and 2-oxo-3-(p-tolyl)propanoic acid or a salt thereof than that of a polypeptide consisting of the amino acid sequence represented by SEQ ID NO: 17 under at least one reaction condition.

[77] The polypeptide according to any of [1] to [76], wherein the polypeptide has higher catalytic activity for the reductive amination reaction between methylamine or a salt thereof and 2-cyclopentyl-2-oxo-acetic acid or a salt thereof than that of a polypeptide consisting of the amino acid sequence represented by SEQ ID NO: 17 under at least one reaction condition.

[78] The polypeptide according to any of [1] to [77], wherein the polypeptide has catalytic activity for the reductive amination reaction between ammonia or a salt thereof and phenylpyruvic acid or a salt thereof under at least one reaction condition.

[79] The polypeptide according to any of [1] to [78], wherein the engineering is one or more selected from the group consisting of substitution, deletion and insertion.

[80] The polypeptide according to any of [1] to [79], wherein the engineering is conservative engineering.

[81] The polypeptide according to any of [1] to [80], wherein the engineering is substitution.

[82] The polypeptide according to any of [1] to [81], wherein the engineering is substitution by a natural amino acid different from the amino acid residue before the engineering.

[83] The polypeptide according to [82], wherein the natural amino acid is one or more selected from the group consisting of glycine, alanine, serine, threonine, valine, leucine, isoleucine, phenylalanine, tyrosine, tryptophan, histidine, glutamine, asparagine, glutamic acid, aspartic acid, methionine, lysine, arginine and proline.

[84] The polypeptide according to any of [1] to [83], wherein the polypeptide comprises one or more selected from the group consisting of a streptavidin-bound peptide tag sequence and a His tag sequence at any one or both of the N terminus and the C terminus.

[85] The polypeptide according to any of [1] to [84], wherein the number of amino acid residues is between 300 and 400 in the form of a monomer.

[86]

[86-1] The polypeptide according to any of [1] to [85], wherein a temperature at which the polypeptide has the higher catalytic activity than that of a polypeptide comprising the amino acid sequence represented by SEQ ID NO: 1 falls within the range of 0°C or higher and 50°C or lower.

[86-2] The polypeptide according to any of [1] to [85] and [86-1], wherein a temperature at which the polypeptide has the higher catalytic activity than that of a polypeptide consisting of the amino acid sequence represented by SEQ ID NO: 17 falls within the range of 0°C or higher and 50°C or lower.

[87]

[87-1] The polypeptide according to any of [1] to [86], wherein pH at which the polypeptide has the higher catalytic activity than that of a polypeptide comprising the amino acid sequence represented by SEQ ID NO: 1 falls within the range of 7 or higher and 11 or lower.

[87-2] The polypeptide according to any of [1] to [86] and [87-1] wherein pH at which the polypeptide has the higher catalytic activity than that of a polypeptide consisting of the amino acid sequence represented by SEQ ID NO: 17 falls within the range of 7 or higher and 11 or lower.

[88] The polypeptide according to any of [1] to [5], wherein the polypeptide has the higher catalytic activity than that of a polypeptide comprising the amino acid sequence represented by SEQ ID NO: 1 at at least one point in a range in which a total concentration of the compound A and a salt thereof at the start of reaction is 100 mM or higher and 3000 mM or lower in the total amount of a reaction solution in performing reductive amination reaction.

[89] The polypeptide according to any of [1] to [5] and [88], wherein the polypeptide has the higher catalytic activity than that of a polypeptide comprising the amino acid sequence represented by SEQ ID NO: 1 at at least one point in a range in which the total concentration of the compound B and a salt thereof at the start of reaction is 0.001 mM or higher and 1000 mM or lower in the total amount of a reaction solution in performing reductive amination reaction.

[90] The polypeptide according to any of [1] to [5] and [88] and [89], wherein the catalytic activity is higher than that of a polypeptide comprising the amino acid sequence represented by SEQ ID NO: 1 at at least one point when a ratio of the total molar number of the compound A and a salt thereof to the total molar number of the compound B

and a salt thereof at the start of reaction is 1 or more in the total amount of a reaction solution in performing reductive amination reaction.
[91]

[91-1] The polypeptide according to any of [1] to [90], wherein the polypeptide has catalytic activity higher than that of a polypeptide comprising the amino acid sequence represented by SEQ ID NO: 1 under reaction conditions of 37°C and pH 8.
[91-2] The polypeptide according to any of [1] to [90] and [91-1] wherein the polypeptide has catalytic activity higher than that of a polypeptide consisting of the amino acid sequence represented by SEQ ID NO: 17 under reaction conditions of 37°C and pH 8.

[92]

[92-1] The polypeptide according to any of [1] to [91], wherein the polypeptide has catalytic activity higher than that of a polypeptide comprising the amino acid sequence represented by SEQ ID NO: 1 under reaction conditions of 25°C and pH 9.
[92-2] The polypeptide according to any of [1] to [91] and [92-1], wherein the polypeptide has catalytic activity higher than that of a polypeptide consisting of the amino acid sequence represented by SEQ ID NO: 17 under reaction conditions of 25°C and pH 9.

[93] The polypeptide according to any of [1] to [92], wherein the catalytic activity is evaluated under the following reaction conditions:
the reaction is started with 50 mM phenylpyruvic acid, 2-oxo-3-(p-tolyl)propanoic acid, or 2-cyclopentyl-2-oxo-acetic acid, 100 mM D(+)-glucose, 500 mM methylamine or ethylamine, a 100 mM phosphate buffer solution, 0.89 mM β-NADPH, a 0.002 units/μL GDH solution, the polypeptide to be evaluated at a concentration of 2.5 μM under conditions of 37°C and pH 8, and a yield of an amino acid formed through reductive amination reaction is determined after a lapse of 19 hours.
[94] The polypeptide according to any of [1] to [93], wherein the polypeptide is an enzyme that catalyzes reductive amination reaction.
[95]

[95-1] The polypeptide according to any of [1] to [94], wherein the catalytic activity is 1.2 or more times the catalytic activity of the polypeptide comprising the amino acid sequence represented by SEQ ID NO: 1.
[95-2] The polypeptide according to any of [1] to [94] and [95-1], wherein the catalytic activity is 1.2 or more times the catalytic activity of the polypeptide consisting of the amino acid sequence represented by SEQ ID NO: 17.

[96] The polypeptide according to any of [1] to [5] and [88] to [90], wherein in the formula (1), $R^1$ is a hydrogen atom, and $R^2$ is a $C_1$ to $C_6$ alkyl group.
[97] The polypeptide according to any of [1] to [5], [88] to [90] and [96], wherein in the formula (1), $R^1$ is a hydrogen atom, and $R^2$ is an ethyl group.
[98] The polypeptide according to any of [1] to [5], [88] to [90] and [96], wherein in the formula (1), $R^1$ is a hydrogen atom, and $R^2$ is a methyl group.
[99] The polypeptide according to any of [1] to [5] and [88] to [90], wherein in the formula (1), each of $R^1$ and $R^2$ is a hydrogen atom.
[100] The polypeptide according to any of [1] to [5], [88] to [90] and [96] to [99], wherein in the formula (2), Y is a $C_3$ to $C_8$ cycloalkyl group or a $C_6$ to $C_9$ aralkyl group, and the aralkyl group is optionally substituted by a $C_1$ to $C_3$ alkyl group or a halogen atom.
[101] The polypeptide according to any of [1] to [5], [88] to [90] and [96] to [100], wherein in the formula (2), Y is a $C_3$ to $C_8$ cycloalkyl group.
[102] The polypeptide according to any of [1] to [5], [88] to [90] and [96] to [100], wherein in the formula (2), Y is a $C_6$ to $C_9$ aralkyl group, and the aralkyl group is optionally substituted by a $C_1$ to $C_3$ alkyl group or a halogen atom.
[103] The polypeptide according to any of [1] to [5] and [88] to [90], wherein

in the formula (1), $R^1$ is a hydrogen atom, and $R^2$ is an ethyl group; and
in the formula (2), each of n and m is 0, Y is a group represented by the formula (3), $R^3$ is a phenyl group, each of $R^4$, $R^5$ and $R^6$ is a hydrogen atom, and each of p, q and r is 1.

[104] The polypeptide according to any of [1] to [5] and [88] to [90], wherein

in the formula (1), $R^1$ is a hydrogen atom, and $R^2$ is an ethyl group; and
in the formula (2), each of n and m is 0, Y is a group represented by the formula (3), $R^3$ is a p-tolyl group, each of $R^4$, $R^5$ and $R^6$ is a hydrogen atom, and each of p, q and r is 1.

[105] The polypeptide according to any of [1] to [5] and [88] to [90], wherein

in the formula (1), $R^1$ is a hydrogen atom, and $R^2$ is a methyl group; and
in the formula (2), each of n and m is 0, Y is a group represented by the formula (3), $R^3$ and $R^4$ are linked to each other to form a cyclopentane ring, each of $R^5$ and $R^6$ is a hydrogen atom, and each of p, q and r is 1.

[106] The polypeptide according to any of [1] to [105], wherein

the polypeptide comprising a sequence has 95% sequence identity to an amino acid sequence represented by SEQ ID NO: 1 with one amino acid residue thereof engineered, and
has higher catalytic activity for a reductive amination reaction between ethylamine or a salt thereof and phenylpyruvic acid or a salt thereof than that of a polypeptide consisting of the amino acid sequence represented by SEQ ID NO: 17 under conditions of 37°C and pH 8.

[107] The polypeptide according to any of [1] to [106], wherein

the polypeptide comprises a sequence having 95% sequence identity to an amino acid sequence represented by SEQ ID NO: 1 with one amino acid residue thereof engineered, and
has higher catalytic activity for a reductive amination reaction between ethylamine or a salt thereof and 2-oxo-3-(p-tolyl)propanoic acid or a salt thereof than that of a polypeptide consisting of the amino acid sequence represented by SEQ ID NO: 17 under conditions of 37°C and pH 8.

[108] The polypeptide according to any of [1] to [107], wherein

the polypeptide comprises a sequence having 95% sequence identity to an amino acid sequence represented by SEQ ID NO: 1 with one amino acid residue thereof engineered, and
has higher catalytic activity for a reductive amination reaction between methylamine or a salt thereof and 2-cyclopentyl-2-oxo-acetic acid or a salt thereof than that of a polypeptide consisting of the amino acid sequence represented by SEQ ID NO: 17 under conditions of 37°C and pH 8.

[109] The polypeptide according to any of [1] to [108], wherein

the polypeptide comprises a sequence having 95% sequence identity to an amino acid sequence represented by SEQ ID NO: 1 with one amino acid residue thereof engineered, and
has higher catalytic activity for a reductive amination reaction between ethylamine or a salt thereof and phenylpyruvic acid or a salt thereof than that of a polypeptide consisting of the amino acid sequence represented by SEQ ID NO: 17 under conditions of 25°C and pH 9.

[110] The polypeptide according to any of [1] to [109], wherein

the polypeptide comprises a sequence having 95% sequence identity to an amino acid sequence represented by SEQ ID NO: 1 with one amino acid residue thereof engineered, and
has higher catalytic activity for a reductive amination reaction between ethylamine or a salt thereof and 2-oxo-3-(p-tolyl)propanoic acid or a salt thereof than that of a polypeptide consisting of the amino acid sequence represented by SEQ ID NO: 17 under conditions of 25°C and pH 9.

[111] The polypeptide according to any of [1] to [110], wherein

the polypeptide comprises a sequence having 95% sequence identity to an amino acid sequence represented by SEQ ID NO: 1 with one amino acid residue thereof engineered, and
has higher catalytic activity for a reductive amination reaction between methylamine or a salt thereof and 2-cyclopentyl-2-oxo-acetic acid or a salt thereof than that of a polypeptide consisting of the amino acid sequence

represented by SEQ ID NO: 17 under conditions of 25°C and pH 9.

[112] An isolated nucleic acid encoding a polypeptide according to any of [1] to [111].
[113] A vector comprising the nucleic acid according to [112].
[114] A transformant comprising the nucleic acid according to [112] or the vector according to [113].
[115] A method for producing a polypeptide, comprising a step of culturing the transformant of [114] so as to produce the polypeptide.
[116] The method according to [115], further comprising a step of recovering the polypeptide.
[117] A polypeptide produced by the method of [115] or [116].
[118] A reductive aminating agent comprising a polypeptide according to any of [1] to [111].
[119] A method for producing an amino acid, comprising a step of reacting one or more selected from the group consisting of amine, an amine analog and salts thereof with one or more selected from the group consisting of keto acid, a keto acid analog and salts thereof, or intramolecularly reacting a compound selected from the group consisting of keto acid, a keto acid analog and salts thereof in the presence of a polypeptide according to any of [1] to [111] and a reducing agent.
[120] The production method according to [119], wherein the reaction is performed under appropriate conditions.
[121] The production method according to [119] or [120], wherein the reaction involves the reductive amination reaction between the amine, the amine analog or a salt thereof and the keto acid, the keto acid analog or a salt thereof.
[122] The production method according to any of [119] to [121], wherein the amine or the amine analog is represented by the formula (1), and $R^1$ and $R^2$ in the formula (1) are synonymous with $R^1$ and $R^2$, respectively, described in the formula (1) of [1].
[123] The production method according to any of [119] to [122], wherein the keto acid or the keto acid analog is represented by the formula (2), and the formula (1'), the formula (3), X, Y, $Z^1$, $Z^2$, $R^{1a}$, $R^3$, $R^4$, $R^5$, $R^6$, m, n, p, q and r in the formula (2) are synonymous with the formula (1')the formula (3), X, Y, $Z^1$, $Z^2$, $R^{1a}$, $R^3$, $R^4$, $R^5$, $R^6$, m, n, p, q and r, respectively, described in the formula (2) of [1].
[124] The production method according to any of [119] to [123], wherein the keto acid or the keto acid analog is represented by the formula (2'), and Y' in the formula (2') is synonymous with Y' described in [6].
[125] The production method according to any of [119] to [124], wherein the reducing agent is one or more selected from the group consisting of nicotinamide adenine dinucleotide phosphate (NADPH), $NADP^+$, nicotinamide adenine dinucleotide (NADH) and $NAD^+$.
[126] The production method according to any of [119] to [125], wherein the reaction is performed in the presence of compound C represented by the following formula (4):

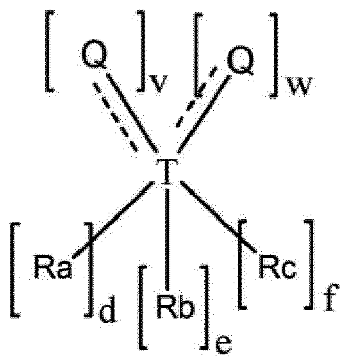
(4)

wherein

in the formula (4),
v and w each independently represent 0 or 1,
at least one of v and w represents 1,
T represents a carbon atom, a phosphorus atom, or a sulfur atom,
the functional group represented by the following formula (4a):

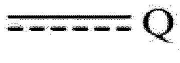
(4a)

represents =O, -ORd, or a hydroxy group,

when each of v and w is 1, two functional groups represented by a plurality of formulas (4a) are the same or different,

Ra, Rb, and Rc each independently represent a hydrogen atom, a $C_1$ to $C_3$ alkyl group, an alkylamino group, or $-CH_2-ORd$,

at least any two of Ra, Rb, and Rc are optionally linked to each other to form a ring structure together with T,

Rd represents a $C_1$ to $C_3$ alkyl group,

d, e, and f each independently represent 0 or 1,

at least one of d, e, and f represents 1,

when each of v and w is 1, at least one of Ra, Rb, and Rc is a methyl group, and none of Ra, Rb, and Rc are linked to each other to form a ring structure together with T,

when at least one of Ra, Rb, and Rc is a methylamino group, none of Ra, Rb, and Rc are linked to each other to form a ring structure together with T, and

when the functional group represented by the formula (4a) is a hydroxy group and T is a carbon atom, v is 1, w is 0, each of d, e, and f is 1, and each of Ra, Rb, and Rc is a hydrogen atom.

[127] The production method according to [126], wherein in the formula (4), T is a phosphorus atom or a sulfur atom, the functional group represented by the formula (4a) is =O, and each of Ra, Rb, and Rc is a methyl group.

[128] The production method according to [126] or [127], wherein the compound C is at least one compound selected from the group consisting of dimethyl sulfoxide, dimethylsulfone, dimethoxyethane, trimethylphosphine oxide, N,N-dimethylformamide, N,N-dimethylacetamide, tetramethylene sulfoxide, diethyl sulfoxide, methanol, and methylformamide.

[129] The production method according to any of [126] to [128], wherein the compound C is dimethyl sulfoxide.

[130] The production method according to any of [126] to [129], comprising the following steps (A) and (B):

step (A); a step of contacting a lithium-containing substance with a mixture to be purified which is a mixture of the following purification target (i) and the following impurities (ii), obtained by the method according to any of [126] to [129]:

(i) the amino acid having a protective group at the N terminus, and

(ii) compounds other than the purification target, and

step (B); a step of precipitating lithium salt of the purification target.

[131] A method for producing a peptide compound, comprising the steps of:

(1) producing an amino acid by the method according to any of [119] to [131]; and

(2) linking the amino acid to one or more selected from the group consisting of other amino acids and a peptide to produce a peptide compound.

The present invention can provide a novel polypeptide usable as an enzyme that catalyzes reductive amination reaction. The present invention can also provide a method for producing an amino acid using the polypeptide.

## BRIEF DESCRIPTION OF THE DRAWINGS

FIG. 1 is $^1$H-NMR measurement spectra of (2S, 3S) - 2-amino-3-phenyl-butanoic acid (I) obtained by the reaction in the example, purchased standard compounds (2S, 3R) - 2-amino-3-phenyl-butanoic acid hydrochloride (II) and (2S, 3S) - 2-amino-3-phenyl-butanoic acid hydrochloride (III).

FIG. 2 is chiral HPLC analysis data of (2S, 3S)-2-amino-3-phenylbutanoic acid (I) obtained by the reaction in the Example and a mixture (V) prepared by mixing the purchased standard compounds (III) and (IV) at a ratio of (III) : (IV) = 7 : 3.

## DETAILED DESCRIPTION

Hereinafter, the embodiments for carrying out the present invention will be described in detail. However, the present invention is not limited by embodiments given below.

In the present specification, the term "one or more" means a number of 1 or 2 or larger. When the term "one

or more" is used in a context related to a substituent for a certain group, this term means a number from 1 to the maximum number of substituents accepted by the group. Examples of the term "one or more" specifically include 1, 2, 3, 4, 5, 6, 7, 8, 9, 10, and/or larger numbers.

In the present specification, the term "to" that indicates a range includes values of both ends thereof. For example, "A to B" and "between A and B" means the range of A or more and B or less.

In the present specification, the term "approximately", when used in combination with a numeric value, means the value range of +10% and -10% of the numeric value.

In the present specification, the term "and/or" is meant to include every combination of the terms "and" and "or" appropriately combined. Specifically, for example, the term "A, B and/or C" includes the following seven engineerings: (i) A, (ii) B, (iii) C, (iv) A and B, (v) A and C, (vi) B and C, and (vii) A, B and C.

In the present specification, the "alkyl group" is a monovalent group induced by the removal of one arbitrary hydrogen atom from aliphatic hydrocarbon, and has a subset of a hydrocarbyl or hydrocarbon group structure containing hydrogen atoms and carbon atoms without containing a heteroatom (which refers to an atom other than carbon atoms and hydrogen atoms) or an unsaturated carbon-carbon bond in the skeleton. The alkyl group includes not only a linear form but a branched form. The alkyl group is specifically an alkyl group having 1 to 20 carbon atoms ($C_1$ to $C_{20}$; hereinafter, "$C_p$ to $C_q$" means that the number of carbon atoms is p to q), preferably a $C_1$ to $C_{10}$ alkyl group, more preferably a $C_1$ to $C_6$ alkyl group. Examples of the alkyl group specifically include a methyl group, an ethyl group, a n-propyl group, an i-propyl group, a n-butyl group, a s-butyl group, a t-butyl group, an isobutyl (2-methylpropyl) group, a n-pentyl group, a s-pentyl (1-methylbutyl) group, a t-pentyl (1,1-dimethylpropyl) group, a neopentyl (2,2-dimethylpropyl) group, an isopentyl (3-methylbutyl) group, a 3-pentyl (1-ethylpropyl) group, a 1,2-dimethylpropyl group, a 2-methylbutyl group, a n-hexyl group, a 1,1,2-trimethylpropyl group, a 1,2,2-trimethylpropyl group, a 1,1,2,2-tetramethylpropyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group, a 1-ethylbutyl group, and a 2-ethylbutyl group, preferably include a methyl group, an ethyl group, a n-propyl group, an i-propyl group, a n-butyl group, a s-butyl group, a t-butyl group, an isobutyl (2-methylpropyl) group, and more preferably include a methyl group, an ethyl group, a n-propyl group, and an i-propyl group and most preferably include a methyl group and an ethyl group.

In the present specification, the "alkenyl group" is a monovalent group having at least one double bond (two adjacent $SP^2$ carbon atoms). Depending on the conformation of the double bond and a substituent (if present), the geometric morphology of the double bond can assume entgegen (E) or zusammen (Z) and cis or trans conformations. The alkenyl group includes not only a linear form but a branched form. The alkenyl group is preferably a $C_2$ to $C_{10}$ alkenyl group, more preferably a $C_2$ to $C_6$ alkenyl group. Examples thereof specifically include a vinyl group, an allyl group, a 1-propenyl group, a 2-propenyl group, a 1-butenyl group, a 2-butenyl group (which includes cis and trans), a 3-butenyl group, a pentenyl group, a 3-methyl-2-butenyl group, and a hexenyl group.

In the present specification, the "alkynyl group" is a monovalent group having at least one triple bond (two adjacent SP carbon atoms). The alkynyl group includes not only a linear form but a branched form. The alkynyl group is preferably a $C_2$ to $C_{10}$ alkynyl group, more preferably a $C_2$ to $C_6$ alkynyl group. Examples thereof specifically include an ethynyl group, a 1-propynyl group, a propargyl group, a 3-butynyl group, a pentynyl group, a hexynyl group, a 3-phenyl-2-propynyl group, a 3-(2'-fluorophenyl)-2-propynyl group, a 2-hydroxy-2-propynyl group, a 3-(3-fluorophenyl)-2-propynyl group, and a 3-methyl-(5-phenyl)-4-pentynyl group.

In the present specification, the "cycloalkyl group" means a saturated or partially saturated cyclic monovalent aliphatic hydrocarbon group and includes a monocyclic ring, a bicyclo ring, and a spiro ring. The cycloalkyl group is preferably a $C_3$ to $C_8$ cycloalkyl group. Examples thereof specifically include a cyclopropyl group, a cyclobutyl group, a cyclopentyl group, a cyclohexyl group, a cycloheptyl group, a cyclooctyl group, a bicyclo[2.2.1]heptyl group, and a spiro[3.3]heptyl group.

In the present specification, the "aryl group" means a monovalent aromatic hydrocarbon ring and is preferably a $C_6$ to $C_{10}$ aryl group. Examples of the aryl group specifically include a phenyl group and a naphthyl (e.g., 1-naphthyl and 2-naphthyl) group.

In the present specification, the "heterocyclyl group" means a nonaromatic cyclic monovalent group containing a carbon atom as well as 1 to 5 heteroatoms. The heterocyclyl group may have a double and/or triple bond in the ring. A carbon atom in the ring may form carbonyl through oxidation, and the ring may be a monocyclic ring or a condensed ring. The number of atoms constituting the ring is preferably 4 to 10 (4- to 10-membered heterocyclyl group), more preferably 4 to 7 (4- to 7-membered heterocyclyl group). Examples of the heterocyclyl group specifically include an azetidinyl group, an oxiranyl group, an oxetanyl group, a dihydrofuryl group, a tetrahydrofuryl group, a dihydropyranyl group, a tetrahydropyranyl group, a tetrahydropyridyl group, a tetrahydropyrimidyl group, a morpholinyl group, a thiomorpholinyl group, a pyrrolidinyl group, a piperidinyl group, a piperazinyl group, a pyrazolidinyl group, an imidazolinyl group, an imidazolidinyl group, an oxazolidinyl group, an isoxazolidinyl group, a thiazolidinyl group, an isothiazolidinyl group, a 1,2-thiazinane group, a thiadiazolidinyl group, an oxazolidone group, a benzodioxanyl group, a benzoxazolyl group, a dioxolanyl group, a dioxanyl group, a tetrahydropyrrolo[1,2-c]imidazole group, a thietanyl group, a 3,6-diazabicyclo[3.1.1]heptanyl group, a 2,5-diazabicyclo[2.2.1]heptanyl group, a 3-oxa-8-azabicyclo[3.2.1]octanyl group, a sultam group, and a 2-oxaspiro[3.3]heptyl group.

In the present specification, the "protected heterocyclyl group" means a group in which one or more functional groups, for example, an amino group, contained in the "heterocyclyl group" defined above, is protected with an arbitrary protective group, and is preferably a protected 4- to 7-membered heterocyclyl group. Examples of the protective group specifically include Boc, Fmoc, Cbz, Troc, and Alloc. Examples of the protected heterocyclyl group specifically include a Boc-protected azetidine group.

In the present specification, the "heterocycloalkylidene group" means a divalent group that results from the removal of two hydrogen atoms from one carbon atom of the "heterocyclyl group" defined above and has a free valence that constitutes a portion of a double bond. The heterocycloalkylidene group is preferably a 4- to 7-membered heterocycloalkylidene group. Examples thereof specifically include a tetrahydropyran-4-ylidene group and an azetidin-3-ylidene group.

In the present specification, the "protected heterocycloalkylidene group" means a group in which one or more functional groups, for example, an amino group, contained in the "heterocycloalkylidene group" defined above, is protected with an arbitrary protective group, and is preferably a protected 4- to 7-membered heterocycloalkylidene group. Examples of the protective group specifically include Boc, Fmoc, Cbz, Troc, and Alloc. Examples of the protected heterocycloalkylidene group specifically include a Boc-protected azetidin-3-ylidene group.

In the present specification, the "heteroaryl group" means an aromatic cyclic monovalent group containing a carbon atom as well as 1 to 5 heteroatoms. The ring may be a monocyclic ring or a condensed ring with another ring and may be partially saturated. The number of atoms constituting the ring may be 5 to 12 (5- to 12-membered heteroaryl), may be 6 to 10 (6- to 10-membered heteroaryl), or may be 6 or 7 (6- or 7-membered heteroaryl). Examples of the heteroaryl group specifically include a furyl group, a thienyl group, a pyrrolyl group, an imidazolyl group, a pyrazolyl group, a thiazolyl group, an isothiazolyl group, an oxazolyl group, an isoxazolyl group, an oxadiazolyl group, a thiadiazolyl group, a triazolyl group, a tetrazolyl group, a pyridyl group, a pyrimidyl group, a pyridazinyl group, a pyrazinyl group, a triazinyl group, a benzofuranyl group, a benzothienyl group, a benzothiadiazolyl group, a benzothiazolyl group, a benzoxazolyl group, a benzoxadiazolyl group, a benzimidazolyl group, an indolyl group, an isoindolyl group, an indazolyl group, a quinolyl group, an isoquinolyl group, a cinnolinyl group, a quinazolinyl group, a quinoxalinyl group, a benzodioxolyl group, an indolizinyl group, and an imidazopyridyl group.

In the present specification, the "alkoxy group" means an oxy group bonded to the "alkyl group" defined above and is preferably a $C_1$ to $C_6$ alkoxy group. Examples of the alkoxy group specifically include a methoxy group, an ethoxy group, a 1-propoxy group, a 2-propoxy group, a n-butoxy group, an i-butoxy group, a s-butoxy group, a t-butoxy group, a pentyloxy group, and a 3-methylbutoxy group.

In the present specification, the "alkylthio group" means a thiol group bonded to the "alkyl group" defined above and is preferably a $C_1$ to $C_6$ alkylthio group. Examples of the alkylthio group specifically include a methylthio group, an ethylthio group, a 1-propylthio group, a 2-propylthio group, a n-butylthio group, an i-butylthio group, a s-butylthio group, and a t-butylthio group.

In the present specification, the "alkenyloxy group" means an oxy group bonded to the "alkenyl group" defined above and is preferably a $C_2$ to $C_6$ alkenyloxy group. Examples of the alkenyloxy group specifically include a vinyloxy group, an allyloxy group, a 1-propenyloxy group, a 2-propenyloxy group, a 1-butenyloxy group, a 2-butenyloxy group (which includes cis and trans), a 3-butenyloxy group, a pentenyloxy group, and a hexenyloxy group.

In the present specification, the "cycloalkoxy group" means an oxy group bonded to the "cycloalkyl group" defined above and is preferably a $C_3$ to $C_8$ cycloalkoxy group. Examples of the cycloalkoxy group specifically include a cyclopropoxy group, a cyclobutoxy group, and a cyclopentyloxy group.

In the present specification, the "aryloxy group" means an oxy group bonded to the "aryl group" defined above and is preferably a $C_6$ to $C_{10}$ aryloxy group. Examples of the aryloxy group specifically include a phenoxy group, a 1-naphthyloxy group, and a 2-naphthyloxy group.

In the present specification, the "amino group" means $-NH_2$ in the narrow sense and means -NRR' in the broad sense. In this context, R and R' are each independently selected from a hydrogen atom, an alkyl group, an alkenyl group, an alkynyl group, a cycloalkyl group, a heterocyclyl group, an aryl group, and a heteroaryl group, or R and R' form a ring together with the nitrogen atom bonded thereto. Examples of the amino group preferably include $-NH_2$, a mono-Ci to $C_6$ alkylamino group, a di-Ci to $C_6$ alkylamino group, and a 4- to 8-membered cyclic amino group.

In the present specification, the "monoalkylamino group" means a group of the "amino group" defined above in which R is a hydrogen atom, and R' is the "alkyl group" defined above, and is preferably a mono-Ci to $C_6$ alkylamino group. Examples of the monoalkylamino group specifically include a methylamino group, an ethylamino group, a n-propylamino group, an i-propylamino group, a n-butylamino group, a s-butylamino group, and a t-butylamino group.

In the present specification, the "dialkylamino group" means a group of the "amino group" defined above in which R and R' are each independently the "alkyl group" defined above, and is preferably a di-Ci to $C_6$ alkylamino group. Examples of the dialkylamino group specifically include a dimethylamino group and a diethylamino group.

In the present specification, the "cyclic amino group" means a group of the "amino group" defined above in which R and R' form a ring together with the nitrogen atom bonded thereto, and is preferably a 4- to 8-membered cyclic amino group. Examples of the cyclic amino group specifically include a 1-azetidyl group, a 1-pyrrolidyl group, a 1-piperidyl group, a 1-piperazyl group, a 4-morpholinyl group, a 3-oxazolidyl group, a 1,1-dioxidothiomorpholinyl-4-yl group, and a 3-oxa-8-azabicyclo[3.2.1]octan-8-yl group.

In the present specification, the "protected amino group" means an amino group protected with an arbitrary protective group. Examples of the protected amino specifically include an amino group protected with a protective group such as Boc, Fmoc, Cbz, Troc, or Alloc.

In the present specification, the "aminocarbonyl group" means a carbonyl group bonded to the "amino group" defined above and is preferably $-CONH_2$, a mono-Ci to $C_6$ alkylaminocarbonyl group, a di-Ci to $C_6$ alkylaminocarbonyl group, or a 4- to 8-membered cyclic aminocarbonyl group. Examples of the aminocarbonyl group specifically include -$CONH_2$, a dimethylaminocarbonyl group, a 1-azetidinylcarbonyl group, a 1-pyrrolidinylcarbonyl group, a 1-piperidinylcarbonyl group, a 1-piperazinylcarbonyl group, a 4-morpholinylcarbonyl group, a 3-oxazolidinylcarbonyl group, a 1,1-dioxidothiomorpholinyl-4-ylcarbonyl group, and a 3-oxa-8-azabicyclo[3.2.1]octane-8-ylcarbonyl group.

In the present specification, the "alkenyloxycarbonyl group" means a carbonyl group bonded to the "alkenyloxy group" defined above and is preferably a $C_2$ to $C_6$ alkenyloxycarbonyl group. Examples of the alkenyloxycarbonyl group specifically include a vinyloxycarbonyl group, an allyloxycarbonyl group, a 1-propenyloxy carbonyl group, a 2-propenyloxycarbonyl group, a 1-butenyloxycarbonyl group, a 2-butenyloxycarbonyl group (which includes cis and trans), a 3-butenyloxycarbonyl group, a pentenyloxycarbonyl group, and a hexenyloxycarbonyl group.

In the present specification, the "alkylsulfonyl group" means a sulfonyl group bonded to the "alkyl group" defined above and is preferably a Ci to $C_6$ alkylsulfonyl group. Examples of the alkylsulfonyl group specifically include a methylsulfonyl group.

In the present specification, the "hydroxyalkyl group" means a group in which one or more hydrogen atoms of the "alkyl group" defined above are replaced with a hydroxy group, and is preferably a $C_1$ to $C_6$ hydroxyalkyl group. Examples of the hydroxyalkyl group specifically include a hydroxymethyl group, a 1-hydroxyethyl group, a 2-hydroxyethyl group, a 2-hydroxy-2-methylpropyl group, and a 5-hydroxypentyl group.

In the present specification, the "haloalkyl group" means a group in which one or more hydrogen atoms of the "alkyl group" defined above are replaced with a halogen atom, and is preferably a $C_1$ to $C_6$ haloalkyl group, more preferably a $C_1$ to $C_6$ fluoroalkyl group. Examples of the haloalkyl group specifically include a difluoromethyl group, a trifluoromethyl group, a 2,2-difluoroethyl group, a 2,2,2-trifluoroethyl group, a 3,3-difluoropropyl group, a 4,4-difluorobutyl group, and a 5,5-difluoropentyl group.

In the present specification, the "cyanoalkyl group" means a group in which one or more hydrogen atoms of the "alkyl group" defined above are replaced with a cyano group, and is preferably a $C_1$ to $C_6$ cyanoalkyl group. Examples of the cyanoalkyl group specifically include a cyanomethyl group and a 2-cyanoethyl group.

In the present specification, the "aminoalkyl group" means a group in which one or more hydrogen atoms of the "alkyl group" defined above are replaced with the "amino group" defined above, and is preferably a $C_1$ to $C_6$ aminoalkyl group. Examples of the aminoalkyl group specifically include a 1-pyridylmethyl group, a 2-(1-piperidyl)ethyl group, a 3-(1-piperidyl)propyl group, and a 4-aminobutyl group.

In the present specification, the "carboxyalkyl group" means a group in which one or more hydrogen atoms of the "alkyl group" defined above are replaced with a carboxy group, and is preferably a $C_2$ to $C_6$ carboxyalkyl group. Examples of the carboxyalkyl group specifically include a carboxymethyl group.

In the present specification, the "alkenyloxycarbonylalkyl group" means a group in which one or more hydrogen atoms of the "alkyl group" defined above are replaced with the "alkenyloxycarbonyl group" defined above, and is preferably a $C_2$ to $C_6$ alkenyloxycarbonyl-Ci to $C_6$ alkyl group, more preferably a $C_2$ to $C_6$ alkenyloxycarbonyl-Ci or $C_2$ alkyl group. Examples of the alkenyloxycarbonylalkyl group specifically include an allyloxycarbonylmethyl group and a 2-(allyloxycarbonyl)ethyl group.

In the present specification, the "alkoxyalkyl group" means a group in which one or more hydrogen atoms of the "alkyl group" defined above are replaced with the "alkoxy group" defined above, and is preferably a $C_1$ to $C_6$ alkoxy-Ci to $C_6$ alkyl group, more preferably a $C_1$ to $C_6$ alkoxy-Ci or $C_2$ alkyl group. Examples of the alkoxyalkyl group specifically include a methoxymethyl group, an ethoxymethyl group, a 1-propoxymethyl group, a 2-propoxymethyl group, a n-butoxymethyl group, an i-butoxymethyl group, a s-butoxymethyl group, a t-butoxymethyl group, a pentyloxymethyl group, a 3-methylbutoxymethyl group, a 1-methoxyethyl group, a 2-methoxyethyl group, and a 2-ethoxyethyl group.

In the present specification, the "alkylthioalkyl group" means a group in which one or more hydrogen atoms of the "alkyl group" defined above are replaced with the "alkylthio group" defined above, and is preferably a $C_1$ to $C_6$ alkylthio-Ci to $C_6$ alkyl group, more preferably a $C_1$ to $C_6$ alkylthio-Ci or $C_2$ alkyl group. Examples of the alkylthioalkyl group specifically include a methylthiomethyl group, an ethylthiomethyl group, a 1-propylthiomethyl group, a 2-propylthiomethyl group, a n-butylthiomethyl group, an i-butylthiomethyl group, a s-butylthiomethyl group, and a t-butylthiomethyl group.

In the present specification, the "alkenyloxyalkyl group" means a group in which one or more hydrogen atoms of the "alkyl group" defined above are replaced with the "alkenyloxy group" defined above, and is preferably a $C_2$ to $C_6$ alkenyloxy-Ci to $C_6$ alkyl group, more preferably a $C_1$ to $C_6$ alkenyloxy-Ci or $C_2$ alkyl group. Examples of the alkenyloxyalkyl group specifically include a vinyloxymethyl group and an allyloxymethyl group.

In the present specification, the "cycloalkylalkyl group" means a group in which one or more hydrogen atoms of the "alkyl group" defined above are replaced with the "cycloalkyl group" defined above, and is preferably a $C_3$ to $C_8$ cycloalkyl-Ci to $C_6$ alkyl group, more preferably a $C_3$ to $C_6$ cycloalkyl-Ci or $C_2$ alkyl group. Examples of the cycloalkylalkyl group specifically include a cyclopropylmethyl group, a cyclobutylmethyl group, a cyclopentylmethyl group, and a cyclohexylmethyl group.

In the present specification, the "cycloalkoxyalkyl group" means a group in which one or more hydrogen atoms of the "alkyl group" defined above are replaced with the "cycloalkoxy group" defined above, and is preferably a $C_3$ to $C_8$ cycloalkoxy-Ci to $C_6$ alkyl group, more preferably a $C_3$ to $C_6$ cycloalkoxy-Ci or $C_2$ alkyl group. Examples of the cycloalkoxyalkyl group specifically include a cyclopropoxymethyl group and a cyclobutoxymethyl group.

In the present specification, the "heterocyclylalkyl group" means a group in which one or more hydrogen atoms of the "alkyl group" defined above are replaced with the "heterocyclyl group" defined above, and is preferably a 4- to 7-membered heterocyclyl-Ci to $C_6$ alkyl group, more preferably a 4- to 7-membered heterocyclyl-Ci or $C_2$ alkyl group. Examples of the heterocyclylalkyl group specifically include a 2-(tetrahydro-2H-pyran-4-yl)ethyl group and a 2-(azetidin-3-yl)ethyl group.

In the present specification, the "alkylsulfonylalkyl group" means a group in which one or more hydrogen atoms of the "alkyl group" defined above are replaced with the "alkylsulfonyl group" defined above, and is preferably a $C_1$ to $C_6$ alkylsulfonyl-Ci to $C_6$ alkyl group, more preferably a $C_1$ to $C_6$ alkylsulfonyl-Ci or $C_2$ alkyl group. Examples of the alkylsulfonylalkyl group specifically include a methylsulfonylmethyl group and a 2-(methylsulfonyl)ethyl group.

In the present specification, the "aminocarbonylalkyl group" means a group in which one or more hydrogen atoms of the "alkyl group" defined above are replaced with the "aminocarbonyl group" defined above, and is preferably an aminocarbonyl-Ci to $C_6$ alkyl group, more preferably an aminocarbonyl-$C_1$ to $C_4$ alkyl group. Examples of the aminocarbonylalkyl group specifically include a methylaminocarbonylmethyl group, a dimethylaminocarbonylmethyl group, a t-butylaminocarbonylmethyl group, a 1-azetidinylcarbonylmethyl group, a 1-pyrrolidinylcarbonylmethyl group, a 1-piperidinylcarbonylmethyl group, a 4-morpholinylcarbonylmethyl group, a 2-(methylaminocarbonyl)ethyl group, a 2-(dimethylaminocarbonyl)ethyl group, a 2-(1-azetidinylcarbonyl)ethyl group, a 2-(1-pyrrolidinylcarbonyl)ethyl group, a 2-(4-morpholinylcarbonyl)ethyl group, a 3-(dimethylaminocarbonyl)propyl group, and a 4-(dimethylaminocarbonyl)butyl group.

In the present specification, the "aryloxyalkyl group" means a group in which one or more hydrogen atoms of the "alkyl group" defined above are replaced with the "aryloxy group" defined above, and is preferably a $C_6$ to $C_{10}$ aryloxy-Ci to $C_6$ alkyl group, more preferably a $C_6$ to $C_{10}$ aryloxy-Ci or $C_2$ alkyl group. Examples of the aryloxyalkyl group specifically include a phenoxymethyl group and a 2-phenoxyethyl group.

In the present specification, the "aralkyl (arylalkyl) group" means a group in which at least one hydrogen atom of the "alkyl group" defined above is replaced with the "aryl group" defined above, and is preferably a $C_7$ to $C_{14}$ aralkyl group, more preferably a $C_7$ to $C_{10}$ aralkyl group. Examples of the aralkyl group specifically include a benzyl group, a phenethyl group, and a 3-phenylpropyl group.

In the present specification, the "aralkoxy group" means an oxy group bonded to the "aralkyl group" defined above and is preferably a $C_7$ to $C_{14}$ aralkoxy group, more preferably a $C_7$ to $C_{10}$ aralkoxy group. Examples of the aralkoxy group specifically include a benzyloxy group, a phenethyloxy group, and a 3-phenylpropoxy group.

In the present specification, the "aralkoxyalkyl group" means a group in which one or more hydrogen atoms of the "alkyl group" defined above are replaced with the "aralkoxy group" defined above, and is preferably a $C_7$ to $C_{14}$ aralkoxy-Ci to $C_6$ alkyl group, more preferably a $C_7$ to $C_{14}$ aralkoxy-Ci or $C_2$ alkyl group. Examples of the aralkoxyalkyl group specifically include a benzyloxymethyl group and a 1-(benzyloxy)ethyl group.

In the present specification, the "heteroarylalkyl group" means a group in which at least one hydrogen atom of the "alkyl group" defined above is replaced with the "heteroaryl group" defined above, and is preferably a 5- to 10-membered heteroaryl-Ci to $C_6$ alkyl group, more preferably a 5- to 10-membered heteroaryl-Ci or $C_2$ alkyl group. Examples of the heteroarylalkyl group specifically include a 3-thienylmethyl group, a 4-thiazolylmethyl group, a 2-pyridylmethyl group, a 3-pyridylmethyl group, a 4-pyridylmethyl group, a 2-(2-pyridyl)ethyl group, a 2-(3-pyridyl)ethyl group, a 2-(4-pyridyl)ethyl group, a 2-(6-quinolyl)ethyl group, a 2-(7-quinolyl)ethyl group, a 2-(6-indolyl)ethyl group, a 2-(5-indolyl)ethyl group, and a 2-(5-benzofuranyl)ethyl group.

In the present specification, the "heteroarylalkoxy group" means an oxy group bonded to the "heteroarylalkyl group" defined above and is preferably a 5- to 10-membered heteroaryl-Ci to $C_6$ alkoxy group, more preferably a 5- to 10-membered heteroaryl-Ci or $C_2$ alkoxy group. Examples of the heteroarylalkoxy group specifically include a 3-thienylmethoxy group and a 3-pyridylmethoxy group.

In the present specification, the "heteroarylalkoxyalkyl group" means a group in which one or more hydrogen

atoms of the "alkyl group" defined above are replaced with the "heteroarylalkoxy group" defined above, and is preferably a 5- to 10-membered heteroaryl-Ci to $C_6$ alkoxy-Ci to $C_6$ alkyl group, more preferably a 5- to 10-membered heteroaryl-Ci or $C_2$ alkoxy-Ci or $C_2$ alkyl group. Examples of the heteroarylalkoxyalkyl group specifically include a 3-pyridylmethoxymethyl group.

In the present specification, the "heterocycloalkylidenealkyl group" means a group in which one or more hydrogen atoms of the "alkyl group" defined above are replaced with the "heterocycloalkylidene group" defined above, and is preferably a 4- to 7-membered heterocycloalkylidene-Ci to $C_6$ alkyl group, more preferably a 4- to 7-membered heterocycloalkylidene-Ci or $C_2$ alkyl group. Examples of the heterocycloalkylidenealkyl group specifically include a tetrahydro-4H-pyran-4-ylidenemethyl and azetidin-3-ylidenemethyl group.

In the present specification, the "alkoxyalkenyl group" means a group in which one or more hydrogen atoms of the "alkenyl group" defined above are replaced with the "alkoxy group" defined above, and is preferably a $C_1$ to $C_6$ alkoxy-$C_2$ to $C_6$ alkenyl group. Examples of the alkoxyalkenyl group specifically include a (E)-4-methoxybut-2-en-1-yl group.

In the present specification, the "aminocarbonylalkenyl group" means a group in which one or more hydrogen atoms of the "alkenyl group" defined above are replaced with the "aminocarbonyl group" defined above, and is preferably an aminocarbonyl-$C_2$ to $C_6$ alkenyl group. Examples of the aminocarbonylalkenyl group specifically include a (E)-3-(dimethylaminocarbonylcarbonyl)-prop-2-en-1-yl group.

In the present specification, the "haloalkoxy group" means a group in which one or more hydrogen atoms of the "alkoxy group" defined above are replaced with a halogen atom, and is preferably a $C_1$ to $C_6$ haloalkoxy group. Examples of the haloalkoxy group specifically include a difluoromethoxy group, a trifluoromethoxy group, a 2,2-difluoroethoxy group, and a 2,2,2-trifluoroethoxy group.

In the present specification, the "alkylene group" means a divalent group induced by the further removal of one arbitrary hydrogen atom from the "alkyl group" described above, and is preferably a $C_4$ to $C_8$ alkylene group. Examples of the alkylene group specifically include - $CH_2$-, -$(CH_2)_2$-, -$(CH_2)_3$-, -$CH(CH_3)CH_2$-, -$C(CH_3)_2$-, -$(CH_2)_4$-, -$CH(CH_3)CH_2CH_2$-, - $C(CH_3)_2CH_2$-, -$CH_2CH(CH_3)CH_2$-, -$CH_2C(CH_3)_2$-, -$CH_2CH_2CH(CH_3)$-, -$(CH_2)_5$-, -$(CH_2)_6$-, -$(CH_2)_7$-, and -$(CH_2)_8$-.

In the present specification, the "cycloalkylene group" means a divalent group induced by the further removal of one arbitrary hydrogen atom from the "cycloalkyl group" described above, and is preferably a $C_3$ to $C_8$ cycloalkylene group. Examples of the cycloalkylene group specifically include a cyclopropane-1,2-diyl group, a cyclobutane-1,2-diyl group, a cyclopentane-1,2-diyl group, and a cyclohexane-1,2-diyl group.

In the present specification, the "alkenylene group" means a divalent group induced by the further removal of one arbitrary hydrogen atom from the "alkenyl group" described above. Depending on the conformation of the double bond and a substituent (if present), the geometric morphology of the double bond can assume entgegen (E) or zusammen (Z) and cis or trans conformations. The alkenylene group includes a linear or branched form and is preferably a $C_2$ to $C_{10}$ alkenylene group, more preferably a $C_2$ to $C_6$ alkenylene group.

In the present specification, the "alkynylene group" means a divalent group induced by the further removal of one arbitrary hydrogen atom from the "alkynyl group" described above. The alkynylene group includes a linear or branched form and is preferably a $C_2$ to $C_{10}$ alkynylene group, more preferably a $C_2$ to $C_6$ alkynylene group.

In the present specification, the "arylene group" means a divalent group induced by the further removal of one arbitrary hydrogen atom from the "aryl group" described above. The arylene group may be a monocyclic ring or a condensed ring. The number of atoms constituting the ring is not particularly limited and is preferably 6 to 10 ($C_6$ to $C_{10}$ arylene group). Examples of the arylene group specifically include a 1,2-phenylene group, a 1,3-phenylene group, a 1,4-phenylene group, a 1,2-naphthylene group, a 1,3-naphthylene group, and a 1,4-naphthylene group.

In the present specification, the "spirocycloalkyl group" means a group formed such that one carbon atom constituting a cycloalkane ring is shared by a carbon atom in a group to be bonded thereto. The spirocycloalkyl group is preferably a $C_3$ to $C_8$ spirocycloalkyl group. Examples thereof specifically include a spirocyclopropyl group, a spirocyclobutyl group, a spirocyclopentyl group, a spirocyclohexyl group, a spirocycloheptyl group, and a spirocyclooctyl group.

In the present specification, the "spiroheterocyclyl group" means a group in which one or more carbon atoms in the "spirocycloalkyl group" are replaced with a heteroatom. The spiroheterocyclyl group is preferably 4- to 10-membered spiroheterocyclyl.

In the present specification, the "alicyclic ring" means a nonaromatic hydrocarbon ring. The alicyclic ring may have an unsaturated bond in the ring and may be a polycyclic ring having two or more rings. A carbon atom constituting the ring may form a carbonyl group through oxidation. The alicyclic ring is preferably a 3- to 8-membered alicyclic ring. Examples thereof specifically include a cyclopropane ring, a cyclobutane ring, a cyclopentane ring, a cyclohexane ring, a cycloheptane ring, a cyclooctane ring, and a bicyclo[2.2.1]heptane ring.

In the present specification, the "heterocyclic ring" means a nonaromatic heterocyclic ring containing preferably 1 to 5, more preferably 1 to 3 heteroatoms among the atoms constituting the ring. The heterocyclic ring may have a double and/or triple bond in the ring. A carbon atom in the ring may form carbonyl through oxidation, and the ring may

be a monocyclic ring, a condensed ring, or a spiro ring. The number of atoms constituting the ring is preferably 3 to 12 (3- to 12-membered heterocyclic ring), more preferably 4 to 8 (4- to 8-membered heterocyclic ring). Examples of the heterocyclic ring specifically include an azetidine ring, an oxetane ring, a tetrahydrofuran ring, a tetrahydropyran ring, a morpholine ring, a thiomorpholine ring, a pyrrolidine ring, a 4-oxopyrrolidine ring, a piperidine ring, a 4-oxopiperidine ring, a piperazine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolane ring, a dioxane ring, a thietane ring, an octahydroindole ring, and an azocane ring, and rings in which one or more single bonds in any of these saturated heterocyclic rings are replaced with a double bond or a triple bond.

In the present specification, the "saturated heterocyclic ring" means a nonaromatic heterocyclic ring containing a carbon atom as well as 1 to 5 heteroatoms without containing a double bond and/or a triple bond in the ring. The saturated heterocyclic ring may be a monocyclic ring or may form a condensed ring with another ring, for example, an aromatic ring such as a benzene ring. When the saturated heterocyclic ring forms a condensed ring, the saturated heterocyclic ring is preferably a 4- to 7-membered saturated heterocyclic ring. Examples thereof specifically include an azetidine ring, an oxetane ring, a tetrahydrofuran ring, a tetrahydropyran ring, a morpholine ring, a thiomorpholine ring, a pyrrolidine ring, a 4-oxopyrrolidine ring, a piperidine ring, a 4-oxopiperidine ring, a piperazine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolane ring, a dioxane ring, a thietane ring, an octahydroindole ring, an indoline ring, and an azepane ring.

In the present specification, the "amino acid" includes a natural amino acid and a non-natural amino acid. In the present specification, the "natural amino acid" refers to Gly, Ala, Ser, Thr, Val, Leu, Ile, Phe, Tyr, Trp, His, Glu, Asp, Gln, Asn, Cys, Met, Lys, Arg, or Pro. Examples of the non-natural amino acid include, but are not particularly limited to, β-amino acids, γ-amino acids, D-amino acids, N-substituted amino acids, α,α-disubstituted amino acids, amino acids having a side chain different from the natural one, and hydroxycarboxylic acid. In the present specification, the non-natural N-substituted amino acid means a N-substituted amino acid other than Pro. In the present specification, the amino acid accepts an arbitrary conformation. The side chain of the amino acid can be selected without particular limitations and is freely selected from a hydrogen atom as well as, for example, an alkyl group, an alkenyl group, an alkynyl group, an aryl group, a heteroaryl group, an aralkyl group, and a cycloalkyl group. One or two non-adjacent methylene groups in any of these groups may be substituted by an oxygen atom, a carbonyl group (-CO-), or a sulfonyl group ($-SO_2-$). A substituent may be added to each of the groups. Such a substituent is not limited and can be one or two or more substituents each independently freely selected from arbitrary substituents including a halogen atom, an O atom, a S atom, a N atom, a B atom, a Si atom, and a P atom. Specifically, examples thereof include an optionally substituted alkyl group, alkenyl group, alkynyl group, aryl group, heteroaryl group, aralkyl group, and cycloalkyl group. In a non-limiting aspect, the amino acid in the present specification may be a compound having a carboxy group and an amino group in the same molecule (even in this case, the amino acid also includes imino acids such as proline and hydroxyproline).

The backbone amino group of the amino acid may be unsubstituted ($NH_2$ group) or may be substituted (i.e., a -NHR group wherein R represents an alkyl group, an alkenyl group, an alkynyl group, an aryl group, a heteroaryl group, an aralkyl group, or a cycloalkyl group optionally having a substituent, and one or two non-adjacent methylene groups in any of these groups may be substituted by an oxygen atom, a carbonyl group (-CO-), or a sulfonyl group ($-SO_2-$); and a carbon chain bonded to a N atom and a carbon atom at position α may form a ring, as in proline). The substituent of R is selected in the same manner as in the substituent for the amino acid side chain mentioned above. In the case of a substituted backbone amino group, the R is contained in the "side chain of the amino acid" in the present specification. In the present specification, such an amino acid having the substituted backbone amino group is referred to as the "N-substituted amino acid". In the present specification, examples of the "N-substituted amino acid" preferably include, but are not limited to, N-alkyl amino acids, N-Ci to $C_6$ alkyl amino acids, N-Ci to $C_4$ alkyl amino acids, and N-methyl amino acids.

In the present specification, the "amino acid" includes all isotopes corresponding to each amino acid. The isotope of the "amino acid" is a form in which at least one atom is replaced with an atom having the same atomic number (proton number) therewith and a different mass number (total number of protons and neutrons) therefrom. In the present specification, examples of the isotope included in the "amino acid" include a hydrogen atom, a carbon atom, a nitrogen atom, an oxygen atom, a phosphorus atom, a sulfur atom, a fluorine atom, and a chlorine atom which respectively include $^{2}H$, $^{3}H$, $^{13}C$, $^{14}C$, $^{15}N$, $^{17}O$, $^{18}O$, $^{31}P$, $^{32}P$, $^{35}S$, $^{18}F$, and $^{36}Cl$.

In the present specification, examples of the substituent containing a halogen atom include an alkyl group, a cycloalkyl group, an alkenyl group, an alkynyl group, an aryl group, a heteroaryl group, and an aralkyl group having halogen as a substituent and more specifically include a fluoroalkyl group, a difluoroalkyl group, and a trifluoroalkyl group.

In the present specification, examples of the substituent containing an oxygen atom include groups such as a hydroxy group (-OH), an oxy group (-OR), a carbonyl group (-C(=O)-R), a carboxy group ($-CO_2H$), an oxycarbonyl group (-C(=O)-OR), a carbonyloxy group (-O-C(=O)-R), a thiocarbonyl group (-C(=O)-SR), a carbonylthio group (-S-C(=O)-R), an aminocarbonyl group (-C(=O)-NHR), a carbonylamino group (-NH-C(=O)-R), an oxycarbonylamino group (-NH-C(=O)-OR), a sulfonylamino group (-NH-$SO_2$-R), an aminosulfonyl group (-$SO_2$-NHR), a sulfamoylamino group (-NH-$SO_2$-NHR), a thiocarboxy group (-C(=O)-SH), and a carboxycarbonyl group (-C(=O)-$CO_2$H).

Examples of the oxy group (-OR) include an alkoxy group, a cycloalkoxy group, an alkenyloxy group, an alkynyloxy group, an aryloxy group, a heteroaryloxy group, and an aralkyloxy group. The alkoxy group is preferably a $C_1$ to $C_4$ alkoxy group or a $C_1$ or $C_2$ alkoxy group, particularly preferably a methoxy group or an ethoxy group.

Examples of the carbonyl group (-C(=O)-R) include a formyl group (-C(=O)-H), an alkylcarbonyl group, a cycloalkylcarbonyl group, an alkenylcarbonyl group, an alkynylcarbonyl group, an arylcarbonyl group, a heteroarylcarbonyl group, and an aralkylcarbonyl group.

Examples of the oxycarbonyl group (-C(=O)-OR) include an alkyloxycarbonyl group, a cycloalkyloxycarbonyl group, an alkenyloxycarbonyl group, an alkynyloxycarbonyl group, an aryloxycarbonyl group, a heteroaryloxycarbonyl group, and an aralkyloxycarbonyl group.

Examples of the carbonyloxy group (-O-C(=O)-R) include an alkylcarbonyloxy group, a cycloalkylcarbonyloxy group, an alkenylcarbonyloxy group, an alkynylcarbonyloxy group, an arylcarbonyloxy group, a heteroarylcarbonyloxy group, and an aralkylcarbonyloxy group.

Examples of the thiocarbonyl group (-C(=O)-SR) include an alkylthiocarbonyl group, a cycloalkylthiocarbonyl group, an alkenylthiocarbonyl group, an alkynylthiocarbonyl group, an arylthiocarbonyl group, a heteroarylthiocarbonyl group, and an aralkylthiocarbonyl group.

Examples of the carbonylthio group (-S-C(=O)-R) include an alkylcarbonylthio group, a cycloalkylcarbonylthio group, an alkenylcarbonylthio group, an alkynylcarbonylthio group, an arylcarbonylthio group, a heteroarylcarbonylthio group, and an aralkylcarbonylthio group.

Examples of the aminocarbonyl group (-C(=O)-NHR) include an alkylaminocarbonyl group (e.g., a $C_1$ to $C_6$ or $C_1$ to $C_4$ alkylaminocarbonyl group, particularly, an ethylaminocarbonyl group and a methylaminocarbonyl group), a cycloalkylaminocarbonyl group, an alkenylaminocarbonyl group, an alkynylaminocarbonyl group, an arylaminocarbonyl group, a heteroarylaminocarbonyl group, and an aralkylaminocarbonyl group. Examples thereof additionally include compounds in which the H atom bonded to the N atom in -C(=O)-NHR is further replaced with an alkyl group, a cycloalkyl group, an alkenyl group, an alkynyl group, an aryl group, a heteroaryl group, or an aralkyl group.

Examples of the carbonylamino group (-NH-C(=O)-R) include an alkylcarbonylamino group, a cycloalkylcarbonylamino group, an alkenylcarbonylamino group, an alkynylcarbonylamino group, an arylcarbonylamino group, a heteroarylcarbonylamino group, and an aralkylcarbonylamino group. Examples thereof additionally include compounds in which the H atom bonded to the N atom in -NH-C(=O)-R is further replaced with an alkyl group, a cycloalkyl group, an alkenyl group, an alkynyl group, an aryl group, a heteroaryl group, or an aralkyl group.

Examples of the oxycarbonylamino group (-NH-C(=O)-OR) include an alkoxycarbonylamino group, a cycloalkoxycarbonylamino group, an alkenyloxycarbonylamino group, an alkynyloxycarbonylamino group, an aryloxycarbonylamino group, a heteroaryloxycarbonylamino group, and an aralkyloxycarbonylamino group. Examples thereof additionally include compounds in which the H atom bonded to the N atom in -NH-C(=O)-OR is further replaced with an alkyl group, a cycloalkyl group, an alkenyl group, an alkynyl group, an aryl group, a heteroaryl group, or an aralkyl group.

Examples of the sulfonylamino group (-NH-$SO_2$-R) include an alkylsulfonylamino group, a cycloalkylsulfonylamino group, an alkenylsulfonylamino group, an alkynylsulfonylamino group, an arylsulfonylamino group, a heteroarylsulfonylamino group, and an aralkylsulfonylamino group. Examples thereof additionally include compounds in which the H atom bonded to the N atom in -NH-$SO_2$-R is further replaced with an alkyl group, a cycloalkyl group, an alkenyl group, an alkynyl group, an aryl group, a heteroaryl group, or an aralkyl group.

Examples of the aminosulfonyl group (-$SO_2$-NHR) include an alkylaminosulfonyl group, a cycloalkylaminosulfonyl group, an alkenylaminosulfonyl group, an alkynylaminosulfonyl group, an arylaminosulfonyl group, a heteroarylaminosulfonyl group, and an aralkylaminosulfonyl group. Examples thereof additionally include compounds in which the H atom bonded to the N atom in -$SO_2$-NHR is further replaced with an alkyl group, a cycloalkyl group, an alkenyl group, an alkynyl group, an aryl group, a heteroaryl group, or an aralkyl group.

Examples of the sulfamoylamino group (-NH-$SO_2$-NHR) include an alkylsulfamoylamino group, a cycloalkylsulfamoylamino group, an alkenylsulfamoylamino group, an alkynylsulfamoylamino group, an arylsulfamoylamino group, a heteroaryl sulfamoyl amino group, and an aralkylsulfamoylamino group. The two H atoms bonded to the N atoms in -NH-$SO_2$-NHR may be substituted by substituents each independently selected from the group consisting of an alkyl group, a cycloalkyl group, an alkenyl group, an alkynyl group, an aryl group, a heteroaryl group, and an aralkyl group, and these two substituents may form a ring.

Examples of the substituent containing a S atom include groups such as a thiol group (-SH), a thio group (-S-R), a sulfinyl group (-S(=O)-R), a sulfonyl group (-$SO_2$-R), and a sulfo group (-$SO_3$H).

Examples of the thio group (-S-R) that can be selected include an alkylthio group, a cycloalkylthio group, an alkenylthio group, an alkynylthio group, an arylthio group, a heteroarylthio group, and an aralkylthio group.

Examples of the sulfonyl group (-$SO_2$-R) include an alkylsulfonyl group, a cycloalkylsulfonyl group, an alkenylsulfonyl group, an alkynylsulfonyl group, an arylsulfonyl group, a heteroarylsulfonyl group, and an aralkylsulfonyl group.

In the present specification, examples of the substituent containing a nitrogen atom include groups such as an azide group ($-N_3$), a cyano group (-CN), a primary amino group ($-NH_2$), a secondary amino group (-NH-R; also referred to as mono-substituted amino), a tertiary amino group (-NR(R'); also referred to as a di-substituted amino group), an amidino group ($-C(=NH)-NH_2$), a substituted amidino group (-C(=NR)-NR'R"), a guanidino group ($-NH-C(=NH)-NH_2$), a substituted guanidino group (-NR-C(=NR‴)-NR'R"), an aminocarbonylamino group (-NR-CO-NR'R"), a pyridyl group, a piperidino group, a morpholino group, and an azetidinyl group.

Examples of the secondary amino group (-NH-R: mono-substituted amino) include an alkylamino group, a cycloalkylamino group, an alkenylamino group, an alkynylamino group, an arylamino group, a heteroarylamino group, and an aralkylamino group.

Examples of the tertiary amino group (-NR(R'): di-substituted amino group) include an amino group having arbitrary two substituents each independently selected from an alkyl group, a cycloalkyl group, an alkenyl group, an alkynyl group, an aryl group, a heteroaryl group, and an aralkyl group, for example, an alkyl(aralkyl)amino group. These arbitrary two substituents may form a ring. Examples thereof specifically include a dialkylamino group, particularly, a $C_1$-$C_6$ dialkylamino group, a $C_1$-$C_4$ dialkylamino group, a dimethylamino group, and a diethylamino group. In the present specification, the "$C_p$-$C_q$ dialkylamino group" refers to a group in which an amino group is substituted by two $C_p$-$C_q$ alkyl groups. The $C_p$-$C_q$ alkyl groups may be the same or different.

Examples of the substituted amidino group (-C(=NR)-NR'R") include groups in which three substituents R, R', and R" on the N atoms are each independently selected from an alkyl group, a cycloalkyl group, an alkenyl group, an alkynyl group, an aryl group, a heteroaryl group, and an aralkyl group, for example, an alkyl(aralkyl)(aryl)amidino group.

Examples of the substituted guanidino group (-NR-C(=NR‴)-NR'R") include groups in which R, R', R", and R‴ are each independently selected from an alkyl group, a cycloalkyl group, an alkenyl group, an alkynyl group, an aryl group, a heteroaryl group, and an aralkyl group, and groups in which these substituents form a ring.

Examples of the aminocarbonylamino group (-NR-CO-NR'R") include groups in which R, R', and R" are each independently selected from a hydrogen atom, an alkyl group, a cycloalkyl group, an alkenyl group, an alkynyl group, an aryl group, a heteroaryl group, and an aralkyl group, and groups in which these substituents form a ring.

In the present specification, the "peptide residue" and the "amino acid residue" are also simply referred to as a "peptide" and an "amino acid", respectively.

In the present specification, the phrase "site corresponding to" may be used for characterizing an amino acid residue in the amino acid sequence of the polypeptide according to the present embodiment with reference to the amino acid sequence represented by SEQ ID NO: 1. The alignment for determining the corresponding site can be achieved by use of various methods within the scope of techniques in the technical field, for example, publicly available computer software such as BLAST, BLAST-2, ALIGN, Megalign (DNASTAR) software, or GENETYX(R) (Genetyx Corp.). Those skilled in the art can determine appropriate parameters for adopting sequence alignment, including an arbitrary algorithm necessary for achieving the maximum alignment over the full lengths of sequences to be compared.

The polypeptide according to one embodiment comprises a sequence having 90% or higher sequence identity to an amino acid sequence represented by SEQ ID NO: 1 with one amino acid residue thereof engineered, and has higher catalytic activity for the reductive amination reaction between at least one compound A represented by the formula (1) mentioned later or a salt thereof and at least one compound B represented by the formula (2) mentioned later or a salt thereof, or the intramolecular reductive amination reaction of at least one compound B represented by the formula (2) or a salt thereof than that of a polypeptide comprising the amino acid sequence represented by SEQ ID NO: 1 under at least one reaction condition.

The polypeptide according to one embodiment comprises a sequence having 90% or higher sequence identity to an amino acid sequence represented by SEQ ID NO: 1 with two amino acid residues thereof engineered, and has higher catalytic activity for the reductive amination reaction between at least one compound A represented by the formula (1) mentioned later or a salt thereof and at least one compound B represented by the formula (2) mentioned later or a salt thereof, or the intramolecular reductive amination reaction of at least one compound B represented by the formula (2) or a salt thereof than that of a polypeptide comprising the amino acid sequence represented by SEQ ID NO: 1 under at least one reaction condition.

The polypeptide according to one embodiment comprises a sequence having 90% or higher sequence identity to an amino acid sequence represented by SEQ ID NO: 1 with three amino acid residues thereof engineered, and has higher catalytic activity for the reductive amination reaction between at least one compound A represented by the formula (1) mentioned later or a salt thereof and at least one compound B represented by the formula (2) mentioned later or a salt thereof, or the intramolecular reductive amination reaction of at least one compound B represented by the formula (2) or a salt thereof than that of a polypeptide comprising the amino acid sequence represented by SEQ ID NO: 1 under at least one reaction condition.

The polypeptide according to one embodiment can be an enzyme that catalyzes reductive amination reaction. The polypeptide according to the present embodiment can have catalytic activity for the reductive amination reaction between compound A or a salt thereof and compound B or a salt thereof or the intramolecular reductive amination

reaction of compound B or a salt thereof under at least one reaction condition.

In the present specification, the compound A and the salt thereof are also collectively referred to as compound A, and the compound B and the salt thereof are also collectively referred to as compound B.

The polypeptide according to one embodiment has catalytic activity for reductive amination reaction and as such, is suitable for the production of an amino acid.

The polypeptide according to one embodiment has high catalytic activity even in the production of an amino acid against which a conventional enzyme such as the polypeptide consisting of the amino acid sequence represented by SEQ ID NO: 17 that catalyzes reductive amination reaction has low catalytic activity. For example, the polypeptide consisting of the amino acid sequence represented by SEQ ID NO: 17 or the protein PP3591 (hereinafter, referred to as a wild-type enzyme) encoded by the genome of the *P. putida* KT2440 strain described in Non Patent Literature 4 has low catalytic activity against amino acids having a nitrogen atom bonded to an alkyl group (e.g., an ethyl group) having a larger number of carbon atoms than that of a methyl group (hereinafter, also referred to as "N-Et or higher N-substituted amino acids"), and amino acids having a branched structure at position β with respect to the amino group (hereinafter, also referred to as "β-branched amino acids"). The polypeptide according to one embodiment has higher catalytic activity against N-Et or higher N-substituted amino acids and/or β-branched amino acids than that of the wild-type enzymes which are described above. Furthermore, the polypeptide according to the present embodiment can produce the amino acid of interest with high stereoselectivity.

The amino acid sequence represented by SEQ ID NO: 1 is an amino acid sequence known in the art described in Non Patent Literature 4.

The polypeptide according to one embodiment comprises a sequence having exemplarily 90% or higher, preferably 95% or higher, more preferably 98% or higher, and most preferably 100% identity to the amino acid sequence represented by SEQ ID NO: 1 with one amino acid residue thereof engineered.

The polypeptide according to one embodiment comprises a sequence having exemplarily 90% or higher, preferably 95% or higher, more preferably 98% or higher, and most preferably 100% identity to the amino acid sequence represented by SEQ ID NO: 1 with two amino acid residues thereof engineered.

The polypeptide according to one embodiment comprises a sequence having exemplarily 90% or higher, preferably 95% or higher, more preferably 98% or higher, and most preferably 100% identity to the amino acid sequence represented by SEQ ID NO: 1 with three amino acid residues thereof engineered.

The sequence identity of an amino acid sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining the sequence identity of an amino acid sequence can be achieved in various ways that are within the skill in the art, for instance, using publicly available computer software such as BLAST, BLAST-2, ALIGN, Megalign (DNASTAR) software, or GENETYX (registered trademark) (Genetyx Co., Ltd.). Those skilled in the art can determine appropriate parameters for aligning sequences, including any algorithms needed to achieve maximal alignment over the full length of the sequences being compared.

The sequence identity of an amino acid sequence can be determined with algorithm BLAST by Karlin and Altschul (Proc. Natl. Acad. Sci. USA (1993) 90: 5873-7). A program called BLASTN or BLASTX has been developed on the basis of this algorithm (Altschul et al., J. Mol. Biol. (1990) 215: 403-10). In the case of analyzing an amino acid sequence with BLASTX on the basis of BLAST, the parameters are set to Score = 50 and Wordlength = 3. In the case of using BLAST and Gapped BLAST programs, the default parameters of each program are used. For specific approaches of these analysis methods, see information on the website of BLAST (basic local alignment search tool) of NCBI (National Center for Biotechnology Information) known in the art.

The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

In the present specification, the "higher catalytic activity than that of a polypeptide comprising the amino acid sequence represented by SEQ ID NO: 1" means that the catalytic activity is higher than that of the polypeptide under at least one reaction condition. The "higher catalytic activity than that of a polypeptide consisting of the amino acid sequence represented by SEQ ID NO: 17" means that the catalytic activity is higher than that of the polypeptide under at least one reaction condition. In one aspect, the catalytic activity of a polypeptide can be evaluated using reaction conditions described in Examples. For example, the concentration of each compound at the start of reaction can be 50 mM as the total concentration of compound B and a salt thereof, 100 mM as the concentration of D(+)-glucose, 500 mM as the total concentration of compound A and a salt thereof, 100 mM as the concentration of a phosphate buffer solution, 0.89 mM as the concentration of β-NADPH, 0.002 units/μL as the concentration of a GDH solution, and 2.5 μM as the concentration of the polypeptide to be evaluated. This reaction can be performed by adding the polypeptide to be evaluated for its catalytic activity to a premix mentioned later. The premix can be prepared by dissolving the compound B and/or a salt thereof and D(+)-glucose in a mixed solvent of an aqueous solution of the compound A and/or a salt thereof adjusted to pH 7.5 to 8.5 with hydrochloric acid, and a phosphate buffer solution, adjusting pH to 8.0 using an aqueous sodium hydroxide solution, then diluting the resultant with ultrapure water, and adding an aqueous β-NADPH solution (β-NADPH dissolved in ultrapure water and thereby adjusted to 89 mM) and a GDH solution (GDH dissolved in 1 × TNG) to this solution.

In the preferrable embodiment, the catalytic activity can be evaluated by starting the reaction with 50 mM phenylpyruvic acid, 2-oxo-3-(p-tolyl)propanoic acid, or 2-cyclopentyl-2-oxo-acetic acid, 100 mM D(+)-glucose, 500 mM methylamine or ethylamine, a 100 mM phosphate buffer solution, 0.89 mM β-NADPH, a 0.002 units/μL GDH solution, the polypeptide to be evaluated at a concentration of 2.5 μM under conditions of 37°C and pH 8, and determining a yield of an amino acid formed through reductive amination reaction after a lapse of 19 hours.

The catalytic activity is calculated on the basis of the amount of an amino acid (hereinafter, also referred to as a "target product") formed through reductive amination reaction. The amount of the target product formed can be measured using a liquid chromatograph mass spectrometer (LCMS). Specifically, the amount can be measured under conditions described in Examples mentioned later.

The catalytic activity of the polypeptide according to the present invention may be exemplarily 1.2 or more times, preferably 1.5 or more times, more preferably 2 or more times, and most preferably 3 or more times the catalytic activity of the polypeptide comprising the amino acid sequence represented by SEQ ID NO: 1. The catalytic activity of the polypeptide according to the present invention may be exemplarily 1.2 or more times, preferably 1.5 or more times, more preferably 2 or more times, and most preferably 3 or more times the catalytic activity of the polypeptide consisting of the amino acid sequence represented by SEQ ID NO: 17. How many times the catalytic activity of the polypeptide according to the present invention is higher than that of the polypeptide comprising the amino acid sequence represented by SEQ ID NO: 1 or SEQ ID NO: 17 can be determined by calculating the ratio of the yield of the target product between both the polypeptides after a lapse of 19 hours from the start of reaction. Specifically, this can be determined by dividing the yield obtained using the polypeptide according to the present invention by the yield obtained using the polypeptide comprising the amino acid sequence represented by SEQ ID NO: 1 or SEQ ID NO: 17. In the present specification, the start of reaction means a later point in time among points in time when the compound A represented by the formula (1) or a salt thereof and the compound B represented by the formula (2) or a salt thereof, an enzyme disclosed in the present specification, and a reducing agent are added into the system.

The yield after a lapse of 19 hours from the start of reaction is calculated by the following method: a reaction solution and a separately prepared calibration curve sample (compound having the same structure or a structure that exhibits the same UV absorption wavelength as that of the target product) are subjected to LCMS analysis to obtain their respective UV charts or extracted ion chromatograms. A UV peak area or a MS peak area derived from the target product is obtained from the UV chart or the extracted ion chromatogram obtained from the reaction solution. Likewise, a UV peak area or a MS peak area of a preparation is obtained from the calibration curve sample. The concentration of the target product contained in the reaction solution is calculated from the UV peak area or the MS peak area on the basis of the correspondence relationship between the concentration of the preparation contained in the calibration curve sample and the UV peak area or the MS peak area. When the compound B and a salt thereof in the reaction solution is completely converted to the target product, it is considered that the concentration of the target product in the reaction solution is equal to the total concentration of the compound B and a salt thereof in the reaction solution at the start of reaction. In actuality, the yield is calculated by dividing the concentration of the target product contained in the reaction solution by the total concentration of the compound B and a salt thereof in the reaction solution at the start of reaction.

The compound A is a compound represented by the following formula (1):

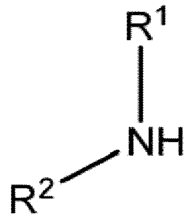

(1)

In the formula (1), $R^1$ and $R^2$ each independently represent a hydrogen atom, an alkyl group, an alkenyl group, an alkynyl group, a cycloalkyl group, an aryl group, a heterocyclyl group, or a heteroaryl group, these groups are optionally substituted, and at least one of $R^1$ and $R^2$ is a hydrogen atom. In the formula (1), $R^1$ may be a hydrogen atom, and $R^2$ may be $C^1$ to $C^6$ alkyl group. The compound A may be amine or an amine analog.

The compound A may be alkylamine of the formula (1) wherein one of $R^1$ and $R^2$ is an alkyl group, and the other moiety is a hydrogen atom. The alkylamine or a salt thereof may be one or more selected from the group consisting of methylamine, ethylamine and salts thereof. The compound A may be ammonia. The compound A may be one or more selected from the group consisting of ammonia, methylamine, ethylamine and salts thereof, most preferably selected from the group consisting of methylamine and ethylamine and salts thereof.

The compound B is a compound represented by the following formula (2):

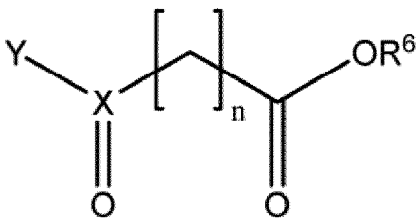

(2)

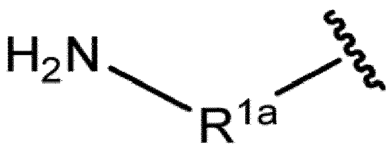

(1')

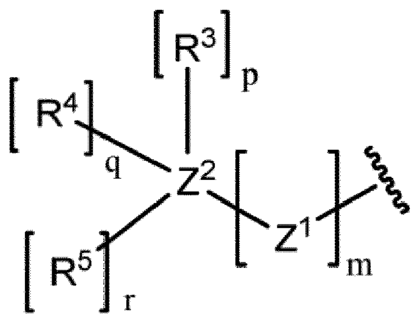

(3)

In the formula (2), X represents a carbon atom, and Y represents a hydrogen atom, a group represented by the formula (1') or a group represented by the formula (3). In the formula (2), Y represents a $C_3$ to $C_8$ cycloalkyl group or a $C_6$ to $C_9$ aralkyl group, and the aralkyl group is optionally substituted by a $C_1$ to $C_3$ alkyl group or a halogen atom.

n represents an integer of between 0 and 2. n may be 0 or 1 and may be 0.

$R^6$ represents a hydrogen atom, an optionally substituted aliphatic hydrocarbon group having between 1 and 6 carbon atoms, an optionally substituted aryl group having between 5 and 12 carbon atoms, an optionally substituted heteroaryl group having between 5 and 12 ring-constituting atoms, a group containing a nitrogen atom, or a group containing an oxygen atom. Specific examples of the group represented by $R^6$ are as mentioned above.

In the formula (1'),

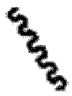

represents a binding point to X.

$R^{1a}$ is a group represented by $R^1$ except for a hydrogen atom in the formula (1), and is not a hydrogen atom.

In the formula (3),

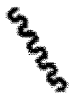

represents a binding point to X.

m represents an integer of between 0 and 6. The lower limit of m is 0 or larger and may be 1 or larger, 2 or larger, 3 or larger, 4 or larger, or 5 or larger. The upper limit of m is 6 or smaller and may be 5 or smaller, 4 or smaller, 3 or smaller, 2 or smaller, or 1 or smaller, p is 0 or 1, q is 0 or 1, and r is 0 or 1.

$Z^1$ represents an optionally substituted alkylene group, or an ether bond-containing group having between 1 and 6 carbon atoms. When m is 2 or larger, a plurality of $Z^1$ are the same or different. Examples of the "ether bond-containing group" include an alkyl group substituted by an alkoxy group, an aryloxy group, or the like. Specific examples of the ether bond-containing group having between 1 and 6 carbon atoms include a methoxymethyl group, an ethoxymethyl group, an allyloxymethyl group, an allyloxyethyl group, and an allyloxypropyl group.

$Z^2$ represents a carbon atom.

$R^3$, $R^4$ and $R^5$ each independently represent a hydrogen atom, an optionally substituted aliphatic hydrocarbon group having between 1 and 6 carbon atoms, an optionally substituted aryl group having between 5 and 12 carbon atoms, an optionally substituted heteroaryl group having between 5 and 12 ring-constituting atoms, a group containing a nitrogen atom, or a group containing an oxygen atom.

Any two or more of $R^3$, $R^4$, and $R^5$ are optionally bonded to each other to form a ring together with $Z^2$, the ring structure is optionally cycloalkyl, aryl, heterocyclyl, or heteroaryl, and these groups are optionally substituted.

$R^3$, $R^4$, and $R^5$ each optionally form a double bond or a triple bond with $Z^2$, and when any one of $R^3$, $R^4$, and $R^5$ is bonded to $Z^2$ through a double bond or a triple bond, at least one of p, q and r is 0.

When one of $R^1$ and $R^2$ in the formula (1) is a methyl group and the other moiety is a hydrogen atom, in the formula (2), Y is a group represented by the formula (3), m is 0, and two or more of $R^3$ to $R^5$ are not a hydrogen atom.

When any two of $R^3$ to $R^5$ are hydrogen atoms, the group other than the hydrogen atoms may be an optionally substituted aliphatic hydrocarbon group having between 1 and 6 carbon atoms, an optionally substituted aryl group having between 5 e and 12 carbon atoms, or an optionally substituted heteroaryl group having between 5 and 12 ring-constituting atoms, may be an optionally substituted aryl group having between 5 and 12 carbon atoms, and may be a phenyl group or a p-tolyl group.

In the formula (1), $R^1$ may be a hydrogen atom, and $R^2$ may be an ethyl group.

In the formula (1), $R^1$ may be a hydrogen atom, and $R^2$ may be a methyl group.

In the formula (1), each of $R^1$ and $R^2$ may be a hydrogen atom.

The compound B may be keto acid or a keto acid analog.

The compound B may be a compound represented by the following formula (2'):

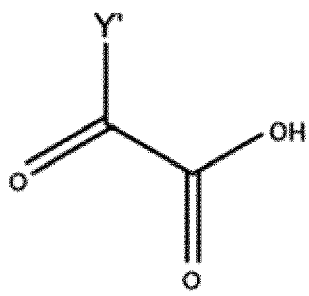

(2')

In the formula (2'), Y' represents a $C_3$ to $C_8$ cycloalkyl group or a $C_6$ to $C_9$ aralkyl group, and the aralkyl group

is optionally substituted by a $C_1$ to $C_3$ alkyl group or a halogen atom.

The compound represented by the formula (2') or a salt thereof may be one or more selected from the group consisting of phenylpyruvic acid, 2-oxo-3-(p-tolyl)propanoic acid, 2-cyclopentyl-2-oxo-acetic acid and salts thereof.

The combination of the compound A and the compound B may be a combination of compound A (ethylamine) of the formula (1) wherein $R^1$ is a hydrogen atom, and $R^2$ is an ethyl group, and compound B (phenylpyruvic acid) of the formula (2) wherein each of n and m is 0, Y is a group represented by the formula (3), $R^3$ is a phenyl group, each of $R^4$, $R^5$ and $R^6$ is a hydrogen atom, and each of p, q and r is 1, may be a combination of compound A (ethylamine) of the formula (1) wherein $R^1$ is a hydrogen atom, and $R^2$ is an ethyl group, and compound B (2-oxo-3-(p-tolyl)propanoic acid) of the formula (2) wherein each of n and m is 0, Y is a group represented by the formula (3), $R^3$ is a p-tolyl group, each of $R^4$, $R^5$ and $R^6$ is a hydrogen atom, and each of p, q and r is 1, and may be a combination of compound A (methylamine) of the formula (1) wherein $R^1$ is a hydrogen atom, and $R^2$ is a methyl group, and compound B (2-cyclopentyl-2-oxo-acetic acid) of the formula (2) wherein each of n and m is 0, Y is a group represented by the formula (3), $R^3$ and $R^4$ are linked to each other to form a cyclopentane ring, each of $R^5$ and $R^6$ is a hydrogen atom, and each of p, q and r is 1.

For the measurement of the catalytic activity, ethylamine as the compound A and phenylpyruvic acid sodium salt as the compound B can be used in combination. In this case, the catalytic activity of the polypeptide can be evaluated with H-EtPhe-OH synthetic activity as an index. In another aspect, for the measurement of the catalytic activity, ethylamine as the compound A and 2-oxo-3-(p-tolyl)propanoic acid sodium salt as the compound B can be used in combination. In this case, the catalytic activity of the polypeptide can be evaluated with H-EtPhe(4-Me)-OH synthetic activity as an index. In another aspect, for the measurement of the catalytic activity, methylamine as the compound A and 2-cyclopentyl-2-oxo-acetic acid as the compound B can be used in combination. In this case, the catalytic activity of the polypeptide can be evaluated with H-MeGly(cPent)-OH synthetic activity as an index.

The polypeptide according to one embodiment of the present invention may be a polypeptide having the engineering of one or more amino acid residues in the amino acid sequence represented by SEQ ID NO: 1, and 1 to 20, 1 to 15, 1 to 10, 1 to 7 or 1 to 5 amino acid residues may be engineered. The number of engineered amino acid residues may be preferably 3 or less, more preferably 2 or less, or most preferably 1.

The number of amino acid residues engineered in the amino acid sequence represented by SEQ ID NO : 1 amino acid sequence represented by SEQ ID NO: 1 is 1 to 5, preferably 1, more preferably 2 or more, most preferably 2 or 3.

The engineering may be one or more selected from the group consisting of substitution, deletion and insertion and can be substitution. The engineering may be conservative engineering. The conservative engineering means the engineering of an amino acid residue so as not to reduce the catalytic activity of interest as compared with the polypeptide before the engineering.

The engineering may be substitution by a natural amino acid different from the amino acid residue before the engineering. The natural amino acid for use in the substitution may be one or more selected from the group consisting of glycine, alanine, serine, threonine, valine, leucine, isoleucine, phenylalanine, tyrosine, tryptophan, histidine, glutamine, asparagine, glutamic acid, aspartic acid, methionine, lysine, arginine, cysteine and proline.

The polypeptide according to one embodiment of the present invention may be a polypeptide comprising a sequence having the engineering of an amino acid residue positioned at a site corresponding to at least one amino acid residue selected from the group consisting of a histidine residue at position 44, a phenylalanine residue at position 117, a methionine residue at position 141, a threonine residue at position 156, a histidine residue at position 182, a glutamine residue at position 186, a tryptophan residue at position 253, and a lysine residue at position 260 in the amino acid sequence represented by SEQ ID NO: 1, may be a polypeptide comprising a sequence having the engineering of an amino acid residue positioned at a site corresponding to at least one amino acid residue selected from the group consisting of a methionine residue at position 141, a histidine residue at position 182, a tryptophan residue at position 253, and a lysine residue at position 260 because the catalytic activity for reductive amination reaction is further improved, and may be a polypeptide comprising a sequence having the engineering of an amino acid residue positioned at a site corresponding to at least one amino acid residue selected from the group consisting of a methionine residue at position 141, a tryptophan residue at position 253, and a lysine residue at position 260.

The polypeptide according to one embodiment of the present invention may be a polypeptide comprising a sequence having the substitution of an amino acid residue positioned at a site corresponding to a histidine residue at position 44 in the amino acid sequence represented by SEQ ID NO: 1 by an amino acid residue other than histidine. The polypeptide may be a polypeptide comprising the amino acid sequence represented by SEQ ID NO: 2. The amino acid residue represented by X in SEQ ID NO: 2 is an alanine residue, an aspartic acid residue, a glutamic acid residue, a phenylalanine residue, a glycine residue, an isoleucine residue, a lysine residue, a leucine residue, a methionine residue, an asparagine residue, a proline residue, a glutamine residue, an arginine residue, a serine residue, a threonine residue, a valine residue, a tryptophan residue, or a tyrosine residue.

The polypeptide may comprise a sequence having the substitution of an amino acid residue positioned at a site corresponding to a histidine residue at position 44 by a methionine residue because the catalytic activity for reductive amination reaction is further improved. Specifically, the polypeptide according to the present embodiment may comprise

an amino acid sequence (H44M) having the substitution of an amino acid residue positioned at a site corresponding to a histidine residue at position 44 in the amino acid sequence represented by SEQ ID NO: 1 by a methionine residue. In this case, the catalytic activity for the reductive amination reaction between ethylamine and 2-oxo-3-(p-tolyl)propanoic acid is more improved.

The polypeptide according to one embodiment of the present invention may be a polypeptide comprising a sequence having the substitution of an amino acid residue positioned at a site corresponding to a phenylalanine residue at position 117 in the amino acid sequence represented by SEQ ID NO: 1 by an amino acid residue other than a phenylalanine residue. The polypeptide may be a polypeptide comprising the amino acid sequence represented by SEQ ID NO: 4. The amino acid residue represented by X in SEQ ID NO: 4 is an alanine residue, an aspartic acid residue, a glutamic acid residue, a glycine residue, a histidine residue, an isoleucine residue, a lysine residue, a leucine residue, a methionine residue, an asparagine residue, a proline residue, a glutamine residue, an arginine residue, a serine residue, a threonine residue, a valine residue, a tryptophan residue, or a tyrosine residue.

The polypeptide may comprise a sequence having the substitution of an amino acid residue positioned at a site corresponding to a phenylalanine residue at position 117 by a leucine residue because the catalytic activity for reductive amination reaction is further improved. Specifically, the polypeptide according to the present embodiment may comprise an amino acid sequence (F1 17L) having the substitution of an amino acid residue positioned at a site corresponding to a phenylalanine residue at position 117 in the amino acid sequence represented by SEQ ID NO: 1 by a leucine residue. In this case, the catalytic activity for the reductive amination reaction between methylamine and 2-cyclopentyl-2-oxo-acetic acid is more improved.

The polypeptide according to one embodiment of the present invention may be a polypeptide comprising a sequence having the substitution of an amino acid residue positioned at a site corresponding to a methionine residue at position 141 in the amino acid sequence represented by SEQ ID NO: 1 by an amino acid residue other than methionine. The polypeptide may be a polypeptide comprising the amino acid sequence represented by SEQ ID NO: 6. The amino acid residue represented by X in SEQ ID NO: 6 is an alanine residue, an aspartic acid residue, a glutamic acid residue, a phenylalanine residue, a glycine residue, a histidine residue, an isoleucine residue, a lysine residue, a leucine residue, an asparagine residue, a proline residue, a glutamine residue, an arginine residue, a serine residue, a threonine residue, a valine residue, a tryptophan residue, or a tyrosine residue.

The polypeptide may comprise a sequence having the substitution of an amino acid residue positioned at a site corresponding to a methionine residue at position 141 by at least one amino acid residue selected from the group consisting of a tyrosine residue, a tryptophan residue, a valine residue, a threonine residue, a serine residue, an arginine residue, a leucine residue, a lysine residue, an isoleucine residue, a histidine residue, a phenylalanine residue and an alanine residue because the catalytic activity for reductive amination reaction is further improved. Specifically, the polypeptide according to the present embodiment may comprise an amino acid sequence having the substitution of an amino acid residue positioned at a site corresponding to a methionine residue at position 141 in the amino acid sequence represented by SEQ ID NO: 1 by a tyrosine residue, a tryptophan residue, a valine residue, a threonine residue, a serine residue, an arginine residue, a leucine residue, a lysine residue, an isoleucine residue, a histidine residue, a phenylalanine residue, or an alanine residue (M141Y, M141W, M141V, M141T, M141S, M141R, M141L, M141K, M141I, M141H, M141F, and M141A). The polypeptide may comprise a sequence having the substitution of an amino acid residue positioned at a site corresponding to a methionine residue at position 141 by at least one amino acid residue selected from the group consisting of a tyrosine residue, a tryptophan residue, a valine residue, a lysine residue, an isoleucine residue, a phenylalanine residue and an alanine residue. Specifically, the polypeptide according to the present embodiment may comprise an amino acid sequence having the substitution of an amino acid residue positioned at a site corresponding to a methionine residue at position 141 in the amino acid sequence represented by SEQ ID NO: 1 by a tyrosine residue, a tryptophan residue, a valine residue, a lysine residue, an isoleucine residue, a histidine residue, a phenylalanine residue, or an alanine residue (M141Y, M141W, M141V, M141K, M141I, M141H, M141F, and M141A).

The polypeptide comprising an amino acid sequence having the substitution of an amino acid residue positioned at a site corresponding to a methionine residue at position 141 in the amino acid sequence represented by SEQ ID NO: 1 by an alanine residue, a lysine residue, a valine residue, a tryptophan residue or a tyrosine residue has more improved catalytic activity for the reductive amination reaction between methylamine and 2-cyclopentyl-2-oxo-acetic acid, the reductive amination reaction between ethylamine and phenylpyruvic acid, and the reductive amination reaction between ethylamine and 2-oxo-3-(p-tolyl)propanoic acid.

The polypeptide comprising an amino acid sequence having the substitution of an amino acid residue positioned at a site corresponding to a methionine residue at position 141 in the amino acid sequence represented by SEQ ID NO: 1 by an arginine residue, a leucine residue or a serine residue has more improved catalytic activity for the reductive amination reaction between methylamine and 2-cyclopentyl-2-oxo-acetic acid, and the reductive amination reaction between ethylamine and phenylpyruvic acid.

The polypeptide comprising an amino acid sequence having the substitution of an amino acid residue positioned at a site corresponding to a methionine residue at position 141 in the amino acid sequence represented by SEQ ID NO:

1 by an isoleucine residue or a threonine residue has more improved catalytic activity for the reductive amination reaction between ethylamine and phenylpyruvic acid, and the reductive amination reaction between ethylamine and 2-oxo-3-(p-tolyl)propanoic acid.

The polypeptide comprising an amino acid sequence having the substitution of an amino acid residue positioned at a site corresponding to a methionine residue at position 141 in the amino acid sequence represented by SEQ ID NO: 1 by a phenylalanine residue or a histidine residue has more improved catalytic activity for the reductive amination reaction between ethylamine and 2-oxo-3-(p-tolyl)propanoic acid.

The polypeptide according to one embodiment of the present invention may be a polypeptide comprising a sequence having the substitution of an amino acid residue positioned at a site corresponding to a threonine residue at position 156 in the amino acid sequence represented by SEQ ID NO: 1 by an amino acid residue other than threonine. The polypeptide may be a polypeptide comprising the amino acid sequence represented by SEQ ID NO: 7. The amino acid residue represented by X in SEQ ID NO: 7 is an alanine residue, an aspartic acid residue, a glutamic acid residue, a phenylalanine residue, a glycine residue, a histidine residue, an isoleucine residue, a lysine residue, a leucine residue, an asparagine residue, a proline residue, a glutamine residue, an arginine residue, a serine residue, a threonine residue, a valine residue, a tryptophan residue, or a tyrosine residue.

The polypeptide can comprise a sequence having the substitution of an amino acid residue positioned at a site corresponding to a threonine residue at position 156 by a serine residue because the catalytic activity for reductive amination reaction is further improved. Specifically, the polypeptide according to the present embodiment can comprise an amino acid sequence having the substitution of an amino acid residue positioned at a site corresponding to a threonine residue at position 156 in the amino acid sequence represented by SEQ ID NO: 1 by a serine residue (T156S). In this case, the catalytic activity for the reductive amination reaction between ethylamine and 2-oxo-3-(p-tolyl)propanoic acid is more improved.

The polypeptide according to one embodiment of the present invention may be a polypeptide comprising a sequence having the substitution of an amino acid residue positioned at a site corresponding to a histidine residue at position 182 in the amino acid sequence represented by SEQ ID NO: 1 by an amino acid residue other than histidine. The polypeptide may be a polypeptide comprising the amino acid sequence represented by SEQ ID NO: 8. The amino acid residue represented by X in SEQ ID NO: 8 is an alanine residue, an aspartic acid residue, a glutamic acid residue, a phenylalanine residue, a glycine residue, an isoleucine residue, a lysine residue, a leucine residue, a methionine residue, an asparagine residue, a proline residue, a glutamine residue, an arginine residue, a serine residue, a threonine residue, a valine residue, a tryptophan residue, or a tyrosine residue.

The polypeptide may comprise a sequence having the substitution of an amino acid residue positioned at a site corresponding to a histidine residue at position 182 by at least one amino acid residue selected from the group consisting of a tyrosine residue, a glutamine residue, a methionine residue, a leucine residue, a glycine residue, a phenylalanine residue and an alanine residue because the catalytic activity for reductive amination reaction is further improved. Specifically, the polypeptide according to the present embodiment may comprise an amino acid sequence having the substitution of an amino acid residue positioned at a site corresponding to a histidine residue at position 182 in the amino acid sequence represented by SEQ ID NO: 1 by a tyrosine residue, a glutamine residue, a methionine residue, a leucine residue, a glycine residue, a phenylalanine residue, or an alanine residue (H182Y, H182Q, H182M, H182L, H182G, H182F, and H182A). The polypeptide may comprise a sequence having the substitution of an amino acid residue positioned at a site corresponding to a histidine residue at position 182 by at least one amino acid residue selected from the group consisting of a methionine residue, a leucine residue, and a phenylalanine residue. Specifically, the polypeptide according to the present embodiment may comprise an amino acid sequence having the substitution of an amino acid residue positioned at a site corresponding to a histidine residue at position 182 in the amino acid sequence represented by SEQ ID NO: 1 by a methionine residue, a leucine residue, or a phenylalanine residue (H182M, H182L, and H182F).

The polypeptide comprising an amino acid sequence having the substitution of an amino acid residue positioned at a site corresponding to a histidine residue at position 182 in the amino acid sequence represented by SEQ ID NO: 1 by an alanine residue, a phenylalanine residue, a leucine residue, a methionine residue or a tyrosine residue has more improved catalytic activity for the reductive amination reaction between ethylamine and phenylpyruvic acid and the reductive amination reaction between ethylamine and 2-oxo-3-(p-tolyl)propanoic acid.

The polypeptide comprising an amino acid sequence having the substitution of an amino acid residue positioned at a site corresponding to a histidine residue at position 182 in the amino acid sequence represented by SEQ ID NO: 1 by a glutamine residue has more improved catalytic activity for the reductive amination reaction between methylamine and 2-cyclopentyl-2-oxo-acetic acid.

The polypeptide comprising an amino acid sequence having the substitution of an amino acid residue positioned at a site corresponding to a histidine residue at position 182 in the amino acid sequence represented by SEQ ID NO: 1 by a glycine residue has more improved catalytic activity for the reductive amination reaction between ethylamine and phenylpyruvic acid.

The polypeptide according to one embodiment of the present invention may be a polypeptide comprising a sequence having the substitution of an amino acid residue positioned at a site corresponding to a glutamine residue at position 186 in the amino acid sequence represented by SEQ ID NO: 1 by an amino acid residue other than a glutamine residue. The polypeptide may be a polypeptide comprising the amino acid sequence represented by SEQ ID NO: 10. The amino acid residue represented by X in SEQ ID NO: 10 is an alanine residue, an aspartic acid residue, a glutamic acid residue, a phenylalanine residue, a glycine residue, a histidine residue, an isoleucine residue, a lysine residue, a leucine residue, a methionine residue, an asparagine residue, a proline residue, an arginine residue, a serine residue, a threonine residue, a valine residue, a tryptophan residue, or a tyrosine residue.

The polypeptide may comprise a sequence having the substitution of an amino acid residue positioned at a site corresponding to a glutamine residue at position 186 in the amino acid sequence represented by SEQ ID NO: 1 by at least one amino acid residue selected from the group consisting of a methionine residue and a glutamic acid residue because the catalytic activity for reductive amination reaction is further improved. Specifically, the polypeptide according to the present embodiment may comprise an amino acid sequence having the substitution of an amino acid residue positioned at a site corresponding to a glutamine residue at position 186 in the amino acid sequence represented by SEQ ID NO: 1 by a methionine residue or a glutamic acid residue (Q186M, and Q186E).

The polypeptide comprising an amino acid sequence having the substitution of an amino acid residue positioned at a site corresponding to a glutamine residue at position 186 in the amino acid sequence represented by SEQ ID NO: 1 by a methionine residue has more improved catalytic activity for the reductive amination reaction between ethylamine and phenylpyruvic acid.

The polypeptide comprising an amino acid sequence having the substitution of an amino acid residue positioned at a site corresponding to a glutamine residue at position 186 in the amino acid sequence represented by SEQ ID NO: 1 by a glutamic acid residue has more improved catalytic activity for the reductive amination reaction between ethylamine and 2-oxo-3-(p-tolyl)propanoic acid and the reductive amination reaction between ethylamine and phenylpyruvic acid.

The polypeptide according to one embodiment of the present invention may be a polypeptide comprising a sequence having the substitution of an amino acid residue positioned at a site corresponding to a tryptophan residue at position 253 in the amino acid sequence represented by SEQ ID NO: 1 by an amino acid residue other than a tryptophan residue. The polypeptide may be a polypeptide comprising the amino acid sequence represented by SEQ ID NO: 11. The amino acid residue represented by X in SEQ ID NO: 11 is an alanine residue, an aspartic acid residue, a glutamic acid residue, a phenylalanine residue, a glycine residue, a histidine residue, an isoleucine residue, a lysine residue, a leucine residue, a methionine residue, an asparagine residue, a proline residue, a glutamine residue, an arginine residue, a serine residue, a threonine residue, a valine residue, or a tyrosine residue.

The polypeptide may comprise a sequence having the substitution of an amino acid residue positioned at a site corresponding to a tryptophan residue at position 253 by at least one amino acid residue selected from the group consisting of a tyrosine residue, a valine residue, a threonine residue, a serine residue, an arginine residue, a glutamine residue, a proline residue, an asparagine residue, a methionine residue, a leucine residue, a lysine residue, an isoleucine residue, a histidine residue, a phenylalanine residue and an alanine residue because the catalytic activity for reductive amination reaction is further improved. Specifically, the polypeptide according to the present embodiment can comprise an amino acid sequence having the substitution of an amino acid residue positioned at a site corresponding to a tryptophan residue at position 253 in the amino acid sequence represented by SEQ ID NO: 1 by a tyrosine residue, a valine residue, a threonine residue, a serine residue, an arginine residue, a glutamine residue, a proline residue, an asparagine residue, a methionine residue, a leucine residue, a lysine residue, an isoleucine residue, a histidine residue, a phenylalanine residue, or an alanine residue (W253Y, W253V, W253T, W253S, W253R, W253Q, W253P, W253N, W253M, W253L, W253K, W253I, W253H, W253F, and W253A). The polypeptide may comprise a sequence having the substitution of an amino acid residue positioned at a site corresponding to a tryptophan residue at position 253 by at least one amino acid residue selected from the group consisting of a leucine residue, an isoleucine residue and a histidine residue, and preferably by a histidine residue. Specifically, the polypeptide according to the present embodiment may comprise an amino acid sequence having the substitution of an amino acid residue positioned at a site corresponding to a tryptophan residue at position 253 in the amino acid sequence represented by SEQ ID NO: 1 by a leucine residue, an isoleucine residue or a histidine residue (W253L, W253I, and W253H) preferably comprise an amino acid sequence having the substitution of an amino acid residue positioned at a site corresponding to a tryptophan residue at position 253 in the amino acid sequence represented by SEQ ID NO: 1 by a histidine residue (W253H).

The polypeptide comprising an amino acid sequence having the substitution of an amino acid residue positioned at a site corresponding to a tryptophan residue at position 253 in the amino acid sequence represented by SEQ ID NO: 1 by a histidine residue has more improved catalytic activity for the reductive amination reaction between methylamine and 2-cyclopentyl-2-oxo-acetic acid, the reductive amination reaction between ethylamine and phenylpyruvic acid, and the reductive amination reaction between ethylamine and 2-oxo-3-(p-tolyl)propanoic acid.

The polypeptide comprising an amino acid sequence having the substitution of an amino acid residue positioned at a site corresponding to a tryptophan residue at position 253 in the amino acid sequence represented by SEQ ID NO:

1 by an alanine residue, a phenylalanine residue, an isoleucine residue, a leucine residue, a methionine residue, a proline residue, a glutamine residue, a threonine residue, a valine residue, or a tyrosine residue has more improved catalytic activity for the reductive amination reaction between methylamine and 2-cyclopentyl-2-oxo-acetic acid and the reductive amination reaction between ethylamine and phenylpyruvic acid.

The polypeptide comprising an amino acid sequence having the substitution of an amino acid residue positioned at a site corresponding to a tryptophan residue at position 253 in the amino acid sequence represented by SEQ ID NO: 1 by a lysine residue, an arginine residue, or a serine residue has more improved catalytic activity for the reductive amination reaction between methylamine and 2-cyclopentyl-2-oxo-acetic acid.

The polypeptide comprising an amino acid sequence having the substitution of an amino acid residue positioned at a site corresponding to a tryptophan residue at position 253 in the amino acid sequence represented by SEQ ID NO: 1 by an asparagine residue has more improved catalytic activity for the reductive amination reaction between ethylamine and phenylpyruvic acid.

The polypeptide according to one embodiment of the present invention may be a polypeptide comprising a sequence having the substitution of an amino acid residue positioned at a site corresponding to a lysine residue at position 260 in the amino acid sequence represented by SEQ ID NO: 1 by an amino acid residue other than a lysine residue. The polypeptide may be a polypeptide comprising the amino acid sequence represented by SEQ ID NO: 12. The amino acid residue represented by X in SEQ ID NO: 12 is an alanine residue, an aspartic acid residue, a glutamic acid residue, a phenylalanine residue, a glycine residue, a histidine residue, an isoleucine residue, a leucine residue, a methionine residue, an asparagine residue, a proline residue, a glutamine residue, an arginine residue, a serine residue, a threonine residue, a valine residue, a tryptophan residue, or a tyrosine residue.

The polypeptide may comprise a sequence having the substitution of an amino acid residue positioned at a site corresponding to a lysine residue at position 260 by at least one amino acid residue selected from the group consisting of a tyrosine residue, a tryptophan residue, a threonine residue, a serine residue, an arginine residue, a glutamine residue, an asparagine residue, a methionine residue, a leucine residue, a histidine residue, a glycine residue, a phenylalanine residue, a glutamic acid residue and an alanine residue because the catalytic activity for reductive amination reaction is further improved. Specifically, the polypeptide according to the present embodiment may comprise an amino acid sequence having the substitution of an amino acid residue positioned at a site corresponding to a lysine residue at position 260 in the amino acid sequence represented by SEQ ID NO: 1 by a tyrosine residue, a tryptophan residue, a threonine residue, a serine residue, an arginine residue, a glutamine residue, a asparagine residue, a methionine residue, a leucine residue, a histidine residue, a glycine residue, a phenylalanine residue, a glutamic acid residue or an alanine residue (K260Y, K260W, K260T, K260S, K260R, K260Q, K260N, K260M, K260L, K260H, K260G, K260F, K260E, and K260A). The polypeptide may further comprise a sequence having the substitution of an amino acid residue positioned at a site corresponding to a lysine residue at position 260 by at least one amino acid residue selected from the group consisting of a glutamine residue, a methionine residue, a glutamic acid residue and an asparagine residue. Specifically, the polypeptide according to the present embodiment may comprise an amino acid sequence having the substitution of an amino acid residue positioned at a site corresponding to a lysine residue at position 260 in the amino acid sequence represented by SEQ ID NO: 1 by a glutamine residue, a methionine residue, a glutamic acid residue or an asparagine residue (K260Q, K260M, K260E, and K260N).

The polypeptide comprising an amino acid sequence having the substitution of an amino acid residue positioned at a site corresponding to a lysine residue at position 260 in the amino acid sequence represented by SEQ ID NO: 1 by a glutamic acid residue or a serine residue has more improved catalytic activity for the reductive amination reaction between methylamine and 2-cyclopentyl-2-oxo-acetic acid, the reductive amination reaction between ethylamine and phenylpyruvic acid, and the reductive amination reaction between ethylamine and 2-oxo-3-(p-tolyl)propanoic acid.

The polypeptide comprising an amino acid sequence having the substitution of an amino acid residue positioned at a site corresponding to a lysine residue at position 260 in the amino acid sequence represented by SEQ ID NO: 1 by a phenylalanine residue, a leucine residue, a methionine residue, a glutamine residue, a threonine residue, or a tyrosine residue has more improved catalytic activity for the reductive amination reaction between ethylamine and phenylpyruvic acid and the reductive amination reaction between ethylamine and 2-oxo-3-(p-tolyl)propanoic acid.

The polypeptide comprising an amino acid sequence having the substitution of an amino acid residue positioned at a site corresponding to a lysine residue at position 260 in the amino acid sequence represented by SEQ ID NO: 1 by an alanine residue, a glycine residue, a histidine residue, an asparagine residue, an arginine residue, or a tryptophan residue has more improved catalytic activity for the reductive amination reaction between ethylamine and phenylpyruvic acid.

The polypeptide according to the present embodiment may comprise amino acid sequence a1 represented by SEQ ID NO: 6 wherein the amino acid residue represented by X is a valine residue (mutation: M141V), amino acid sequence a2 represented by SEQ ID NO: 6 wherein the amino acid residue represented by X is a tyrosine residue (mutation: M141Y), amino acid sequence a3 represented by SEQ ID NO: 8 wherein the amino acid residue represented by X is a leucine residue (mutation: H182L), amino acid sequence a4 represented by SEQ ID NO: 11 wherein the amino

acid residue represented by X is a histidine residue (mutation: W253H), or amino acid sequence a5 represented by SEQ ID NO: 12 wherein the amino acid residue represented by X is a glutamic acid residue (mutation: K260E).

The polypeptide according to the present embodiment may comprise a sequence having exemplarily 90% or higher sequence identity to the amino acid sequence a1, a2, a3, a4, or a5. The sequence identity may be preferably 95% or higher, more preferably 98% or higher, and most preferably 100%.

The polypeptide according to the present embodiment may comprise a sequence having exemplarily 90% or higher sequence identity to the amino acid sequence a4. The sequence identity may be preferably 95% or higher, more preferably 98% or higher, and most preferably 100%.

The polypeptide according to the present embodiment may be a polypeptide comprising an amino acid sequence a1, a2, a3, a4, or a5 with one or more amino acid residue thereof engineered, and 1 to 20, 1 to 15, 1 to 10, 1 to 7 or 1 to 5 amino acid residues may be engineered. The number of engineered amino acid residues may be preferably 3 or less, more preferably 2 or less, and most preferably 1.

The polypeptide according to the present embodiment may be a polypeptide comprising an amino acid sequence a4 with one or more amino acid residue thereof engineered, and 1 to 20, 1 to 15, 1 to 10, 1 to 7 or 1 to 5 amino acid residues may be engineered. The number of engineered amino acid residues may be preferably 3 or less, more preferably 2 or less, and most preferably 1.

In the polypeptide according to the present embodiment, the amino acid sequence represented by SEQ ID NO: 1 may have two or more engineering sites, or two engineering sites. Hereinafter, a preferred embodiment of the polypeptide having two or more modified portions will be described.

The polypeptide may preferably comprise a sequence comprising the engineering of an amino acid residue positioned at a site corresponding to at least one amino acid residue selected from the group consisting of a methionine residue at position 141, a histidine residue at position 182, and a tryptophan residue at position 253 in the amino acid sequence represented by SEQ ID NO: 1 as a first engineering site, and the engineering of an amino acid residue positioned at a site corresponding to at least one amino acid residue selected from the group consisting of a histidine residue at position 182, a tryptophan residue at position 253, and a lysine residue at position 260 in the amino acid sequence represented by SEQ ID NO: 1 as a second engineering site, wherein the amino acid reside engineered at the second engineering site is positioned at a site corresponding to one amino acid residue different from the amino acid residue engineered at the first engineering site.

The polypeptide may preferably comprise a sequence having the engineering of an amino acid residue positioned at a site corresponding to a methionine residue at position 141 and a histidine residue at position 182, the engineering of an amino acid residue positioned at a site corresponding to a methionine residue at position 141 and a tryptophan residue at position 253, the engineering of an amino acid residue positioned at a site corresponding to a methionine residue at position 141 and a lysine residue at position 260, the engineering of an amino acid residue positioned at a site corresponding to a histidine residue at position 182 and a tryptophan residue at position 253, the engineering of an amino acid residue positioned at a site corresponding to a histidine residue at position 182 and a lysine residue at position 260, or the engineering of an amino acid residue positioned at a site corresponding to a tryptophan residue at position 253 and a lysine residue at position 260 in the amino acid sequence represented by SEQ ID NO: 1. The polypeptide may more preferably comprise a sequence having the engineering of an amino acid residue positioned at a site corresponding to a methionine residue at position 141 and a lysine residue at position 260, the engineering of an amino acid residue positioned at a site corresponding to a histidine residue at position 182 and a lysine residue at position 260, or the engineering of an amino acid residue positioned at a site corresponding to a tryptophan residue at position 253 and a lysine residue at position 260 in the amino acid sequence represented by SEQ ID NO: 1.

The polypeptide may preferably comprise a sequence having the substitution of an amino acid residue positioned at a site corresponding to a methionine residue at position 141 in the amino acid sequence represented by SEQ ID NO: 1 by one amino acid residue selected from the group consisting of a tyrosine residue, a tryptophan residue, a valine residue, a threonine residue, an arginine residue, a lysine residue, an isoleucine residue and an alanine residue, or more preferably comprise a sequence having the substitution of an amino acid residue positioned at a site corresponding to a methionine residue at position 141 in the amino acid sequence represented by SEQ ID NO: 1 by one amino acid residue selected from the group consisting of a tyrosine residue, a valine residue and an alanine residue.

The polypeptide may preferably comprise a sequence having the substitution of an amino acid residue positioned at a site corresponding to a histidine residue at position 182 in the amino acid sequence represented by SEQ ID NO: 1 by one amino acid residue selected from the group consisting of a tyrosine residue, a methionine residue, a leucine residue and a phenylalanine residue, or more preferably comprise a sequence having the substitution of an amino acid residue positioned at a site corresponding to a histidine residue at position 182 in the amino acid sequence represented by SEQ ID NO: 1 by one amino acid residue selected from the group consisting of a methionine residue and a leucine residue.

The polypeptide may preferably comprise a sequence having the substitution of an amino acid residue positioned

at a site corresponding to a tryptophan residue at position 253 in the amino acid sequence represented by SEQ ID NO: 1 by one amino acid residue selected from the group consisting of a tyrosine residue, a threonine residue, a serine residue, an asparagine residue, a methionine residue, a histidine residue and an alanine residue, or more preferably comprise a sequence having the substitution of an amino acid residue positioned at a site corresponding to a tryptophan residue at position 253 in the amino acid sequence represented by SEQ ID NO: 1 by a histidine residue.

The polypeptide may preferably comprise a sequence having the substitution of an amino acid residue positioned at a site corresponding to a lysine residue at position 260 in the amino acid sequence represented by SEQ ID NO: 1 by one amino acid residue selected from the group consisting of a serine residue, a glutamine residue, an asparagine residue, a leucine residue, an isoleucine residue, a histidine residue, a glycine residue, a phenylalanine residue, a glutamic acid residue and an alanine residue, or more preferably comprise a sequence having the substitution of an amino acid residue positioned at a site corresponding to a lysine residue at position 260 in the amino acid sequence represented by SEQ ID NO: 1 by one amino acid residue selected from the group consisting of a glutamine residue and a glutamic acid residue.

Hereinafter, in the amino acid sequence represented by SEQ ID NO: 1, a sequence in which an amino acid residue located at a site corresponding to an amino acid residue $X^1$ at position $m^1$ is substituted by another amino acid residue $x^1$ other than $X^1$, and an amino acid residue located at a site corresponding to amino acid residue $X^2$ at a site $m^2$ is substituted by another amino acid residue $x^2$ other than $X^2$ is referred to as $X^1m^1x^1_X^2m^2x^2$. For example, the amino acid sequence represented by SEQ ID NO: 23 is expressed as M141X_H182X because each of the amino acid residues located at positions corresponding to amino acid residues 141 and 182 is substituted by another amino acid residue X. Specifically, an amino acid sequence represented by SEQ ID NO: 23 in which X located at the site corresponding to the amino acid residue at position 141 is a tyrosine residue and X located at the site corresponding to the amino acid residue at position 182 is a methionine residue is denoted as M141Y_H182M. Amino acid sequences represented by SEQ ID NOs: 24 to 30 are expressed in the same manner.

The amino acid sequence represented by SEQ ID NO : 1 with two amino acid residues engineered thereof may be, for example, an amino acid sequence selected from the group a1 to group a4 consisting of the following amino acid sequences, may be preferably an amino acid sequence selected from the group a1 to group a3, may be more preferably an amino acid sequence selected from the group a1 and group a2, and may be most preferably an amino acid sequence selected from group a1.

Group a1:
M141Y_K260E, H182L_K260Q, M141A_K260E, H182M_K260Q, M141A_K260Q, H182M_K260E, M141V_K260E, W253H _K260E, and H182L_K260E.
Group a2:
M141V_K260Q, W253H_K260Q, W253M _K260E, M141Y_H182M, M141Y _K260Q, H182F _K260Q, M141V_K260F, M141V _H182L, M141V_K260L, M141Y_K260G, H182L_W253H, H182F_K260E, and M141T_K260Q
Group a3:
M141Y_H182L, W253M_K260Q, M141V_W253M, M141V_K260S, M141V _K260A, M141A_K260F, M141A_K260H, W253A _K260E, M141V_W253A, M141V_W253H, M141K_K260L, M141V_K260G, and M141A_K260L.
Group a4:
M141W_K260A, M141V_W253Y, M141K_K260G, M141A_K260G, M141A_K260S, M141A_K260N, M141Y_W253H, M141A_K260A, M141K_K260S, W253A _K260Q, M141V_H182M, M141A _K260I, M141T_K260G, W253M _K260L, M141I_W253A, W253T_K260L, M141R_K260G, W253A _K260L, M141K_W253S, M141A _H182L, W253N_K260L, M141K_K260A, M141K_W253M, H182Y_W253A, H182M_W253H, M141R_K260A, H182M _W253A and M141K _W253A.

The polypeptide may comprise, for example, a sequence having 90% or more sequence identity to an amino acid sequence selected from the group consisting of group a1 to group a4, preferably comprise a sequence having 90% or more sequence identity to an amino acid sequence selected from the group consisting of group a1 to group a3, more preferably comprises a sequence having 90% or more sequence identity to an amino acid sequence selected from the group consisting of group a1 and group a2, and most preferably comprises a sequence having 90% or more sequence identity to an amino acid sequence selected from group a1.

The polypeptide may comprise an amino acid sequence selected from the group consisting of groups a1 to a4, preferably comprises an amino acid sequence selected from the group consisting of groups a1 to a3, more preferably comprises an amino acid sequence selected from the group consisting of groups a1 and a2, and most preferably comprises an amino acid sequence selected from group a1.

The polypeptide may have three or more engineering sites or three engineering sites in the amino acid sequence

represented by SEQ ID NO: 1. Hereinafter, a preferred embodiment of the polypeptide having three or more engineering sites is described.

The polypeptide may preferable comprise a sequence comprising the engineering of an amino acid residue positioned at a site corresponding to at least one amino acid residue selected from the group consisting of a methionine residue at position 141, and a histidine residue at position 182 in the amino acid sequence represented by SEQ ID NO: 1 as a first engineering site, the engineering of an amino acid residue positioned at a site corresponding to at least one amino acid residue selected from the group consisting of a histidine residue at position 182, and a tryptophan residue at position 253 in the amino acid sequence represented by SEQ ID NO: 1 as a second engineering site, wherein the amino acid reside engineered at the second engineering site is positioned at a site corresponding to one amino acid residue different from the amino acid residue engineered at the first engineering site, and the engineering of an amino acid residue positioned at a site corresponding to at least one amino acid residue selected from the group consisting of a tryptophan residue at position 253, and a lysine residue at position 260 in the amino acid sequence represented by SEQ ID NO: 1 as a third engineering site, wherein the amino acid reside engineered at the third engineering site is positioned at a site corresponding to one amino acid residue different from the amino acid residue engineered at the first engineering site and the second engineering site.

The polypeptide may preferably comprise a sequence having the engineering of an amino acid residue positioned at a site corresponding to a methionine residue at position 141, a histidine residue at position 182, and a tryptophan residue at position 253, the engineering of an amino acid residue positioned at a site corresponding to a methionine residue at position 141, a histidine residue at position 182 and a lysine residue at position 260, the engineering of an amino acid residue positioned at a site corresponding to a methionine residue at position 141, a tryptophan residue at position 253 and a lysine residue at position 260, or the engineering of an amino acid residue positioned at a site corresponding to a histidine residue at position 182, a tryptophan residue at position 253 and a lysine residue at position 260 in the amino acid sequence represented by SEQ ID NO: 1. The polypeptide may more preferably comprise a sequence having the engineering of an amino acid residue positioned at a site corresponding to a methionine residue at position 141, a histidine residue at position 182 and a lysine residue at position 260, the engineering of an amino acid residue positioned at a site corresponding to a methionine residue at position 141, a tryptophan residue at position 253 and a lysine residue at position 260, or the engineering of an amino acid residue positioned at a site corresponding to a histidine residue at position 182, a tryptophan residue at position 253 and a lysine residue at position 260 in the amino acid sequence represented by SEQ ID NO: 1.

The polypeptide may preferably have three engineering sites in the amino acid sequence represented by SEQ ID NO: 1 and comprise a sequence having the substitution of an amino acid residue positioned at a site corresponding to a methionine residue at position 141 in the amino acid sequence represented by SEQ ID NO: 1 by one amino acid residue selected from the group consisting of a tyrosine residue, a valine residue, a threonine residue, a serine residue, a leucine residue, a lysine residue, an isoleucine residue and an alanine residue. The polypeptide may more preferably have three engineering sites in the amino acid sequence represented by SEQ ID NO: 1 and comprise sequence having the substitution of an amino acid residue positioned at a site corresponding to a methionine residue at position 141 in the amino acid sequence represented by SEQ ID NO: 1 by one amino acid residue selected from the group consisting of a tyrosine residue, a valine residue and an alanine residue.

The polypeptide may preferably have three engineering sites in the amino acid sequence represented by SEQ ID NO: 1 and comprise a sequence having the substitution of an amino acid residue positioned at a site corresponding to a histidine residue at position 182 in the amino acid sequence represented by SEQ ID NO: 1 by one amino acid residue selected from the group consisting of a methionine residue, a leucine residue and a phenylalanine residue. The polypeptide may more preferably have three engineering sites in the amino acid sequence represented by SEQ ID NO: 1 and comprise a sequence having the substitution of an amino acid residue positioned at a site corresponding to a histidine residue at position 182 in the amino acid sequence represented by SEQ ID NO: 1 by one amino acid residue selected from the group consisting of a methionine residue and a leucine residue.

The polypeptide may preferably have three engineering sites in the amino acid sequence represented by SEQ ID NO: 1 and comprise a sequence having the substitution of an amino acid residue positioned at a site corresponding to a tryptophan residue at position 253 in the amino acid sequence represented by SEQ ID NO: 1 by one amino acid residue selected from the group consisting of a valine residue, a threonine residue, a serine residue, a glutamine residue, a methionine residue, a leucine residue, a histidine residue, a phenylalanine residue and a glutamic acid residue. The polypeptide may more preferably have three engineering sites in the amino acid sequence represented by SEQ ID NO: 1 and comprise a sequence having the substitution of an amino acid residue positioned at a site corresponding to a tryptophan residue at position 253 in the amino acid sequence represented by SEQ ID NO: 1 by one amino acid residue selected from the group consisting of a glutamine residue, a methionine residue, a leucine residue and a histidine residue.

The polypeptide may preferably have three engineering sites in the amino acid sequence represented by SEQ ID NO: 1 and comprise a sequence having the substitution of an amino acid residue positioned at a site corresponding to a lysine residue at position 260 in the amino acid sequence represented by SEQ ID NO: 1 by one amino acid residue

selected from the group consisting of a glutamine residue and a glutamic acid residue.

Hereinafter, in the amino acid sequence represented by SEQ ID NO: 1, an amino acid sequence at a site corresponding to an amino acid residue $X^1$ at position $m^1$ is substituted by another amino acid residue $x^1$ other than $X^1$, an amino acid residue at a site corresponding to an amino acid residue $X^2$ at position $m^2$ is substituted by another amino acid residue $x^2$ other than $X^2$, and an amino acid residue at a site corresponding to an amino acid residue $X^3$ at position $m^3$ is substituted by another amino acid residue $x^3$ other than $X^3$ is referred to as $X^1m^1x^1_X^2m^2x^2_X^3m^3x^3$. For example, the amino acid sequence represented by SEQ ID NO: 31 is expressed as M141X_W253X_K260X because each amino acid residue located at sites corresponding to amino acid residues 141, 253 and 260 is substituted by another amino acid residue X. Specifically, an amino acid sequence represented by SEQ ID NO: 31, in which X located at the site corresponding to the amino acid residue at position 141 is a valine residue, X located at the site corresponding to the amino acid residue at position 253 is a methionine residue, and X located at the site corresponding to the amino acid residue at position 260 is a glutamic acid residue, is denoted as M141V_W253M_K260E. Amino acid sequences represented by SEQ ID NOs: 32 to 35 are expressed in the same manner.

The amino acid sequence by SEQ ID NO: 1 with three amino acid residues engineered thereof may be, for example, an amino acid sequence selected from the group b1 to group b4 consisting of the following amino acid sequences, may be preferably an amino acid sequence selected from the group b1 to group b3, may be more preferably an amino acid sequence selected from the group b1 and group b2, and may be most preferably an amino acid sequence selected from group b 1.

Group b1:
M141Y_H182M_K260E, M141V_W253M_K260E, H182L_W253H_K260E, M141Y_H182M_K260Q, M141V_H182M_K260E, M141V_H182L_K260Q, M141Y_H182L_K260Q, M141V_W253Q _K260E, H182L_W253Q_K260E, M141I_W253H_K260E, M141V_H182L_K260E, M141Y_H182L_K260E, H182L W253M K260E, M141V_W253Q_K260Q, M141V _W253H_K260Q, M141V_W253L_K260Q, M141I_W253H_K260Q, and M141V_W253M_K260Q.
Group b2:
H182M W253H K260E, M141V W253V K260Q, H182L W253H K260Q, M141V_W253H_K260E, H182L W253Q _K260Q, M141L_W253H_K260E, H182M W253Q K260E, M141V _W253F_K260Q, M141V _W253L_K260E, M141V_W253T_K260Q, M141Y_H182F_K260E, H182M_W253M_K260E, H182L W253M K260Q, M141Y_W253H_K260E, M141Y_W253Q_K260E, M141V_H182M_K260Q, H182M_W253L_K260E, H182F_W253H_K260E, M141A_H182F_K260Q, M141Y_W253M_K260E, H182M W253Q _K260Q, and H182M_W253M_K260Q.
Group b3:
H182L_W253T_K260Q, M141Y_W253S_K260E, M141V_H182F_K260E, M141A_H182M_K260E, M141T_H182L_K260Q, M141Y_W253T_K260E, H182M_W253L_K260Q, M141V_H182F_K260Q, M141S_W253H_K260E, M141T_W253H_K260E, H182M_W253HK260Q, M141A_H182L_K260Q, H182M_W253F_K260Q, M141A_H182M _K260Q, M141A _W253Q_K260E, M141A_H182L_K260E, M141A_W253H_K260E, M141V_W253E_K260Q, M141A_W253M_K260E, M141A_W253H_K260Q, M141K_H182L_W253H, and M141T_H182M_K260Q.
Group b4:
M141A_W253M_K260Q and M141A _H182F_K260E

The polypeptide may comprise, for example, an amino acid sequence having 90% or more sequence identity to an amino acid sequence selected from the group consisting of groups b1 to b4, preferably comprises a sequence having 90% or more sequence identity to an amino acid sequence selected from the group consisting of groups b1 to b3, more preferably comprises a sequence having 90% or more sequence identity to an amino acid sequence selected from the group consisting of groups b1 and b2, and most preferably comprises a sequence having 90% or more sequence identity to an amino acid sequence selected from the group consisting of group b1.

The polypeptide may comprise an amino acid sequence selected from the group consisting of groups b1 to b4, preferably comprises an amino acid sequence selected from the group consisting of groups b1 to b3, more preferably comprises an amino acid sequence selected from the group consisting of groups b1 and b2, and most preferably comprises an amino acid sequence selected from group b1.

The polypeptide according to the present embodiment has higher catalytic activity for the reductive amination reaction between at least one compound A represented by the formula (1) mentioned above or a salt thereof and at least one compound B represented by the formula (2) mentioned above or a salt thereof or the intramolecular reductive amination reaction of at least one compound B mentioned above or a salt thereof than that of a polypeptide comprising the amino acid sequence represented by SEQ ID NO: 1 under at least one reaction condition. In one aspect, the polypeptide according to the present embodiment has the higher catalytic activity than that of a polypeptide consisting

of the amino acid sequence represented by SEQ ID NO: 17 under at least one reaction condition.

Since the sequence represented by SEQ ID NO: 17 is the sequence represented by SEQ ID NO: 1 C-terminally tagged with a streptavidin-binding peptide tag sequence (GTDEKTTGWRGGHVVEGLAGELEQLRARLEHHPQ) and a His tag sequence (HHHHHH), all definitions for the polypeptide comprising the amino acid sequence represented by SEQ ID NO: 1 also apply to the polypeptide consisting of the amino acid sequence represented by SEQ ID NO: 17 and vice versa.

In one aspect, the polypeptide according to the present embodiment has higher catalytic activity for the reductive amination reaction between ethylamine or a salt thereof and phenylpyruvic acid or a salt thereof than that of a polypeptide consisting of the amino acid sequence represented by SEQ ID NO: 17 under at least one reaction condition.

In one aspect, the polypeptide according to the present embodiment has higher catalytic activity for the reductive amination reaction between ethylamine or a salt thereof and 2-oxo-3-(p-tolyl)propanoic acid or a salt thereof than that of a polypeptide consisting of the amino acid sequence represented by SEQ ID NO: 17 under at least one reaction condition.

In one aspect, the polypeptide according to the present embodiment has higher catalytic activity for the reductive amination reaction between methylamine or a salt thereof and 2-cyclopentyl-2-oxo-acetic acid or a salt thereof than that of a polypeptide consisting of the amino acid sequence represented by SEQ ID NO: 17 under at least one reaction condition.

In one aspect, the polypeptide according to the present embodiment has catalytic activity for the reductive amination reaction between ammonia or a salt thereof and phenylpyruvic acid or a salt thereof under at least one reaction condition.

The polypeptide according to the present embodiment may be fused with another polypeptide or a protein. Specifically, the polypeptide according to the present embodiment may comprise an amino acid sequence (additional amino acid sequence) other than the sequence having exemplarily 90% or higher sequence identity to an amino acid sequence represented by SEQ ID NO: 1 with one amino acid residue thereof engineered at any one or both of the N terminus and the C terminus. The additional amino acid sequence may be, for example, a tag sequence.

Examples of a polypeptide or a protein having the additional amino acid sequence include His tag ($6 \times$ His, $10 \times$ His, etc.) which is a tag consisting of several (e.g., 6 and 10) His (histidine) residues, streptavidin-bound peptide tag (SBP tag) comprising an amino acid sequence having the ability to bind to a biotin binding site of streptavidin, GST (glutathione S-transferase), HA (influenza hemagglutinin), immunoglobulin constant regions, B-galactosidase, MBP (maltose binding protein), FLAG (Hopp, T.P. et al., BioTechnology (1988) 6, 1204-1210), human c-myc fragments, VSV-GP fragments, p18 HIV fragments, T7-tag, HSV-tag, E-tag, SV40T antigen fragments, lck tag, $\alpha$-tubulin fragments, B-tag, protein C fragments, Stag, StrepTag, and HaloTag. The His tag may be, for example, the amino acid sequence represented by SEQ ID NO: 14. The SBP tag may be, for example, the amino acid sequence represented by SEQ ID NO: 15.

The polypeptide according to the present embodiment may comprise one or more selected from the group consisting of a streptavidin-bound peptide tag sequence and a His tag sequence at any one or both of the N terminus and the C terminus, and may comprise a streptavidin-bound peptide tag sequence and a His tag sequence at the C terminus. The linker sequence may comprise, for example, the amino acid sequence represented by GGSS or GGS. One example of the amino acid sequence comprising a streptavidin-bound peptide tag sequence, a linker sequence, and a His tag sequence includes the amino acid sequence represented by SEQ ID NO: 16.

The polypeptide according to the present embodiment may comprise the amino acid sequence a1, a2, a3, a4, or a5 and a tag sequence. The polypeptide according to the present embodiment may comprise, in addition to the amino acid sequence a1, a2, a3, a4, or a5 and a tag sequence, a linker sequence that links the amino acid sequence a1, a2, a3, a4, or a5 to the tag sequence.

The polypeptide according to one embodiment may comprise

the amino acid sequence represented by SEQ ID NO: 18 having amino acid sequence a1 represented by SEQ ID NO: 6 wherein the amino acid residue represented by X is a valine residue (mutation: M141V), and the amino acid sequence represented by SEQ ID NO: 16 linked to the C terminus of the amino acid sequence a1,

the amino acid sequence represented by SEQ ID NO: 19 having amino acid sequence a2 represented by SEQ ID NO: 6 wherein the amino acid residue represented by X is a tyrosine residue (mutation: M141Y), and the amino acid sequence represented by SEQ ID NO: 16 linked to the C terminus of the amino acid sequence a2,

the amino acid sequence represented by SEQ ID NO: 20 having amino acid sequence a3 represented by SEQ ID NO: 8 wherein the amino acid residue represented by X is a leucine residue (mutation: H182L), and the amino acid sequence represented by SEQ ID NO: 16 linked to the C terminus of the amino acid sequence a3,

the amino acid sequence represented by SEQ ID NO: 21 having amino acid sequence a4 represented by SEQ ID NO: 11 wherein the amino acid residue represented by X is a histidine residue (mutation: W253H), and the amino acid sequence represented by SEQ ID NO: 16 linked to the C terminus of the amino acid sequence a4, or

the amino acid sequence represented by SEQ ID NO: 22 having amino acid sequence a5 represented by SEQ ID NO: 12 wherein the amino acid residue represented by X is a glutamic acid residue (mutation: K260E), and the amino acid sequence represented by SEQ ID NO: 16 linked to the C terminus of the amino acid sequence a5.

One or more amino acid residues in the amino acid sequence represented by SEQ ID NO: 18, SEQ ID NO: 19, SEQ ID NO: 20, SEQ ID NO: 21, or SEQ ID NO: 22 may be engineered, and 1 to 20, 1 to 15, 1 to 10, 1 to 7, or 1 to 5 amino acid residues may be engineered. The number of engineered amino acid residues in the amino acid sequence represented by SEQ ID NO: 18, SEQ ID NO: 19, SEQ ID NO: 20, SEQ ID NO: 21, or SEQ ID NO: 22 may be preferably 3 or less, more preferably 2 or less, and most preferably 1.

The polypeptide according to a present embodiment preferably has an amino acid sequence represented by any of SEQ ID NOs: 36 to 44.

The polypeptide according to a present embodiment preferably has an amino acid sequence represented by any of SEQ ID NOs: 45 to 62.

The polypeptide according to the present embodiment may be used as a mixture with another polypeptide and may be used in an isolated or purified state. In the case of using the polypeptide as a mixture with another polypeptide, the amino acid of interest may be obtained through the step of isolating or purifying the target product.

The number of amino acid residues in the polypeptide according to the present embodiment in the form of a monomer may be exemplarily between 300 and 400, preferably between 320 and 390 more preferably between 325 and 380, and most preferably between 330 and 375.

When the additional amino acid sequence is added to neither the N terminus nor the C terminus, the number of amino acid residues in the polypeptide according to the present embodiment may be exemplarily between 300 and 360, preferably between 320 and 350, more preferably between 325 and 340, and most preferably between 330 and 335.

When the additional amino acid sequence is added to any one or both of the N terminus and the C terminus, the number of amino acid residues in the polypeptide according to the present embodiment may be exemplarily between 340 and 400, preferably between 350 and 390,more preferably between 360 and 380, and most preferably between 370 and 375.

The polypeptide according to the present embodiment may be used as a monomer or may be used in an associated form of two or more monomers. The polypeptide according to the present embodiment may be a homodimer.

When the polypeptide according to the present embodiment is a homodimer, the homodimer has two times the number of amino acid residues in the form of a monomer.

In one aspect, the polypeptide according to the present embodiment has the catalytic activity higher than that of a polypeptide comprising the amino acid sequence represented by SEQ ID NO: 1 under at least one reaction condition.

In one aspect, a temperature at which the polypeptide according to the present embodiment has the higher catalytic activity than that of a polypeptide comprising the amino acid sequence represented by SEQ ID NO: 1 falls within the range of 0°C or higher and 50°C or lower.

The polypeptide according to the present embodiment may have catalytic activity for the reductive amination reaction between at least one compound A or a salt thereof and at least one compound B or a salt thereof or the intramolecular reductive amination reaction of at least one compound B or a salt thereof at at least one point under a reaction condition of 0°C or higher and 50°C or lower. The lower limit of the temperature may be 0°C or higher, 10°C or higher, 20°C or higher, or 30°C or higher. The upper limit of the reaction temperature may be 50°C or lower, 40°C or lower, or 30°C or lower. The reaction temperature may be exemplarily between 0°C and 50°C, preferably between 10°C and 40°C, more preferably between 20°C and 45°C and most preferably between 20°C and 40°C. In one aspect, the temperature may be 25°C or may be 37°C. The reaction temperature may be changed stepwise or continuously.

In one aspect, pH at which the polypeptide according to the present embodiment has the higher catalytic activity than that of a polypeptide comprising the amino acid sequence represented by SEQ ID NO: 1 falls within the range of between 7 and 11. The polypeptide according to the present embodiment may have catalytic activity at at least one point under a reaction condition of pH 7 or higher and 11 or lower. The lower limit of the pH may be 7 or higher, 7.5 or higher, 8 or higher, or 8.5 or higher. The upper limit of the pH may be 11 or lower, 10 or lower, 9.5 or lower, or 9 or lower. The pH may be exemplarily between pH 7and 11, preferably between 7.5 and 10.5, more preferably between 8 and 10,and most preferably between 8 and 9.5.. In this context, the pH refers to pH at the start of reaction, and a pH engineering during reaction is accepted. The pH engineering during reaction may be within exemplarily 2, preferably within 1.5, and most preferably within 1.

In one aspect, the polypeptide according to the present embodiment has the higher catalytic activity than that of a polypeptide comprising the amino acid sequence represented by SEQ ID NO: 1 at at least one point in a range in which the total concentration of the compound A and a salt thereof at the start of reaction is 100 mM or higher and 3000 mM or lower in the total amount of a reaction solution in performing reductive amination reaction. In one aspect, the polypeptide according to the present embodiment has the higher catalytic activity than that of a polypeptide comprising the amino acid sequence represented by SEQ ID NO: 1 under reaction conditions of 37°C and pH 8.

In one aspect, the polypeptide according to the present embodiment has the higher catalytic activity than that of a polypeptide consisting of the amino acid sequence represented by SEQ ID NO: 17 at least one point in a range in which the total concentration of the compound A and a salt thereof at the start of reaction is 100 mM or higher and 3000 mM or lower in the total amount of a reaction solution in performing reductive amination reaction. In one aspect, the polypeptide according to the present embodiment has the higher catalytic activity than that of a polypeptide consisting of the amino acid sequence represented by SEQ ID NO: 17 under reaction conditions of 37°C and pH 8.

In one aspect, the polypeptide according to the present embodiment has the higher catalytic activity than that of a polypeptide comprising the amino acid sequence represented by SEQ ID NO: 1 under reaction conditions of 25°C and pH 9.

In one aspect, the polypeptide according to the present embodiment has the higher catalytic activity than that of a polypeptide consisting of the amino acid sequence represented by SEQ ID NO: 17 under reaction conditions of 25°C and pH 9.

The polypeptide according to the present embodiment may have catalytic activity under a reaction condition where the total concentration of the compound A and a salt thereof is 100 mM or higher and 3000 mM or lower. The lower limit of the total concentration of the compound A and a salt thereof may be 100 mM or higher, 300 mM or higher, 500 mM or higher, 700 mM or higher, 900 mM or higher, 1100 mM or higher, 1300 mM or higher, 1500 mM or higher, or 1700 mM or higher. The upper limit of the total concentration of the compound A and a salt thereof may be 3000 mM or lower, 2500 mM or lower, or 2000 mM or lower. The total concentration of the compound A and a salt thereof may be exemplarily between 100 mM and 3000 mM, preferably between 300 mM and 2500 mM, more preferably between 500 mM and 2000 mM, and most preferably between 500 mM and 1750 mM. In one aspect, the total concentration of the compound A and a salt thereof may be 500 mM or may be 1750 mM. The total concentration of the compound A and a salt thereof is the total concentration of the compound A and a salt thereof at the start of reaction in the total amount of a reaction solution in performing reductive amination reaction.

In one aspect, the polypeptide according to the present embodiment has the higher catalytic activity than that of a polypeptide comprising the amino acid sequence represented by SEQ ID NO: 1 at at least one point in a range in which the total concentration of the compound B and a salt thereof at the start of reaction is 0.001 mM or higher and 1000 mM or lower in the total amount of a reaction solution in performing reductive amination reaction.

In one aspect, the polypeptide according to the present embodiment has the higher catalytic activity than that of a polypeptide consisting of the amino acid sequence represented by SEQ ID NO: 17 at least one point in a range in which the total concentration of the compound B and a salt thereof at the start of reaction is 0.001 mM or higher and 1000 mM or lower in the total amount of a reaction solution in performing reductive amination reaction.

The polypeptide according to the present embodiment may have catalytic activity under a reaction condition where the total concentration of the compound B and a salt thereof is 10 mM or higher and 500 mM or lower. The lower limit of the total concentration of the compound B and a salt thereof may be 10 mM or higher, 30 mM or higher, 50 mM or higher, 100 mM or higher, 150 mM or higher, 200 mM or higher, 250 mM or higher, 300 mM or higher, or 330 mM or higher. The upper limit of the total concentration of the compound B and a salt thereof may be 500 mM or lower, 450 mM or lower, 400 mM or lower, or 380 mM or lower. The total concentration of the compound B and a salt thereof may be exemplarily between 0.001 mM and 1000 mM, preferably between 10 mM and 500 mM, more preferably between 30 mM and 450mM and most preferably between 50 mM and 400 mM. In one aspect, the total concentration of the compound B and a salt thereof may be 50 mM or may be 350 mM. The total concentration of the compound B and a salt thereof is the total concentration of the compound B and a salt thereof at the start of reaction in the total amount of a reaction solution in performing reductive amination reaction.

The polypeptide according to the present embodiment may have catalytic activity under a reaction condition exemplarily where the total concentration of the compound A and a salt thereof is between 100mM and 3000mM, and the total concentration of the B and a salt thereof is 0.001mM and 1000mM, and temperature is 25°C and pH is 9, preferably where the total concentration of the compound A and a salt thereof is between 300mM and 2500mM, and the total concentration of the B and a salt thereof is 10mM and 500mM, and temperature is 25°C and pH is 9, more preferably where the total concentration of the compound A and a salt thereof is between 500mM and 2000mM, and the total concentration of the B and a salt thereof is 30mM and 450mM, and temperature is 25°C and pH is 9, most preferably where the total concentration of the compound A and a salt thereof is between 500mM and 1750mM, and the total concentration of the B and a salt thereof is 50mM and 400mM, and temperature is 25°C and pH is 9.

The polypeptide according to the present embodiment may have catalytic activity under a reaction condition exemplarily where the total concentration of the compound A and a salt thereof is between 100mM and 3000mM, and the total concentration of the B and a salt thereof is 0.001mM and 1000mM, and temperature is 37°C and pH is 8, preferably where the total concentration of the compound A and a salt thereof is between 300mM and 2500mM, and the total concentration of the B and a salt thereof is 10mM and 500mM, and temperature is 37°C and pH is 8, more preferably where the total concentration of the compound A and a salt thereof is between 500mM and 2000mM, and the total concentration of the B and a salt thereof is 30mM and 450mM, and temperature is 37°C and pH is 8, most

preferably where the total concentration of the compound A and a salt thereof is between 500mM and 1750mM, and the total concentration of the B and a salt thereof is 50mM and 400mM, and temperature is 37°C and pH is 8.

In one aspect, in the polypeptide according to the present embodiment, the catalytic activity is higher than that of a polypeptide comprising the amino acid sequence represented by SEQ ID NO: 1 at at least one point when a ratio of the total molar number of the compound A and a salt thereof to the total molar number of the compound B and a salt thereof at the start of reaction is 1 or more in the total amount of a reaction solution in performing reductive amination reaction.

The polypeptide according to the present embodiment may have catalytic activity under a reaction condition where the ratio of the total molar number of the compound A and a salt thereof to the total molar number of the compound B and a salt thereof (the total molar number of the compound A and a salt thereof / the total molar number of the compound B and a salt thereof) is 1 or more.

The ratio of the total molar number of the compound A and a salt thereof to the total molar number of the compound B and a salt thereof (the total molar number of the compound A and a salt thereof / the total molar number of the compound B and a salt thereof) may be exemplarily between 1 and 50, preferably between 3 and 30, more preferably between 5 and 15, most preferably between 5 and 10. The ratio of the total molar number of the compound A and a salt thereof to the total molar number of the compound B and a salt thereof is the ratio of the total molar number of the compound A and a salt thereof to the total molar number of the compound B and a salt thereof at the start of reaction in the total amount of a reaction solution in performing reductive amination reaction.

The polypeptide according to the present embodiment may be used in a method for producing an amino acid. The method for producing an amino acid comprises the step of reacting one or more selected from the group consisting of amine, an amine analog and salts thereof with one or more selected from the group consisting of keto acid, a keto acid analog and salts thereof, or intramolecularly reacting a compound selected from the group consisting of keto acid, a keto acid analog and salts thereof in the presence of the polypeptide and a reducing agent. For the intramolecular reaction, the keto acid and the keto acid analog may have a primary amino group or a secondary amino group.

In the method for producing an amino acid, the reaction may be performed under appropriate conditions. In this context, the appropriate conditions refer to appropriately set conditions such as the temperature, the pH, and the total concentration of the compound A and a salt thereof, the total concentration of the compound B and a salt thereof and the ratio of the total molar number of the compound A and a salt thereof to the total molar number of the compound B and a salt thereof, in a reaction solution at the start of reaction as described in the paragraphs [0198] to [0208].

The reaction may be performed in a reaction solution containing amine or an amine analog, keto acid or a keto acid analog, the polypeptide, and a reducing agent. In one aspect, the compound A may be used as the amine or the amine analog. The amine or the amine analog may be alkylamine or ammonia. The alkylamine may be $C_1$ to $C_6$ alkylamine or $C_1$ to $C_4$ alkylamine. Examples thereof more specifically include methylamine, ethylamine, propylamine, and ammonia. In one aspect, the compound B may be used as the keto acid or the keto acid analog. The keto acid or the keto acid analog may be of the formula (2) wherein Y is $C_3$ to $C_8$ cycloalkyl or $C_6$ to $C_9$ aralkyl. Examples thereof more specifically include 2-cyclopentyl-2-oxo-acetic acid, phenylpyruvic acid, and 2-oxo-3-(p-tolyl)propanoic acid. The amine or the amine analog and the keto acid or the keto acid analog may each be added in a salt state to the reaction solution. The materials for use in reaction may be mixed simultaneously or separately in an arbitrary order. The salt may be preferably a chemically or pharmaceutically acceptable salt. Examples of the salt include: hydrochloride; hydrobromide; hydroiodide; phosphate; phosphonate; sulfate; sulfonate such as methanesulfonate and p-toluenesulfonate; carboxylate such as acetate, citrate, malate, tartrate, succinate, and salicylate; alkali metal salts such as lithium salt, sodium salt, and potassium salt; alkaline earth metal salts such as magnesium salt and calcium salt; and ammonium salts such as ammonium salt, alkylammonium salt, dialkylammonium salt, trialkylammonium salt, and tetraalkylammonium salt.

Examples of the reducing agent include reduced nicotinamide adenine dinucleotide phosphate (NADPH), oxidized nicotinamide adenine dinucleotide phosphate ($NADP^+$), reduced nicotinamide adenine dinucleotide (NADH) and oxidized nicotinamide adenine dinucleotide ($NAD^+$). In one embodiment, the reducing agent can be β-NADPH.

For example, additives, such as glucose dehydrogenase (GDH) and glucose, which reduce $NADP^+$ and $NAD^+$ into NADPH and NADH, respectively, can be further added together therewith.

In the case of using, for example, NADPH, as the reducing agent, a reactant that reduces $NADP^+$ (oxidized nicotinamide adenine dinucleotide phosphate) which may appear in the reaction solution into NADPH may be used in the reaction step because the amount of NADPH used can be reduced. Examples of the reactant include a combination of glucose and GDH (glucose dehydrogenase). In the case of using glucose and GDH together with NADPH, the amount of NADPH used can be reduced to a catalytic amount.

The concentration of the reducing agent in the reaction solution at the start of reaction may be, for example, 0.001 mMto 100 mM, or 0.01 to 10 mM.

In the reaction, reaction conditions such as temperature, pH, and the concentration of each compound may be the conditions mentioned above.

In the method for producing an amino acid, the reductive amination reaction may be performed in the presence

of compound C represented by the following formula (4):

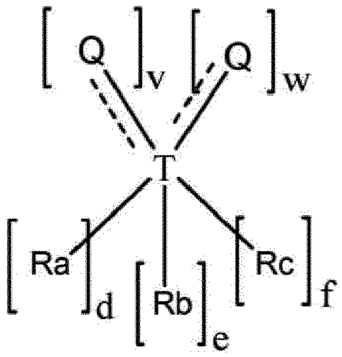

(4)

In the formula (4), v and w each independently represent 0 or 1, at least one of v and w represents 1, and T represents a carbon atom, a phosphorus atom, or a sulfur atom.

In the formula (4), the functional group represented by the following formula (4a):

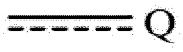

(4a)

represents =O, -ORd, or a hydroxy group. In this context, =O which is the functional group represented by the formula (4a) means that T is bonded to the oxygen atom through a double bond. Specifically, the compound C wherein the functional group represented by the formula (4a) is =O has a structure represented by T=O. -ORd which is the functional group represented by the formula (4a) means that T is bonded to ORd through a single bond. Specifically, the compound C wherein the functional group represented by the formula (4a) is -ORd has a structure represented by T-ORd. The hydroxy group which is the functional group represented by the formula (4a) means that T is bonded directly to a hydroxy group. Specifically, the compound C wherein the functional group represented by the formula (4a) is -OH has a structure represented by T-OH.

In the formula (4), when each of v and w is 1, two functional groups represented by a plurality of formulas (4a) are the same or different. Ra, Rb, and Rc each independently represent a hydrogen atom, a $C_1$ to $C_3$ alkyl group, an alkylamino group, or $-CH_2$-ORd, and at least any two of Ra, Rb, and Rc are optionally linked to each other to form a ring structure together with T. Rd represents a $C_1$ to $C_3$ alkyl group. d, e, and f each independently represent 0 or 1, and at least one of d, e and f represents 1.

In the formula (4), when each of v and w is 1, at least one of Ra, Rb, and Rc is a methyl group, and none of Ra, Rb, and Rc are linked to each other to form a ring structure together with T. For example, when each of v and w is 1, T is a sulfur atom, each functional group represented by the formula (4a) is =O, f is 0, and at least one of Ra and Rb may be a methyl group.

When at least one of Ra, Rb, and Rc is a methylamino group, none of Ra, Rb, and Rc are linked to each other to form a ring structure together with T. When the functional group represented by the formula (4a) is a hydroxy group and T is a carbon atom, v is 1, w is 0, each of d, e, and f is 1, and each of Ra, Rb, and Rc is a hydrogen atom.

The compound C may be a compound represented by the following formula (4-1) wherein T in the formula (1) is a carbon atom:

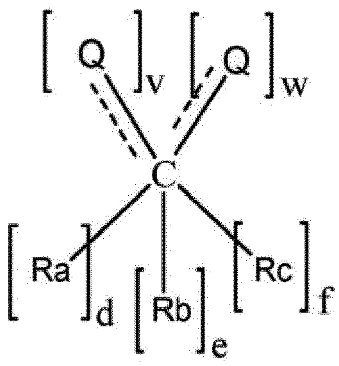

(4−1)

The compound represented by the formula (4-1) may be a compound represented by the following formula (4-1a) wherein v is 1, and w is 0:

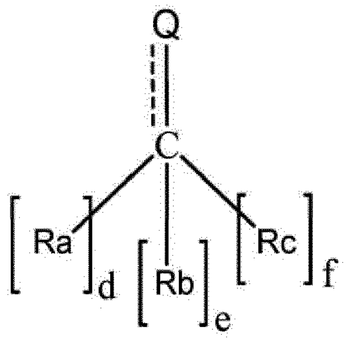

(4-1a)

In the formula (4-1a), when the functional group represented by the formula (4a) is =O, two of d, e, and f are 1 and the remaining one is 0. When the functional group represented by the formula (4a) is =O, the compound represented by the formula (4-1a) may be a compound represented by the following formula (4-1b) wherein each of d and e is 1, and f is 0:

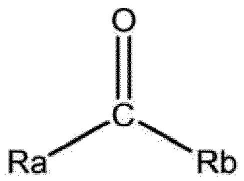

(4-1b)

The compound represented by the formula (4-1b) may be a compound wherein one of Ra and Rb is a hydrogen atom or a $C_1$ to $C_3$ alkyl group, and the other moiety is an alkylamino group. The alkylamino group may be a monomethylamino group or a dimethylamino group. The compound represented by the formula (4-1b) may be, for example, N,N-dimethylformamide, N,N-dimethylacetamide, or N-methylformamide.

In the formula (4-1a), when the functional group represented by the formula (4a) is a hydroxy group, each of d, e, and f is 1. When the functional group represented by the formula (4a) is a hydroxy group, the compound represented by the formula (4-1a) may be a compound represented by the following formula (4-1c) wherein each of d, e, and f is 1:

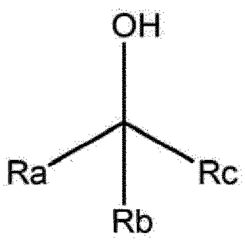

(4-1c)

Ra, Rb, and Rc may each independently be a hydrogen atom or a $C_1$ to $C_3$ alkyl group. The compound represented by the formula (4-1c) may be methanol wherein each of Ra, Rb, and Rc is a hydrogen atom.

In the formula (4-1a), when the functional group represented by the formula (4a) is -ORd, each of d, e, and f may be 1 and each of Rb and Rd may be a hydrogen atom. When the functional group represented by the formula (4a) is -ORd, the compound represented by the formula (4-1a) may be a compound represented by the formula (4-1d) given below wherein each of d, e, and f is 1, and each of Rb and Rd is a hydrogen atom. In the compound represented by the formula (4-1d) given below, Ra may be $-CH_2-ORd$. The compound represented by the formula (4-1d) given below may be dimethoxyethane wherein Ra is $-CH_2-ORd$, and Rd is $-CH_3$.

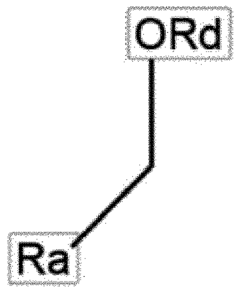

(4-1d)

The compound C may be a compound represented by the following formula (4-2) wherein T in the formula (4) is a phosphorus atom:

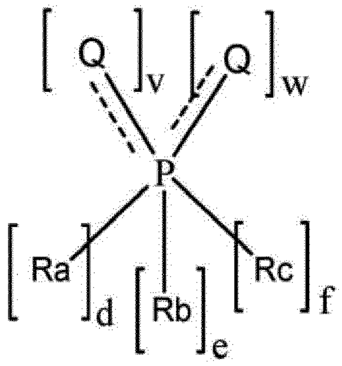

(4-2)

The compound represented by the formula (4-2) may be a compound represented by the following formula (4-2a) wherein v is 1, w is 0, and the functional group represented by the formula (4a) is =O:

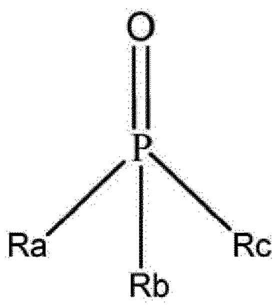

(4-2a)

In the formula (4-2a), each of Ra, Rb, and Rc may be a $C_1$ to $C_3$ alkyl group. The compound represented by the formula (4-2a) may be trimethylphosphine oxide wherein each of Ra, Rb, and Rc is a methyl group.

The compound C may be a compound represented by the following formula (4-3) wherein T in the formula (4) is a sulfur atom:

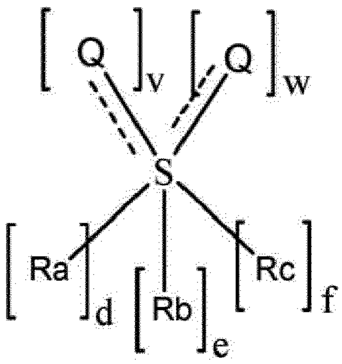

(4−3)

The compound represented by the formula (4-3) may be a compound represented by the following formula (4-3a) wherein v is 1, w is 0, the functional group represented by the formula (4a) is =O, each of d and e is 1, and f is 0:

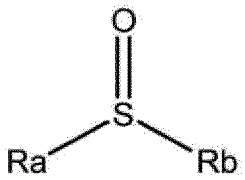

(4−3a)

In the formula (4-3a), each of Ra and Rb may be a $C_1$ to $C_3$ alkyl group, and Ra and Rb may be linked to each other to form a ring structure together with the sulfur atom (S).

In the compound represented by the formula (4-3a), Ra and Rb may each independently be a methyl group or an ethyl group. The compound represented by the formula (4-3a) may be dimethyl sulfoxide wherein each of Ra and Rb is a methyl group, or diethyl sulfoxide wherein each of Ra and Rb is an ethyl group. The compound represented by the formula (4-3a) may be tetramethylene sulfoxide wherein each of Ra and Rb is a $C_1$ to $C_3$ alkyl group, and these alkyl groups are linked to each other to form a ring structure together with S.

The compound represented by the formula (4-3) may be a compound represented by the following formula (4-3b) wherein each of v and w is 1, the functional group represented by the formula (4a) is =O, each of d and e is 1, and f is 0:

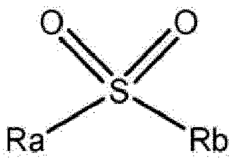

(4−3b)

In the compound represented by the formula (4-3b), at least one of Ra and Rb is a methyl group. The compound represented by the formula (4-3b) may be dimethylsulfone wherein each of Ra and Rb is a methyl group.

The compound C may be at least one compound selected from the group consisting of dimethyl sulfoxide, dimethylsulfone, trimethylphosphine oxide, dimethoxyethane, N,N-dimethylformamide, N,N-dimethylacetamide, tetramethylene sulfoxide, diethyl sulfoxide, methanol, and methylformamide because the amino acid can be produced with a better yield. The compound C may be at least one selected from the group consisting of dimethyl sulfoxide, dimethyl sulfone, and trimethylphosphine oxide and may be dimethyl sulfoxide because the amino acid can be produced with a better yield.

The compound C may be a liquid or a solid under conditions of 25°C and 1 atm, and may be a liquid under conditions of 25°C and 1 atm because the production efficiency of the amino acid is more improved. Examples of the compound C which is a liquid under conditions of 25°C and 1 atm include dimethyl sulfoxide, dimethoxyethane, N,N-dimethylformamide, N,N-dimethylacetamide, methanol, and N-methylformamide.

The method for producing an amino acid may comprise the following steps (A) and (B):

step (A); a step of contacting a lithium-containing substance with a mixture to be purified which is a mixture of the following purification target (i) and the following impurities (ii), obtained by the method mentioned above:

(i) the amino acid having a protective group at the N terminus, and
(ii) compounds other than the purification target, and

step (B); a step of precipitating lithium salt of the purification target.

An alternative embodiment of the present invention provides a method for removing impurities from a mixture to be purified, comprising the steps (A) and (B).

A further alternative embodiment of the present invention provides lithium salt of an amino acid or a solvate thereof, wherein the lithium salt of an amino acid contains lithium salt of an amino acid represented by the following general formula (5) with a purity of 99% by mol or higher or an apparent purity of 99% or higher:

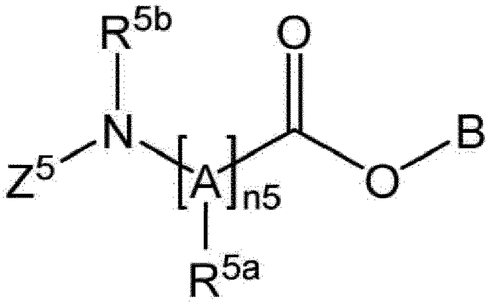
(5)

In the general formula (5), n5 represents a number of between 1 and 3, when a plurality of $R^{5a}$ are present, $R^{5a}$ are the same as or different from each other, $R^{5a}$ and $R^{5b}$ each independently represent a hydrogen atom, an alkyl group, an alkenyl group, an alkynyl group, a cycloalkyl group, an aryl group, a heterocyclyl group, or a heteroaryl group, or a substituent in which two or more selected from the group consisting of these substituents are bonded, these substituents optionally have a substituent, each of these groups is optionally a saturated hydrocarbon group or an unsaturated hydrocarbon group, A represents a carbon atom, B represents a hydrogen atom or a binding point to an amino acid or a peptide compound, $Z^5$ represents a fluorenylmethoxycarbonyl group, a tert-butoxycarbonyl group, a benzyloxycarbonyl group, an allyloxycarbonyl group, a 2,2,2-trichloroethoxycarbonyl group, or a 2-(trimethylsilyl)ethoxycarbonyl group, $R^{5a}$ and $R^{5b}$ are optionally bonded to form a ring together with N and A, wherein each of $R^{5a}$ and $R^{5b}$ is a substituent having a structure obtained by eliminating one hydrogen atom from the structure of the substituent that is not involved in ring formation, and when n5 is 2, at least one of $R^{5b}$ and two $R^{5a}$ is not a hydrogen atom.

For conventional purification of the purification target mentioned above, it is difficult to purify the purification target with a high purity. In general, the purification target is used as a starting material for peptide compound and protein synthesis, etc. Obtainment of a peptide compound and a protein with a high purity is important for suppressing adverse reaction ascribable to impurities when the peptide compound and the protein are medicaments. Specifically, purification of the starting material constituting them with a high purity is important. As a result of conducting diligent studies, the present inventors have newly found that, surprisingly, the purification target is converted to lithium salt and can thereby be purified with a high purity. Thus, the present inventors have further improved the usefulness of the purification target and its usefulness in peptide compound and protein synthesis using the purification target.

The salt of the amino acid or the solvate thereof, more preferably the salt of the amino acid, still more preferably the lithium salt of the amino acid, according to the present invention is obtained by treating the mixture to be purified by the step (A) and the step (B). In the present specification, the mixture to be purified is a mixture of the purification target and the impurities. In the present specification, the purification target refers to the amino acid having a protective group at the N terminus. The purification target is preferably an amino acid produced by the production method as described herein, more preferably, an amino acid produced by the production method as described herein in the presence of the polypeptide of the invention. Examples of the purification target include an amino acid represented by the general formula (5). In the present specification, the impurities refer to compounds other than the purification target contained in the mixture to be purified. Examples of the impurities include semi-specific impurities (amino acids having a protective group at the N terminus, other than the purification target). More specific examples thereof include β-alanine and derivatives thereof. In the present specification, the derivative refers to, assuming that a certain organic compound is a matrix, a compound engineered by introduction of a functional group, oxidation, reduction, replacement of an atom, or the like to the extent that the structure or properties of the matrix are not drastically changed. Examples of the derivative of β-alanine include specific impurities (β-alanine having a protective group at the N terminus).

The step (A) is the step of contacting a lithium-containing substance with the mixture to be purified. The step (A) may form lithium salt of the purification target and may form lithium salt of the impurities. The step (B) is the step of precipitating lithium salt of the purification target. A portion of the step (A) and a portion of the step (B) may be performed

such that their times overlap with each other. Before the step (A), the mixture to be purified may be purified by a method other than the method of the present invention. For example, the purification target may be precipitated in a free form and purified, without forming lithium salt of the purification target. At least one of the step (A) and the step (B) may be performed in the presence of a solvent. In the present specification, generation of a solid from a solvent refers to "precipitation".

The protective group of the purification target may be a carbamate group. The carbamate group refers to a fluorenylmethoxycarbonyl group, a tert-butoxycarbonyl group, a benzyloxycarbonyl group, an allyloxycarbonyl group, a 2,2,2-trichloroethoxycarbonyl group, or a 2-(trimethylsilyl)ethoxycarbonyl group and may be a fluorenylmethoxycarbonyl group, a tert-butoxycarbonyl group, or a benzyloxycarbonyl group, may be a fluorenylmethoxycarbonyl group or a benzyloxycarbonyl group, may be a fluorenylmethoxycarbonyl group, or may be a benzyloxycarbonyl group.

The protective group of the impurities may be a carbamate group. The carbamate group refers to a fluorenylmethoxycarbonyl group, a tert-butoxycarbonyl group, a benzyloxycarbonyl group, an allyloxycarbonyl group, a 2,2,2-trichloroethoxycarbonyl group, or a 2-(trimethylsilyl)ethoxycarbonyl group and may be a fluorenylmethoxycarbonyl group, a tert-butoxycarbonyl group, or a benzyloxycarbonyl group, may be a fluorenylmethoxycarbonyl group or a benzyloxycarbonyl group, may be a fluorenylmethoxycarbonyl group, or may be a benzyloxycarbonyl group. These protective groups may be protective groups that protect the N terminus of the impurities.

The protective group of the purification target and the protective group of the impurities may be different or may be the same. The protective group of the purification target and the protective group of the impurities are preferably the same.

The step (A) may be performed in the presence of a first organic solvent. The first organic solvent is a solvent that can dissolve the purification target from the viewpoint of allowing the contact of the lithium-containing substance with the purification target to proceed efficiently. The solubility of the purification target in the first organic solvent is preferably 20 g/L or more. The solubility of the lithium salt of the purification target in the first organic solvent is preferably 20 g/L or less. The solubility of the impurities in the first organic solvent is preferably 20 g/L or more.

The first organic solvent is, for example, at least one selected from the group consisting of nitriles such as acetonitrile, alcohols such as methanol, ethanol, 1-propanol, 2-propanol, 1-butanol, 2-butanol, 2-methyl-2-propanol, and ethylene glycol, ethers such as tetrahydrofuran, 1,4-dioxane, 2-methyltetrahydrofuran, methyl tert-butyl ether, diisopropyl ether, diethyl ether, and 1,2-dimethoxyethane, ketones such as acetone, methyl ethyl ketone, and methyl isobutyl ketone, amides such as dimethylformamide, dimethylacetamide, and N-methyl-2-pyrrolidone, ureas such as 1,3-dimethyl-2-imidazolidinone, sulfoxides such as dimethyl sulfoxide, sulfones such as sulfolane, alkanes such as heptane, methylcyclohexane, hexane, cyclohexane, and pentane, aromatic compounds such as benzene, toluene, 1,2-dimethylbenzene, 1,3-dimethylbenzene, and 1,4-dimethylbenzene, esters such as ethyl acetate and isopropyl ester, and alkyl halides such as dichloroethane, dichloromethane, chloroform, and carbon tetrachloride. The first solvent may be of plural types and is preferably of one type from the viewpoint of the simplified step (A).

The step (A) may be performed in the presence of water.

The step (B) may be performed in the presence of a second organic solvent. The second organic solvent is a solvent in which the solubility of the lithium salt of the purification target is small, i.e., a solvent that functions as a poor solvent for the lithium salt of the purification target. The solubility of the lithium salt of the purification target in the second organic solvent is preferably 10 g/L or less.

The second organic solvent is, for example, at least one selected from the group consisting of nitriles such as acetonitrile, alcohols such as methanol, ethanol, 1-propanol, 2-propanol, 1-butanol, 2-butanol, 2-methyl-2-propanol, and ethylene glycol, ethers such as tetrahydrofuran, 1,4-dioxane, 2-methyltetrahydrofuran, methyl tert-butyl ether, diisopropyl ether, diethyl ether, and 1,2-dimethoxyethane, ketones such as acetone, methyl ethyl ketone, and methyl isobutyl ketone, amides such as dimethylformamide, dimethylacetamide, and N-methyl-2-pyrrolidone, ureas such as 1,3-dimethyl-2-imidazolidinone, sulfoxides such as dimethyl sulfoxide, sulfones such as sulfolane, alkanes such as heptane, methylcyclohexane, hexane, cyclohexane, and pentane, aromatic compounds such as benzene, toluene, 1,2-dimethylbenzene, 1,3-dimethylbenzene, and 1,4-dimethylbenzene, esters such as ethyl acetate and isopropyl ester, and alkyl halides such as dichloroethane, dichloromethane, chloroform, and carbon tetrachloride. The second solvent may be of plural types and is preferably of one type from the viewpoint of the simplified step (B).

The step (B) may be performed in the presence of water and is preferably performed in the presence of water from the viewpoint of dissolving lithium salt of the impurities. The solubility of the lithium salt of the impurities in water is preferably 10 g/L or more.

The second organic solvent may be used with the first organic solvent in the step (A).

In the step (B), lithium salt of the purification target is precipitated. The precipitation of the lithium salt of the purification target may be promoted by adding the second organic solvent upon precipitation and increasing its proportion. In the case of increasing the proportion of the second organic solvent, the second organic solvent may be added to the first organic solvent used in the step (A).

The proportion of the first organic solvent or the proportion of the second organic solvent means a volume/mass

ratio (ml/g; hereinafter, also referred to as v/w) calculated by dividing the volume of the first organic solvent or the second organic solvent by the mass of the purification target.

When the lithium salt of the purification target has sufficiently small solubility in the first organic solvent used in the step (A), the first organic solvent and the second organic solvent may be the same solvent. In this case, it is not necessary to add the second organic solvent in the step (B). In this case, the step (B) starts immediately after contact of the lithium-containing substance with the purification target in the step (A) so that lithium salt of the purification target is precipitated.

The lithium salt of the purification target is preferably precipitated as a solid. In this context, the solid includes crystals or an amorphous form. The lithium salt is more preferably precipitated as crystals (crystallized). In this case, the step (B) may be regarded as the step of crystallizing lithium salt of the purification target.

The volume of the first organic solvent per g of the mixture to be purified may be 1 ml or more, 2 ml or more, or 3 ml or more and may be 100 ml or less, 80 ml or less, 60 ml or less, or 40 ml or less, over the step (A) and the step (B). The volume of the first organic solvent per g of the mixture to be purified may be between 1 ml and 100 ml, between 1 ml and 80 ml, between 2 ml and 60 ml, or between 3 ml and 40 ml over the step (A) and the step (B).

The volume of the first organic solvent per g of the mixture to be purified at the completion of the step (A) (initial concentration of the first organic solvent) may be 1 ml or more, 2 ml or more, or 3 ml or more and may be 50 ml or less, 40 ml or less, 30 ml or less, or 20 ml or less when the proportion of the second organic solvent is increased in the step (B). The initial concentration of the first organic solvent may be between 1 ml and 50 ml, between 1 ml and 40 ml, between 2 ml and 30 ml, or between 3 ml and 20 ml.

The volume of the first organic solvent per g of the mixture to be purified at the completion of the step (A) (initial concentration of the first organic solvent) may be 1 ml or more, 10 ml or more, 20 ml or more, 30 ml or more, or 35 ml or more and may be 100 ml or less, 75 ml or less, 50 ml or less, or 37 ml or less when the proportion of the second organic solvent is not increased in the step (B). The initial concentration of the first organic solvent may be between 1 ml and 100 ml, between 10 ml and 75 ml, between 20 ml and 50 ml, between 30 ml and 37 ml, or between 35 ml and 37 ml.

The volume of the second organic solvent per g of the mixture to be purified may be 0 ml or more and may be 40 ml or less, 60 ml or less, 80 ml or less, or 100 ml or less, over the steps (A) and (B). The volume of the second organic solvent per g of the mixture to be purified may be between 0 ml and 100 ml, between 0 ml and 80 ml, between 1 ml and 60 ml, or between 1 ml and 40 ml, over the steps (A) and (B).

The volume of the second organic solvent per g of the mixture to be purified at the completion of the step (B) may be 0 ml or more and may be 40 ml or less, 60 ml or less, 80 ml or less, or 100 ml or less. The volume of the second organic solvent per g of the mixture to be purified at the completion of the step (B) may be between 0 ml and 100 ml, between 0 ml and 80 ml, between 0 ml and 60 ml, or between 0 ml and 40 ml.

The volume of the water per g of the mixture to be purified may be 0.1 ml or more, 0.3 ml or more, or 0.5 ml or more and may be 50 ml or less, 30 ml or less, 10 ml or less, or 5 ml or less, over the steps (A) and (B). The volume of the water in the solvents may be between 0.1 ml and 50 ml, between 0.1 ml and 30 ml, between 0.3 ml and 10 ml, or between 0.5 ml and 5 ml, over the steps (A) and (B).

The volume of the water per g of the mixture to be purified at the completion of the step (A) (initial concentration of the water) may be 0.1 ml or more, 0.3 ml or more, or 0.5 ml or more and may be 50 ml or less, 30 ml or less, 10 ml or less, or 4 ml or less. The initial concentration of the water may be between 0.1 ml and 50 ml, between 0.1 ml and 30 ml, between 0.3 ml and 10 ml, or between 0.5 ml and 4 ml.

The volume of the water per g of the mixture to be purified at the completion of the step (B) (initial concentration of the water) may be 0.1 ml or more, 0.3 ml or more, or 0.5 ml or more and may be 50 ml or less, 30 ml or less, 10 ml or less, or 4 ml or less. The initial concentration of the water may be between 0.1 ml and 50 ml, between 0.1 ml and 30 ml, between 0.3 ml and 10 ml, or between 0.5 ml and 4 ml.

The step (A) and the step (B) may be performed in the presence of an additional solvent other than the first organic solvent and the second organic solvent. Examples of the additional solvent include buffer solutions such as phosphate buffer solutions, CHES (N-cyclohexyl-2-aminoethanesulfonic acid), Tris (trishydroxymethylaminomethane), and Bicine (N,N-di(2-hydroxyethyl)glycine).

The temperature at which the step (A) is performed may be -20°C or higher, may be 0°C or higher, may be 10°C or higher, or may be 20°C or higher. The temperature at which the step (A) is performed may be 100°C or lower, may be 80°C or lower, may be 60°C or lower, may be 40°C or lower, or may be 30°C or lower. The temperature at which the step (A) is performed may be -20 to 100°C, may be 0 to 80°C, may be 10 to 60°C, may be 10 to 40°C, or may be 20 to 30°C.

The temperature at which the step (B) is performed may be -20°C or higher, may be 0°C or higher, may be 10°C or higher, may be 20°C or higher, may be 30°C or higher, or may be 35°C or higher. The temperature at which the step (B) is performed may be 100°C or lower, may be 80°C or lower, may be 60°C or lower, may be 40°C or lower, or may be 30°C or lower. The temperature at which the step (B) is performed may be -20 to 100°C, may be 0 to 80°C, may be 10 to 60°C, may be 10 to 40°C, or may be 20 to 30°C.

The conditions, such as temperature and concentrations of substances contained in various solutions, of the step (A) and the step (B) may be the same or may be different. When these conditions are different between these steps, conditions selected from among various conditions of the step (A) mentioned above may be combined. The amount of substance of the lithium-containing substance at the completion of the step (A) may be 0.5 equivalents or more, 0.8 equivalents or more, or 1.0 equivalent or more and may be 2.0 equivalents or less, 1.5 equivalents or less, or 1.1 equivalents or less, based on the amount of substance of the purification target. The amount of the lithium-containing substance based on the amount of the purification target may be between 0.5 equivalents and 2.0 equivalents, between 0.8 equivalents and 1.5 equivalents, or between 1.0 equivalent and 1.1 equivalents.

In the present specification, the lithium-containing substance refers to a substance that contains lithium for contact with the purification target in the step (A). The lithium-containing substance for use in the step (A) is, for example, at least one selected from the group consisting of lithium hydroxide, lithium tert-butoxide, lithium carbonate, lithium hydride, trilithium phosphate, lithium methoxide, lithium ethoxide, lithium isopropoxide, methyllithium, n-butyllithium, sec-butyllithium, tert-butyllithium, lithium amide, lithium diisopropylamide, lithium hexamethyldisilazide, and lithium tetramethylpiperidide.

The method for contacting the lithium-containing substance with the mixture to be purified is not limited, and the mixture to be purified and the lithium-containing substance may be contacted with each other in the presence of the first organic solvent. In this case, the mixture to be purified, the lithium-containing substance, and the first organic solvent may be mixed in the same container from the beginning; the mixture to be purified and the first organic solvent may be mixed, and the lithium-containing substance can be added to the obtained mixed solution; or the lithium-containing substance and the first organic solvent may be mixed, and the mixture to be purified may be added to the obtained mixed solution.

The content of the purification target in the mixture to be purified, based on which the amounts of the first organic solvent, the second organic solvent, and the lithium-containing substance are determined may be an actually measured value, an estimated value, or a calculated value and is preferably an actually measured value or a calculated value, most preferably an actually measured value.

Examples of the amino acid having a protective group at the N terminus or an amino acid constituting a peptide compound having a protective group at the N terminus, included in the purification target include an amino acid represented by the general formula (5) (also referred to as an amino acid (1)), hydrophobic amino acids, aliphatic amino acids, and aromatic amino acids.

The amino acid having a protective group at the N terminus is preferably an amino acid as produced according to the methods of the invention in the presence of the polypeptide of the invention.

n5 represents a number of between 1 and 3 and may be an integer of 1 or larger and 3 or smaller. n5 may be 1, 2, or 3. When n5 is 2, at least one of $R^{5b}$ and two $R^{5a}$ is not a hydrogen atom, two or more of $R^{5b}$ and two $R^{5a}$ may not be a hydrogen atom, and all three of $R^{5b}$ and two $R^{5a}$ may not be a hydrogen atom.

Each of $R^{5a}$ and $R^{5b}$ represents a hydrogen atom, an alkyl group, an alkenyl group, an alkynyl group, a cycloalkyl group, an aryl group, a heterocyclyl group, or a heteroaryl group, or a substituent in which two or more selected from the group consisting of these substituents are bonded (e.g., a benzyl group), these substituents optionally have a substituent, and these groups may be saturated or unsaturated.

A represents a carbon atom, and B represents a hydrogen atom or a binding point to an amino acid or a peptide compound.

$Z^5$ represents a fluorenylmethoxycarbonyl group, a tert-butoxycarbonyl group, a benzyloxycarbonyl group, an allyloxycarbonyl group, a 2,2,2-trichloroethoxycarbonyl group, or a 2-(trimethylsilyl)ethoxycarbonyl group.

$R^{5a}$ and $R^{5b}$ are optionally bonded to form a ring together with N and A. In this case, each of $R^{5a}$ and $R^{5b}$ is a substituent having a structure obtained by eliminating one hydrogen atom from the structure of the substituent that is not involved in ring formation.

In the step (B), a purified product may be precipitated so as to reduce the content of semi-specific impurities contained in the precipitate (purified product) obtained in the step (B). In the step (B), the purified product may be precipitated such that the purity of the purified product is 95% by mol or higher. The purity is more preferably 96% by mol or higher, further preferably 97% by mol or higher, still further preferably 98% by mol or higher, particularly preferably 99% by mol or higher, most preferably 100% by mol. In the present specification, the "purity" means the abundance ratio of the substance of interest contained in a substance whose purity is to be measured in the broad sense, and means the ratio of the amount of substance of the lithium salt of the purification target based on the total amount of substance of the lithium salt of the purification target and lithium salt of specific impurities contained in the purified product in the narrow sense.

In the step (B), the purified product may be precipitated such that the content percentage of impurities in the purified product is 5% by mol or lower. The content percentage of impurities is preferably 4% by mol or lower, more preferably 3% by mol or lower, further preferably 2% by mol or lower, particularly preferably 1% by mol or lower, most preferably 0% by mol. In the present specification, the "content percentage of impurities" means the amount of substance

of lithium salt of specific impurities based on the total amount of substance of the lithium salt of the purification target and the lithium salt of specific impurities contained in the purified product.

The purity or the content percentage of impurities may be measured by use of, for example, a nuclear magnetic resonance apparatus, gas chromatography, or high-performance liquid chromatography. In the case of using high-performance liquid chromatography, the purity or the content percentage of impurities can be measured from the area ratio of a UV absorption peak. The following expression 1a represents an expression to determine the purity, and the following expression 2a represents an expression to determine the content percentage of impurities.

[Expression 1]

$$\text{Purity} = \frac{\text{UV absorption peak area of the lithium salt of the purification target} \times \text{Amount of substance per unit UV absorption peak area of the lithium salt of the purification target}}{(\text{UV absorption peak area of the lithium salt of specific impurities} \times \text{Amount of substance per unit UV absorption peak area of the lithium salt of specific impurities})} \times 100\ (\%\ \text{by mol}) \quad \cdots \text{Expression 1a}$$

[Expression 2]

$$\text{Content percentage of impurities} = \frac{\text{UV absorption peak area of the lithium salt of specific impurities} \times \text{Amount of substance per unit UV absorption peak area of the lithium salt of specific impurities}}{(\text{UV absorption peak area of the lithium salt of the purification target} \times \text{Amount of substance per unit UV absorption peak area of the lithium salt of the purification target})} \times 100\ (\%\ \text{by mol}) \quad \cdots \text{Expression 2a}$$

In this context, the amount of substance per unit UV absorption peak area is a value obtained by dividing the UV absorption peak area of the purification target or the specific impurities by the amount of substance.

In the step (B), the purified product may be precipitated such that the apparent purity of the purified product is 95% or higher. The apparent purity is more preferably 96% or higher, further preferably 97% or higher, still further preferably 98% or higher, particularly preferably 99% or higher, most preferably 100%. In the present specification, the "apparent purity" means the ratio of the UV absorption peak area at a particular wavelength of the purification target based on the total UV absorption peak area at the particular wavelength of the purification target and specific impurities measured after separation of the purified product into the purification target and the specific impurities by high-performance liquid chromatography.

In the step (B), the purified product may be precipitated such that the apparent content percentage of impurities of the purified product is 5% or lower. The apparent content percentage of impurities is preferably 4% or lower, more preferably 3% or lower, further preferably 2% or lower, particularly preferably 1% or lower, most preferably 0%. In the present specification, the "apparent content percentage of impurities" means the ratio of the UV absorption peak area at a particular wavelength of specific impurities based on the total UV absorption peak area at the particular wavelength of the purification target and the specific impurities measured after separation of the purified product into the purification target and the specific impurities by high-performance liquid chromatography.

The following expression 1b represents an expression to determine the apparent purity, and the following expression 2b represents an expression to determine the apparent content percentage of impurities.

[Expression 3]

$$\text{Apparent purity} = \frac{\text{UV absorption peak area at the particular wavelength of the lithium salt of the purification target}}{(\text{UV absorption peak area at the particular wavelength of the lithium salt of specific impurities})} \times 100\ (\%) \quad \cdots \text{Expression 1b}$$

[Expression 4]

$$\text{Apparent content percentage of impurities} = \frac{\text{UV absorption peak area at the particular wavelength of the lithium salt of specific impurities}}{(\text{UV absorption peak area at the particular wavelength of the lithium salt of the purification target})} \times 100\ (\%) \quad \cdots \text{Expression 2b}$$

In the present specification, the particular wavelength is a wavelength within ±10% of the maximum absorption wavelength exhibited by the protective group of the purification target. When a plurality of maximum absorption wavelengths are present, any maximum absorption wavelength that exhibits 30% or more absorption intensity based on absorption intensity at the largest absorption wavelength among them may be selected as the maximum absorption wavelength. The influence of noise can be reduced by selecting a longer wavelength from among a plurality of maximum absorption wavelengths. A sample concentration can be adjusted depending on absorption intensity at the selected maximum absorption wavelength. For measurement, a maximum absorption wavelength that seems most preferable from the viewpoint of easy adjustment of a sample concentration or noise reduction can be selected.

For example, UV light at 254 nm for a Fmoc group, 197 nm for a Boc group, or 210 nm for a Cbz group can be used as UV light at the particular wavelength.

In the production method according to one embodiment, the content percentage of β-alanine or a derivative thereof in the mixture to be purified can be reduced by obtaining lithium salt. In other words, the production method according to the present embodiment may comprise the step of reducing the content percentage of β-alanine or a derivative thereof.

The production method according to the present embodiment may further comprise the step of preparing a salt other than the lithium salt. Specifically, the lithium salt obtained by the production method according to one embodiment may be desalted, for example, to obtain an amino acid in a free form or a peptide compound in a free form. The desalting can be performed by a known method. Further, a salt other than the lithium salt can be produced from the obtained amino acid or peptide compound in a free form. Examples of the salt other than the lithium salt can include: salts with inorganic acids such as hydrochloric acid, hydrobromic acid, sulfuric acid, nitric acid, and phosphoric acid; salts with organic acids such as acetic acid, succinic acid, fumaric acid, maleic acid, tartaric acid, citric acid, lactic acid, stearic acid, benzoic acid, methanesulfonic acid, ethanesulfonic acid, and p-toluenesulfonic acid; salts with alkali metals (sodium or potassium); salts with alkaline earth metals such as calcium and magnesium; and ammonium salt.

The polypeptide according to the present invention can be used in a method for producing a peptide compound.

In one aspect, the method for producing a peptide compound can comprise the steps of:

(1) producing an amino acid or an amino acid derivative by the method mentioned above; and
(2) linking the amino acid to one or more selected from the group consisting of other amino acids and a peptide.

The step (2) may be performed in a conditions described in "SOLID PHASE PEPTIDE SYNTHESIS" (https://www.bachem.com/wpfd_file/solid-phase-peptide-synthesis/,2002363 published by Global Marketing, Bachem AG, December 2019).

The reaction may be performed in an aqueous vehicle or in a mixed solution of an aqueous vehicle and an organic solvent. The aqueous vehicle may be water or a buffer solution. Examples of the buffer solution include phosphate buffer solutions, CHES (N-cyclohexyl-2-aminoethanesulfonic acid), Tris (trishydroxymethylaminomethane), and Bicine (N,N-di(2-hydroxyethyl)glycine). Examples of the organic solvent include dimethyl sulfoxide.

The peptide compound obtained by the method for producing a peptide compound according to the present embodiment may be, for example, a peptide compound having a cyclic moiety (cyclic peptide compound).

The number of amino acid residues in the cyclic peptide compound according to the present embodiment may be 30 or less, 25 or less, 20 or less, 15 or less, 14 or less, 13 or less, 12 or less, 11 or less, 10 or less, 9 or less, 8 or less, 7 or less, 6 or less, 5 or less, 4 or less, 3 or less, or 2 or less. The cyclic peptide compound according to the present embodiment may be a peptide compound consisting of 2 or more, 3 or more, 4 or more, 5 or more, 6 or more, 7 or more, 8 or more, 9 or more, 10 or more, 11 or more, 12 or more, 13 or more, 14 or more, or 15 or more amino acids. The number of amino acid residues in the cyclic peptide compound according to the present embodiment may be between 2 and 10, between 2 and 8, between 2 and 6, or between 2 and 4. The number of amino acid residues in the cyclic peptide compound according to the present embodiment may be exemplarily between 8 and 20, preferably between 9 and 15, more preferably between 10 and 14, and most preferably between 11 and 13.

The "cyclic moiety" of the cyclic peptide compound means a cyclic portion formed by the linkage of two or more amino acid residues. The number of amino acid residues constituting the cyclic moiety of the cyclic peptide compound may be exemplarily between 8 and 20, preferably between 9 and 15, more preferably between 10 or and 14, and most preferably 8 and 13. The cyclic moiety is preferably formed via a covalent bond such as an amide bond, carbon-carbon bond, a S-S bond, a thioether bond, or a triazole bond, and may be formed by bonding the N-terminal group of a linear peptide compound having linked amino acid residues to the C-terminal group thereof. Specifically, the cyclic moiety can be formed, for example, by bonding the N-terminal amino group of a linear peptide compound having linked amino acid residues to the C-terminal carboxyl group thereof. The cyclization may be in any form such as cyclization through a carbon-nitrogen bond (e.g., an amide bond), cyclization through a carbon-oxygen bond (e.g., an ester bond and an ether bond), cyclization through a carbon-sulfur bond (e.g., a thioether bond), cyclization through a carbon-carbon bond, cyclization through a sulfur-sulfur bond, or cyclization by heterocyclic ring construction. Among them, cyclization via a

covalent bond such as an amide bond or a carbon-carbon bond is preferred, and cyclization via the amide bond between a side chain carboxy group and a backbone amino group is more preferred. The carboxy group and the amino group, etc. for use in cyclization may be positioned on the backbone or may be positioned on a side chain without particular limitations as long as the positions permit cyclization.

The number of ring atoms in the cyclic peptide compound according to the present embodiment may be exemplarily between 15 and 46. In the present specification, the "number of ring atoms" means the number of atoms in a cyclic compound (ring atoms) including the innermost portion of the ring. When the compound has a plurality of rings, the number of ring atoms is defined as the number of ring atoms in a ring having the largest number of ring atoms. When two rings share some atoms, a ring having a smaller number of shared atoms is used to calculate the number of ring atoms in each ring. The "number of ring atoms" will be further described with reference to specific examples. According to this method used, for example, the number of ring atoms in tetrahydrofuran (THF) is 5, and the number of ring atoms in tacrolimus (FK506) is 21.

The number of ring atoms in the cyclic peptide compound according to the present embodiment may be preferably between 34 and 46more preferably 34 and 40, and most preferably between 34 and 36. The ring atoms for use in the calculation of the number of ring atoms may be selected from the group consisting of a carbon atom, a hydrogen atom, a nitrogen atom, an oxygen atom, a sulfur atom, a phosphorus atom, and a silicon atom or may be selected from the group consisting of a carbon atom, a hydrogen atom, a nitrogen atom, and an oxygen atom.

The cyclic peptide compound according to the present embodiment may have a linear moiety in addition to the cyclic moiety. A specific aspect of the number of amino acid residues in the cyclic peptide compound is the same as that of the number of amino acid residues in the peptide compound mentioned above. When the cyclic peptide compound has a linear moiety, the total number of amino acid residues in the cyclic moiety and the linear moiety preferably falls within the presented range. When the cyclic peptide compound has a linear moiety, the number of amino acid residues constituting the cyclic moiety is exemplarily between 5 and 15, preferably between 8 and 14, more preferably between 9 and 13 and most preferably between 10 or 11, and the number of amino acid residues constituting the linear moiety is exemplarily between 1 and 8, preferably between 1 and 5, more preferably between 1 and 4, and most preferably between 1 and 3.

The molecular weight of the cyclic peptide compound according to the present embodiment is not particularly limited and may be, for example, 500 or higher, 550 or higher, 600 or higher, 650 or higher, 700 or higher, 750 or higher, 800 or higher, 850 or higher, 900 or higher, or 950 or higher and is preferably 1000 or higher, 1100 or higher, 1200 or higher, 1300 or higher, or 1400 or higher. The upper limit of the molecular weight of the peptide compound according to the present embodiment is not particularly limited and is preferably 5000 or lower, 4000 or lower, 3000 or lower, 2500 or lower, or 2000 or lower. The molecular weight of the cyclic peptide compound according to the present embodiment is exemplarily between 500 and 2000, preferably between 1000 and 1800, more preferably between 1300 and 1600, and most preferably between 1400 and 1500. In the present specification, the molecular weight means the total sum of atomic weights of atoms constituting a compound molecule (unit: "g/mol") and is obtained by calculating the total sum of atomic weights of atoms included in a molecular structural formula (unit: "g/mol"). In the present specification, the unit of the molecular weight may be omitted. The molecular weight of the peptide compound can be calculated by liquid chromatography mass spectrometry (LC/MS) described in Examples.

The cyclic peptide compound according to the present embodiment may contain one or more N-substituted amino acid residues, preferably contains at least three N-substituted amino acid residues, more preferably contains at least four N-substituted amino acid residues, and further preferably contains at least five N-substituted amino acid residues. The cyclic peptide compound according to the present embodiment may contain exemplarily between land 13 N-substituted amino acid residues, preferably contains between 1 and 12 N-substituted amino acid residues, more preferably contains between 4 and 11 N-substituted amino acid residues, and most preferably contains between 5 and 10 N-substituted amino acid residues. The N-substituted amino acid residues may be present continuously or discontinuously in the N-substituted cyclic peptide compound.

One embodiment of the present invention provides DNA encoding the polypeptide. The DNA encompasses, for example, all of genomic DNA, cDNA and DNA artificially prepared on the basis thereof. The genomic DNA contains exons and introns. Specifically, the genomic DNA may or may not contain introns and may or may not contain an untranslated region (5' UTR and/or 3' UTR), a transcriptional control region, etc. The cDNA is a nucleic acid sequence derived from a portion of intron sequences and may contain a nucleic acid sequence encoding an amino acid sequence. The DNA also includes a degenerate polynucleotide constituted by any of codons as long as the codons encode the same amino acid. The DNA according to the present embodiment may be DNA derived from a desired organism.

The DNA according to the present embodiment may be obtained by any method. The DNA also encompasses, for example, all of complementary DNA (cDNA) prepared from mRNA, DNA prepared from genomic DNA, DNA obtained by chemical synthesis, DNA obtained by PCR amplification with RNA or DNA as a template, and DNA constructed by an appropriate combination of these methods. The DNA according to the present embodiment can be prepared in accordance with a routine method, for example, by cloning the DNA from genomic DNA or RNA encoding the polypeptide,

and introducing a mutation thereto.

For example, in a method for cloning cDNA from mRNA encoding the polypeptide encoded by the DNA according to the present embodiment, mRNA encoding the polypeptide is first prepared in accordance with a routine method from arbitrary tissues or cells expressing and producing the polypeptide. This can be performed, for example, by preparing total RNA by use of a method such as guanidine thiocyanate method, thermal phenol method or AGPC (acid guanidium-phenol-chloroform), and subjecting the total RNA to affinity chromatography using oligo(dT) cellulose, poly-U-Sepharose, or the like.

Subsequently, a cDNA strand is synthesized with the obtained mRNA as a template, for example, by a method known in the art (Mil. Cell. Biol., Vol. 2, p. 161, 1982; Mol. Cell. Biol., Vol. 3, p. 280, 1983; and Gene, Vol. 25, p. 263, 1983) using reverse transcriptase or the like, and converted to double-stranded cDNA. This cDNA is integrated into a plasmid vector, a phage vector or a cosmid vector, etc. *E. coli* is transformed with the vector, or transfected with the vector after *in vitro* packaging to prepare a cDNA library.

The prepared cDNA library can be screened with the DNA according to the present embodiment or a portion thereof as a probe to obtain the gene of interest. Alternatively, the DNA according to the present embodiment may be amplified directly by PCR using the DNA or a portion thereof as primers. The sites and lengths of the probe and the primers can be appropriately determined.

One embodiment of the present invention provides a recombinant vector having the DNA. The recombinant vector is not particularly limited as long as the recombinant vector can maintain its replication or self-propagate in any host of prokaryotic cells and/or eukaryotic cells. The recombinant vector encompasses, for example, a plasmid vector, a phage vector, and a virus vector.

Examples of the vector for cloning include pUC19, λgt10, and λgt11. In the case of isolating cells capable of expressing the polypeptide according to the present embodiment within the host cells, a vector having a promoter that permits expression of the DNA according to the present embodiment is preferred.

The recombinant vector can be prepared, conveniently, by ligating the DNA encoding the polypeptide according to the present embodiment to a vector for recombination available in the art (plasmid DNA and bacteriophage DNA) in accordance with a routine method.

Examples of the vector for recombination used include *E. coli*-derived plasmids (pBR322, pBR325, pUC12, pUC13, pUC19, etc.), yeast-derived plasmids (pSH19, pSH15, etc.), and *Bacillus subtilis*-derived plasmids (pUBl10, pTP5, pC194, etc.).

Examples of the phage include bacteriophages such as λ phages as well as animal or insect viruses (e.g., pVL1393, manufactured by Invitrogen Corp.) such as retrovirus, vaccinia virus, nucleopolyhedrovirus, and lentivirus.

An expression vector is useful for the purpose of expressing the DNA encoding the polypeptide according to the present embodiment, and producing the polypeptide. The expression vector is not particularly limited as long as the expression vector has the function of allowing any host of prokaryotic cells and/or eukaryotic cells to express the DNA encoding the polypeptide and to produce the polypeptide.

Examples thereof can include pMAL C2, pEF-BOS (Nucleic Acid Research, Vol. 18, 1990, p. 5322, etc.) and pME18S (Experimental Medicine, separate volume, "Idenshi Kogaku (Genetic Engineering in English) Handbook", 1992, etc.).

In the case of using a bacterium, particularly, *E. coli,* as host cells, the recombinant vector preferably contains at least a promoter-operator region, a start codon, the DNA encoding the polypeptide, a stop codon, a terminator region and a replicable unit.

In the case of using a yeast, animal cells or insect cells as a host, the recombinant vector preferably contains at least a promoter, a start codon, the DNA encoding the polypeptide, and a stop codon.

The recombinant vector may contain DNA encoding a signal peptide, an enhancer sequence, 5' and 3' untranslated regions of the gene encoding the polypeptide, a splice junction, a polyadenylation site, a selective marker region or a replicable unit, etc.

The recombinant vector may optionally contain a marker gene (amplified gene, drug resistance gene, etc.) which enables an amplified gene-containing transformed host to be selected.

Examples of the marker gene can include dihydrofolate reductase (DHFR) gene, thymidine kinase gene, neomycin resistance gene, glutamate synthase gene, adenosine deaminase gene, omithine decarboxylase gene, hygromycin-B-phosphotransferase gene, and aspartate transcarbamylase gene.

The promoter-operator region for expressing the polypeptide in a bacterium can contain a promoter, an operator and a Shine-Dalgarno (SD) sequence (e.g., AAGG).

When the host is a bacterium of the genus *Escherichia,* examples of the promoter include Trp promoter, lac promoter, recA promoter, λPL promoter, LPP promoter, and tac promoter.

Examples of the promoter for expressing the polypeptide in a yeast include PHOS promoter, PGK promoter, GAP promoter, and ADH promoter. When the host is a bacterium of the genus *Bacillus,* examples of the promoter include SLO1 promoter, SP02 promoter, and penP promoter.

When the host is eukaryotic cells such as mammalian cells, examples of the promoter include SV40-derived promoter, retrovirus promoter, and heat shock promoter. SV40 or retrovirus is preferred. However, the promoter is not particularly limited thereto. Use of an enhancer is also an effective method for expression.

Preferred examples of the start codon include methionine codon (ATG). Examples of the stop codon include common stop codons (e.g., TAG-TGA and TM). A natural or synthetic terminator usually used can be used as the terminator region.

The replicable unit refers to DNA having the ability to replicate its total DNA sequence in host cells, and includes, for example, a natural plasmid, an artificially modified plasmid (DNA fragment prepared from a natural plasmid) and a synthetic plasmid. Preferred examples of the plasmid include: for A. *coli,* a plasmid pBR322 and artificially modified products thereof (DNA fragments obtained by treating pBR322 with appropriate restriction enzymes); for yeasts, yeast 2$\mu$ plasmid and yeast chromosomal DNA; and for mammalian cells, a plasmid pRSVneo (ATCC 37198), a plasmid pSV2dhfr (ATCC 37145), a plasmid pdBPV-MMTneo (ATCC 37224), and a plasmid pSV2neo (ATCC 37149).

As for the enhancer sequence, the polyadenylation site and the splice junction site, for example, ones usually used by those skilled in the art can be used, such as those derived from SV40.

The expression vector can be prepared by linking at least the promoter, the start codon, the DNA encoding the polypeptide, the stop codon and the terminator region mentioned above to an appropriate replicable unit in a continuous and circular manner. In this respect, an appropriate DNA fragment (e.g., a linker and an additional restriction site) can be used, if desired, by a routine method such as digestion with a restriction enzyme or ligation using T4 DNA ligase.

One embodiment of the present invention provides a transformant having the DNA encoding the polypeptide. The transformant can be obtained as a transformed recombinant cell, for example, by transferring the expression vector mentioned above to a host cell.

The host cell is not particularly limited as long as the host cell is compatible with the expression vector and can be transformed therewith. Examples thereof include various cells such as natural cells and artificially established recombinant cells usually used in the technical field of the present invention, for example, bacteria (bacteria of the genus *Escherichia,* bacteria of the genus *Bacillus,* etc.), yeasts (the genus *Saccharomyces,* the genus *Pichia,* etc.), animal cells and insect cells.

The host cell is preferably *E. coli* or an animal cell. Examples of the *E. coli* or the animal cell include *E. coli* (DH5a, TB1, HB101, etc.), mouse-derived cells (Cop, L, C127, Sp2/0, NS-1, NIH3T3, etc.), rat-derived cells (PC12, PC12h, etc.), hamster-derived cells (BHK, CHO, etc.), monkey-derived cells (COS1, COS3, COS7, CV1, Velo, etc.) and human-derived cells (Hela, cells derived from diploid fibroblasts, myeloma cells, HepG2, etc.).

The transfer of the expression vector to the host cell (transformation (transfection)) can be performed in accordance with a routine method ([in the case of *E. coli, Bacillus subtilis,* etc.]: Proc. Natl. Acad. Sci. USA., Vol. 69, p. 2110, 1972; Mil. Gen. Genet., Vol. 168, p. 111, 1979; and J. Mol. Biol., Vol. 56, p. 209, 1971; [in the case of *Saccharomyces cerevisiae]:* Proc. Natl. Acad. Sci. USA., Vol. 75, p. 1927, 1978; and J. Bacteriol., Vol. 153, p. 163,1983); [in the case of animal cells]: Virology, Vol. 52, p. 456, 1973; [in the case of insect cells]: Mol. Cell. Biol., Vol. 3, p. 2156-2165, 1983).

The polypeptide according to the present embodiment can be produced by, for example, a method comprising: culturing a recombinant cell which is the transformant in accordance with a routine method; and obtaining the polypeptide of interest from the cultures thus obtained by culture.

An isolation or purification method usually used can be used as a method for obtaining the polypeptide of interest from the cultures. Examples of the isolation or purification method include: methods exploiting solubility, such as salting out and solvent precipitation; methods exploiting difference in molecular weight, such as dialysis, ultrafiltration, gel filtration, and sodium dodecyl sulfate-polyacrylamide gel electrophoresis; methods exploiting electric charge, such as ion-exchange chromatography and hydroxylapatite chromatography; methods exploiting specific affinity, such as affinity chromatography; methods exploiting difference in hydrophobicity, such as reverse-phase high-performance liquid chromatography; and methods exploiting difference in isoelectric point, such as isoelectric focusing electrophoresis.

When the polypeptide of interest is present in the periplasm or cytoplasm of the cultured recombinant cell *(E. coli,* etc.), the cultures are subjected to a routine method such as filtration or centrifugation, bacterial cells or cells are collected and suspended in an appropriate buffer solution, and the cell walls and/or cell membranes of the cells, etc. are disrupted by a method, for example, ultrasonic wave, lysozyme, freezing and thawing, followed by a method such as centrifugation or filtration to obtain a fraction containing the polypeptide of interest. The fraction is solubilized using a surfactant such as Triton(TM)- X100 to obtain a crude solution. Then, the polypeptide of interest can be isolated or purified from the crude solution by use of the routine method given above.

The polypeptide according to the present embodiment can be prepared by the gene recombination technique known in the art as mentioned above and, in general, can be prepared as follows: a site-directed mutation is introduced to a particular position and a particular amino acid by PCR with a plasmid containing the wild-type polypeptide gene as a template using primers therefor, and the template plasmid is digested with a restriction enzyme, followed by the transformation of *E. coli,* etc. to clone a plasmid harboring the mutation of interest.

In the case of introducing an amino acid mutation to the second amino acid, site-directed mutagenesis with the

plasmid harboring the mutation at the particular site as a template using primers is subsequently repeated in the same manner as above to construct plasmid DNA encoding a 2-amino acid substitution mutant. The further introduction of an amino acid mutation can also be performed in the same manner as above.

An *E. coli* BL21 strain or the like is cotransformed with the prepared DNA and, for example, a plasmid pREP4 encoding lac repressor (Laci). The obtained transformed strain is isolated and cultured, followed by the induction of expression with IPTG. The obtained bacterial strain is then disrupted, and the polypeptide of interest is purified, for example, by applying a supernatant to an affinity column through the use of His tag.

Alternatively, the polypeptide can also be prepared by the following method: a gene having a nucleotide sequence encoding the polypeptide of interest is synthesized, and the nucleotide sequence is transferred to an expression vector, followed by protein expression and the purification of the polypeptide of interest using an affinity column through the use of various purification tags.

The method for producing the polypeptide according to the present embodiment is not limited to the methods mentioned above. Various gene manipulation techniques, such as point mutation techniques, gene synthesis techniques, and methods for introducing variant fragments using restriction enzymes, known in the art can be used. The expression is not limited by *E. coli,* and animal cells or a cell-free translation system may be used. The purification method is not limited by an affinity column using polyhistidine, and various peptide tags or purification columns can be used.

[Examples]

The present invention will be further illustrated with reference to Examples given below and however, is not limited by these examples.

In Examples, the following abbreviations were used.

[Table 1]

| Abbreviation | Formal name |
|---|---|
| F A | Formic acid |
| β - N A D P H | Reduced nicotinamide adenine dinucleotide phosphate |
| H E P E S | 4-(2-Hydroxyethyl)-1-piperazine ethanesulfonic acid |
| 1 x T N G | 50 mM Tris (trishydroxymethylaminomethane), 150 mM sodium chloride, 10% (v/v) glycerol |
| G D H | Glucose dehydrogenase |
| P V D F | Polyvinylidene fluoride |
| P T F E | Ethylene tetrafluoride |

[Table 2]

| Abbreviation | Structural formula |
|---|---|
| **Fmoc-MeGly(cPent)-OH** | 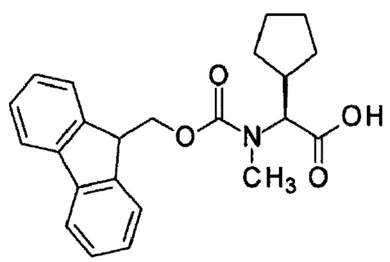 |
| **H-MeGly(cPent) -OH** | 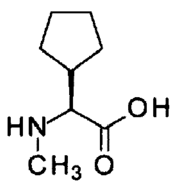 |

(continued)

| Abbreviation | Structural formula |
|---|---|
| **H-EtPhe-OH** | 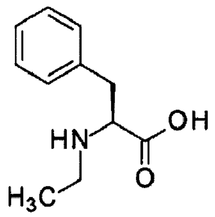 |
| **H-EtPhe(4-Me)-OH** | 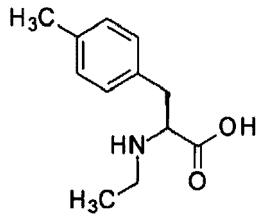 |
| **H-MePhe-OH** | 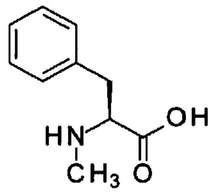 |
| **Phe** | 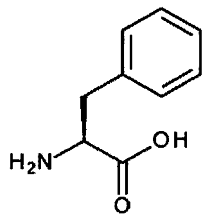 |

The analysis conditions of LCMS are as described below.

[Table 3]

| Condition name | Apparatus | Column (I.D. × length (mm)) | Mobile phase | Gradient (A/B) | Flow rate (ml/min) | Column temperature (°C) | Wavelength |
|---|---|---|---|---|---|---|---|
| FA 05-1 | Acquity UPLC/ SQD | Aldrich Ascentis Express C18 (2.1 x 50) | A) 0.1% FA, $H_2O$ B) 0.1% FA $CH_3CN$ | 95/5~0/100 (1.0 min) => 0/100 (0.4 min) | 0.9 | 35 | 210-400 nm PDA total |
| FA 05-2 | Acquity UPLC/ SQD | Aldrich Ascentis Express C18 (2.1 x 50) | A) 0.1% FA, $H_2O$ B) 0.1% FA $CH_3CN$ | 95/5~80/20 (0.8 min) => 80/20~0/100 (0.2 min) => 0/100 (0.4 min) | 0.9 | 35 | 210-400 nm PDA total |

(continued)

| Condition name | Apparatus | Column (I.D. × length (mm)) | Mobile phase | Gradient (A/B) | Flow rate (ml/min) | Column temperature (°C) | Wavelength |
|---|---|---|---|---|---|---|---|
| HILIC | Acquity UPLC/ SQD | Intrada amino acid, 3 um (3.0 x 50) | A) 50 mM ammonium formate, $H_2O$ B) 0.1% FA $CH_3CN$/50 mM ammonium formate, $H_2O$ (86:14) | 0/100 (3 min) => 0/100~100/ 0 (0.5 min) => 100/0 (0.9 min) | 0.6 | 35 | 210-400 nm PDA total |

Reagents whose distributor was not particularly described were purchased from any of Sigma-Aldrich Japan G.K., FUJIFILM Wako Pure Chemical Corp., Tokyo Chemical Industry Co., Ltd. (TCI), Nacalai Tesque, Inc., and Watanabe Chemical Industries, Ltd.

Comparative Example 1: Expression and purification of wild-type enzyme

A gene of a wild-type enzyme sequence (SEQ ID NO: 1) C-terminally tagged with a streptavidin-binding peptide tag sequence (GTDEKTTGWRGGHVVEGLAGELEQLRARLEHHPQ) and a His tag sequence (HHHHHH) was synthesized and cloned into a vector for expression in *E. coli.* This expression vector was transferred to a BL21 (DE3) *E. coli* strain (Novagen), which was then cultured at 18°C for 2 days using Overnight Express Instant TB Medium (Novagen) to express the protein of interest.

The obtained bacterial cells were recovered by centrifugation, and the bacterial cells were ultrasonically disrupted. The lysate was fractionated by centrifugation, and a supernatant fraction was purified by affinity chromatography using Ni Sepharose 6 Fast Flow (Cytiva). A fraction containing the protein of interest was recovered and used as a final preparation (#02-001 (SEQ ID NO: 17)).

Example 1: Expression and purification of variant enzyme

A gene of each variant enzyme sequence (SEQ ID NOs: 2 to 13 and 23 to 35) C-terminally tagged with a streptavidin-binding peptide tag sequence (GTDEKTTGWRGGHVVEGLAGELEQLRARLEHHPQ), linker sequence (GGS), and a His tag sequence (HHHHHH) was synthesized and cloned into a vector for expression in *E. coli.* This expression vector was transferred to a BL21 (DE3) *E. coli* strain (Novagen), which was then cultured at 18°C for 2 days using Overnight Express Instant TB Medium (Novagen) to express the protein of interest.

The obtained bacterial cells were recovered by centrifugation, and the bacterial cells were ultrasonically disrupted. The lysate was fractionated by centrifugation, and a supernatant fraction was purified by affinity chromatography using Ni Sepharose 6 Fast Flow (Cytiva). A fraction containing the protein of interest was recovered and used as a final preparation (#02-002 to -217, #03-001 to -130 and #04-001 to -0072).

Example 2: Expression and purification of variant enzyme

The same method as in Example 1 was carried out (#02-169) except that: a supernatant fraction was purified by affinity chromatography using cOmplete™ His-Tag Purification Resin (Roche) instead of Ni Sepharose 6 Fast Flow (Cytiva); and after recovery of a fraction containing the protein of interest, the sample was dialyzed against 50 mM Tris-hydrochloric acid (pH 8.0)/150 mM sodium chloride/10% glycerol and used as a final preparation.

Synthesis Example 1: Synthesis of H-MeGly(cPent)-OH

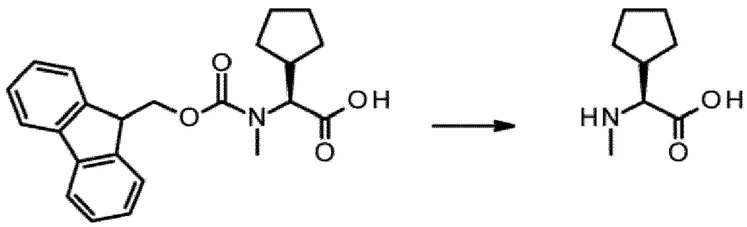

4-(3-Phenylpropyl)piperidine (TCI, cat. No. P0760) (0.419 ml, 1.977 mmol) was added to a suspension of Fmoc-MeGly(cPent)-OH (CAS No. 187475-29-2) (250 mg, 0.659 mmol) in dichloromethane (Kanto Chemical Co., Inc., cat. No. 11338-25) (1.0 ml), and the mixture was stirred at room temperature for 1 minute. Then, water (1.0 ml) was added thereto, and the mixture was then stirred overnight at room temperature. An aqueous layer in the reaction solution was separated and purified by reverse-phase silica gel column chromatography (0.1% aqueous formic acid solution/0.1% solution of formic acid in acetonitrile = 100/0 -> 95/5) to give H-MeGly(cPent)-OH (88.2 mg, 85%).

LCMS (ESI) m/z = 158.1 $(M + H)^+$
Retention time: 0.25 min (analysis condition FA05-1)
$^1$H-NMR (JNM-ECZ400S, 400 MHz, $D_2O$) δppm 3.28 (1H, d, 7.6 Hz), 2.53 (3H, s), 2.07 (1H, m), 1.69 (1H, m), 1.60-1.38 (5H, m), 1.22 (2H, m)

Evaluation Example 1: Screening of variant enzyme with H-MeGly(cPent)-OH synthetic activity as index

In order to screen for each variant enzyme (#02-002 to -217) prepared in Example 1 with H-MeGly(cPent)-OH synthetic activity as an index, a solution containing substrates (hereinafter, referred to as premix 1) was prepared. Specifically, two compounds, 2-cyclopentyl-2-oxo-acetic acid (purchased from Enamine Ltd.) and D(+)-glucose (purchased from FUJIFILM Wako Pure Chemical Corp.), were dissolved in a mixed solvent of an aqueous methylamine solution (an aqueous solution purchased from FUJIFILM Wako Pure Chemical Corp. was adjusted to pH 8.5 with hydrochloric acid) and a phosphate buffer solution (400 mM, pH 7.4). The pH of the solution was adjusted to 8.0 using an aqueous sodium hydroxide solution, and the resultant was then diluted with ultrapure water. An aqueous β-NADPH solution (a powder purchased from Oriental Yeast Co., Ltd. was dissolved at 89 mM in ultrapure water) and a GDH solution (a powder purchased from FUJIFILM Wako Pure Chemical Corp. was dissolved in 1 × TNG) were added to this solution to give premix 1. The concentration of each compound in the premix 1 was 55.6 mM 2-cyclopentyl-2-oxo-acetic acid, 111 mM D(+)-glucose, 556 mM methylamine, a 111 mM phosphate buffer solution, 0.99 mM β-NADPH, and a 0.0022 units/μL GDH solution.

Any one of #02-001 to -217 was added to the prepared premix 1 to carry out H-MeGly(cPent)-OH synthesis reaction. Specifically, a solution of any one of #02-001 to -217 was added to the premix 1. For a negative control enzyme(-), an imidazole-containing buffer solution (250 mM imidazole, 50 mM HEPES (pH 7.5), 150 mM sodium chloride) was added thereto. The resultant was well mixed and then incubated at 37°C for 19 hours. The concentration of each compound at the start of reaction was 50 mM 2-cyclopentyl-2-oxo-acetic acid, 100 mM D(+)-glucose, 500 mM methylamine, a 100 mM phosphate buffer solution, 0.89 mM β-NADPH, a 0.002 units/μL GDH solution, and 2.5 μM of any one of #02-001 to -217.

The reaction solution thus incubated was aftertreated to prepare a sample for LCMS analysis. Specifically, hydrochloric acid was added in 9 times the amount of the reaction solution such that the final concentration was 30 mM after the addition of hydrogen chloride (hereinafter, this solution is referred to as aftertreated reaction solution 1).

Serial dilutions of H-MeGly(cPent)-OH synthesized in Synthesis Example 1 were prepared for the purpose of quantifying the concentration of H-MeGly(cPent)-OH dissolved in the aftertreated reaction solution 1 by LCMS. Specifically, H-MeGly(cPent)-OH was dissolved at 1000 mM in a 1 M aqueous sodium hydroxide solution. Then, a two-fold dilution operation with ultrapure water was repeated.

The quantification of the concentration using the serial dilutions was difficult due to ion suppression ascribable to various compounds dissolved in the aftertreated reaction solution 1. Therefore, the serial dilutions were added to a solution that mimicked the composition of the aftertreated reaction solution 1. First, two compounds, 2-cyclopentyl-2-oxo-acetic acid and D(+)-glucose, were dissolved in a mixed solvent of an aqueous methylamine solution (pH 8.3) and a phosphate buffer solution. The pH of the solution was adjusted to 8.0 using an aqueous sodium hydroxide solution and hydrochloric acid, and the resultant was then diluted with ultrapure water (hereinafter, this solution is referred to as premix 2). Next, an aqueous β-NADPH solution, 1 × TNG, an imidazole-containing buffer solution, and the serial dilutions were added to the premix 2 and well mixed. The concentration of each compound at this point in time was 25 mM 2-cyclopentyl-2-oxo-acetic acid, 75 mM D(+)-glucose, 475 mM methylamine, a 100 mM phosphate buffer solution, and

0.89 mM β-NADPH, and the 1 × TNG and the imidazole-containing buffer solution each had a volume ratio of 10%. Finally, hydrochloric acid was added in 9 times the amount of the reaction solution such that the final concentration was 30 mM after the addition of hydrogen chloride to obtain a calibration curve sample for LCMS analysis (hereinafter, this solution is referred to as aftertreated calibration curve sample 1).

A dilution operation was performed in consideration of the possibility that H-MeGly(cPent)-OH was dissolved, at a level exceeding the calibration curve range for the aftertreated calibration curve sample 1, in the aftertreated reaction solution 1. First, a solution for dilution that mimicked the composition of various compounds dissolved in the aftertreated reaction solution 1 was prepared as compensation for the ion suppression mentioned above. Specifically, the premix 2, 1 × TNG, an imidazole-containing buffer solution, an aqueous β-NADPH solution, and hydrochloric acid were mixed and diluted with ultrapure water. The concentrations of β-NADPH and hydrogen chloride in the solution thus diluted were 0.089 mM and 30 mM, respectively, and the premix 2, the 1 × TNG, and the imidazole-containing buffer solution had final volume ratios of 6%, 1%, and 1%, respectively. The aftertreated reaction solution 1 was diluted 32-fold with this solution for dilution to obtain aftertreated reaction solution 2.

All of the aftertreated reaction solution 1, the aftertreated reaction solution 2, and the aftertreated calibration curve sample 1 were passed through a 0.2 μm PVDF membrane filter (purchased from Corning, Inc.) and then subjected to LCMS measurement. The liquid volume used in measurement was 1 μL. The function (TargetLynx) of detecting peak areas of extracted ion chromatograms, on MassLynx was used to evaluate the synthetic activity of each of #02-001 to -217 against H-MeGly(cPent)-OH. The results are as described in the tables given below. A plurality of variant enzymes having higher H-MeGly(cPent)-OH synthetic activity than that of the wild-type enzyme (#02-001) were able to be found.

LCMS (ESI) m/z = 158.10 $(M + H)^+$
Retention time: 0.23 min (analysis condition FA05-1)

The synthetic activity was determined by dividing the yield of the target product obtained using each of #02-001 to -217 by the yield of the target product obtained using the wild-type enzyme (#02-001). The calculated ratios of the synthetic activity are shown in Tables 4 and 5. The yield was 21% when the wild-type enzyme was used.

[Table 4]

| No. | Name | Activity ratio (fold) |
|---|---|---|
| **Enzyme(-)** | **Enzyme(-)** | **0.00** |
| **#02-001** | **WT** | **1.00** |
| **#02-002** | **H44A** | **0.00** |
| **#02-003** | **H44D** | **0.00** |
| **#02-004** | **H44E** | **0.01** |
| **#02-005** | **H44F** | **0.00** |
| **#02-006** | **H44G** | **0.00** |
| **#02-007** | **H44I** | **0.00** |
| **#02-008** | **H44K** | **0.00** |
| **#02-009** | **H44L** | **0.00** |
| **#02-010** | **H44M** | **0.00** |
| **#02-011** | **H44N** | **0.01** |
| **#02-013** | **H44Q** | **0.00** |
| **#02-014** | **H44R** | **0.00** |
| **#02-015** | **H44S** | **0.04** |
| **#02-016** | **H44T** | **0.00** |
| **#02-017** | **H44V** | **0.00** |
| **#02-018** | **H44W** | **0.00** |
| **#02-019** | **H44Y** | **0.00** |

(continued)

| No. | Name | Activity ratio (fold) |
|---|---|---|
| #02-020 | R48A | 0.02 |
| #02-021 | R48D | 0.00 |
| #02-022 | R48E | 0.00 |
| #02-023 | R48F | 0.00 |
| #02-024 | R48G | 0.03 |
| #02-025 | R48H | 0.04 |
| #02-026 | R48I | 0.00 |
| #02-027 | R48K | 0.01 |
| #02-028 | R48L | 0.01 |
| #02-029 | R48M | 0.02 |
| #02-030 | R48N | 0.03 |
| #02-031 | R48P | 0.00 |
| #02-032 | R48Q | 0.08 |
| #02-033 | R48S | 0.02 |
| #02-034 | R48T | 0.03 |
| #02-035 | R48V | 0.01 |
| #02-036 | R48W | 0.00 |
| #02-037 | R48Y | 0.03 |
| #02-038 | F117A | 0.08 |
| #02-039 | F117D | 0.00 |
| #02-040 | F117E | 0.00 |
| #02-041 | F117G | 0.03 |
| #02-042 | F117H | 0.07 |
| #02-043 | F117I | 0.28 |
| #02-044 | F117K | 0.04 |
| #02-045 | F117L | 1.33 |
| #02-046 | F117M | 0.20 |
| #02-047 | F117N | 0.24 |
| #02-048 | F117P | 0.00 |
| #02-049 | F117Q | 0.09 |
| #02-051 | F117S | 0.06 |
| #02-052 | F117T | 0.10 |
| #02-053 | F117V | 0.10 |
| #02-054 | F117W | 0.14 |
| #02-055 | F117Y | 0.51 |
| #02-057 | S140D | 0.00 |
| #02-058 | S140E | 0.00 |
| #02-059 | S140F | 0.00 |

(continued)

| No. | Name | Activity ratio (fold) |
|---|---|---|
| #02-060 | S140G | 0.04 |
| #02-061 | S140H | 0.00 |
| #02-062 | S140I | 0.00 |
| #02-063 | S140K | 0.00 |
| #02-064 | S140L | 0.00 |
| #02-065 | S140M | 0.00 |
| #02-066 | S140N | 0.24 |
| #02-067 | S140P | 0.00 |
| #02-068 | S140Q | 0.00 |
| #02-069 | S140R | 0.00 |
| #02-070 | S140T | 0.03 |
| #02-071 | S140V | 0.00 |
| #02-072 | S140W | 0.00 |
| #02-073 | S140Y | 0.00 |
| #02-074 | M141A | 2.47 |
| #02-075 | M141D | 0.13 |
| #02-076 | M141E | 0.13 |
| #02-077 | M141F | 0.10 |
| #02-078 | M141G | 0.64 |
| #02-079 | M141H | 0.26 |
| #02-080 | M141I | 0.98 |
| #02-081 | M141K | 2.01 |
| #02-082 | M141L | 1.55 |
| #02-083 | M141N | 0.22 |
| #02-084 | M141P | 0.13 |
| #02-085 | M141Q | 0.59 |
| #02-086 | M141R | 1.61 |
| #02-087 | M141S | 1.84 |
| #02-088 | M141T | 0.86 |
| #02-089 | M141V | 1.59 |
| #02-090 | M141W | 1.88 |
| #02-091 | M141Y | 1.20 |
| #02-092 | T156A | 0.03 |
| #02-093 | T156D | 0.00 |
| #02-094 | T156E | 0.00 |
| #02-095 | T156F | 0.00 |
| #02-096 | T156G | 0.00 |
| #02-097 | T156H | 0.00 |

(continued)

| No. | Name | Activity ratio (fold) |
|---|---|---|
| #02-098 | T156I | 0.00 |
| #02-099 | T156K | 0.00 |
| #02-100 | T156L | 0.00 |
| #02-101 | T156M | 0.00 |
| #02-102 | T156N | 0.03 |
| #02-103 | T156P | 0.05 |
| #02-104 | T156Q | 0.00 |
| #02-105 | T156R | 0.00 |
| #02-106 | T156S | 0.27 |
| #02-107 | T156V | 0.00 |
| #02-108 | T156W | 0.00 |
| #02-109 | T156Y | 0.00 |

[Table 5]

| No. | Name | Activity ratio (fold) |
|---|---|---|
| #02-110 | H182A | 0.26 |
| #02-111 | H182D | 0.04 |
| #02-112 | H182E | 0.55 |
| #02-113 | H182F | 0.26 |
| #02-114 | H182G | 0.07 |
| #02-115 | H182I | 0.1 3 |
| #02-116 | H182K | 0.48 |
| #02-117 | H182L | 0.93 |
| #02-118 | H182M | 0.32 |
| #02-119 | H182N | 0.09 |
| #02-120 | H182P | 0.00 |
| #02-121 | H182Q | 1.76 |
| #02-122 | H182R | 0.78 |
| #02-123 | H182S | 0.09 |
| #02-124 | H182T | 0.05 |
| #02-125 | H182V | 0.1 9 |
| #02-126 | H182W | 0.20 |
| #02-127 | H182Y | 0.25 |
| #02-128 | G183A | 0.01 |
| #02-129 | G183D | 0.00 |
| #02-1 30 | G183E | 0.00 |
| #02-131 | G183F | 0.00 |

(continued)

| No. | Name | Activity ratio (fold) |
|---|---|---|
| #02-132 | G183H | 0.00 |
| #02-133 | G183I | 0.00 |
| #02-1 34 | G183K | 0.00 |
| #02-135 | G183L | 0.00 |
| #02-136 | G183M | 0.00 |
| #02-137 | G183N | 0.00 |
| #02-138 | G183P | 0.00 |
| #02-139 | G183Q | 0.00 |
| #02-140 | G183R | 0.00 |
| #02-141 | G183S | 0.00 |
| #02-142 | G183T | 0.00 |
| #02-143 | G183V | 0.00 |
| #02-144 | G183W | 0.00 |
| #02-145 | G183Y | 0.00 |
| #02-146 | Q186A | 0.26 |
| #02-147 | Q186D | 0.14 |
| #02-148 | Q186E | 0.63 |
| #02-149 | Q186F | 0.08 |
| #02-150 | Q186G | 0.06 |
| #02-151 | Q186H | 0.08 |
| #02-152 | Q186I | 0.15 |
| #02-153 | Q186K | 0.22 |
| #02-154 | Q186L | 0.20 |
| #02-155 | Q186M | 0.23 |
| #02-156 | Q186N | 0.06 |
| #02-157 | Q186P | 0.00 |
| #02-158 | Q186R | 0.14 |
| #02-159 | Q186S | 0.12 |
| #02-160 | Q186T | 0.19 |
| #02-161 | Q186V | 0.28 |
| #02-162 | Q186W | 0.07 |
| #02-163 | Q186Y | 0.07 |
| #02-164 | W253A | 1.18 |
| #02-165 | W253D | 0.25 |
| #02-166 | W253E | 0.19 |
| #02-167 | W253F | 1.95 |
| #02-168 | W253G | 0.69 |
| #02-169 | W253H | 3.07 |

(continued)

| No. | Name | Activity ratio (fold) |
|---|---|---|
| #02-170 | W253I | 2.00 |
| #02-171 | W253K | 1.14 |
| #02-172 | W253L | 2.17 |
| #02-173 | W253M | 1.62 |
| #02-174 | W253N | 1.00 |
| #02-175 | W253P | 1.93 |
| #02-176 | W253Q | 1.77 |
| #02-177 | W253R | 1.44 |
| #02-178 | W253S | 1.30 |
| #02-179 | W253T | 1.67 |
| #02-180 | W253V | 1.96 |
| #02-181 | W253Y | 1.11 |
| #02-182 | K260A | 0.95 |
| #02-183 | K260D | 0.23 |
| #02-184 | K260E | 1.20 |
| #02-1 85 | K260F | 0.97 |
| #02-186 | K260G | 0.85 |
| #02-187 | K260H | 0.91 |
| #02-188 | K260I | 0.20 |
| #02-189 | K260L | 0.47 |
| #02-190 | K260M | 0.50 |
| #02-191 | K260N | 0.77 |
| #02-192 | K260P | 0.11 |
| #02-193 | K260Q | 0.93 |
| #02-194 | K260R | 0.76 |
| #02-195 | K260S | 1.02 |
| #02-196 | K260T | 0.93 |
| #02-197 | K260V | 0.63 |
| #02-198 | K260W | 0.43 |
| #02-199 | K260Y | 0.66 |
| #02-200 | P262A | 0.31 |
| #02-201 | P262D | 0.01 |
| #02-202 | P262E | 0.03 |
| #02-203 | P262F | 0.00 |
| #02-204 | P262G | 0.05 |
| #02-205 | P262H | 0.00 |
| #02-206 | P262I | 0.07 |
| #02-207 | P262K | 0.09 |

(continued)

| No. | Name | Activity ratio (fold) |
|---|---|---|
| **#02-208** | **P262L** | **0.05** |
| **#02-209** | **P262M** | **0.03** |
| **#02-210** | **P262N** | **0.00** |
| **#02-211** | **P262Q** | **0.04** |
| **#02-212** | **P262R** | **0.04** |
| **#02-213** | **P262S** | **0.02** |
| **#02-214** | **P262T** | **0.04** |
| **#02-215** | **P262V** | **0.05** |
| **#02-216** | **P262W** | **0.01** |
| **#02-217** | **P262Y** | **0.00** |

Evaluation Example 2: Screening of variant enzyme with H-EtPhe-OH synthetic activity as index(Single mutant screening)

In order to screen for each variant enzyme (#02-002 to -217) prepared in Example 1 with H-EtPhe-OH synthetic activity as an index, a solution containing substrates (hereinafter, referred to as premix 3) was prepared. Specifically, two compounds, phenylpyruvic acid sodium salt (purchased from Sigma-Aldrich Japan G.K.) and D(+)-glucose, were dissolved in a mixed solvent of an aqueous ethylamine solution (an aqueous solution purchased from Tokyo Chemical Industry Co., Ltd. was adjusted to pH 7.7 with hydrochloric acid) and a phosphate buffer solution. The pH of the solution was adjusted to 8.0 using an aqueous sodium hydroxide solution and hydrochloric acid, and the resultant was then diluted with ultrapure water. An aqueous β-NADPH solution and a GDH solution were added to this solution to give premix 3. The concentration of each compound in the premix 3 was 55.6 mM phenylpyruvic acid sodium salt, 111 mM D(+)-glucose, 556 mM ethylamine, a 111 mM phosphate buffer solution, 0.99 mM β-NADPH, and a 0.0022 units/μL GDH solution.

Any one of #02-001 to -217 was added to the prepared premix 3 to carry out H-EtPhe-OH synthesis reaction. Specifically, a solution of any one of #02-001 to -217 was added to the premix 3. For a negative control enzyme(-), an imidazole-containing buffer solution was added thereto. The resultant was well mixed and then incubated at 37°C for 19 hours. The concentration of each compound at the start of reaction was 50 mM phenylpyruvic acid sodium salt, 100 mM D(+)-glucose, 500 mM ethylamine, a 100 mM phosphate buffer solution, 0.89 mM β-NADPH, a 0.002 units/μL GDH solution, and 2.5 μM of any one of #02-001 to -217.

The reaction solution thus incubated was aftertreated to prepare a sample for LCMS analysis. Specifically, hydrochloric acid was added in 9 times the amount of the reaction solution such that the final concentration was 30 mM after the addition of hydrogen chloride (hereinafter, this solution is referred to as aftertreated reaction solution 3).

Serial dilutions of H-MePhe-OH presumed to have a molar absorbance coefficient equivalent to that of H-EtPhe-OH were prepared for the purpose of quantifying the concentration of H-EtPhe-OH dissolved in the aftertreated reaction solution 3 by LCMS. Specifically, H-MePhe-OH (purchased from Watanabe Chemical Industries, Ltd.) was dissolved at 500 mM in a 1 M aqueous potassium hydroxide solution. Then, a two-fold dilution operation with ultrapure water was repeated. The serial dilutions were added to a mixed solvent containing a phosphate buffer solution, 1 × TNG, and an imidazole-containing buffer solution to prepare a calibration curve sample. The concentration of the phosphate buffer solution at this point in time was 100 mM (pH 7.4), and the 1 × TNG, the imidazole-containing buffer solution, and the H-MePhe-OH serial dilutions had volume ratios of 10%, 10%, and 20%, respectively, with ultrapure water constituting the volume balance. Finally, hydrochloric acid was added in 9 times the amount of the reaction solution such that the final concentration was 30 mM after the addition of hydrogen chloride to give a calibration curve sample for LCMS analysis (hereinafter, this solution is referred to as aftertreated calibration curve sample 2).

All of the aftertreated reaction solution 3 and the aftertreated calibration curve sample 2 were passed through a 0.2 μm PVDF membrane filter (purchased from Corning, Inc.) and then subjected to LCMS measurement. The liquid volume used in measurement was 1 μL. The area of a peak derived from H-EtPhe-OH on a UV chart (254 nm) was detected using TargetLynx to evaluate the synthetic activity of each of #02-001 to -217 against H-EtPhe-OH. The results are as described in the tables given below. A plurality of variant enzymes having higher H-EtPhe-OH synthetic activity than that of the wild-type enzyme (#02-001) were able to be found.

LCMS (ESI) m/z = 194.18 $(M + H)^+$
Retention time: 0.37 min (analysis condition FA05-2; retention time in the UV chart (254 nm))

The synthetic activity was determined by dividing the yield of the target product obtained using each of #02-001 to -217 by the yield of the target product obtained using the wild-type enzyme (#02-001). The calculated ratios of the synthetic activity are shown in Tables 6 and 7. The yields of the wild-type enzymes were 22% for those corresponding to #02-193 to -200 and 28% for others.

[Table 6]

| No. | Name | Activity ratio (fold) |
|---|---|---|
| Enzyme (-) | Enzyme (-) | 0.00 |
| #02-001 | WT | 1.00 |
| #02-002 | H44A | 0.00 |
| #02-003 | H44D | 0.00 |
| #02-004 | H44E | 0.00 |
| #02-005 | H44F | 0.00 |
| #02-006 | H44G | 0.00 |
| #02-007 | H44I | 0.24 |
| #02-008 | H44K | 0.00 |
| #02-009 | H44L | 0.14 |
| #02-010 | H44M | 0.47 |
| #02-011 | H44N | 0.15 |
| #02-013 | H44Q | 0.00 |
| #02-014 | H44R | 0.00 |
| #02-015 | H44S | 0.18 |
| #02-016 | H44T | 0.21 |
| #02-017 | H44V | 0.22 |
| #02-018 | H44W | 0.00 |
| #02-019 | H44Y | 0.00 |
| #02-020 | R48A | 0.10 |
| #02-021 | R48D | 0.00 |
| #02-022 | R48E | 0.00 |
| #02-023 | R48F | 0.00 |
| #02-024 | R48G | 0.14 |
| #02-025 | R48H | 0.06 |
| #02-026 | R48I | 0.05 |
| #02-027 | R48K | 0.00 |
| #02-028 | R48L | 0.18 |
| #02-029 | R48M | 0.16 |
| #02-030 | R48N | 0.13 |
| #02-032 | R48Q | 0.19 |
| #02-033 | R48S | 0.11 |

(continued)

| No. | Name | Activity ratio (fold) |
|---|---|---|
| #02-034 | R48T | 0.25 |
| #02-035 | R48V | 0.22 |
| #02-036 | R48W | 0.00 |
| #02-038 | F117A | 0.66 |
| #02-039 | F117D | 0.00 |
| #02-040 | F117E | 0.00 |
| #02-041 | F117G | 0.53 |
| #02-042 | F117H | 0.34 |
| #02-045 | F117L | 0.79 |
| #02-046 | F117M | 0.96 |
| #02-047 | F117N | 0.64 |
| #02-048 | F117P | 0.00 |
| #02-049 | F117Q | 0.30 |
| #02-052 | F117T | 0.15 |
| #02-053 | F117V | 0.27 |
| #02-054 | F117W | 0.08 |
| #02-055 | F117Y | 0.36 |
| #02-057 | S140D | 0.00 |
| #02-058 | S140E | 0.00 |
| #02-059 | S140F | 0.00 |
| #02-060 | S140G | 0.00 |
| #02-061 | S140H | 0.00 |
| #02-062 | S140I | 0.00 |
| #02-063 | S140K | 0.00 |
| #02-064 | S140L | 0.00 |
| #02-065 | S140M | 0.00 |
| #02-066 | S140N | 0.14 |
| #02-067 | S140P | 0.00 |
| #02-068 | S140Q | 0.00 |
| #02-069 | S140R | 0.00 |
| #02-070 | S140T | 0.00 |
| #02-071 | S140V | 0.00 |
| #02-072 | S140W | 0.00 |
| #02-073 | S140Y | 0.00 |
| #02-074 | M141A | 1.57 |
| #02-075 | M141D | 0.00 |
| #02-076 | M141E | 0.05 |
| #02-077 | M141F | 0.83 |

(continued)

| No. | Name | Activity ratio (fold) |
|---|---|---|
| #02-078 | M141G | 0.07 |
| #02-079 | M141H | 0.63 |
| #02-080 | M141I | 1.32 |
| #02-081 | M141K | 1.44 |
| #02-082 | M141L | 1.43 |
| #02-083 | M141N | 0.22 |
| #02-084 | M141P | 0.12 |
| #02-085 | M141Q | 0.26 |
| #02-086 | M141R | 1.16 |
| #02-087 | M141S | 1.08 |
| #02-088 | M141T | 1.25 |
| #02-089 | M141V | 1.76 |
| #02-090 | M141W | 1.47 |
| #02-091 | M141Y | 1.90 |
| #02-092 | T156A | 0.00 |
| #02-093 | T156D | 0.00 |
| #02-094 | T156E | 0.00 |
| #02-095 | T156F | 0.00 |
| #02-096 | T156G | 0.00 |
| #02-097 | T156H | 0.00 |
| #02-098 | T1561 | 0.00 |
| #02-099 | T156K | 0.00 |
| #02-100 | T156L | 0.00 |
| #02-101 | T156M | 0.00 |
| #02-102 | T156N | 0.00 |
| #02-103 | T156P | 0.00 |
| #02-104 | T156Q | 0.00 |
| #02-105 | T156R | 0.00 |
| #02-106 | T156S | 0.74 |
| #02-107 | T156V | 0.00 |
| #02-108 | T156W | 0.00 |
| #02-109 | T156Y | 0.00 |
| #02-110 | H182A | 1.45 |
| #02-111 | H182D | 0.13 |
| #02-112 | H182E | 0.30 |
| #02-113 | H182F | 1.53 |
| #02-114 | H182G | 1.20 |
| #02-115 | H182I | 0.45 |

[Table 7]

| No. | Name | Activity ratio (fold) |
|---|---|---|
| #02-116 | H182K | 0.83 |
| #02-117 | H182L | 2.03 |
| #02-118 | H182M | 1.85 |
| #02-119 | H182N | 0.32 |
| #02-120 | H182P | 0.00 |
| #02-121 | H182Q | 0.56 |
| #02-122 | H182R | 0.98 |
| #02-123 | H182S | 0.15 |
| #02-124 | H182T | 0.09 |
| #02-125 | H182V | 0.70 |
| #02-126 | H182W | 0.80 |
| #02-127 | H182Y | 1.39 |
| #02-128 | G183A | 0.17 |
| #02-129 | G183D | 0.08 |
| #02-130 | G183E | 0.00 |
| #02-131 | G183F | 0.00 |
| #02-132 | G183H | 0.00 |
| #02-133 | G183I | 0.00 |
| #02-134 | G183K | 0.00 |
| #02-135 | G183L | 0.06 |
| #02-136 | G183M | 0.05 |
| #02-137 | G183N | 0.00 |
| #02-138 | G183P | 0.00 |
| #02-139 | G183Q | 0.00 |
| #02-140 | G183R | 0.00 |
| #02-141 | G183S | 0.15 |
| #02-142 | G183T | 0.08 |
| #02-143 | G183V | 0.00 |
| #02-144 | G183W | 0.00 |
| #02-145 | G183Y | 0.00 |
| #02-146 | Q186A | 0.76 |
| #02-147 | Q186D | 0.76 |
| #02-148 | Q186E | 1.15 |
| #02-149 | Q186F | 0.29 |
| #02-150 | Q186G | 0.30 |
| #02-151 | Q186H | 0.23 |
| #02-152 | Q186I | 0.56 |

(continued)

| No. | Name | Activity ratio (fold) |
|---|---|---|
| #02-153 | Q186K | 0.46 |
| #02-154 | Q186L | 0.89 |
| #02-155 | Q186M | 1.35 |
| #02-156 | Q186N | 0.28 |
| #02-157 | Q186P | 0.00 |
| #02-158 | Q186R | 0.20 |
| #02-159 | Q186S | 0.67 |
| #02-160 | Q186T | 0.66 |
| #02-161 | Q186V | 0.58 |
| #02-162 | Q186W | 0.24 |
| #02-163 | Q186Y | 0.23 |
| #02-164 | W253A | 1.07 |
| #02-165 | W253D | 0.49 |
| #02-166 | W253E | 0.59 |
| #02-167 | W253F | 1.25 |
| #02-168 | W253G | 0.69 |
| #02-169 | W253H | 1.70 |
| #02-170 | W253I | 1.32 |
| #02-171 | W253K | 0.94 |
| #02-172 | W253L | 1.35 |
| #02-173 | W253M | 1.30 |
| #02-174 | W253N | 1.16 |
| #02-175 | W253P | 1.25 |
| #02-176 | W253Q | 1.40 |
| #02-177 | W253R | 0.89 |
| #02-178 | W253S | 0.91 |
| #02-179 | W253T | 1.15 |
| #02-180 | W253V | 1.13 |
| #02-181 | W253Y | 1.01 |
| #02-182 | K260A | 1.03 |
| #02-183 | K260D | 0.49 |
| #02-184 | K260E | 1.90 |
| #02-185 | K260F | 1.30 |
| #02-186 | K260G | 1.25 |
| #02-187 | K260H | 1.17 |
| #02-188 | K260I | 0.99 |
| #02-189 | K260L | 1.22 |
| #02-190 | K260M | 1.40 |

(continued)

| No. | Name | Activity ratio (fold) |
|---|---|---|
| #02-191 | K260N | 1.62 |
| #02-192 | K260P | 0.18 |
| #02-193 | K260Q | 1.88 |
| #02-194 | K260R | 1.25 |
| #02-195 | K260S | 1.21 |
| #02-196 | K260T | 1.44 |
| #02-197 | K260V | 0.56 |
| #02-198 | K260W | 1.17 |
| #02-199 | K260Y | 1.34 |
| #02-200 | P262A | 0.68 |
| #02-201 | P262D | 0.00 |
| #02-202 | P262E | 0.05 |
| #02-203 | P262F | 0.00 |
| #02-204 | P262G | 0.00 |
| #02-205 | P262H | 0.08 |
| #02-206 | P262I | 0.00 |
| #02-207 | P262K | 0.07 |
| #02-208 | P262L | 0.00 |
| #02-209 | P262M | 0.00 |
| #02-210 | P262N | 0.00 |
| #02-211 | P262Q | 0.00 |
| #02-212 | P262R | 0.00 |
| #02-213 | P262S | 0.00 |
| #02-214 | P262T | 0.00 |
| #02-215 | P262V | 0.00 |
| #02-216 | P262W | 0.00 |
| #02-217 | P262Y | 0.00 |

Synthesis Example 2: Conversion of 2-oxo-3-(p-tolyl)propanoic acid into sodium salt

2-Oxo-3-(p-tolyl)propanoic acid, a starting material keto acid for H-EtPhe(4-Me)-OH, was converted into sodium salt. Specifically, 2-oxo-3-(p-tolyl)propanoic acid (purchased from ChemSpace) was suspended in an acetonitrile-ultrapure water (1: 1) mixed solvent. Then, a 1 M aqueous sodium hydroxide solution was added at 1 equivalent based on the keto acid, and the mixture was well stirred. After confirmation of complete dissolution, the solution was passed through a 0.5 μm PTFE membrane filter (purchased from Tosoh Corp.) and freeze-dried.

Evaluation Example 3: Screening of variant enzyme with H-EtPhe(4-Me)-OH synthetic activity as index

In order to screen for each variant enzyme (#02-002 to -217) prepared in Example 1 with H-EtPhe(4-Me)-OH synthetic activity as an index, a solution containing substrates (hereinafter, referred to as premix 4) was prepared. Specifically, two compounds, the keto acid sodium salt prepared in Synthesis Example 2 and D(+)-glucose, were dissolved in a mixed solvent of an aqueous ethylamine solution and a phosphate buffer solution. The pH of the solution was

adjusted to 8.0 using an aqueous sodium hydroxide solution, and the resultant was then diluted with ultrapure water. An aqueous β-NADPH solution and a GDH solution were added to this solution to give premix 4. The concentration of each compound in the premix 4 was 55.6 mM keto acid sodium salt prepared in Synthesis Example 2, 111 mM D(+)-glucose, 556 mM ethylamine, a 111 mM phosphate buffer solution, 0.99 mM β-NADPH, and a 0.0022 units/μL GDH solution.

Any one of #02-001 to -217 was added to the prepared premix 4 to carry out H-EtPhe(4-Me)-OH synthesis reaction. Specifically, a solution of any one of #02-001 to -217 was added to the premix 4. For a negative control enzyme(-), an imidazole-containing buffer solution was added thereto. The resultant was well mixed and then incubated at 37°C for 19 hours. The concentration of each compound at the start of reaction was 50 mM keto acid sodium salt prepared in Synthesis Example 2, 100 mM D(+)-glucose, 500 mM ethylamine, a 100 mM phosphate buffer solution, 0.89 mM β-NADPH, a 0.002 units/μL GDH solution, and 2.5 μM of any one of #02-001 to -217.

The reaction solution thus incubated was aftertreated to prepare a sample for LCMS analysis. Specifically, a hydrochloric acid-containing DMSO solution (volume ratio of hydrochloric acid and DMSO: 3:87) was added in 9 times the amount of the reaction solution such that the final concentration was 30 mM after the addition of hydrogen chloride (hereinafter, this solution is referred to as aftertreated reaction solution 4).

For quantifying the concentration of H-EtPhe(4-Me)-OH dissolved in the aftertreated reaction solution 4 by LCMS, serial dilutions of H-MePhe-OH (aftertreated calibration curve sample 2) were prepared in the same manner as in H-EtPhe-OH. Hydrochloric acid or a hydrochloric acid-containing DMSO solution was used in the final dilution process (the composition of the hydrochloric acid-containing DMSO solution was the same as that used in the aftertreated reaction solution 4).

All of the aftertreated reaction solution 4 and the aftertreated calibration curve sample 2 were passed through a 0.2 μm PVDF membrane filter (purchased from Corning, Inc.) and then subjected to LCMS measurement. The liquid volume used in measurement was 1 μL. The area of a peak derived from H-EtPhe(4-Me)-OH on a UV chart (254 nm) was detected using TargetLynx to evaluate the synthetic activity of each of #02-001 to -217 against H-EtPhe(4-Me)-OH. The results are as described in the tables given below. A plurality of variant enzymes having higher H-EtPhe(4-Me)-OH synthetic activity than that of the wild-type enzyme (#02-001) were able to be found.

LCMS (ESI) m/z = 208.21 $(M + H)^+$
Retention time: 0.59 min (analysis condition FA05-2; retention time in the UV chart (254 nm))

The synthetic activity was determined by dividing the yield of the target product obtained using each of #02-001 to -217 by the yield of the target product obtained using the wild-type enzyme (#02-001). The calculated ratios of the synthetic activity are shown in Tables 8 and 9. The yields of the wild-type enzyme were 10% for #02-002 to -073, #02-092 to -109, #02-128 to - 145 and #02-200 to -217, and 6% for the others.

[Table 8]

| No. | Name | Activity ratio (fold) |
|---|---|---|
| Enzyme(-) | Enzyme(-) | 0.00 |
| #02-001 | WT | 1.00 |
| #02-003 | H44D | 0.00 |
| #02-004 | H44E | 0.00 |
| #02-005 | H44F | 0.00 |
| #02-006 | H44G | 0.00 |
| #02-007 | H44I | 0.00 |
| #02-008 | H44K | 0.00 |
| #02-009 | H44L | 0.69 |
| #02-010 | H44M | 2.08 |
| #02-011 | H44N | 0.39 |
| #02-013 | H44Q | 0.00 |
| #02-014 | H44R | 0.00 |
| #02-015 | H44S | 0.45 |

(continued)

| No. | Name | Activity ratio (fold) |
|---|---|---|
| #02-016 | H44T | 0.76 |
| #02-018 | H44W | 0.23 |
| #02-019 | H44Y | 0.00 |
| #02-020 | R48A | 0.00 |
| #02-021 | R48D | 0.00 |
| #02-022 | R48E | 0.00 |
| #02-024 | R48G | 0.00 |
| #02-025 | R48H | 0.00 |
| #02-026 | R48I | 0.00 |
| #02-027 | R48K | 0.00 |
| #02-028 | R48L | 0.40 |
| #02-029 | R48M | 0.00 |
| #02-030 | R48N | 0.21 |
| #02-032 | R48Q | 0.34 |
| #02-033 | R48S | 0.29 |
| #02-034 | R48T | 0.35 |
| #02-035 | R48V | 0.25 |
| #02-036 | R48W | 0.00 |
| #02-038 | F117A | 0.64 |
| #02-039 | F117D | 0.00 |
| #02-040 | F117E | 0.00 |
| #02-041 | F117G | 0.74 |
| #02-042 | F117H | 0.47 |
| #02-045 | F117L | 0.34 |
| #02-046 | F117M | 0.71 |
| #02-047 | F117N | 0.79 |
| #02-049 | F117Q | 0.00 |
| #02-054 | F117W | 0.00 |
| #02-055 | F117Y | 0.55 |
| #02-057 | S140D | 0.00 |
| #02-058 | S140E | 0.00 |
| #02-059 | S140F | 0.00 |
| #02-060 | S140G | 0.00 |
| #02-061 | S140H | 0.00 |
| #02-062 | S140I | 0.00 |
| #02-065 | S140M | 0.00 |
| #02-066 | S140N | 0.27 |
| #02-067 | S140P | 0.00 |

(continued)

| No. | Name | Activity ratio (fold) |
|---|---|---|
| #02-068 | S140Q | 0.00 |
| #02-069 | S140R | 0.00 |
| #02-071 | S140V | 0.00 |
| #02-072 | S140W | 0.00 |
| #02-073 | S140Y | 0.00 |
| #02-074 | M141A | 1.55 |
| #02-075 | M141D | 0.00 |
| #02-076 | M141E | 0.00 |
| #02-077 | M141F | 3.01 |
| #02-078 | M141G | 0.00 |
| #02-079 | M141H | 1.98 |
| #02-080 | M141I | 2.18 |
| #02-081 | M141K | 2.05 |
| #02-082 | M141L | 0.97 |
| #02-083 | M141N | 0.00 |
| #02-084 | M141P | 0.00 |
| #02-085 | M141Q | 0.00 |
| #02-086 | M141R | 0.73 |
| #02-087 | M141S | 0.86 |
| #02-088 | M141T | 1.91 |
| #02-089 | M141V | 7.93 |
| #02-090 | M141W | 3.89 |
| #02-091 | M141Y | 5.96 |
| #02-092 | T156A | 0.00 |
| #02-093 | T156D | 0.00 |
| #02-094 | T156E | 0.00 |
| #02-095 | T156F | 0.00 |
| #02-096 | T156G | 0.00 |
| #02-097 | T156H | 0.00 |
| #02-098 | T156I | 0.00 |
| #02-099 | T156K | 0.00 |
| #02-100 | T156L | 0.00 |
| #02-101 | T156M | 0.00 |
| #02-102 | T156N | 0.00 |
| #02-103 | T156P | 0.25 |
| #02-104 | T156Q | 0.00 |
| #02-105 | T156R | 0.00 |
| #02-106 | T156S | 1.21 |

(continued)

| No. | Name | Activity ratio (fold) |
|---|---|---|
| #02-107 | T156V | 0.00 |
| #02-108 | T156W | 0.00 |
| #02-109 | T156Y | 0.00 |
| #02-110 | H182A | 1.17 |
| #02-111 | H182D | 0.00 |
| #02-112 | H182E | 0.00 |
| #02-113 | H182F | 2.31 |
| #02-114 | H182G | 0.00 |
| #02-115 | H1821 | 0.59 |
| #02-116 | H182K | 0.00 |
| #02-117 | H182L | 4.48 |
| #02-118 | H182M | 2.62 |
| #02-119 | H182N | 0.00 |
| #02-120 | H182P | 0.00 |
| #02-121 | H182Q | 0.00 |

[Table 9]

| No. | Name | Activity ratio (fold) |
|---|---|---|
| #02-122 | H182R | 0.16 |
| #02-123 | H182S | 0.00 |
| #02-124 | H182T | 0.00 |
| #02-125 | H182V | 0.35 |
| #02-126 | H182W | 0.37 |
| #02-127 | H182Y | 1.89 |
| #02-128 | G183A | 0.32 |
| #02-129 | G183D | 0.00 |
| #02-130 | G183E | 0.00 |
| #02-132 | G183H | 0.00 |
| #02-133 | G183I | 0.00 |
| #02-134 | G183K | 0.00 |
| #02-135 | G183L | 0.00 |
| #02-136 | G183M | 0.23 |
| #02-137 | G183N | 0.00 |
| #02-138 | G183P | 0.00 |
| #02-139 | G183Q | 0.00 |
| #02-140 | G183R | 0.00 |
| #02-141 | G183S | 0.40 |

(continued)

| No. | Name | Activity ratio (fold) |
|---|---|---|
| #02-142 | G183T | 0.29 |
| #02-143 | G183V | 0.22 |
| #02-144 | G183W | 0.00 |
| #02-145 | G183Y | 0.00 |
| #02-146 | Q186A | 0.21 |
| #02-147 | Q186D | 0.00 |
| #02-148 | Q186E | 1.22 |
| #02-149 | Q186F | 0.00 |
| #02-150 | Q186G | 0.00 |
| #02-151 | Q186H | 0.00 |
| #02-152 | Q186I | 0.22 |
| #02-153 | Q186K | 0.06 |
| #02-154 | Q186L | 0.39 |
| #02-155 | Q186M | 0.56 |
| #02-156 | Q186N | 0.00 |
| #02-157 | Q186P | 0.00 |
| #02-158 | Q186R | 0.00 |
| #02-159 | Q186S | 0.00 |
| #02-160 | Q186T | 0.37 |
| #02-161 | Q186V | 0.43 |
| #02-162 | Q186W | 0.00 |
| #02-163 | Q186Y | 0.00 |
| #02-164 | W253A | 0.08 |
| #02-165 | W253D | 0.00 |
| #02-166 | W253E | 0.00 |
| #02-167 | W253F | 0.31 |
| #02-168 | W253G | 0.00 |
| #02-169 | W253H | 1.33 |
| #02-170 | W253I | 0.86 |
| #02-171 | W253K | 0.00 |
| #02-172 | W253L | 0.63 |
| #02-173 | W253M | 0.43 |
| #02-174 | W253N | 0.05 |
| #02-175 | W253P | 0.58 |
| #02-176 | W253Q | 0.69 |
| #02-177 | W253R | 0.27 |
| #02-178 | W253S | 0.24 |
| #02-179 | W253T | 0.41 |

(continued)

| No. | Name | Activity ratio (fold) |
|---|---|---|
| #02-180 | W253V | 0.34 |
| #02-181 | W253Y | 0.08 |
| #02-182 | K260A | 0.54 |
| #02-183 | K260D | 0.00 |
| #02-184 | K260E | 3.52 |
| #02-185 | K260F | 1.15 |
| #02-186 | K260G | 0.15 |
| #02-187 | K260H | 0.93 |
| #02-188 | K260I | 0.53 |
| #02-189 | K260L | 1.98 |
| #02-190 | K260M | 2.41 |
| #02-191 | K260N | 0.54 |
| #02-192 | K260P | 0.00 |
| #02-193 | K260Q | 3.20 |
| #02-194 | K260R | 0.99 |
| #02-195 | K260S | 1.16 |
| #02-196 | K260T | 1.97 |
| #02-197 | K260V | 0.80 |
| #02-198 | K260W | 0.85 |
| #02-199 | K260Y | 1.35 |
| #02-200 | P262A | 0.37 |
| #02-201 | P262D | 0.00 |
| #02-202 | P262E | 0.00 |
| #02-203 | P262F | 0.00 |
| #02-204 | P262G | 0.00 |
| #02-205 | P262H | 0.00 |
| #02-206 | P262I | 0.00 |
| #02-207 | P262K | 0.00 |
| #02-208 | P262L | 0.00 |
| #02-209 | P262M | 0.00 |
| #02-210 | P262N | 0.00 |
| #02-211 | P262Q | 0.00 |
| #02-212 | P262R | 0.00 |
| #02-213 | P262S | 0.00 |
| #02-214 | P262T | 0.00 |
| #02-215 | P262V | 0.00 |
| #02-216 | P262W | 0.00 |
| #02-217 | P262Y | 0.00 |

Evaluation example 4 : Screening of variant enzyme with H-EtPhe-OH synthetic activity as index (double mutant screening)

Screenings of the variant enzymes (#03-001 to -130) prepared in Example 1 were performed with H-EtPhe-OH synthetic activity as an index. The protocol was performed in the same manner as in Evaluation Example 2 except that #03 -001 to -130 was used instead of #02-002 to -217 as the variant enzyme. Although the activities of #02-021 and -117 have already been evaluated in Evaluation Example 2, they were used as controls in an experimental system different from WT.

The results are as described in the tables given below. A plurality of variant enzymes having higher H-EtPhe-OH synthetic activity than that of the wild-type enzyme (#02-001) were able to be found.

LCMS(ESI) m/z=194.11$(M+H)^+$
Retention time: 0.38 min (analysis condition FA05-2, retention time in UV chart (254 nm))

The synthetic activity was determined by dividing the yield of the target product using each of #02-001 and #03-001 to -130 by the yield of the target product using the wild-type enzyme (#02-001). The calculated ratios of synthetic activity are shown in Tables 10 and 11. The yield of the wild-type enzyme was 32%.

[Table 10]

| No. | Name | Activity ratio (fold) |
|---|---|---|
| Enzyme(-) | Enzyme(-) | **0.00** |
| **#02-001** | **WT** | **1.00** |
| **#02-021** | **R48D** | **0.00** |
| **#02-117** | **H182L** | **1.80** |
| **#03-001** | **M141A_H182F** | **0.45** |
| **#03-002** | **M141A_H182L** | **1.08** |
| **#03-003** | **M141A_H182M** | **0.75** |
| **#03-004** | **M141A_W253H** | **0.84** |
| **#03-005** | **M141A_W253M** | **0.68** |
| **#03-006** | **M141A_K260E** | **2.68** |
| **#03-007** | **M141A_K260Q** | **2.65** |
| **#03-008** | **M141K_W253M** | **1.05** |
| **#03-009** | **M141V_H182F** | **0.80** |
| **#03-010** | **M141V_H182L** | **2.21** |
| **#03-011** | **M141V_H182M** | **1.30** |
| **#03-012** | **M141V_W253H** | **1.60** |
| **#03-013** | **M141V_W253M** | **1.80** |
| **#03-014** | **M141V_K260E** | **2.65** |
| **#03-015** | **M141V_K260Q** | **2.49** |
| **#03-016** | **M141Y_H182F** | **0.92** |
| **#03-017** | **M141Y_H182L** | **1.89** |
| **#03-018** | **M141Y_H182M** | **2.28** |
| **#03-019** | **M141Y_W253H** | **1.40** |
| **#03-020** | **M141Y_K260E** | **> 3.00** |
| **#03-022** | **H182F_W253H** | **0.96** |
| **#03-023** | **H182F_K260E** | **2.10** |

(continued)

| No. | Name | Activity ratio (fold) |
|---|---|---|
| #03-024 | H182F_K260Q | 2.23 |
| #03-025 | H182L_W253H | 2.12 |
| #03-026 | H182L_K260E | 2.51 |
| #03-027 | H182L_K260Q | 2.76 |
| #03-028 | H182M_W253H | 1.03 |
| #03-029 | H182M_K260E | 2.65 |
| #03-030 | H182M_K260Q | 2.65 |
| #03-031 | W253H_K260E | 2.62 |
| #03-032 | W253H_K260Q | 2.29 |
| #03-033 | W253M_K260E | 2.29 |
| #03-034 | W253M_K260Q | 1.85 |
| #03-035 | W253A_K260E | 1.65 |
| #03-036 | W253M_K260G | 0.72 |
| #03-037 | M141V_K260G | 1.54 |
| #03-038 | W253A_K260G | 0.44 |
| #03-039 | W253A_K260W | 0.61 |
| #03-040 | M141A_W253A | 0.55 |
| #03-041 | W253A_K260L | 1.11 |
| #03-042 | W253M_K260L | 1.20 |
| #03-043 | W253A_K260T | 0.45 |
| #03-044 | W253A_K260Q | 1.32 |
| #03-045 | M141A_H182W | 0.25 |
| #03-046 | M141K_W253A | 1.02 |
| #03-048 | W253A_K260A | 0.71 |
| #03-049 | M141V_K260F | 2.21 |
| #03-050 | M141A_K260G | 1.48 |
| #03-051 | W253A_K260I | 0.79 |
| #03-052 | W253A_K260F | 0.83 |
| #03-053 | M141A_W253S | 0.58 |
| #03-054 | M141R_K260G | 1.11 |
| #03-055 | M141V_W253A | 1.64 |
| #03-056 | W253K_K260G | 0.32 |
| #03-057 | W253A_K260S | 0.66 |
| #03-058 | W253M_K260A | 0.99 |
| #03-059 | W253Y_K260L | 0.83 |
| #03-060 | H182W_K260G | 0.58 |
| #03-061 | M141K_K260L | 1.56 |
| #03-062 | M141Q_W253A | 0.55 |

(continued)

| No. | Name | Activity ratio (fold) |
|---|---|---|
| **#03-063** | **M141V_K260S** | **1.80** |

[Table 11]

| No. | Name | Activity ratio (fold) |
|---|---|---|
| **#03-064** | **M141K_K260G** | **1.48** |
| **#03-065** | **M141A_K260A** | **1.38** |
| **#03-066** | **W253K_K260L** | **0.82** |
| **#03-067** | **M141Q_K260G** | **0.30** |
| **#03-068** | **M141A_K260S** | **1.46** |
| **#03-069** | **M141A_W253T** | **0.48** |
| **#03-070** | **H182W_K260L** | **0.46** |
| **#03-071** | **M141Y_K260G** | **2.13** |
| **#03-072** | **W253R_K260F** | **0.63** |
| **#03-073** | **H182G_W253A** | **0.79** |
| **#03-074** | **W253A_K260H** | **0.78** |
| **#03-075** | **W253Y_K260G** | **0.41** |
| **#03-076** | **M141A_K260V** | **0.75** |
| **#03-077** | **W253R_K260A** | **0.48** |
| **#03-078** | **W253A_K260V** | **0.48** |
| **#03-079** | **F117N_M141A** | **0.68** |
| **#03-080** | **W253S_K260G** | **0.40** |
| **#03-081** | **W253A_K260R** | **0.73** |
| **#03-082** | **W253T_K260L** | **1.17** |
| **#03-083** | **M141A_K260I** | **1.22** |
| **#03-084** | **W253M_K260S** | **0.79** |
| **#03-085** | **H182W_W253A** | **0.54** |
| **#03-086** | **M141V_W253Y** | **1.49** |
| **#03-087** | **M141A_W253Y** | **0.38** |
| **#03-088** | **W253T_K260G** | **0.47** |
| **#03-089** | **W253A_K260M** | **0.94** |
| **#03-090** | **W253Y_K260F** | **0.68** |
| **#03-091** | **M141V_K260L** | **2.14** |
| **#03-092** | **W253A_K260N** | **0.56** |
| **#03-093** | **W253R_K260G** | **0.30** |
| **#03-094** | **H182S_K260G** | **0.10** |
| **#03-095** | **M141K_K260S** | **1.34** |
| **#03-096** | **M141R_K260A** | **1.03** |

(continued)

| No. | Name | Activity ratio (fold) |
|---|---|---|
| **#03-097** | **M141T_K260G** | **1.21** |
| **#03-098** | **H182M_W253A** | **1.02** |
| **#03-099** | **W253R_K260L** | **0.84** |
| **#03-100** | **M141K_K260A** | **1.07** |
| **#03-101** | **H182S_W253A** | **0.11** |
| **#03-102** | **M141R_W253M** | **0.61** |
| **#03-103** | **M141R_W253A** | **0.60** |
| **#03-104** | **M141T_K260Q** | **2.07** |
| **#03-105** | **M141S_K260L** | **0.94** |
| **#03-106** | **M141W_K260A** | **1.49** |
| **#03-107** | **M141A_K260L** | **1.51** |
| **#03-108** | **H182S_K260L** | **0.15** |
| **#03-109** | **W253N_K260L** | **1.07** |
| **#03-110** | **W253Y_K260I** | **0.51** |
| **#03-111** | **M141Q_K260A** | **0.25** |
| **#03-112** | **W253L_K260G** | **0.76** |
| **#03-113** | **M141T_H182S** | **0.10** |
| **#03-114** | **W253Y_K260V** | **0.41** |
| **#03-115** | **M141V_K260A** | **1.74** |
| **#03-116** | **M141A_K260N** | **1.43** |
| **#03-117** | **W253M_K260H** | **0.97** |
| **#03-118** | **W253R_K260T** | **0.33** |
| **#03-119** | **M141A_K260F** | **1.72** |
| **#03-120** | **W253R_K260V** | **0.48** |
| **#03-121** | **H182Y_W253A** | **1.04** |
| **#03-122** | **M141K_W253S** | **1.09** |
| **#03-123** | **W253Q_K260G** | **0.40** |
| **#03-124** | **M141K_H182S** | **0.11** |
| **#03-125** | **H182G_K260G** | **0.49** |
| **#03-126** | **W253M_K260V** | **0.53** |
| **#03-127** | **M141A_H182S** | **0.12** |
| **#03-128** | **W253N_K260G** | **0.41** |
| **#03-129** | **M141A_K260H** | **1.66** |
| **#03-130** | **M141I_W253A** | **1.19** |

Evaluation Example 5: Screening of variant enzyme with H-EtPhe-OH synthetic activity (triple mutant screening)

The variant enzymes (#04-001 to 072) prepared in Example 1 were screened using H-EtPhe-OH synthetic activity as an index. The protocol was performed in the same manner as in Evaluation Example 2 except that #04-001

to -072 was used instead of #02-002 to -217 as the variant enzyme. Although #02-021, -117 and #03-006, -020 have been evaluated for activity in Evaluation Examples 2 and 3, they were used as controls in an experimental system different from WT.

The results are as described in the Tables given below. A plurality of variant enzymes having higher H- EtPhe-OH synthetic activity than that of the wild-type enzyme (#02-001) were able to be found.

LCMS(ESI) m/z=194.07$(M+H)^+$

Retention time: 0.37 min (analysis condition FA05-2, retention time in UV chart (254 nm))

The synthetic activity was determined by dividing the yield of the target product using #02-001 and #04-001 to -072 by the yield of the desired product using the wild-type enzyme (#02-001). The calculated ratios of synthetic activity are shown in Table 12. The yield of the wild-type enzyme was 16%.

[Table 12]

| No. | Name | Activity ratio (fold) |
|---|---|---|
| **Enzyme(-)** | **Enzyme(-)** | **0.00** |
| **#04-001** | **M141V_W253H_K260E** | **3.41** |
| **#04-002** | **H182L_W253H_K260Q** | **3.45** |
| **#04-003** | **M141V_W253H_K260Q** | **3.61** |
| **#04-004** | **H182L_W253H_K260E** | **4.28** |
| **#04-005** | **M141Y_W253H_K260E** | **3.14** |
| **#04-006** | **H182M_W253H_K260Q** | **2.17** |
| **#04-007** | **H182M_W253H_K260E** | **3.49** |
| **#04-008** | **M141A_W253H_K260E** | **1.74** |
| **#04-009** | **M141V_W253Q_K260E** | **3.90** |
| **#04-010** | **M1411_W253H_K260E** | **3.82** |
| **#04-011** | **M141V_W253L_K260Q** | **3.60** |
| **#04-012** | **M141V_W253L_K260E** | **3.35** |
| **#04-013** | **H182M_W253M_K260E** | **3.16** |
| **#04-014** | **H182M_W253Q_K260E** | **3.36** |
| **#04-015** | **M141A_W253Q_K260E** | **1.89** |
| **#04-016** | **M1411_W253H_K260Q** | **3.57** |
| **#04-017** | **H182L_W253Q_K260E** | **3.86** |
| **#04-018** | **H182L_W253M_K260Q** | **3.15** |
| **#04-019** | **M141V_W253E_K260Q** | **1.73** |
| **#04-020** | **H182M_W253M_K260Q** | **2.51** |
| **#04-021** | **M141A_W253H_K260Q** | **1.66** |
| **#04-022** | **M141Y_W253T_K260E** | **2.30** |
| **#04-023** | **H182L_W253M_K260E** | **3.71** |
| **#04-024** | **M141L_W253H_K260E** | **3.37** |
| **#04-025** | **M141V_W253Q_K260Q** | **3.65** |
| **#04-026** | **H182L_W253T_K260Q** | **2.48** |
| **#04-027** | **H182L_W253Q_K260Q** | **3.40** |
| **#04-028** | **H182M_W253L_K260Q** | **2.28** |

(continued)

| No. | Name | Activity ratio (fold) |
|---|---|---|
| #04-029 | H182M_W253Q_K260Q | 2.67 |
| #04-030 | M141V_W253F_K260Q | 3.36 |
| #04-031 | M141V_W253T_K260Q | 3.31 |
| #04-032 | M141Y_W253Q_K260E | 3.09 |
| #04-033 | H182M_W253F_K260Q | 1.95 |
| #04-034 | M141T_W253H_K260E | 2.19 |
| #04-035 | M141Y_W253S_K260E | 2.46 |
| #04-036 | M141V_W253V_K260Q | 3.48 |
| #04-037 | H182M_W253L_K260E | 2.91 |
| #04-038 | M141S_W253H_K260E | 2.20 |
| #04-039 | T156V_Q186R | 0.00 |
| #04-040 | T156I_Q186H | 0.00 |
| #04-041 | T156I_Q186R | 0.00 |
| #04-042 | M141Y_W253M_K260E | 2.70 |
| #04-043 | M141V_W253M_K260Q | 3.55 |
| #04-044 | M141V_H182L_K260Q | 3.98 |
| #04-045 | M141V_H182M_K260Q | 3.06 |
| #04-046 | M141V_W253M_K260E | 4.34 |
| #04-047 | H182F_W253H_K260E | 2.89 |
| #04-048 | M141Y_H182M_K260Q | 4.03 |
| #04-049 | M141Y_H182M_K260E | 4.67 |
| #04-050 | M141A_H182M_K260Q | 1.89 |
| #04-051 | M141A_W253M_K260E | 1.71 |
| #04-052 | M141A_H182L_K260Q | 2.17 |
| #04-053 | M141V_H182L_K260E | 3.79 |
| #04-054 | M141V_H182M_K260E | 4.01 |
| #04-055 | M141T_H182L_K260Q | 2.43 |
| #04-056 | M141A_H182M_K260E | 2.44 |
| #04-057 | M141A_W253M_K260Q | 1.39 |
| #04-058 | M141A_H182L_K260E | 1.85 |
| #04-059 | M141T_H182M_K260Q | 1.62 |
| #04-060 | M141Y_H182L_K260E | 3.75 |
| #04-061 | M141Y_H182L_K260Q | 3.97 |
| #04-062 | M141V_H182F_K260Q | 2.25 |
| #04-063 | M141A_H182F_K260Q | 2.78 |
| #04-064 | M141A_H182F_K260E | 1.37 |
| #04-065 | M141V_H182F_K260E | 2.46 |
| #04-066 | M141Y_H182F_K260E | 3.26 |

(continued)

| No. | Name | Activity ratio (fold) |
|---|---|---|
| **#04-067** | **M141I_H182M** | **1.67** |
| **#04-068** | **M141K_H182L_W253H** | **1.63** |
| **#04-069** | **F117I_M141A_K260Q** | **0.34** |
| **#04-070** | **W253L_K260D** | **0.77** |
| **#04-071** | **M141Y_H182G_W253H** | **0.41** |
| **#04-072** | **M141Y_H182A** | **1.62** |
| **#02-001** | **WT** | **1.00** |
| **#02-021** | **R48D** | **0.00** |
| **#02-117** | **H182L** | **2.15** |
| **#03-006** | **M141A_K260E** | **4.23** |
| **#03-020** | **M141Y_K260E** | **5.00** |

Synthesis Example 3: Synthesis of H-MeVal-OH

In order to test H-MeVal-OH synthesis by the variant enzyme (#02-169) prepared in Example 1, a solution containing a substrate (hereinafter referred to as premix 5) was prepared. Specifically, two compounds, sodium 3-methyl-2-oxobutanoate (purchased from Sigma-Aldrich Japan LLC) and D(+)-glucose, were dissolved in a mixed solvent of aqueous methyl amine solution and a phosphate buffer (400mM, pH7. 4). The pH of the solution was adjusted to 8.0 using an aqueous sodium hydroxide solution, and the resultant was then diluted with ultrapure water. An aqueous β -NADPH solution and a GDH solution were added to this solution to give a premix 5. The concentrations of each compound in the premix 5 was 55. 6mM sodium 3-methyl-2-oxobutanoate, 111mM D(+)- glucose, 556mM methyl amine, 111mM phosphate buffer solution, 0. 99mM β-NADPH, and a 0.0022 unit/μL GDH solution.

#02-169 was added to the prepared premix 5 to carry out H-MeVal-OH synthesis reaction. Specifically, a solution of #02-169 was added to the premix 5. The resultant was well mixed and then incubated at 37°C for 19 hours. The concentration of each compound at the start of reaction was 50mM sodium 3-methyl-2-oxobutanoate, 100mM D(+)-glucose, 500mM methyl amine, 100mM phosphate buffer, 0. 89mM β-NADPH, a 0. 002 unit/μL GDH solution and 2.5 μ M of #02-169.

The reaction solution thus incubated was after treated to prepare a sample for LCMS analysis. Specifically, hydrochloric acid was added in 9 times the amount of the reaction solution such that the final concentration was 30 mM after the addition of hydrogen chloride (hereinafter, this solution is referred to as aftertreated solution 5).

Serial dilutions of H-MeVal-OH were prepared for the purpose of quantifying the concentration of H-MeVal-OH dissolved in the aftertreated reaction solution 5 by LCMS. Specifically, H-MeVal-OH was dissolved at 1000mM in ultrapure water, and then a two fold dilution operation with ultrapure water was repeated.

The quantification of the concentration using the serial dilutions was difficult due to ion suppression ascribable to various compounds dissolved in the aftertreated reaction solution 5. Therefore, the serial dilutions were added to a solution that mimicked the composition of the aftertreated reaction solution 5. First, two compounds, sodium 3-methyl-2-oxobutanoate and D (+)-glucose, were dissolved in a mixed solvent of an aqueous methylamine solution and a phosphate buffer solution. The pH of the solution was adjusted to 8.0 using an aqueous sodium hydroxide solution, and the mixture was diluted with ultrapure water (hereinafter, this solution is referred to as premix 6). Next, an aqueous β - NADPH solution, 1 × TNG, imidazole-containing buffer, and serial dilutions were added to Premix 6 and well mixed. The concentration of each compound at this point was 25mM sodium 3-methyl-2-oxobutanoate, 75mM D(+)-glucose, 475mM methyl amine, a 100mM phosphate buffer solution and a 0. 89mM β - NADPH, and the imidazole-containing buffer solution each had a volume ratio of 10%. Finally, hydrochloric acid in 9 times the amount of the reaction solution such that the final concentration was 30mM after addition of hydrogen chloride to obtain a calibration curve sample for LCMS analysis (hereinafter referred to as aftertreated calibration curve sample 3).

A dilution operation was performed in consideration of the possibility that H-MeVal-OH was dissolved, at a level exceeding the calibration curve range for the aftertreated calibration curve sample 3, in the aftertreated reaction solution 5. First, a solution for dilution that mimicked the composition of various compounds dissolved in the aftertreated reaction solution 5 was prepared as compensation for the ion suppression mentioned above. Specifically, premix 6, 1 × TNG,

an imidazole-containing buffer solution, an aqueous β - NADPH solution, and hydrochloric acid were mixed, and diluted with ultrapure water. The concentrations of β - NADPH and hydrochloride in the solution thus diluted were 0. 089mM and 30mM, respectively, and the premix 6, the 1 × TNG, and the imidazole-containing buffer solution had final volume ratios of 6%, 1%, and 1%, respectively. The aftertreated reaction solution 5 was diluted 32-fold with this solution for dilution to obtain aftertreated reaction solution 6.

All of the aftertreated reaction solution 5, aftertreated reaction solution 6, and the aftertreated calibration curve sample 3 were passed through a 0.2 μm PVDF membrane filter, and then subjected to LCMS measurement. The liquid volume used in measurement was 1 μL. The function (TargetLynx) of detecting peak areas of extracted ion chromatograms, on MassLynx was used to evaluate the synthetic activity of #02-169 against H-MeVal-OH. #02-169 was shown to have the synthesis activity of H-MeVal-OH.

LCMS(ESI) m/z=132.02$(M+H)^+$
Retention time: 2.85 min (analysis condition HILIC)
Yield : 26%

Synthesis Example 4: Synthesis of H-MeGly(cPent)-OH

LC/MS was carried out under the following analysis conditions.

UPLC method
Apparatus: Waters AQUITY UPLC H-Class/QDa
Column: ACQUITY UPLC HSS T3 2.1 × 50 mm, 1.8 μm
Solvent: A) 0.05% TFA-$H_2O$, B) 0.05% TFA-$CH_3CN$
Gradient: 0% B (0 min) -> 98% B (5.0 min) -> 98% B (6.0 min) → 0% B (6.01 min) → 0% B (8.0 min)
Flow rate: 0.5 mL/min
Injection volume: 1.0 μl
Temperature: 30°C
Wavelength: 197 nm

The following reagents were used in this Example.

[Table 13]

| Reagent name | CAS registration No. | Supplier |
|---|---|---|
| 2-Cyclopentyl-2-oxo-acetic acid sodium salt | 22342-04-6 | Hebei Yaocheng Pharmac eutical Technology |
| Methylamine hydrochloride | 593-51-1 | Tokyo Chemical Industry Co., Ltd. |
| D-Glucose | 50-99-7 | FUJIFILM Wako Pure Chemical Corp. |
| N,N-Di(2-Hydroxyethyl)glycine | 150-25-4 | Tokyo Chemical Industry Co., Ltd. |
| 50 w/v% aqueous sodium hydroxide solution | 1310-73-2 | FUJIFILM Wako Pure Chemical Corp. |
| Oxidized nicotinamide adenine dinucleotide phosphate | 1184-16-3 | Oriental Yeast Co., Ltd. |
| D-Glucose dehydrogenase (CDX-901) | 9028-53-9 | Codexis |
| Variant enzyme solution | N/A | Prepared in Example 2 |
| Concentrated hydrochloric acid | 7647-01-0 | FUJIFILM Wako Pure Chemical Corp. |

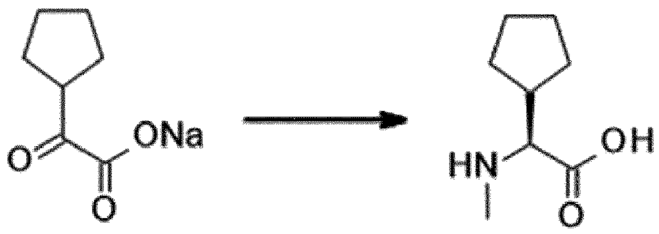

2-Cyclopentyl-2-oxo-acetic acid sodium salt (5.3 g, 32.5 mmol), methylamine hydrochloride (11.0 g, 162.7 mmol), D-glucose (11.7 g, 65.1 mmol), and N,N-di(2-hydroxyethyl)glycine (10.6 g, 65.1 mmol) were dissolved in distilled water (69.4 mL), and the outside temperature was set to 25°C. The pH of the solution was adjusted to 9.2 by the addition of a 50 w/v% aqueous sodium hydroxide solution (4.8 mL). Oxidized nicotinamide adenine dinucleotide phosphate (249.0 mg, 0.3 mmol), D-glucose dehydrogenase (26.0 mg, 0.5 wt%, 1300 U), and a variant enzyme solution (2.0 mL, 53 mg in terms of the enzyme; corresponding to 1.0 wt%) were added thereto, and the mixture was stirred at an inside temperature of 25°C for 21 hours (rate of reaction conversion: 99% or more). The pH of the solution was adjusted to 1.9 by the addition of concentrated hydrochloric acid (7.5 mL), and the mixture was stirred for 1 hour. Insoluble matter was filtered off by celite filtration, and the celite was washed with distilled water (10.7 mL) to give a H-MeGly(cPent)-OH solution as a filtrate. Retention time: 1.41 min, MS (ESI): m/z = 158.10 $(M+H)^+$

Synthesis Example 4-2 : Synthesis of (S)-3,3-dimethyl-2-(methylamino)butyric acid

LC/MS was carried out under the following analytical conditions.

UPLC method
Apparatus: Waters AQUITY UPLC H-Class/QDa
Column: ACQUITY UPLC HSS T3 2.1x50mm, 1.8 μ m
Solvents: A) 0.05% TFA-$H_2O$, B) 0.05% TFA-$CH_3CN$
Gradient: 0% B (0 min) -> 98% B (5.0 min) -> 98% B (6.0 min) -> 0% B (6.01 min) -> 0% B (8.0 min)
Flow rate: 0.5mL/min
Injection volume: 0.5 μl
Temperature: 30°C
Wavelength: 197 nm

In this example, reagents described in the following table were used. The reagents not listed were those in Table 13.

[Table 14]

| Reagent name | CAS registration No. | supplier |
|---|---|---|
| 3,3-Dimethyl-2-oxobutyric Acid | 815-17-8 | Tokyo Chemical Industry Co., Ltd. |
| (S)-2-Amino-3,3-dimethylbutyric Acid | 20859-02-3 | Tokyo Chemical Industry Co., Ltd. |

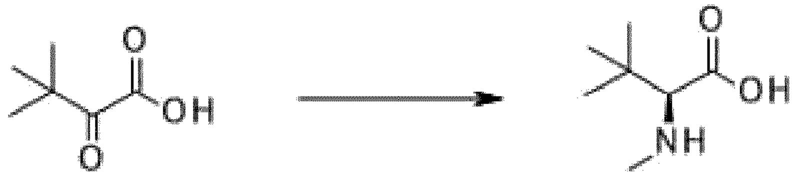

Oxidized nicotinamide adenine dinucleotide phosphate (50 mg) was dissolved in 0.4 mol/L of N, N-di (2-hydroxyethyl) glycine solution (1.0 mL, pH9.0) to prepare an oxidized nicotinamide adenine dinucleotide phosphate solution (solution A).
D-Glucose dehydrogenase (50 mg) was dissolved in 0.4 mol/L of N, N-di (2-hydroxyethyl) glycine solution (1.0 mL, pH9.0) to prepare D-glucose dehydrogenase solution (solution B).
3, 3-Dimethyl-2-oxobutyric acid (50 mg, 0.38 mmol), N, N-di (2-hydroxyethyl) glycine (130 mg, 0.77 mmol), methylamine hydrochloride (130 mg, 1.9 mmol), and D-glucose (140 mg, 0.77 mmol) were dissolved in distilled water

(0.80 mL) and dimethyl sulfoxide (0.20 mL). A 50w/v% sodium hydroxide aqueous solution (88 μ L) was added to adjust the pH to 9.0 to prepare a substrate solution (solution C).

Solution A (59 μL, 3.8 μmol) and Solution B (10 μL, 1.0 wt%) were added to Solution C, the modified enzyme solution prepared in Example 2 (1.0 to 100 wt%, Each was added as a 73.3 mg/ml solution) was added thereto, the mixture was stirred at an external temperature of 25 ° C. for 17 hours, and then shaken at an external temperature of 37 °C for 24 hours.

The yield of (S) -3, 3-dimethyl-2 - (methylamino) butyric acid in the reaction solution was calculated from the UV absorption peak area at 197 nm in LC/MS using (S) - 2-amino-3, 3-dimethylbutyric acid as a standard. The results are shown in Table 15.

[Table 15]

| Amount of variant enzyme (wt%) | Yield(%) | |
|---|---|---|
| | 25°C, 15hours | 37°C, 24hours |
| 1.0 | 0 | 0 |
| 10 | 0 | 1 |
| 100 | 1 | 4 |

Evaluation Example 6: Synthesis of MeHph(2-Cl) using variant enzyme W253H-SBP-His and additive (dimethyl sulfoxide)

The abbreviation MeHph(2-Cl) represents a compound represented by the following structural formula.

[Table 16]

| Abbreviation | Structural formula |
|---|---|
| **MeHph(2-Cl)** | 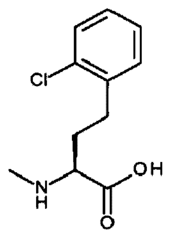 |

LCMS analysis conditions are as follows.

LCMS method
Condition name: FA05
Apparatus: Waters Acquity UPLC/SQD
Column (I.D. x length (mm), particle size (μm)): Aldrich Ascentis Express C18 (2.1 × 50, 2.7) Mobile phase: A) 0.1% FA $H_2O$, B) 0.1% FA MeCN
Gradient (A/B): 95/5 to 0/100 (1.0 min) -> 0/100 (0.4 min)
Flow rate (ml/min): 1
Column temperature: 35°C
Wavelength: 210-400 nm PDA total
Condition name: TFA00
Apparatus: Waters H class
Column: (I.D. x length (mm), particle size (μm)): ACQUITY UPLC HSS T3 (2.1 × 50, 1.8)
Mobile phase: A) 0.05% TFA $H_2O$, B) 0.05% TFA MeCN
Gradient (A/B): 100/0 to 2/98 (5.0 min) -> 2/98 (1.0 min) -> 100/0 (0.01 min) -> 100/0 (2.0 min)
Flow rate (ml/min): 0.5
Column temperature: 30°C
Wavelength: 197 nm
Condition name: TFA00-$QD_a$
Apparatus: Waters H class/QDa
Column: (I.D. x length (mm), particle size (μm)): ACQUITY UPLC HSS T3 (2.1 × 50, 1.8) Mobile phase: A) 0.05%

TFA $H_2O$, B) 0.05% TFA MeCN
Gradient (A/B): 100/0 to 2/98 (5.0 min) -> 2/98 (1.0 min) -> 100/0 (0.01 min) -> 100/0 (2.0 min)
Flow rate (ml/min): 0.5
Column temperature: 30°C
Wavelength: 197 nm

The following reagents were used in this Example.

[Table 17]

| Reagent name | CAS registration No | Supplier |
|---|---|---|
| Sodium pyruvate | 113-24-6 | Tokyo Chemical Industry Co., Ltd. |
| 2-Chlorobenzaldehyde | 89-98-5 | Tokyo Chemical Industry Co., Ltd. |
| Palladium/carbon PE type | 7440-05-3 | NE Chemcat |
| Diphenyl sulfide | 139-66-2 | FUJIFILM Wako Pure Chemical Corp. |
| D(+)-Glucose | 50-99-7 | FUJIFILM Wako Pure Chemical Corp. |
| 40% aqueous methylamine solution | 74-89-5 | FUJIFILM Wako Pure Chemical Corp. |
| Phosphate buffer powder (1/15 mol/L, pH 7.4) | - | FUJIFILM Wako Pure Chemical Corp. |
| NADPH | 2646-71 -1 | Oriental Yeast Co., Ltd. |
| GDH | 9028-53-9 | FUJIFILM Wako Pure Chemical Corp. |
| Sodium 2-cyclopentyl-2-oxoacetate | 223422-04-6 | Hebei Yaocheng Pharmaceutical Technology |
| N,N-Di(2-hydroxyethyl)glycine | 150-25-4 | |
| Ammonium hydroxide solution (28 to 30% $NH_3$) | 1336-21-6 | Sigma-Aldrich Japan G.K. |

The following reagents were preliminarily prepared as follow and used.

[Table 18]

| Reagent name | Preliminary preparation |
|---|---|
| Methylamine solution | The 40% aqueous methylamine solution was prepared into a 2.b M solution of pH 8.5 using hydrochloric acid and ultrapure water. |
| Phosphate buffer solution | The phosphate buffer powder was prepared into a 1 M solution of pH 8.5 using an aqueous sodium hydroxide solution and ultrapure water. |
| NADPH solution | A 100 mM solution was prepared using ultrapure water. |
| GDH solution | A 0.02 units/μL solution was prepared using 1 x TNG. |
| Ammonia solution | The ammonia concentration of the ammonium hydroxide solution (28 to 30% $NH_3$) was adjusted to 28%, and a 2.5 M solution of pH 8.5 was prepared using hydrochloric acid and ultrapure water. |
| N,N-Di(2-hydroxyethyl)glycine buffer solution | N,N-Di(2-hydroxyethyl)glycine was prepared into a 1 M solution of pH 9 using an aqueous sodium hydroxide solution and ultrapure water. |

Synthesis of starting material intermediate (E)-4-(2-chlorophenyl)-2-oxobut-3-enoic acid

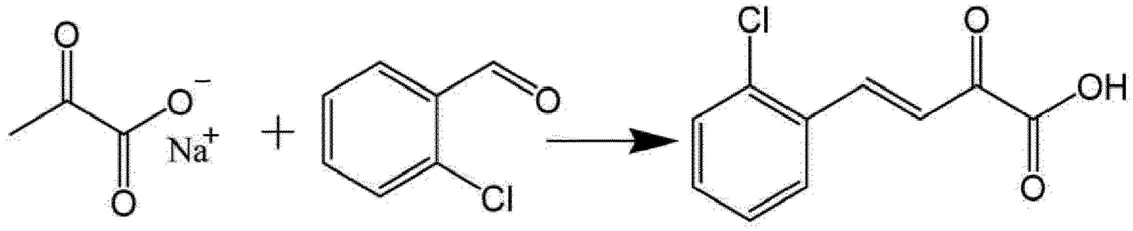

Sodium pyruvate (2.42 g, 0.022 mol) was added to a mixed solution of a 1 N aqueous sodium hydroxide solution (60 ml) and ethanol (3 ml) at 0°C, then 2-chlorobenzaldehyde (2.25 ml, 0.02 mol) was added dropwise thereto over 20 minutes or longer, and the reaction solution was stirred at 0°C for 2 hours. Then, water (160 ml) was added to the reaction solution, and the mixture was washed with toluene (100 ml). 5 N hydrochloric acid was added dropwise to the obtained aqueous layer at 0°C until pH became 1. The solution was stirred for 30 minutes. The resulting precipitate in the solution was recovered by filtration, and the precipitate was washed with cold water. The obtained wet solid was heated and dried under reduced pressure to give (E)-4-(2-chlorophenyl)-2-oxobut-2-enoic acid (2.7 g, 0.013 mol, 64% yield).

LCMS (ESI) m/z = 209.0 $(M - H)^-$
Retention time: 0.52 min (analysis condition FA05)

Synthesis of starting material sodium 4-(2-chlorophenyl)-2-oxobutanoate

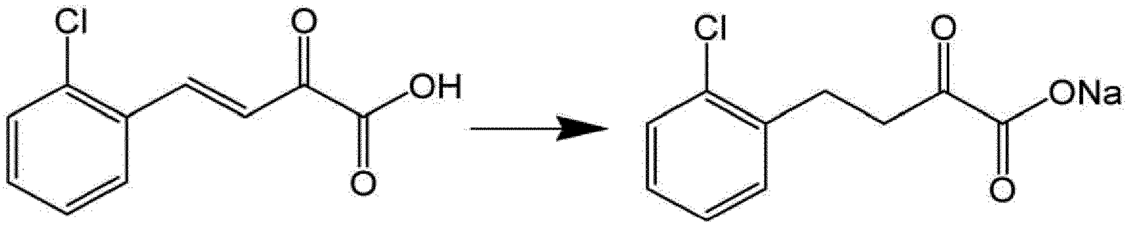

A suspension of (E)-4-(2-chlorophenyl)-2-oxobut-2-enoic acid (1.00 g, 4.75 mol) and palladium/carbon (0.20 g, 1.88 mol) in methanol (20 ml) and diphenyl sulfide (7.97 μl, 0.047 mmol) was stirred at room temperature for 1 hour in a hydrogen atmosphere. The reaction solution was filtered, and the obtained filtrate was then concentrated under a reduced pressure condition. A 1 N aqueous sodium hydroxide solution (15 ml) was added to the obtained residue, and the mixture was washed (twice) with toluene (15 ml). The aqueous layer was stirred at 0°C for 30 minutes. Then, the resulting precipitate in the solution was recovered by filtration, and the precipitate was washed with cold water. The obtained wet solid was heated and dried under reduced pressure to give sodium 4-(2-chlorophenyl)-2-oxobutanoate (847 mg, 3.61 mmol, 75% yield).

LCMS (ESI) m/z = 211.0 $(M - H)^-$
Retention time: 0.59 min (analysis condition FA05)

Synthesis of MeHph(2-Cl) using variant enzyme W253H-SBP-His and additive

A mixed solution (pH 8 to 9) of sodium 4-(2-chlorophenyl)-2-oxobutanoate (final concentration: 50 mM), D(+)-glucose (final concentration: 100 mM), a methylamine solution (final concentration: 500 mM), a phosphate buffer solution (final concentration: 0.1 M), a NADPH solution (final concentration: 1 mM), a GDH solution (final concentration: 0.002 units/μL), dimethyl sulfoxide (DMSO) (20 v/v% based on the reaction solution), the variant enzyme W253H-SBP-His (final concentration: 2.5 μM) prepared in Example 2, and ultrapure water was prepared and incubated at 25°C or 37°C for 3 hours.

To the reaction solution thus incubated, a hydrochloric acid-containing DMSO solution was added in 19 times the amount of the reaction solution such that the final concentration of added hydrogen chloride was 300 mM. The obtained solution was allowed to pass through a 0.45 μm PTFE membrane filter (Cosmospin Filter H, Nacalai Tesque, Inc.) to prepare a sample for LCMS analysis, which was then subjected to LCMS measurement (analysis condition: FA05). The UV peak area at 254 nm of the title compound MeHph(2-Cl) was calculated to determine a reaction yield.

The results are shown in Table 19.

LCMS (ESI) m/z = 228.1 $(M + H)^+$
Retention time: 0.38 min (analysis condition FA05)

[Table 19]

| Additive | Yield (%) at reaction temperature of 25°C | Yield (%) at reaction temperature of 37°C |
|---|---|---|
| **DMSO** | **98** | **99** |

In this example, the following reagents were used.

[Table 20]

| Reagent Name | CAS registration No. | Supplier |
|---|---|---|
| Sodium 2-oxo-3-phenylbutanoic acid | 125116-72-5 | Chemieliva Pharmaceutical |
| D(+)-glucose | 50-99-7 | Fujifilm Wako Pure Chemical Corporation |
| Ammonium hydroxide solution (28~30%$NH_3$) | 1336-21-6 | Sigma-Aldrich |
| β-NADPH | 2646-71-1 | ORIENTAL YEAST CO., LTD |
| GDH | 9028-53-9 | Fujifilm Wako Pure Chemical Corporation |
| N, N-Di (2-hydroxyethyl) glycine | 150-25-4 | Fujifilm Wako Pure Chemical Corporation |
| 5mol/L Hydrochloric acid | 7647-01-0 | Fujifilm Wako Pure Chemical Corporation |
| (2S, 3R) -2-amino-3-phenylbutanoic acid hydrochloride | 143251-58-5 | J&W Pharmlab |
| (2S, 3S) -2-amino-3-phenylbutanoic acid hydrochloride | 53331-55-8 | J&W Pharmlab |
| (2R, 3R) -2-amino-3-phenylbutanoic acid hydrochloride | 143251-57-4 | J&W Pharmlab |

The following reagents were pre-prepared and used as follows.

Table 21

| Reagent Name | Preliminary preparation |
|---|---|
| Ammonia solution | The ammonia concentration of the ammonium hydroxide solution (28 to 30% $NH_3$) was adjusted to 28%, and a 2.5 M solution of pH 8.5 was prepared using hydrochloric acid and ultrapure water. |
| NADPH solution | A 100 mM solution was prepared using ultrapure water. |
| GDH solution | A 0.02 units/μL solution was prepared using 1 x TNG |
| N,N-Di(2-hydroxyethyl)glycin e buffer solution | N,N-Di(2-hydroxyethyl)glycine was prepared into a 1 M solution of pH 9 using an aqueous sodium hydroxide solution and ultrapure water. |

LCMS analysis conditions are as follows.

LCMS method

Condition Name: FA05

Apparatus: Waters Acquity UPLC/SQD

Column (I.D. × length (mm), particle size (μm)): Aldrich Ascentis Express C18 (2.1 × 50, 2.7) Mobile phase: A) 0. 1% FA $H_2O$, B) 0.1% FA MeCN

Gradient (A/B): 95/5 to 0/100 (1.0 min) -> 0/100 (0.4 min)

Flow rate (ml/min): 1

Column temperature: 35°C

Wavelength: 210-400nm PDA total

$^1$H-NMR analysis was carried out using the following apparatus and deuterated solvent. Apparatus: AVANCE III HD 400 SMART - BBFO probe (400 MHz, Bruker)

deuterated solvent: Trifluoroacetic acid-$d_1$

The following abbreviations are used in this Example.

[Table 22]

| Abbreviation | Compound |
|---|---|
| (I) | Compound obtained by the reaction: (2S, 3S) - 2-amino-3-phenylbutanoic acid |
| (II) | Standard compound: (2S, 3R) - 2-amino-3-phenylbutanoic acid hydrochloride salt |
| (III) | Standard compound: (2S, 3S) - 2-amino-3-phenylbutanoic acid hydrochloride salt |
| (IV) | Standard Compound: (2R, 3R) - 2-amino-3-phenylbutanoic acid hydrochloride |

Evaluation Example 7: Synthesis of (2S, 3S) - 2-amino-3-phenyl-butanoic acid using variant enzyme W253H-SBP-His and an additive (dimethyl sulfoxide)

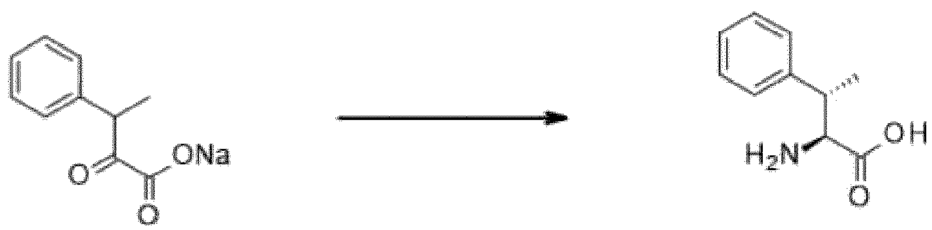

sodium 2-oxo-3-phenylbutanoate (2S, 3S) - 2-amino-3-phenyl-butanoic acid (I)

A mixed solution of sodium 2-oxo-3-phenylbutanoate (final concentration 50 mM), D(+)-glucose (final concentration 100 mM), ammonia solution (final concentration 500 mM), N, N-di (2-hydroxyethyl) glycine buffer solution (final concentration 0.1M), NADPH solution (final concentration 1 mM), GDH solution (final concentration 0.002 unit/μL), additive DMSO (20 vol%), the variant enzyme W253H-SBP-His (final concentration 20 μM) prepared in Example 2, and ultrapure water was prepared and incubated at 37 °C for 47 hours.

600 μL of 5 mol/L hydrochloric acid was added to the reaction solution after the incubation, and the obtained solution was purified by reversed-phase silica gel column chromatography (0. 1% formic acid aqueous solution / 0. 1% formic acid acetonitrile solution = 100/0 to 95/5) to obtain (2S, 3S) - 2-amino-3-phenyl-butanoic acid (I) (16.5 mg, 18%).

LCMS(ESI) m/z=180.1$(M+H)^+$
Retention Time : 0.31 min (Analysis Condition FA05)

$^1$H-NMR (400 MHz, Trifluoroacid - $d_1$, 298K) δ 7.32 - 7.21 (3H, m), 7.18 - 7.16 (2H, m), 4.94 (1H, d, $J$ = 5.6 hz), 3.55 - 3.48 (1H, m), 1.45 (1H, d, $J$ = 6.8 hz)

$^1$H-NMR and chiral HPLC analysis data of the compound (2S, 3S) - 2-amino-3-phenyl-butanoic acid (I) obtained by the reaction were compared with those of the standard compound (2S, 3R) - 2-amino-3-phenyl-butanoic acid hydrochloride (II), (2S, 3S) - 2-amino-3-phenyl-butanoic acid hydrochloride (III), and (2R, 3R) - 2-amino-3-phenyl-butanoic acid hydrochloride (IV) which were purchased.

The structure of the compound (I) obtained in the reaction was specified as (2S, 3S) - 2-amino-3-phenylbutanoic acid from the comparison result of characteristic proton peaks at the 2 - and 3-positions in the $^1$H-NMR analysis (FIG. 1) and the comparison result of chiral HPLC analysis (FIG. 2 and Table 23).
chiral HPLC analysis condition was as follows.

Apparatus: SHIMADZU Nexera X3
Column (I.D. x Length (mm), Particle Size (μm)) : DAICEL CORPORATION CHIRALPAK ZWIX (+) (3.0 × 150, 3.0)
Mobile phase: MeOH : MeCN : $H_2O$ (49 : 49 : 2, v / v) solution containing 50 mM formic acid and 25 mM diethylamine.
Elution Method: Isocratic
Analysis time: 10 minutes.
Flow rate (ml/min): 0.5
Column temperature: 25°C
Wavelength: 254 nm
(V) in FIG. 2 and Table 23 was prepared by mixing the purchased standard compounds (III) and (IV) at a ratio of (III) : (IV) = 7 : 3.

[Table 23]

| | | | | |
|---|---|---|---|---|
| (I) | PDA Ch 1 254 nm | | | |
| | Peak # | Retention time | Area | Area % |
| | 1 | 4.103 | 96765 | 100.000 |
| | Total | | 96765 | 100.000 |
| (V) | PDA Ch 1 254 nm | | | |
| | Peak # | Retention time | Area | Area % |
| | 1 | 4.082 | 133994 | 71.853 |
| | 2 | 5.344 | 52490 | 28.147 |
| | Total | | 186484 | 100.000 |

Evaluation Example 8: Evaluation of additive (DMSO) in MeGly(cPent) synthesis using variant enzyme W253H-SBP-His and the additive

Sodium 2-cyclopentyl-2-oxoacetate (final concentration: 100 mM), D(+)-glucose (final concentration: 200 mM), methylamine hydrochloride (final concentration: 500 mM), NADPH (final concentration: 2.0 mM), and N,N-di(2-hydroxyethyl)glycine (final concentration: 100 mM) were dissolved in distilled water. To the solution, the additive DMSO (0 to 40 v/v%) was added, followed by pH adjustment to 8.8 to 8.9 with a 5 M aqueous sodium hydroxide solution. A GDH solution (0.10 units/μL, 1 x TNG solution, final concentration: 0.0040 units/μL) and the variant enzyme W253H-SBP-His (0.67 mM, final concentration: 5.0 μM) prepared in Example 2 were added thereto, and the mixture was incubated at 25°C for 24 hours.

During the incubation, 50 μL of the reaction solution was sampled, and 750 μL of methanol and 200 μL of 1 M hydrochloric acid were added to the sampled solution. The obtained solution was filtered through a 0.5 μm PTFE membrane filter (DISMIC, ADVANTEC) to prepare a sample for LCMS analysis, which was then subjected to LCMS measurement (analysis condition: TFA00). A UV peak area at 197 nm was measured by LC, and the ratio of the absorbance coefficient of each compound was corrected according to the expression X given below to calculate a reaction yield. Changes in reaction yield are shown in Table 24.

Retention time: 1.1 min (analysis condition :TFA00)

The obtained solution was measured by LCMS (analysis condition: TFA00-QDa) to confirm a MS peak of MeGly(cPent).

Retention time: 1.5 min (analysis condition :TFA00-QDa)
LCMS (ESI) m/z = 158.1 $(M + H)^+$

$$\text{Expression X: Reaction yield (\%)} = S_{1a} / ((S_{2a} / 8.0) + S_{1a}) \times 100$$

In the expression X, $S_{1a}$ represents the UV peak area of MeGly(cPent). $S_{2a}$ represents the UV peak area of sodium 2-cyclopentyl-2-oxoacetate.

[Table 24]

| DMSO(v/v%) | Reaction yield (%) | | |
|---|---|---|---|
| | 3h | 9h | 24h |
| 0 | 26 | 64 | 97 |
| 10 | 37 | 79 | 99 |
| 20 | 47 | 86 | 99 |
| 30 | 47 | 85 | 95 |
| 40 | 47 | 84 | 97 |

Evaluation Example 9: Evaluation of additive in Phe synthesis using variant enzyme W253H-SBP-His

A mixed solution (pH 8 to 9) of phenylpyruvic acid (final concentration: 50 mM), D(+)-glucose (final concentration: 100 mM), an ammonia solution (final concentration: 500 mM), a N,N-di(2-hydroxyethyl)glycine buffer solution (final concentration: 0.1 M), a NADPH solution (final concentration: 1 mM), a GDH solution (final concentration: 0.002 units/μL), the additive DMSO (20 v/v% based on the reaction solution), the variant enzyme W253H-SBP-His (final concentration: 2.5 μM) prepared in Example 2, and ultrapure water was prepared and incubated at 25°C, followed by the evaluation of the reaction. For an additive-free (0 v/v%) condition as a comparative condition, ultrapure water was added instead of the additive. To the reaction solution thus incubated, a hydrochloric acid-containing DMSO solution was added in 19 times the amount of the reaction solution such that the final concentration of added hydrogen chloride was 300 mM. The obtained solution was allowed to pass through a 0.45 μm PTFE membrane filter (Cosmospin Filter H, Nacalai Tesque, Inc.) to prepare a sample for LCMS analysis, which was then subjected to LCMS measurement (analysis condition: FA05-1). The UV peak area at 254 nm of the title compound Phe was calculated to determine a reaction yield. In this respect, the reaction yield was determined after 3 hours and after 5 hours at the reaction temperature of 25°C. The results are shown in Table 25.

LCMS (ESI) m/z = 166.1 $(M + H)^+$
Retention time: 0.27 min (analysis condition FA05-1)

[Table 25]

| Additive | Yield (%) at reaction temperature of 25°C | |
|---|---|---|
| | 3 hr | 5 hr |
| Additive-free | 32 | 68 |
| DMSO | 50 | 86 |

High-performance liquid chromatography conditions are as follows.

Condition 1 of high-performance liquid chromatography
Apparatus: Waters AQUITY H-class/QDa
Column: (I.D. x length (mm), particle size (μm)): ACQUITY UPLC HSS T3 (2.1 × 50, 1.8) Mobile phase: A) 0.05% TFA H2O, B) 0.05% TFA MeCN
Gradient (A/B): 100/0 to 2/98 (5.0 min) → 2/98 (1.0 min) → 100/0 (0.01 min) → 100/0 (2.0 min)
Flow rate (ml/min): 0.5
Column temperature: 30°C

Condition 2 of high-performance liquid chromatography

Apparatus: Waters AQUITY H-class
Column: (I.D. x length (mm), particle size (μm)): Ascentis Express RP-Amide (2.1 × 50, 2.7) Mobile phase: A) 0.05% TFA H2O, B) 0.05% TFA MeCN
Gradient (A/B): 95/5 to 0/100 (4.0 min) -> 0/100 (0.5 min) -> 95/5 (0.1 min) -> 95/5 (1.4 min) Flow rate (ml/min): 0.5
Column temperature: 35°C

Condition 3 of high-performance liquid chromatography

Apparatus: Waters AQUITY H-class/QDa
Column: (I.D. x length (mm), particle size (μm)): Ascentis Express C18 (3.0 × 50, 2.7)
Mobile phase: A) 0.05% TFA $H_2O$, B) 0.05% TFA MeCN
Gradient (A/B): 95/5 to 0/100 (5.0 min) → 0/100 (1.0 min) → 95/5 (0.01 min) → 95/5 (2.0 min) Flow rate (ml/min): 0.5
Column temperature: 30°C

Condition 4 for high-performance liquid chromatography

Apparatus: Waters AQUITY H-class / QDa
Column (I.D. x length (mm), particle size (μm)): DAICEL CHIRALPAK IC-3 (4.6 × 150, 3) Mobile phase : A) 0.05% TFA $H_2O$, B) 0.05% TFA MeCN
Gradient (A/B): 95/5 to 40 / 60 (20.0 min) → 95/5 (0.1 min) → 95/5 (4.9 min)
Flow Rate (ml / min): 1.0
Column temperature : 30°C

Synthesis Example 5: Synthesis of partially purified (S)-2-(benzyloxycarbonyl(methyl)amino)-2-cyclopentyl-acetic acid

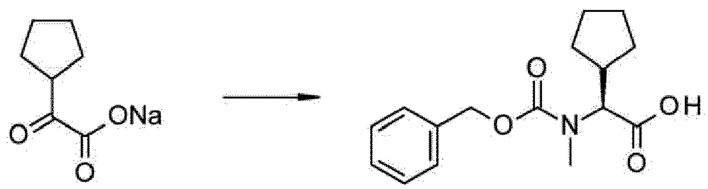

2-Cyclopentyl-2-oxo-acetic acid sodium salt (3.9 g, 24 mmol), methylamine hydrochloride (8.1 g, 120 mmol), D-glucose (8.7 g, 48 mmol), and N,N-di(2-hydroxyethyl)glycine (7.8 g, 48 mmol) were dissolved in distilled water (50 mL), and the outside temperature of the reaction container was set to 25°C. The pH of the solution was adjusted to 8.8 by the addition of a 5 M aqueous sodium hydroxide solution (6.0 mL). NADP+ (0.18 g, 0.24 mmol), D-glucose dehydrogenase (3.8 mg, 960 U, 250 U/mg), and the variant enzyme W253H-SBP-His (1.6 mL; corresponding to 43 mg in terms of the enzyme, 1.1% by mass) prepared in Example 2 were added thereto, and the mixture was stirred at an outside temperature of 25°C of the reaction container for 24 hours. The pH of the solution was adjusted to 1.9 by the addition of concentrated hydrochloric acid (3.0 mL), and the mixture was stirred for 1 hour. Insoluble matter was filtered off through a celite pad, and the celite pad was washed with distilled water (20 mL) to give a (S)-2-cyclopentyl-2-(methylamino)acetic acid solution (120 g).
Mass spectrometry (MS (ESI)) was performed under the condition 1 of high-performance liquid chromatography. A peak with m/z = 158.1 $(M + H)^+$ was confirmed at a retention time of 1.5 minutes to confirm that the (S)-2-cyclopentyl-2-(methylamino)acetic acid solution of interest was obtained.
The pH of the (S)-2-cyclopentyl-2-(methylamino)acetic acid solution (60 g; corresponding to 12 mmol) was

adjusted to 12.0 by the addition of a 10 M aqueous sodium hydroxide solution (10 mL). The obtained solution was concentrated into dryness under reduced pressure at an outside temperature of 40°C of the reaction container. Distilled water (30 mL) was added thereto, and the pH of the solution was adjusted to pH 9.7 by the addition of concentrated hydrochloric acid (0.75 mL). Acetonitrile (10 mL), N-carbobenzoxyoxysuccinimide (4.2 g, 17 mmol), and methyl tert-butyl ether (10 mL) were added thereto, and the mixture was stirred for 1 hour. A 50 w/v% aqueous sodium hydroxide solution (1.5 mL) was added thereto, and the mixture was stirred for 3 hours. N-Carbobenzoxyoxysuccinimide (1.2 g, 4.8 mmol) was added thereto, and the mixture was stirred for 90 minutes. A 50 w/v% aqueous sodium hydroxide solution (0.5 mL) was added thereto, and the mixture was stirred for 18 hours. The title compound was extracted into an organic layer by the addition of concentrated hydrochloric acid (8.0 mL). The obtained organic layer was concentrated into dryness under reduced pressure at an outside temperature of 40°C of the reaction container. Methyl tert-butyl ether (15 mL) was added thereto, and the organic layer was washed twice with a 5% aqueous disodium hydrogen phosphate solution (10 mL × 2) and once with a 2 M aqueous sodium hydroxide solution (10 mL) to extract the title compound into an aqueous layer. The obtained aqueous layers were combined, and the title compound was reextracted into an organic layer by the addition of methyl tert-butyl ether (20 mL) and concentrated hydrochloric acid (1.5 mL). The obtained organic layer was concentrated into dryness under reduced pressure at an outside temperature of 40°C of the reaction container. The title compound was extracted into an aqueous layer by the addition of methyl tert-butyl ether (30 mL) and a 1 M aqueous sodium hydroxide solution (30 mL). The obtained aqueous layer was washed with methyl tert-butyl ether (30 mL). The title compound was reextracted into an organic layer by the addition of toluene (30 mL) and concentrated hydrochloric acid (3.0 mL). The obtained organic layer was concentrated into dryness under reduced pressure at an outside temperature of 40°C of the reaction container to give partially purified (S)-2-(benzyloxycarbonyl(methyl)amino)-2-cyclopentyl-acetic acid (2.8 g).

Synthesis Example 6: Synthesis of partially purified (S)-2-(benzyloxycarbonyl(methyl)amino)-2-cyclopentyl-acetic acid

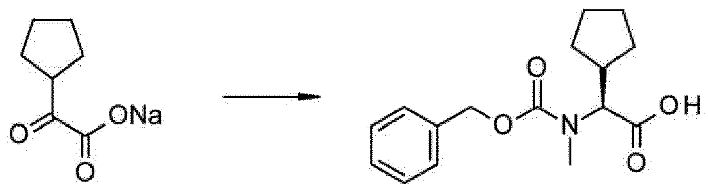

2-Cyclopentyl-2-oxo-acetic acid sodium salt (5.3 g, 33 mmol), methylamine hydrochloride (11 g, 160 mmol), D-glucose (12 g, 65 mmol), and N,N-di(2-hydroxyethyl)glycine (11 g, 65 mmol) were dissolved in distilled water (69 mL), and the outside temperature of the reaction container was set to 25°C. The pH of the solution was adjusted to 9.2 by the addition of a 50 w/v% aqueous sodium hydroxide solution (4.8 mL). NADP+ (0.25 g, 0.33 mmol), D-glucose dehydrogenase (26 mg, 1300 U, 50 U/mg), and the variant enzyme W253H-SBP-His (2.0 mL; corresponding to 53 mg in terms of the enzyme, 1.0% by mass) prepared in Example 2 were added thereto, and the mixture was stirred at an outside temperature of 25°C of the reaction container for 21 hours (rate of reaction conversion: 99% or more). The pH of the solution was adjusted to 1.9 by the addition of concentrated hydrochloric acid (7.5 mL), and the mixture was stirred for 1 hour. Insoluble matter was filtered off through a celite pad, and the celite pad was washed with distilled water (11 mL) to give a (S)-2-cyclopentyl-2-(methylamino)acetic acid solution (130 g).

Mass spectrometry (MS (ESI)) was performed under the condition 1 of high-performance liquid chromatography. A peak with m/z = 158.1 $(M + H)^+$ was confirmed at a retention time of 1.4 minutes to confirm that the (S)-2-cyclopentyl-2-(methylamino)acetic acid solution of interest was obtained.

The pH of the (S)-2-cyclopentyl-2-(methylamino)acetic acid solution (27 g; corresponding to 6.5 mmol) was adjusted to 12.3 by the addition of a 50 w/v% aqueous sodium hydroxide solution (3.2 mL). The obtained solution was concentrated into dryness at an outside temperature of 40°C of the reaction container. The pH of the solution was adjusted to pH 9.2 by the addition of distilled water (24 mL) and concentrated hydrochloric acid (0.53 mL) at an outside temperature of 25°C of the reaction container. Methyl tert-butyl ether (5.3 mL), acetonitrile (2.7 mL), and N-carbobenzoxyoxysuccinimide (1.8 g, 7.2 mmol) were added thereto, and the mixture was stirred for 105 minutes. Acetonitrile (1.6 mL) was added thereto, and the mixture was further stirred for 30 minutes. The pH of the solution was adjusted to 9.3 by the addition of a 50 w/v% aqueous sodium hydroxide solution (0.8 mL), and the mixture was stirred for 105 minutes. N-Carbobenzoxyoxysuccinimide (1.0 g, 3.9 mmol) was added thereto, and the mixture was stirred for 105 minutes. The pH of the solution was adjusted to 9.2 by the addition of a 50 w/v% aqueous sodium hydroxide solution (0.5 mL). Then, N-carbobenzoxyoxysuccinimide (1.1 g, 4.6 mmol) was added thereto, and the mixture was stirred for 13 hours. The pH of the solution was adjusted to 0.4 by the addition of concentrated hydrochloric acid (4.3 mL). Then, the title compound

was extracted into an organic layer by the addition of methyl tert-butyl ether (3.0 mL). The title compound was extracted into an organic layer by the addition of methyl tert-butyl ether (5.3 mL) to the obtained aqueous layer. The obtained organic layers were combined and rendered basic by the addition of a 2 M aqueous sodium hydroxide solution (11 mL). The title compound was extracted into an aqueous layer by the addition of methyl tert-butyl ether (5.3 mL). Methyl tert-butyl ether (11 mL) was added to the aqueous layer for washing. The pH of the aqueous layer was adjusted to 0.0 by the addition of methyl tert-butyl ether (11 mL) and concentrated hydrochloric acid (2.1 mL). The title compound was extracted into an organic layer to give a partially purified (S)-2-(benzyloxycarbonyl(methyl)amino)-2-cyclopentyl-acetic acid solution (14 g).

Mass spectrometry (MS (ESI)) was performed under the condition 1 of high-performance liquid chromatography. A peak with m/z = 292.1 $(M + H)^+$ was confirmed at a retention time of 3.9 minutes to confirm that the partially purified (S)-2-(benzyloxycarbonyl(methyl)amino)-2-cyclopentyl-acetic acid of interest was obtained.

Synthesis Example 7: Method for synthesizing (S)-2-(benzyloxycarbonyl(methyl)amino)-2-cyclopentyl-acetic acid lithium salt seed crystals

(S)-2-(Benzyloxycarbonyl(methyl)amino)-2-cyclopentyl-acetic acid (1.0 g, 3.4 mmol, commercially available product) was dissolved in tetrahydrofuran (5.0 ml, 5.0 v/w), and the solution was stirred at an outside temperature of 25°C of the reaction container. Lithium tert-butoxide (0.30 g, 3.8 mmol) was added thereto, and precipitated crystals were confirmed. Tetrahydrofuran (20 ml, 20 v/w) was added thereto, and the mixture was stirred for 1 hour. The crystals were filtered, and the obtained crystals were washed with tetrahydrofuran (5.0 ml, 5.0 v/w). The crystals were dried under reduced pressure at an outside temperature of 40°C of the reaction container for 3 hours to give (S)-2-(benzyloxycarbonyl(methyl)amino)-2-cyclopentyl-acetic acid lithium salt (0.90 g, 88% yield).

Comparative Example 2 and Example 1-1: Purification of amino acid derivative in free form and in lithium salt form

The purification of an amino acid derivative was compared between a free form (carboxylic acid) and lithium salt. An amino acid derivative containing impurities was isolated in a free form or in a lithium salt form, and apparent purity was compared therebetween. The apparent purity or the apparent content percentage of impurities was calculated from a UV absorption peak area ratio of high-performance liquid chromatography. The apparent purity was calculated according to the expression 1b, and the apparent content percentage of impurities was calculated according to the expression 2b.

Comparative Example 2: Purification of (S)-2-(benzyloxycarbonyl(methyl)amino)-2-cyclopentyl-acetic acid (free form)

The partially purified (S)-2-(benzyloxycarbonyl(methyl)amino)-2-cyclopentyl-acetic acid (1.4 g, theoretical yield quantity as (S)-2-(benzyloxycarbonyl(methyl)amino)-2-cyclopentyl-acetic acid: 1.8 g; corresponding to 6.0 mmol) obtained in Synthesis Example 5 was dissolved in toluene (3.0 mL, 1.7 v/w), and the solution was stirred at an outside temperature of 25°C of the reaction container using a stirring blade. Heptane (1.0 mL, 0.6 v/w) and seed crystals (crystals of (S)-2-(benzyloxycarbonyl(methyl)amino)-2-cyclopentyl-acetic acid (free form), commercially available product, 5.0 mg) were added thereto. The mixture was stirred for 15 minutes, and precipitated crystals were confirmed. Heptane (5.5 mL, 3.1 v/w) was added dropwise thereto over 1 hour, and the mixture was further stirred for 30 minutes. The mixture was further stirred at an outside temperature of 0°C of the reaction container for 1 hour. The crystals were filtered, and the obtained crystals were washed with heptane/toluene (v/v) = 4/1 (4.0 mL, 2.3 v/w). The crystals were dried under reduced pressure at an outside temperature of 40°C of the reaction container for 1 hour to give (S)-2-(benzyloxycarbonyl(methyl)amino)-2-cyclopentyl-acetic acid (1.3 g, 73% yield (calculated based on the 2-cyclopentyl-2-oxo-acetic acid sodium salt of Synthesis Example 5)).

The structure was confirmed by mass spectrometry (MS (ESI)) under the condition 3 of high-performance liquid chromatography. The peak with m/z = 224.1 $(M + H)^+$ of Cbz-β-Ala-OH, one of the impurities, was confirmed at a retention time of 2.6 minutes. The peak with m/z = 292.1 $(M + H)^+$ of Cbz-MeGly(cPent)-OH was confirmed at a retention time of 3.9 minutes. The UV absorption peak areas of Cbz-MeGly(cPent)-OH and Cbz-β-Ala-OH were measured at a measurement wavelength of 210 nm, and the apparent purity and the apparent content percentage of impurities were calculated according to the expressions 1b and 2b. The calculation of the apparent purity and the apparent content percentage of impurities was performed both for the partially purified (S)-2-(benzyloxycarbonyl(methyl)amino)-2-cyclopentyl-acetic acid obtained in Synthesis Example 5 (before purification) and for the (S)-2-(benzyloxycarbonyl(methyl)amino)-2-cyclopentyl-acetic acid obtained in Comparative Example 2 (after purification). The results are shown in Table 26.

[Table 26]

| | Cbz-β-Ala-OH (Apparent content percentage of impurities) | Cbz-MeGly(cPent)-OH Apparent purity |
|---|---|---|
| Before purification | 1.5% | 98.5% |
| After purification | 1.3% | 98.7% |

Example 1-1: Purification of (S)-2-(benzyloxycarbonyl(methyl)amino)-2-cyclopentyl-acetic acid in lithium salt form

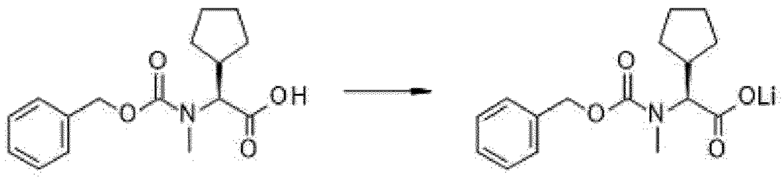

The (S)-2-(benzyloxycarbonyl(methyl)amino)-2-cyclopentyl-acetic acid (500 mg, 1.7 mmol) obtained in Comparative Example 2 was dissolved in acetonitrile (2.5 ml, 5.0 v/w), and the solution was stirred at an outside temperature of 25°C of the reaction container using a stirring blade. A 4 M aqueous lithium hydroxide solution (0.47 ml, 1.9 mmol) and distilled water (0.50 ml, 1.0 v/w) were added thereto, and the mixture was stirred at an outside temperature of 40°C of the reaction container for 20 minutes. Acetonitrile (5.0 ml, 10 v/w) was added thereto over 10 minutes, and seed crystals (synthesized in Synthesis Example 7, 5.0 mg) were added thereto. The mixture was stirred for 40 minutes, and precipitated crystals were confirmed. The outside temperature of the reaction container was set to 25°C, and the mixture was stirred for 3 hours. The outside temperature of the reaction container was set to 0°C, and the mixture was stirred for 30 minutes. Acetonitrile (2.5 ml, 5.0 v/w) was added dropwise thereto over 5 minutes, and the mixture was further stirred for 30 minutes. Acetonitrile (5.0 ml, 10 v/w) was added dropwise thereto over 5 minutes, and the mixture was stirred for 30 minutes. The crystals were filtered, and the obtained crystals were washed with distilled water/acetonitrile (v/v) = 5/95 (2.0 mL, 4.0 v/w). The crystals were dried under reduced pressure at an outside temperature of 40°C of the reaction container for 3 hours to give (S)-2-(benzyloxycarbonyl(methyl)amino)-2-cyclopentyl-acetic acid lithium salt (360 mg, 71% yield, calculated based on the (S)-2-(benzyloxycarbonyl(methyl)amino)-2-cyclopentyl-acetic acid obtained in Comparative Example 2).

Mass spectrometry (MS (ESI)) and apparent purity analysis based thereon were performed under the condition 3 of high-performance liquid chromatography. The peak of Cbz-β-Ala-OH, one of the impurities, was confirmed at a retention time of 2.6 minutes. The peak with m/z = 292.1 $(M + H)^+$ of Cbz-MeGly(cPent)-OH was confirmed at a retention time of 3.9 minutes. The UV absorption peak areas of Cbz-MeGly(cPent)-OH and Cbz-β-Ala-OH were measured at a measurement wavelength of 210 nm, and the apparent purity and the apparent content percentage of impurities were calculated according to the expressions 1b and 2b. The calculation of the apparent purity and the apparent content percentage of impurities was performed both for the (S)-2-(benzyloxycarbonyl(methyl)amino)-2-cyclopentyl-acetic acid obtained in Comparative Example 2 (before purification) and for the (S)-2-(benzyloxycarbonyl(methyl)amino)-2-cyclopentyl-acetic acid lithium salt obtained in Example 1-1 (after purification). The results are shown in Table 27.

[Table 27]

| | Cbz-β-Ala-OH (Apparent content percentage of impurities) | Cbz-MeGly(cPent)-OH Apparent purity |
|---|---|---|
| Before purification | 1.3% | 98.7% |
| After purification | N.D. | 100% |
| **N.D.** : not detected | | |

Example 1-2: Purification of (S)-2-(benzyloxycarbonyl(methyl)amino)-2-cyclopentyl-acetic acid in lithium salt form

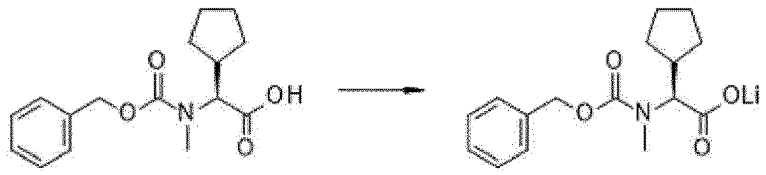

The partially purified (S)-2-(benzyloxycarbonyl(methyl)amino)-2-cyclopentyl-acetic acid solution (7.0 g, theoretical yield quantity as (S)-2-(benzyloxycarbonyl(methyl)amino)-2-cyclopentyl-acetic acid: 0.95 g; corresponding to 3.3 mmol) obtained in Synthesis Example 6 was concentrated into dryness at an outside temperature of 40°C of the reaction container. Acetonitrile (3.2 mL, 3.4 v/w) was added thereto, and the mixture was stirred at an outside temperature of 25°C of the reaction container using a stirring blade. Distilled water (0.53 mL, 0.6 v/w) and a 4 M aqueous lithium hydroxide solution (0.90 mL, 3.6 mmol) were added thereto, and the mixture was stirred for 20 minutes. Acetonitrile (2.7 mL, 2.8 v/w) was added thereto over 10 minutes, and seed crystals (synthesized in Synthesis Example 7, 5.0 mg) were added thereto. The mixture was stirred for 15 minutes. Acetonitrile (5.3 mL, 5.6 v/w) was added dropwise thereto over 20 minutes, and the mixture was stirred for 17 hours. Acetonitrile (5.3 mL, 5.6 v/w) was added dropwise thereto over 30 minutes, and the mixture was stirred for 1 hour. Acetonitrile (5.3 mL, 5.6 v/w) was added dropwise thereto over 30 minutes, and the mixture was stirred for 1 hour. The crystals were filtered, and the obtained crystals were washed with distilled water/acetonitrile (v/v) = 5/95 (3.2 mL, 3.4 v/w). The crystals were dried under reduced pressure at an outside temperature of 40°C of the reaction container for 2.5 hours to give (S)-2-(benzyloxycarbonyl(methyl)amino)-2-cyclopentyl-acetic acid lithium salt (yield quantity: 620 mg, yield: 64%, calculated based on the 2-cyclopentyl-2-oxo-acetic acid sodium salt of Synthesis Example 6).

Mass spectrometry (MS (ESI)) and apparent purity analysis based thereon were performed under the condition 1 of high-performance liquid chromatography. The peak of Cbz-β-Ala-OH, one of the impurities, was confirmed at a retention time of 2.8 minutes. The peak with m/z = 292.1 $(M + H)^+$ of Cbz-MeGly(cPent)-OH was confirmed at a retention time of 3.9 minutes. The UV absorption peak areas of Cbz-MeGly(cPent)-OH and Cbz-β-Ala-OH were measured at a measurement wavelength of 210 nm, and the apparent purity and the apparent content percentage of impurities were calculated according to the expressions 1b and 2b. The calculation of the apparent purity and the apparent content percentage of impurities was performed both for the partially purified (S)-2-(benzyloxycarbonyl(methyl)amino)-2-cyclopentyl-acetic acid solution obtained in Synthesis Example 6 (before purification) and for the (S)-2-(benzyloxycarbonyl(methyl)amino)-2-cyclopentyl-acetic acid lithium salt obtained in Example 1-2 (after purification). The results are shown in Table 28.

[Table 19]

| | Cbz-*β*-Ala-OH (Apparent content percentage of impurities) | Cbz-MeGly(cPent)-OH Apparent purity |
|---|---|---|
| Before purification | 3.6% | 96.4% |
| After purification | N.D. | 100% |
| **N.D.** : not detected | | |

Chiral analysis was carried out using the condition 4 for high performance liquid chromatography. The measurement wavelength was 210 nm. The retention times of (S) compound and (R) compound were confirmed to be 16.7 min and 17.3 min, respectively, by analysis of a standard sample (purchased from Amatek). As a result of the chiral analysis, the (R) compound was not detected in the (S)-2-(benzyloxycarbonyl (methyl) amino)-2-cyclopentyl-acetic acid lithium salt (after purification) obtained in Example 1-2 and had an optical purity of 99.9% ee or more.

## Claims

**1.** A polypeptide

comprising a sequence having 90% or higher sequence identity to an amino acid sequence represented by SEQ ID NO: 1 with one amino acid residue thereof engineered, and
having higher catalytic activity for a reductive amination reaction between at least one compound A represented by the following formula (1) or a salt thereof and at least one compound B represented by the following formula

(2) or a salt thereof or an intramolecular reductive amination reaction of at least one compound B represented by the formula (2) or a salt thereof than that of a polypeptide comprising the amino acid sequence represented by SEQ ID NO: 1 under at least one reaction condition:

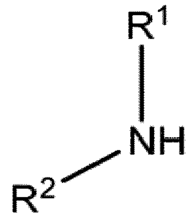

(1)

wherein
in the formula (1),
$R^1$ and $R^2$ each independently represent a hydrogen atom, an alkyl group, an alkenyl group, an alkynyl group, a cycloalkyl group, an aryl group, a heterocyclyl group, or a heteroaryl group, these groups are optionally substituted, and at least one of $R^1$ and $R^2$ is a hydrogen atom,

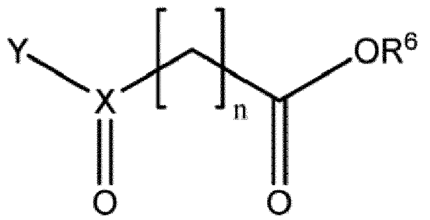

(2)

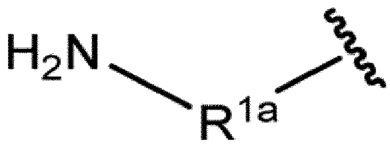

(1')

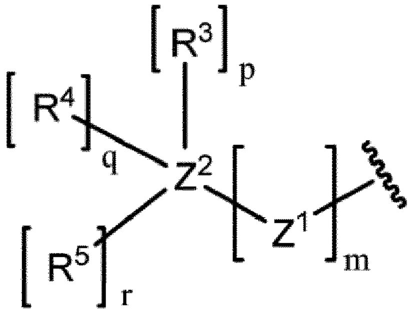

(3)

wherein
in the formula (2),

X represents a carbon atom,
Y represents a hydrogen atom, a group represented by the formula (1') or a group represented by the formula (3),
n represents an integer of between 0 and 2, and
$R^6$ represents a hydrogen atom, an optionally substituted aliphatic hydrocarbon group having between 1 and 6 carbon atoms, an optionally substituted aryl group having between 5 and 12 carbon atoms, an optionally substituted heteroaryl group having between 5 and 12 ring-constituting atoms, a group containing a nitrogen atom, or a group containing an oxygen atom;

in the formula (1'),

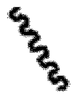

represents a binding point to X, and
$R^{1a}$ is a group formed by removing a hydrogen atom from the group represented by $R^1$ in the formula (1), and is not a hydrogen atom;

in the formula (3),

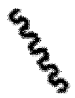

represents a binding point to X,
m represents an integer of between 0 and 6,
p is 0 or 1,
q is 0 or 1,
r is 0 or 1,
$Z^1$ represents an optionally substituted alkylene group, or an ether bond-containing group having between 1 and 6 carbon atoms, and when m is 2 or larger, a plurality of $Z^1$ are the same or different,
$Z^2$ represents a carbon atom,
$R^3$, $R^4$ and $R^5$ each independently represent a hydrogen atom, an optionally substituted aliphatic hydrocarbon group having between 1 and 6 carbon atoms, an optionally substituted aryl group having between 5 and 12 carbon atoms, an optionally substituted heteroaryl group having between 5 and 12 ring-constituting atoms, a group containing a nitrogen atom, or a group containing an oxygen atom,
any two or more of $R^3$, $R^4$, and $R^5$ are optionally bonded to each other to form a ring structure together with $Z^2$, the ring structure is optionally a cycloalkyl group, an aryl group, a heterocyclyl group, or a heteroaryl group, and these groups are optionally substituted,
$R^3$, $R^4$, and $R^5$ each optionally form a double bond or a triple bond with $Z^2$, and when any one of $R^3$, $R^4$, and $R^5$ is bonded to $Z^2$ through a double bond or a triple bond, at least one of p, q and r is 0; and
when one of $R^1$ and $R^2$ in the formula (1) is a methyl group and the other moiety is a hydrogen atom, in the formula (2), Y is a group represented by the formula (3), m is 0, and two or more of $R^3$ to $R^5$ are not a hydrogen atom.

**2.** The polypeptide according to claim 1, wherein in the formula (1), $R^1$ is a hydrogen atom, and $R^2$ is a $C_1$ to $C_6$ alkyl group.

**3.** The polypeptide according to claim 1 or 2, wherein in the formula (2), Y is a $C_3$ to $C_8$ cycloalkyl group or a $C_6$ to $C_9$ aralkyl group, and the aralkyl group is optionally substituted by a $C_1$ to $C_3$ alkyl group or a halogen atom.

**4.** A polypeptide comprising a sequence having 90% or higher sequence identity to an amino acid sequence represented by SEQ ID NO: 1 with one amino acid residue thereof engineered, and having higher catalytic activity for a reductive amination reaction between an alkylamine or a salt thereof and at least one compound represented by the following formula (2') or a salt thereof than that of a polypeptide consisting of the amino acid sequence represented by SEQ ID NO: 17 under at least one reaction condition:

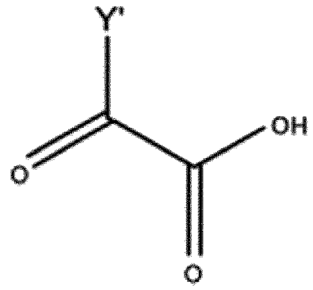

(2')

wherein Y' represents a $C_3$ to $C_8$ cycloalkyl group or a $C_6$ to $C_9$ aralkyl group, and the aralkyl group is optionally substituted by a $C_1$ to $C_3$ alkyl group or a halogen atom.

5. The polypeptide according to claim 4, wherein the alkylamine or a salt thereof is one or more selected from the group consisting of methylamine, ethylamine and salts thereof.

6. The polypeptide according to claim 4 or 5, wherein the compound represented by the formula (2') or a salt thereof is one or more selected from the group consisting of phenylpyruvic acid, 2-oxo-3-(p-tolyl)propanoic acid, 2-cyclopentyl-2-oxo-acetic acid and salts thereof.

7. The polypeptide according to any one of claims 1 to 6, wherein the amino acid sequence represented by SEQ ID NO: 1 with one amino acid residue thereof engineered is the amino acid sequence represented by SEQ ID NO: 6 wherein X is a valine residue, the amino acid sequence represented by SEQ ID NO: 6 wherein X is a tyrosine residue, the amino acid sequence represented by SEQ ID NO: 8 wherein X is a leucine residue, the amino acid sequence represented by SEQ ID NO: 11 wherein X is a histidine residue, or the amino acid sequence represented by SEQ ID NO: 12 wherein X is a glutamic acid residue.

8. A polypeptide comprising a sequence having the engineering of an amino acid residue positioned at a site corresponding to at least one amino acid residue selected from the group consisting of a histidine residue at position 44, a phenylalanine residue at position 117, a methionine residue at position 141, a threonine residue at position 156, a histidine residue at position 182, a glutamine residue at position 186, a tryptophan residue at position 253, and a lysine residue at position 260 in the amino acid sequence represented by SEQ ID NO: 1.

9. The polypeptide according to claim 8, wherein the polypeptide has higher catalytic activity for a reductive amination reaction between at least one compound A represented by the formula (1) or a salt thereof and at least one compound B represented by the formula (2) or a salt thereof or an intramolecular reductive amination reaction of at least one compound B represented by the formula (2) or a salt thereof than that of a polypeptide comprising the amino acid sequence represented by SEQ ID NO: 1 under at least one reaction condition.

10. The polypeptide according to any one of claims 1 to 9, wherein the polypeptide comprises a sequence having substitution of an amino acid residue positioned at a site corresponding to a tryptophan residue at position 253 in the amino acid sequence represented by SEQ ID NO: 1 by at least one amino acid residue selected from the group consisting of a tyrosine residue, a valine residue, a threonine residue, a serine residue, an arginine residue, a glutamine residue, a proline residue, an asparagine residue, a methionine residue, a leucine residue, a lysine residue, an isoleucine residue, a histidine residue, a phenylalanine residue and an alanine residue.

11. The polypeptide according to any one of claims 1 to 10, wherein the polypeptide comprises the amino acid sequence represented by SEQ ID NO: 6 wherein X is a valine residue, the amino acid sequence represented by SEQ ID NO: 6 wherein X is a tyrosine residue, the amino acid sequence represented by SEQ ID NO: 8 wherein X is a leucine residue, the amino acid sequence represented by SEQ ID NO: 11 wherein X is a histidine residue, or the amino acid sequence represented by SEQ ID NO: 12 wherein X is a glutamic acid residue.

12. A method for producing an amino acid, comprising a step of

reacting one or more selected from the group consisting of amine, an amine analog and salts thereof with one or more selected from the group consisting of keto acid, a keto acid analog and salts thereof, or

intramolecularly reacting a compound selected from the group consisting of keto acid, a keto acid analog and salts thereof
in the presence of a polypeptide according to any one of claims 1 to 11 and a reducing agent.

**13.** The production method according to claim 12, wherein the amine or the amine analog is represented by the formula (1), and $R^1$ and $R^2$ in the formula (1) are synonymous with $R^1$ and $R^2$, respectively, described in the formula (1) of claim 1.

**14.** The production method according to claim 12 or 13, wherein the keto acid or the keto acid analog is represented by the formula (2), and the formula (1'), the formula (3), X, Y, $Z^1$, $Z^2$, $R^{1a}$, $R^3$, $R^4$, $R^5$, $R^6$, m, n, p, q and r in the formula (2) are synonymous with the formula (1'), the formula (3), X, Y, $Z^1$, $Z^2$, $R^{1a}$, $R^3$, $R^4$, $R^5$, $R^6$, m, n, p, q and r, respectively, described in the formula (2) of claim 1.

**15.** The production method according to any one of claims 12 to 14, wherein the reaction is performed in the presence of compound C represented by the following formula (4):

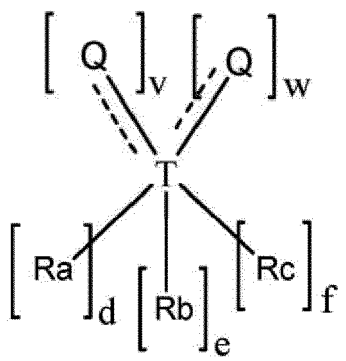

(4)

wherein

in the formula (4),
v and w each independently represent 0 or 1,
at least one of v and w represents 1,
T represents a carbon atom, a phosphorus atom, or a sulfur atom,
the functional group represented by the following formula (4a):

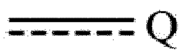

(4a)

represents =O, -ORd, or a hydroxy group,
when each of v and w is 1, two functional groups represented by a plurality of formulas (4a) are the same or different,
Ra, Rb, and Rc each independently represent a hydrogen atom, a $C_1$ to $C_3$ alkyl group, an alkylamino group, or $-CH_2-ORd$,
at least any two of Ra, Rb, and Rc are optionally linked to each other to form a ring structure together with T,
Rd represents a $C_1$ to $C_3$ alkyl group,
d, e, and f each independently represent 0 or 1,
at least one of d, e, and f represents 1,
when each of v and w is 1, at least one of Ra, Rb, and Rc is a methyl group, and none of Ra, Rb, and Rc are linked to each other to form a ring structure together with T,
when at least one of Ra, Rb, and Rc is a methylamino group, none of Ra, Rb, and Rc are linked to each other to form a ring structure together with T, and
when the functional group represented by the formula (4a) is a hydroxy group and T is a carbon atom, v is 1, w is 0, each of d, e, and f is 1, and each of Ra, Rb, and Rc is a hydrogen atom.

**16.** The production method according to any one of claims 12 to 15, comprising the following steps (A) and (B):

step (A); a step of contacting a lithium-containing substance with a mixture to be purified which is a mixture of the following purification target (i) and the following impurities (ii), obtained by the method according to any one of claims 12 to 15:

(i) the amino acid having a protective group at the N terminus, and
(ii) compounds other than the purification target, and

step (B); a step of precipitating lithium salt of the purification target.

**17.** A method for producing a peptide compound, comprising the steps of:

(1) producing an amino acid by the method according to any one of claims 12 to 16; and
(2) linking the amino acid to one or more selected from the group consisting of other amino acids and a peptide to produce a peptide compound.

Fig.1

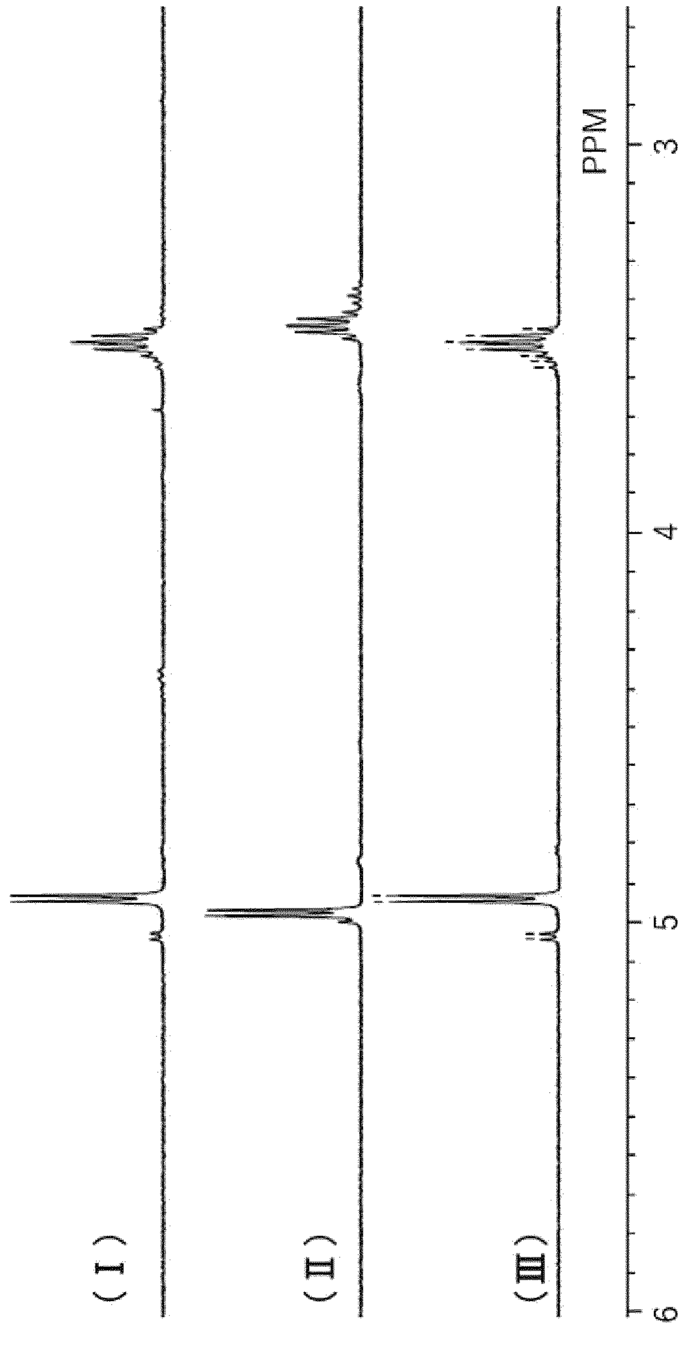

# Fig.2

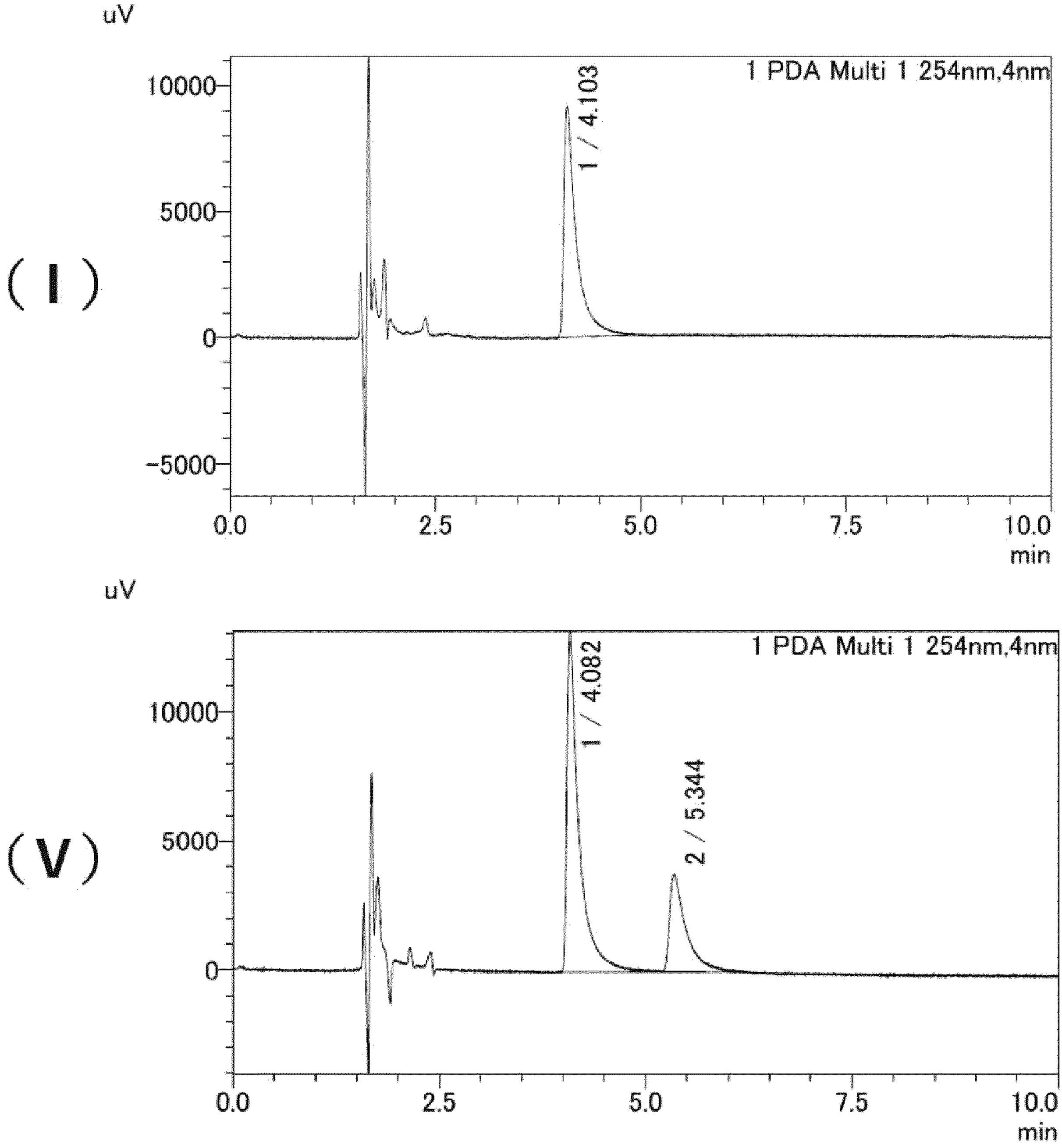
(I)
uV
10000
5000
0
-5000
1 PDA Multi 1 254nm,4nm
1 / 4.103
0.0
2.5
5.0
7.5
10.0
min
(V)
uV
10000
5000
0
1 PDA Multi 1 254nm,4nm
1 / 4.082
2 / 5.344
0.0
2.5
5.0
7.5
10.0
min

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003072770 A
- WO 2002077183 A


### Non-patent literature cited in the description


- **J. F. HYSLOP ET.** *J. Biotechnol.,* 2019, vol. 293, 56-65
- **H. MIHARA et al.** *FEBS Journal,* 2005, vol. 272, 1117-1123
- **HISAAKI MIHARA.** *Seikagaku (Biochemistry in English), the journal of the Japanese Biochemical Society,* 2015, vol. 87, 326-332
- **K. E. NELSON et al.** *Environ. Microbiol.,* 2002, vol. 4, 799-808
- **M. GOTO et al.** *J. Biol. Chem.,* 2005, vol. 280, 40875-40884
- **M. MINDET et al.** *Front. Bioeng. Biotechnol.,* 2019, vol. 7
- **A. KERBS et al.** *Microorganisms,* 2021, vol. 9, 824
- **KARLIN ; ALTSCHUL.** *Proc. Natl. Acad. Sci. USA,* 1993, vol. 90, 5873-7
- **ALTSCHUL et al.** *J. Mol. Biol.,* 1990, vol. 215, 403-10
- **HOPP, T.P. et al.** *BioTechnology,* 1988, vol. 6, 1204-1210
- SOLID PHASE PEPTIDE SYNTHESIS. Global Marketing, December 2019
- *Mil. Cell. Biol.,* 1982, vol. 2, 161
- *Mol. Cell. Biol.,* 1983, vol. 3, 280
- *Gene,* 1983, vol. 25, 263
- *Nucleic Acid Research,* 1990, vol. 18, 5322
- Experimental Medicine. Idenshi Kogaku (Genetic Engineering in English) Handbook. 1992
- *Proc. Natl. Acad. Sci. USA.,* 1972, vol. 69, 2110
- *Mil. Gen. Genet.,* 1979, vol. 168, 111
- *J. Mol. Biol.,* 1971, vol. 56, 209
- *Proc. Natl. Acad. Sci. USA.,* 1978, vol. 75, 1927
- *J. Bacteriol.,* 1983, vol. 153, 163
- *Virology,* 1973, vol. 52, 456
- *Mol. Cell. Biol.,* 1983, vol. 3, 2156-2165
- *CHEMICAL ABSTRACTS,* 187475-29-2